(19) Europäisches Patentamt
European Patent Office
Office européen des brevets

(11) EP 4 332 221 A1

(12) # EUROPEAN PATENT APPLICATION

(43) Date of publication:
**06.03.2024 Bulletin 2024/10**

(21) Application number: **22192656.1**

(22) Date of filing: **29.08.2022**

(51) International Patent Classification (IPC):
***C12N 15/113*** *(2010.01)* ***C12N 15/11*** *(2006.01)*

(52) Cooperative Patent Classification (CPC):
(C-Sets available)
**C12N 15/111;** C12N 15/113; C12N 2310/11; C12N 2310/315; C12N 2310/319; C12N 2310/32; C12N 2310/3231; C12N 2310/3341; C12N 2310/341 (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Roche Innovation Center Copenhagen A/S**
  **2970 Hørsholm (DK)**
- **F. Hoffmann-La Roche AG**
  **4070 Basel (CH)**

(72) Inventor: **The designation of the inventor has not yet been filed**

(74) Representative: **Rodriguez Novoa, Lorena**
**F. Hoffmann-La Roche AG Patent Department**
**Grenzacherstrasse 124**
**4070 Basel (CH)**

Remarks:
- The complete document including Reference Table(s) and the Sequence Listing(s) can be downloaded from the EPO website
- Claims 16 - 98 are deemed to be abandoned due to non-payment of the claims fees (Rule 45(3) EPC).

(54) **THREOSE NUCLEIC ACID ANTISENSE OLIGONUCLEOTIDES AND METHODS THEREOF**

(57) Described are antisense oligonucleotides comprising one or more α-L-threofuranosyl (TNA) nucleosides linked to an adjacent nucleoside via a phosphodiester (PC) internucleoside linkage, as well as methods to modulate the properties of antisense oligonucleotides by the introduction of such TNA nucleosides. These are particularly applicable to antisense gapmer oligonucleotides.

EP 4 332 221 A1

(52) Cooperative Patent Classification (CPC): (Cont.)

C-Sets
C12N 2310/321, C12N 2310/3525

**Description**

FIELD OF THE INVENTION

The present invention relates to antisense oligonucleotides comprising one or more α-L-threofuranosyl (TNA) nucleosides linked to an adjacent nucleoside by a phosphodiester internucleoside linkage, as well as methods to modulate the properties of antisense oligonucleotides by the introduction of such TNA nucleosides. The invention is particularly applicable for antisense gapmer oligonucleotides.

BACKGROUND OF THE INVENTION

Synthetic oligonucleotides as therapeutic agents have witnessed remarkable progress over recent years leading to a broad portfolio of clinically validated molecules acting by diverse mechanisms including antisense oligonucleotides such as ribonuclease H (RNase H) activating gapmers, splice switching oligonucleotides, micro-RNA inhibitors, small interfering RNA (siRNA) and aptamers (S. T. Crooke, Antisense drug technology: principles, strategies, and applications, 2nd ed. Boca Raton, FL: CRC Press, 2008).

Arguably one of the most successful modifications is the introduction of phosphorothioate (PS) linkages, where one of the non-bridging phosphate oxygen atoms is replaced with a sulfur atom (Eckstein, Antisense and Nucleic Acid Drug Development 2009;10: 117-121). Phosphorothioate oligodeoxynucleotides show an increased protein binding as well as a distinctly higher stability to nucleolytic degradation and thus a higher half-life in plasma, tissues and cells than their unmodified phosphodiester analogues. For example, a recent review (Crooke et al., Nucleic Acids Research 2020;48(10):5235-5253) described the PS moiety as being the primary determinant of the distribution of single stranded antisense oligonucleotides after all routes of administration. Other modifications include Locked Nucleic Acids (LNAs) as well as a variety of other modified nucleosides. TNAs, for example, have been used, e.g., in double-stranded siRNA molecules and in the form of oligomers (Matsuda et al., XXIII International Round Table on Nucleosides, Nucleotides and Nucleic acids; 2018, Liu et al., ACS Appl. Mater. Interfaces 2018;10:9736-9743, WO 2012/078536, WO 2012/118911, and WO 2013/179292).

There remains, however, a need for stable, safe, and efficient antisense oligonucleotide-based therapeutic agents.

SUMMARY OF THE INVENTION

It has been found by the present inventor(s) that one or more α-L-threofuranosyl (TNA) nucleosides can be introduced into antisense oligonucleotides via linkages other than PS linkages, particularly into antisense gapmer oligonucleotides, to modulate properties of the antisense oligonucleotides. Surprisingly, TNA nucleosides, particularly when introduced into gapmer designs via phosphodiester (PO) internucleoside linkages as described herein, can yield potent molecules with favourable properties for therapeutic use.

Accordingly, the present invention relates to antisense oligonucleotides comprising at least one TNA nucleoside linked to at least one adjacent nucleoside via an internucleoside linkage different from a PS linkage, particularly to antisense gapmer oligonucleotides comprising at least one such TNA nucleoside. Particularly preferred are PO internucleoside linkages. A TNA nucleoside linked to at least one adjacent nucleoside via a PO linkage can hereinafter be referred to as a TNA(PO) nucleoside. Contemplated TNA(PO) nucleosides include those linked by a 2'-PO linkage, a 3'-PO linkage, or both; hereinafter referred to as 2'-PO linked, 3'-PO linked, and 2',3'-PO linked TNA nucleosides, respectively.

The invention also relates to an antisense gapmer oligonucleotide comprising a contiguous nucleotide sequence of formula 5'-F-G-F'-3' (I) which is capable of recruiting RNase H, wherein the contiguous nucleotide sequence comprises at least one TNA nucleoside which is linked to an adjacent nucleoside by a linkage different than a PS internucleoside linkage, such as by a PO internucleoside linkage.

The invention also relates to an antisense gapmer oligonucleotide comprising a contiguous nucleotide sequence of formula 5'-F-G-F'-3' (I) which is capable of recruiting ribonuclease (RNase) H, wherein

G is a gap region of up to 18 linked nucleosides which comprises at least 3 contiguous DNA nucleosides,
each of F and F' is a flanking region of up to 15 linked nucleosides which independently comprises or consists of 1 to 15 sugar-modified nucleosides,
at least one of F, F' and G comprises a sugar-modified nucleoside which is an α-L-threofuranosyl (TNA) nucleoside and which is linked to an adjacent nucleoside by an internucleoside linkage different than a PS internucleoside linkage, such as by a PO internucleoside linkage.

The invention also relates to a conjugate comprising an antisense gapmer oligonucleotide according to the invention and at least one conjugate moiety covalently attached to the antisense gapmer oligonucleotide, optionally via a linker.
The invention also relates to a pharmaceutically acceptable salt of an antisense gapmer oligonucleotide or conjugate according to the invention.
The invention also relates to a pharmaceutical composition comprising an antisense gapmer oligonucleotide, conjugate, or pharmaceutically acceptable salt according to the invention, and a pharmaceutically acceptable diluent, solvent, carrier, salt and/or adjuvant.
The invention also relates to an antisense oligonucleotide, conjugate, pharmaceutically acceptable salt, or pharmaceutical composition according to the invention, for use as a medicament.
The invention also relates to a method of preparing a modified version of a parent antisense gapmer oligonucleotide, wherein the parent antisense gapmer comprises a contiguous nucleotide sequence of formula 5' F-G-F' 3' (I) which is capable of recruiting RNase H, wherein G is a gap region of 5 to 18 linked DNA nucleosides and each of F and F' is a flanking region of up to 8 linked nucleosides which independently comprises or consists of 1 to 8 sugar-modified nucleosides other than TNA nucleosides, and wherein, in the modified version, at least one nucleoside in F, F', and/or G of the parent antisense gapmer oligonucleotide has been replaced with a TNA nucleoside linked to an adjacent nucleoside by an internucleoside linkage different than a PS internucleoside linkage, such as by a PO internucleoside linkage,

> the method comprising the step of manufacturing the modified antisense gapmer oligonucleotide by reacting nucleotide units to form covalently linked contiguous nucleotide units comprised in the oligonucleotide, wherein at least one of the nucleotide units comprises a TNA nucleoside, and,
> optionally purifying or isolating the modified antisense gapmer oligonucleotide.

The invention also relates to an antisense gapmer oligonucleotide obtained or obtainable by the method of the invention.
Further details of these and other aspects and embodiments of the invention are provided in the following detailed disclosure and the claims.

## DETAILED DISCLOSURE

### Definitions

In order that the present invention may be more readily understood, certain terms are defined and described in the following.
Throughout this description, the word "comprise" or variations such as "comprises" or "comprising" will be understood to imply the inclusion of a stated integer (or components) or group of integers (or components) but not the exclusion of any other integer (or components) or group of integers (or components).

*Oligonucleotide*

The term "oligonucleotide" as used herein is defined as it is generally understood by the skilled person as a molecule comprising two or more covalently linked nucleosides, including modified nucleosides or nucleotides. Such covalently bound nucleosides may also be referred to as nucleic acid molecules or oligomers. Oligonucleotides are commonly made in the laboratory by solid-phase chemical synthesis followed by purification. When referring to an oligonucleotide sequence, reference is made to the sequence or order of nucleobase moieties, or modifications thereof, of the covalently linked nucleotides or nucleosides. The oligonucleotide of the invention is man-made, chemically synthesized, and is typically purified or isolated. The nucleosides may be linked by phosphodiester (PO) linkages or by modified internucleoside linkages.

*Antisense oligonucleotides*

The term "antisense oligonucleotide" as used herein is defined as oligonucleotides capable of modulating expression of a target gene by hybridizing to a target nucleic acid, particularly to a contiguous sequence on a target nucleic acid. The contemplated antisense oligonucleotides are not essentially double stranded and are therefore not siRNAs or short hairpin RNAs (shRNAs). Preferably, the antisense oligonucleotides of the present invention are single stranded. It is understood that single stranded oligonucleotides of the present invention can form hairpins or intermolecular duplex structures (duplex between two molecules of the same oligonucleotide) if the degree of intra or inter self-complementarity is more than 50% across of the full length of the oligonucleotide.

*Contiguous nucleotide sequence*

The term "contiguous nucleotide sequence" refers to the region of the oligonucleotide, which is complementary to a target nucleic acid. The term is used interchangeably herein with the term "contiguous nucleobase sequence" and the term "oligonucleotide motif sequence". For example, all the nucleotides of the oligonucleotide may constitute the contiguous nucleotide sequence. Alternatively, the oligonucleotide may comprise the contiguous nucleotide sequence, such as an F-G-F' gapmer region, and may optionally comprise further nucleotide(s), for example a nucleotide linker region which may be used to attach a functional group to the contiguous nucleotide sequence. The nucleotide linker region may or may not be complementary to the target nucleic acid. Advantageously, the contiguous nucleotide sequence is 100% complementary to the target nucleic acid.

*Nucleotides*

Nucleotides are the building blocks of oligonucleotides and polynucleotides, and include, for the purposes of the present invention, both naturally occurring and non-naturally occurring nucleotides. In nature, nucleotides, such as DNA and RNA nucleotides comprise a ribose sugar moiety, a nucleobase moiety and one or more phosphate groups (which is absent in nucleosides). Nucleosides and nucleotides may also interchangeably be referred to as "units" or "monomers".

*Nucleobase*

The term nucleobase includes the purine (e.g., adenine and guanine) and pyrimidine (e.g., uracil, thymine and cytosine) moiety present in nucleosides and nucleotides, which form hydrogen bonds in nucleic acid hybridization. In the context of the present invention, the term nucleobase also encompasses modified nucleobases, which may differ from naturally occurring nucleobases but are functional during nucleic acid hybridization. In this context, "nucleobase" refers to both naturally occurring nucleobases such as adenine, guanine, cytosine, thymidine, uracil, xanthine and hypoxanthine, as well as non-naturally occurring variants. Such variants are for example described in Hirao et al (2012) Accounts of Chemical Research vol 45. page 2055 and Bergstrom (2009) Current Protocols in Nucleic Acid Chemistry Suppl. 37, 1.4.1.

The nucleobase moiety can optionally be modified by changing the purine or pyrimidine into a modified purine or pyrimidine, such as substituted purine or substituted pyrimidine, such as a nucleobase selected from isocytosine, pseudoisocytosine, 5-methyl cytosine, 5-thiozolo-cytosine, 5-propynyl-cytosine, 5-propynyl-uracil, 5-bromouracil 5-thiazolo-uracil, 2-thio-uracil, 2'-thio-thymine, inosine, diaminopurine, 6-aminopurine, 2-aminopurine, 2,6-diaminopurine and 2-chloro-6-aminopurine.

The nucleobase moieties may be indicated by the letter code for each corresponding nucleobase, *e.g.*, A, T, G, C or U, wherein each letter may optionally include modified nucleobases of equivalent function. For example, in some oligonucleotides, the nucleobase moieties are selected from A, T, G, C, and 5-methyl cytosine ($^{m}C$).

*Modified nucleoside*

The term "modified nucleoside" or "nucleoside modification" as used herein refers to nucleosides modified as compared to the equivalent DNA or RNA nucleoside by the introduction of one or more modifications of the sugar moiety or the (nucleo)base moiety. Preferably, the modified nucleoside comprises a modified sugar moiety. The term "modified nucleoside" may also be used herein interchangeably with the term "nucleoside analogue" or modified "unit" or modified "monomer". Nucleosides with an unmodified DNA or RNA sugar moiety are termed DNA or RNA nucleosides herein. Nucleosides with modifications in the base region of the DNA or RNA nucleoside are still generally termed DNA or RNA if they allow Watson-Crick base pairing.

*Sugar-modified nucleosides*

The antisense oligonucleotides of the invention may comprise one or more nucleosides, which have a modified sugar moiety, *i.e.,* a modification of the sugar moiety when compared to the ribose sugar moiety found in DNA and RNA.

Numerous nucleosides with modification of the ribose sugar moiety have been made, primarily with the aim of improving certain properties of oligonucleotides, such as affinity and/or nuclease resistance.

Such modifications include those where the ribose ring structure is modified, *e.g.*, by replacement with a hexose ring (HNA), or a bicyclic ring, which typically have a bridge between the C2 and C4 carbons on the ribose ring (LNA),

or an unlinked ribose ring which typically lacks a bond between the C2 and C3 carbons (*e.g.*, UNA). Other sugar modified nucleosides include, for example, bicyclohexose nucleic acids (WO2011/017521) or tricyclic nucleic acids (WO2013/154798). Modified nucleosides also include nucleosides where the sugar moiety is replaced with a non-sugar moiety, for example in the case of peptide nucleic acids (PNA), or morpholino nucleic acids.

Sugar modifications also include modifications made via altering the substituent groups on the ribose ring to groups other than hydrogen, or the 2'-OH group naturally found in DNA and RNA nucleosides. Substituents may, for example, be introduced at the 2', 3', 4' or 5' positions.

Non-limiting examples of modified sugar moieties include the following:

o-L-threofuranosyl (as in threose nucleic acid; TNA),
2'-methoxy-ribose (2'-OMe),
2'-O-methoxyethyl-ribose (2'-O-MOE),
5'-methyl-2'-O-methoxyethyl ribose (5'-Me-2'-O-MOE),
2'-O-[2-(methylthio)ethyl]-ribose (2'-O-MTE),
2-(N-methylcarbamoyl)-ethyl]-ribose (2'-O-MCE),
2'-O-[2-(methylamino)-2-oxoethyl]-ribose (2'-O-NMA),
2'-deoxy-2'-fluoro-ribose (as in 2'-deoxy-2'-fluororibo-nucleic acid; 2'-F-RNA),
2'-fluoro-2'-arabinose (as in 2'-fluoro-2'-arabinose nucleic acid; 2'-F-ANA),
2'-O-benzyl-ribose,
oxy, amino or thio β-D-locked ribose (as in β-D-LNA),
oxy, amino or thio o-L-locked ribose (as in o-L-LNA),
2',4'-constrained 2'-O-ethyl ribose (as in constrained ethyl locked nucleic acid; cEt),
tricyclo-deoxyribose (as in tricyclo-deoxyribose DNA; TcDNA),
3'-deoxy-ribose (as in 3'-deoxy-ribose DNA; 3'-DNA),
unlocked ribose (as in unlocked nucleic acid; UNA),
glycol (as in glycol nucleic acid; GNA),
hexitol (as in hexitol nucleic acid; HNA),
3'-fluoro hexitol (as in 3'-fluoro hexitol nucleic acid; FHNA),
3'-arabino-fluoro hexitol (as in 3'-arabino-fluoro hexitol nucleic acid; Ara-FHNA),
cyclohexene (as in cyclohexene nucleic acid; CeNA), and
fluoro-cyclohexenenyl (as in 2'-fluoro-cyclohexenyl nucleic acid; F-CeNA).

Unless otherwise specified or contradicted by context, the term "MOE" may herein refer to any nucleoside which comprises an O-methoxyethyl-group at the 2' position of the ribose ring, including, but not limited to 2'-O-MOE and 5'-Me-2'-O-MOE.

*Threose nucleic acids (TNA)*

As used herein, an "a-L-threofuranosyl nucleoside", "o-L-threose nucleic acid nucleoside", "TNA nucleoside", "TNA-modified nucleoside", "TNA unit", "TNA moiety" and the like refers to a sugar-modified nucleoside which comprises an o-L-threofuranosyl moiety.

TNA nucleosides are linked to adjacent nucleosides by (2'->3') internucleoside linkages, e.g., phosphodiester (PO) or modified internucleoside linkages, as illustrated below for two adjacent TNA nucleosides.

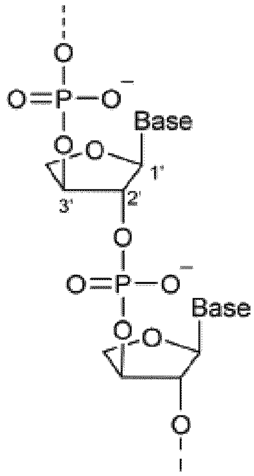

(Formula VIII)

A sugar-modified nucleoside which comprises an o-L-threofuranosyl moiety and is linked to at least one adjacent nucleoside via a PO linkage can be referred to herein as a "a-L-threofuranosyl (PO) nucleoside", "o-L-threose nucleic acid (PO) nucleoside", "TNA(PO) nucleoside", "TNA(PO)-modified nucleoside", "TNA(PO) unit", "TNA(PO) moiety" and the like. Contemplated TNA(PO) nucleosides include those linked by a 2'-PO linkage, a 3'-PO linkage, or both; hereinafter referred to as a "2'-PO linked TNA nucleoside", "3'-PO linked TNA nucleoside", and "2',3'-PO linked TNA nucleoside," respectively.

When the nucleobase (B) is cytosine, the TNA or TNA(PO) nucleoside is advantageously a 5-methyl-cytosine ($^{m}$C) TNA or TNA(PO) nucleoside.

*2'-Sugar-modified nucleosides*

A 2'-sugar modified nucleoside is a nucleoside which has a substituent other than H or -OH at the 2'-position (2'-substituted nucleoside). This includes a nucleoside which comprises a 2'-linked biradical capable of forming a bridge between the 2'-carbon and a second carbon in the ribose ring, such as LNA (2' - 4' bridged) nucleosides.

For the purpose of the present disclosure, a TNA or TNA(PO) nucleoside is not a 2'-substituted nucleoside.

Numerous 2' substituted nucleosides have been found to have beneficial properties when incorporated into oligonucleotides. For example, a 2'-modified sugar may provide enhanced binding affinity and/or increased nuclease resistance to the oligonucleotide. Examples of 2'-substituted modified nucleosides are 2'-O-alkyl-RNA, 2'-O-methyl-RNA, 2'-alkoxy-RNA, 2'-O-methoxyethyl-RNA (2'-O-MOE), 2'-amino-DNA, 2'-fluoro-RNA, 2'-F-ANA, and 2'-bridged molecules like LNA. For further examples, see, *e.g.*, Freier & Altmann; Nucl. Acid Res., 1997, 25, 4429-4443 and Uhlmann; Curr. Opinion in Drug Development, 2000, 3(2), 293-213, and Deleavey and Damha, Chemistry and Biology 2012, 19, 937. Scheme 1 below shows illustrations of some 2'-substituted modified nucleosides.

Scheme 1:

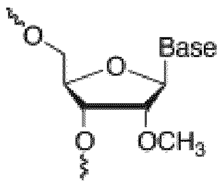

2'-*O*-Me

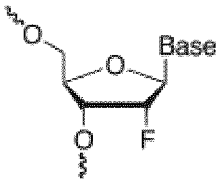

2'F-RNA

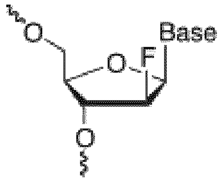

2'F-ANA

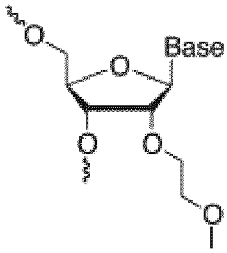

2'-*O*-MOE

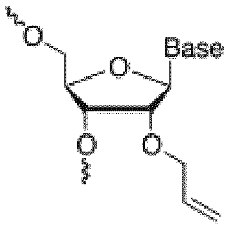

2'-*O*-Allyl

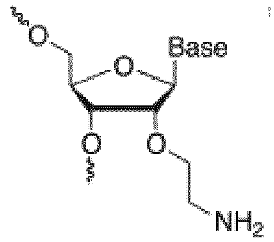

2'-*O*-Ethylamine

*Locked nucleic acids (LNA)*

An "LNA nucleoside" is a 2'-modified nucleoside which comprises a biradical linking the C2' and C4' of the ribose sugar ring of said nucleoside (also referred to as a "2'- 4' bridge"), which restricts or locks the conformation of the ribose ring. These nucleosides are also termed bridged nucleic acid or bicyclic nucleic acid (BNA) in the literature. The locking of the conformation of the ribose is associated with an enhanced affinity of hybridization (duplex stabilization) when the LNA is incorporated into an oligonucleotide for a complementary RNA or DNA molecule. This can be routinely determined by measuring the melting temperature of the oligonucleotide/complement duplex.

Non-limiting, exemplary LNA nucleosides are disclosed in WO 99/014226, WO 00/66604, WO 98/039352, WO 2004/046160, WO 00/047599, WO 2007/134181, WO 2010/077578, WO 2010/036698, WO 2007/090071, WO 2009/006478, WO 2011/156202, WO 2008/154401, WO 2009/067647, WO 2008/150729, Morita et al., Bioorganic & Med. Chem. Lett. 2002, 12, 73-76, Seth et al. J. Org. Chem. 2010, Vol 75(5) pp. 1569-81, and Mitsuoka et al., Nucleic Acids Research 2009, 37(4), 1225-1238, and Wan and Seth, J. Medical Chemistry 2016, 59, 9645-9667.

Further non limiting, exemplary LNA nucleosides are disclosed in Scheme 2.

Scheme 2:

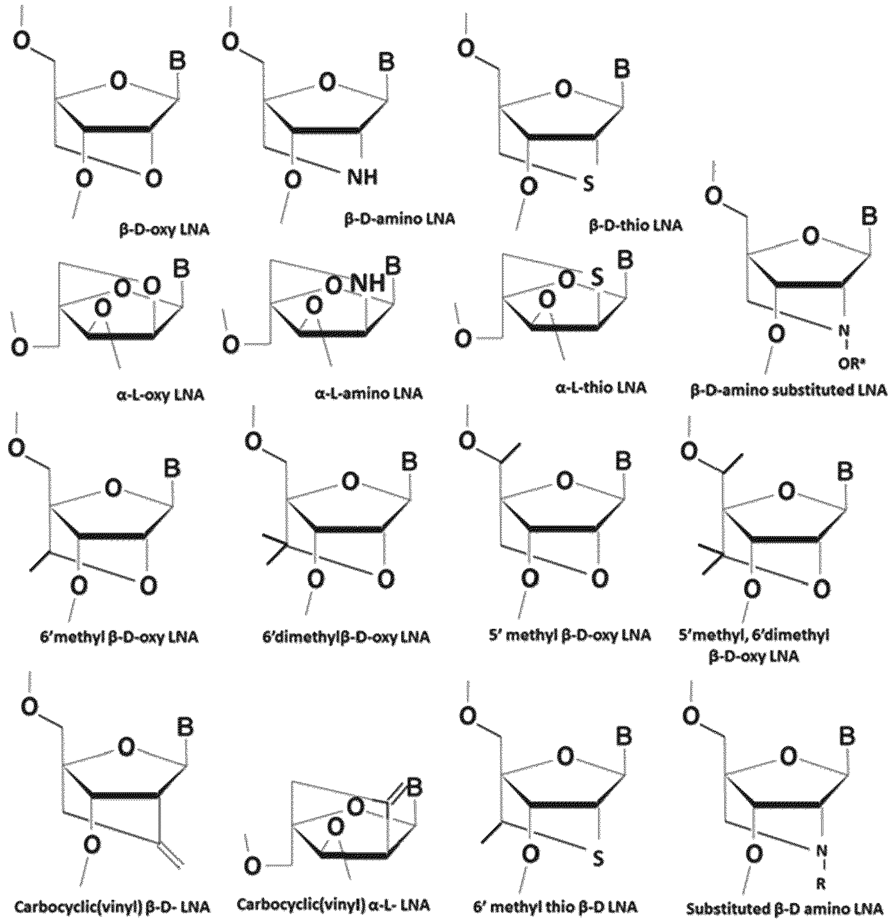

β-D-oxy LNA, β-D-amino LNA, β-D-thio LNA, β-D-amino substituted LNA, α-L-oxy LNA, α-L-amino LNA, α-L-thio LNA, 6'methyl β-D-oxy LNA, 6'dimethylβ-D-oxy LNA, 5' methyl β-D-oxy LNA, 5'methyl, 6'dimethyl β-D-oxy LNA, Carbocyclic(vinyl) β-D- LNA, Carbocyclic(vinyl) α-L- LNA, 6' methyl thio β-D LNA, Substituted β-D amino LNA

Particular LNA nucleosides are beta-D-oxy-LNA, 6'-methyl-beta-D-oxy LNA such as (S)-6'-methyl-beta-D-oxy-LNA (ScET) and ENA. A particularly advantageous LNA is beta-D-oxy-LNA.

*Internucleoside linkages*

The term "internucleoside linkage" is defined, as generally understood by the skilled person, as a linkage that covalently couples two nucleosides together. In antisense oligonucleotides as described herein, internucleoside linkages covalently couple adjacent nucleosides together, typically forming a bond between the sugar moieties of the adjacent nucleosides. Non-limiting examples of internucleoside linkages include phosphodiester (PO) linkages and modified internucleoside linkages.

*Modified internucleoside linkages*

The term "modified internucleoside linkage" is defined as generally understood by the skilled person as a linkage other than a phosphodiester (PO) linkage that covalently couples two nucleosides together. Modified internucleoside linkages may increase the nuclease resistance of the oligonucleotide compared to a phosphodiester (PO) linkage. Modified internucleoside linkages can stabilize oligonucleotides for *in vivo* use and may protect against nuclease cleavage at regions of DNA or RNA nucleosides in an oligonucleotide, for example within the gap region of a gapmer oligonucleotide, as well as in regions of modified nucleosides, such as region F and F'.

Nuclease resistance may be determined by incubating the oligonucleotide in blood serum or by using a nuclease resistance assay (*e.g.*, snake venom phosphodiesterase (SVPD)), both are well known in the art. In some oligonucleotides, all of the internucleoside linkages of an oligonucleotide, or contiguous nucleotide sequence thereof, may be nuclease resistant internucleoside linkages. It is contemplated that nucleosides, which link an oligonucleotide to a non-nucleotide functional group, such as a conjugate, may be phosphodiester.

*Phosphorothioate internucleoside linkages*

A preferred modified internucleoside linkage is phosphorothioate (PS). Phosphorothioate internucleoside linkages can be useful due to nuclease resistance, beneficial pharmacokinetics and ease of manufacture. In some oligonucleotides, all modified internucleoside linkages of the oligonucleotide, or contiguous nucleotide sequence thereof, are phosphorothioate linkages.

Nuclease resistant linkages, such as phosphorothioate linkages, can, for example, be useful in oligonucleotide regions capable of recruiting nuclease when forming a duplex with the target nucleic acid, such as region G for gapmers. Phosphorothioate linkages may, however, also be useful in non-RNase H recruiting regions and/or affinity enhancing regions such as regions F and F' for gapmers.

*Complementarity*

The term "complementarity" describes the capacity for Watson-Crick base-pairing of nucleosides/nucleotides. Watson-Crick base pairs are guanine (G)-cytosine (C) and adenine (A) - thymine (T)/uracil (U). It will be understood that oligonucleotides may comprise nucleosides with modified nucleobases, for example 5-methyl cytosine is often used in place of cytosine, and as such the term complementarity encompasses Watson Crick base-pairing between non-modified and modified nucleobases (see for example Hirao et al (2012) Accounts of Chemical Research vol 45, page 2055 and Bergstrom (2009) Current Protocols in Nucleic Acid Chemistry Suppl. 37 1.4.1).

The term "% complementary" as used herein, refers to the number of nucleotides in percent of a contiguous nucleotide sequence in a nucleic acid molecule (e.g., oligonucleotide) which, at a given position, are complementary to (*i.e.*, form Watson Crick base pairs with) a contiguous sequence of nucleotides, at a given position of a separate nucleic acid molecule (*e.g.*, the target nucleic acid or target sequence). The percentage is calculated by counting the number of aligned bases that form pairs between the two sequences (when aligned with the target sequence 5'-3' and the oligonucleotide sequence from 3'-5'), dividing by the total number of nucleotides in the oligonucleotide and multiplying by 100. In such a comparison a nucleobase/nucleotide which does not align (form a base pair) is termed a mismatch. Preferably, insertions and deletions are not allowed in the calculation of % complementarity of a contiguous nucleotide sequence.

The term "fully complementary", refers to 100% complementarity.

*Identity*

The term "identity" as used herein, refers to the proportion of nucleotides (expressed in percent) of a contiguous nucleotide sequence in a nucleic acid molecule (*e.g.*, oligonucleotide) which across the contiguous nucleotide sequence, are identical to a reference sequence (*e.g.*, a sequence motif). The percentage of identity is thus calculated by counting the number of aligned bases that are identical (a match) between two sequences (*e.g.*, in the contiguous nucleotide sequence of the compound of the invention and in the reference sequence), dividing that number by the total number of nucleotides in the aligned region and multiplying by 100. Therefore, Percentage of Identity = (Matches x 100)/Length of aligned region (*e.g.*, the contiguous nucleotide sequence). Insertions and deletions are not allowed in the calculation of the percentage of identity of a contiguous nucleotide sequence. It will be understood that in determining identity, chemical modifications of the nucleobases are disregarded as long as the functional capacity of the nucleobase to form Watson Crick base pairing is retained (*e.g.*, 5-methyl cytosine is considered identical to a cytosine for the purpose of calculating % identity).

*Hybridization*

The term "hybridizing" or "hybridizes" as used herein is to be understood as two nucleic acid strands (*e.g.*, an oligonucleotide and a target nucleic acid) forming hydrogen bonds between base pairs on opposite strands thereby forming a duplex. The affinity of the binding between two nucleic acid strands is the strength of the hybridization. It is often described in terms of the melting temperature ($T_m$) defined as the temperature at which half of the oligonucleotides are duplexed with the target nucleic acid. At physiological conditions $T_m$ is not strictly proportional to the affinity (Mergny and Lacroix, 2003, Oligonucleotides 13:515-537). The standard state Gibbs free energy $\Delta G°$ is a more accurate representation of binding affinity and is related to the dissociation constant ($K_d$) of the reaction by $\Delta G° = -RT\ln(K_d)$, where R is the gas constant and T is the absolute temperature. Therefore, a very low $\Delta G°$ of the reaction between an oligonucleotide and the target nucleic acid reflects a strong hybridization between the oligonucleotide and target nucleic acid. $\Delta G°$ is the energy associated with a reaction where aqueous concentrations are 1M, the pH is 7, and the temperature is 37°C. The hybridization of oligonucleotides to a target nucleic acid is a spontaneous reaction and for spontaneous reactions $\Delta G°$ is less than zero. $\Delta G°$ can be measured experimentally, for example, by use of the isothermal titration calorimetry

(ITC) method as described in Hansen et al., 1965, Chem. Comm. 36-38 and Holdgate et al., 2005, Drug Discov Today. The skilled person will know that commercial equipment is available for ΔG° measurements. ΔG° can also be estimated numerically by using the nearest neighbor model as described by SantaLucia, 1998, Proc Natl Acad Sci USA. 95: 1460-1465 using appropriately derived thermodynamic parameters described by Sugimoto et al., 1995, Biochemistry 34: 11211-11216 and McTigue et al., 2004, Biochemistry 43:5388-5405. In order to have the possibility of modulating its intended nucleic acid target by hybridization, oligonucleotides of the present invention hybridize to a target nucleic acid with estimated ΔG° values below -10 kcal for oligonucleotides that are 10-30 nucleotides in length. For example, the degree or strength of hybridization can be measured by the standard state Gibbs free energy ΔG°. The oligonucleotides may hybridize to a target nucleic acid with estimated ΔG° values below the range of -10 kcal, such as below -15 kcal, such as below -20 kcal and such as below -25 kcal for oligonucleotides that are 8-30 nucleotides in length. The oligonucleotides may, for example, hybridize to a target nucleic acid with an estimated ΔG° value of -10 to -60 kcal, such as -12 to -40, such as from -15 to -30 kcal or-16 to -27 kcal such as -18 to -25 kcal.

*Target nucleic acid*

A target nucleic acid is a nucleic acid to which an antisense oligonucleotide can hybridize and thereby modulate the expression of a target gene. The target nucleic acid can, for example, be a gene, an RNA, an mRNA, a pre-mRNA, a long non-coding RNA (lncRNA), a mature mRNA or a cDNA sequence or a synthetic nucleic acid derived from DNA or RNA. A target nucleic acid which is an RNA can be referred to as an "RNA target sequence", a "target RNA sequence" or the like.

*Target sequence*

The term "target sequence" as used herein refers to a sequence of nucleotides present in the target nucleic acid, which comprises the nucleobase sequence, which is complementary to an antisense oligonucleotide as described herein. The target sequence may, for example, consist of a region on the target nucleic acid, which is complementary to the contiguous nucleotide sequence of the oligonucleotide of the invention.

*Target cell*

The term a "target cell" as used herein refers to a cell, which is expressing the target nucleic acid. Suitably, the target cell comprises at least one copy of the target gene in its genome. The target cell may be *in vivo* or *in vitro.* The target cell may, for example, be a mammalian cell such as a rodent cell, such as a mouse cell or a rat cell, or a primate cell such as a monkey cell (*e.g.*, a cynomolgus monkey cell) or a human cell.

*Modulation of expression*

The term "modulation of expression" as used herein is to be understood as an overall term for an oligonucleotide's ability to alter the amount of protein expressed or RNA transcribed from the target gene. Modulation of expression may be determined by reference to a control experiment. The control may be an individual or target cell treated with a saline composition or an individual or target cell treated with a non-targeting oligonucleotide (mock).

*High affinity modified nucleosides*

A high affinity modified nucleoside is a modified nucleotide which, when incorporated into the oligonucleotide enhances the affinity of the oligonucleotide for its complementary target, for example as measured by the melting temperature ($T^m$). A high affinity modified nucleoside preferably results in an increase in melting temperature between +0.5 to +12°C, more preferably between +1.5 to +10°C and most preferably between+3 to +8°C per modified nucleoside. Numerous high-affinity modified nucleosides are known in the art and include for example, many 2'-sugar substituted nucleosides such as 2'-O-MOE, 2'-F-RNA, and LNA and analogs thereof (see *e.g.*, Freier & Altmann; Nucl. Acid Res., 1997, 25, 4429-4443 and Uhlmann; Curr. Opinion in Drug Development, 2000, 3(2), 293-213).

*RNase H activity and recruitment*

The ribonuclease (RNase) H activity of an antisense oligonucleotide refers to its ability to recruit RNase H when in a duplex with a complementary RNA molecule. WO 01/23613 provides *in vitro* methods for determining RNaseH activity, which may be used to determine the ability to recruit RNaseH. Recombinant human RNase H1 is available from Lubio Science GmbH, Lucerne, Switzerland. Typically, an oligonucleotide is deemed capable of recruiting RNase H if

it, when provided with a complementary target nucleic acid sequence, has an initial cleavage rate of target RNA molecules, as measured in pmol/l/min, which is at least 5%, such as at least 10% or more than 20% of the initial cleavage rate determined when using a oligonucleotide having the same base sequence as the antisense oligonucleotide being tested, but containing only DNA monomers with phosphorothioate linkages between all monomers in the oligonucleotide, and using the methodology provided by Example 91 - 95 of WO 01/23613 (hereby incorporated by reference).

*Gapmer*

The antisense oligonucleotide, or contiguous nucleotide sequence thereof, may be or comprise a gapmer. Gapmers are commonly used to inhibit a target nucleic acid via RNase H mediated degradation. A gapmer comprises at least three distinct structural regions - a 5'-flank, a gap and a 3'-flank - in the '5 -> 3' orientation, herein represented as 5'-F-G-F'-3' (Formula I). The "gap" region (G) comprises a stretch of contiguous DNA nucleotides, which enable the oligonucleotide to recruit RNase H. The gap region is flanked by a 5' flanking region (F) comprising one or more sugar-modified nucleosides, and by a 3' flanking region (F') comprising one or more sugar-modified nucleosides. The one or more sugar-modified nucleosides in region F and F' may enhance the affinity of the oligonucleotide for the target nucleic acid (*i.e.*, are affinity enhancing sugar-modified nucleosides, such as high-affinity modified nucleosides) or may modulate other properties as desired.

In a gapmer design, the 5'- and 3'-most nucleosides of the gap region are typically DNA nucleosides and are positioned adjacent to a sugar-modified nucleoside of the 5' (F) or 3' (F') region respectively. The flanks may further be defined by having at least one sugar modified nucleoside at the end most distant from the gap region, *i.e.,* at the 5' end of the 5' flank and at the 3' end of the 3' flank.

Regions F-G-F' form a contiguous nucleotide sequence. An antisense oligonucleotide, or a contiguous nucleotide sequence thereof, may comprise or consist of a gapmer of formula I, *i.e.,* F-G-F'.

The overall length of the gapmer design F-G-F' is typically from 12 to 32 nucleosides, such as from 12 to 28, such as from 12 to 26, such as from 14 to 26, such as from 14 to 24, such as from 14 to 22, such as from 16 to 22 nucleosides, such as from 16 to 20 nucleosides.

Traditional gapmer designs, which do not comprise TNA nucleosides, include, for example, $F_{1-8}$-$G_{5-18}$-$F'_{1-8}$ (II), such as $F_{1-8}$-$G_{7-16}$-$F'_{2-8}$ (III), typically with the proviso that the overall length of the gapmer regions F-G-F' is at least 12, such as at least 14 nucleotides in length.

Designs suitable for TNA(PO) gapmers according to the present invention include, for example, $F_{1-15}$-$G_{3-18}$-$F'_{1-15}$ (IV), typically with the proviso that the overall length of the gapmer regions F-G-F' is at least 12, such as at least 14 nucleotides in length. Additional designs (e.g., Formulas IV to VII) for such gapmers are described in more details elsewhere herein.

Regions F, G and F' are further described below and can be incorporated into any of the F-G-F' formulae.

*Gapmer - Region G*

Region G (gap region) of the gapmer is a region of nucleosides, which enables the oligonucleotide to recruit RNase H, such as human RNase H1, typically DNA nucleosides. RNaseH is a cellular enzyme, which recognizes the duplex between DNA and RNA, and enzymatically cleaves the RNA molecule.

Traditional gapmers, which do not comprise TNA nucleosides, include, for example, a gap region (G) of at least 5 or 6 contiguous DNA nucleosides, such as 5 - 16 contiguous DNA nucleosides, such as 6 - 15 contiguous DNA nucleosides, such as 7-14 contiguous DNA nucleosides, such as 8 - 12 contiguous DNA nucleotides, such as 8 - 12 contiguous DNA nucleotides in length.

Suitable gapmers according to the present invention, particularly gapmers comprising one or more TNA(PO) nucleosides as described herein, may have a gap region (G) comprising at least 3 contiguous DNA nucleosides. The gap region G may, for example, comprise or consist of 3, 4, 5, 6, 7, 8, 9, 10, 11, 12, 13, 14, 15, 16, 17 or 18 contiguous DNA nucleosides. Preferably, the gap region G comprises at least 4, at least 5, or at least 6 contiguous DNA nucleosides.

A gap region (G) which comprises one or more TNA(PO) nucleosides as described herein has a DNA nucleoside at the 5' end of the gap (adjacent to the 3' nucleoside of region F), and a DNA nucleoside at the 3' end of the gap (adjacent to the 5' nucleoside of region F'), typically retaining a region of at least 3 or 4 contiguous DNA nucleosides at either the 5' end, the 3' end, or both, of the gap region.

The total length of a gap region G is typically up to 18 contiguous nucleosides. For example, the total length of the gap region G may be from 3 to 18 contiguous nucleosides, such as from 3 to 16 contiguous nucleosides, such as from 4 to 18, 4 to 16, 4 to 14, 4 to 12, or 4 to 10 contiguous nucleosides, such as from 5 to 18, 5 to 16, 5 to 14, 5 to 12, or 5 to 10 contiguous nucleosides, such as from 6 to 18, 6 to 16, 6 to 14, 6 to 12, or 6 to 10 contiguous nucleosides. Shorter gap regions are also contemplated, such as a region G comprising or consisting of 4, 5, 6, 7, 8 or 9 contiguous nucleosides, *e.g.*, contiguous DNA nucleosides.

One or more cytosine (C) DNA nucleosides in the gap region may in some instances be methylated (*e.g.*, when a C DNA nucleoside is followed by a guanine (G) DNA nucleoside and annotated as 5-methyl-cytosine ($^{me}C$ or $^{m}C$).

Oligonucleotides contemplated include those where all the internucleoside linkages in the gap are phosphorothioate linkages, or where all modified internucleoside linkages in the gap are phosphorothioate linkages.

Whilst traditional gapmers have a DNA gap region, there are numerous examples of modified nucleosides, which allow for RNase H recruitment when they are used within the gap region. Modified nucleosides which have been reported as being capable of recruiting RNase H when included within a gap region include, for example, alpha-L-LNA, C4' alkylated DNA (as described in PCT/EP2009/050349 and Vester et al., Bioorg. Med. Chem. Lett. 18 (2008) 2296 - 2300, both incorporated herein by reference), arabinose derived nucleosides like ANA and 2'F-ANA (Mangos et al. 2003 J. AM. CHEM. SOC. 125, 654-661), UNA (unlocked nucleic acid) (as described in Fluiter et al., Mol. Biosyst., 2009, 10, 1039 incorporated herein by reference). UNA or "unlocked nucleic acid" is typically where the bond between C2 and C3 of the ribose has been removed, forming an unlocked "sugar" residue. The modified nucleosides used in gapmers may be nucleosides which adopt a 2'-endo (DNA-like) structure when introduced into the gap region, allowing for RNaseH recruitment. The DNA Gap region (G) described herein may, for example, optionally contain 1 or more (e.g., 1 to 3) sugar modified nucleosides. Any two or more sugar-modified nucleosides in the gap may be consecutive or separated by one or more DNA nucleosides.

As described herein, modified nucleosides which may be used in the gap region include TNA(PO) nucleosides.

*Region G - "Gap-breaker"*

There are also numerous reports on the insertion of modified nucleosides, which confer a 3' endo conformation into the gap region of gapmers, whilst retaining some RnaseH activity. Gapmers with a gap region comprising one or more 3' endo modified nucleosides are referred to as "gap-breaker" or "gap-disrupted" gapmers, see for example WO2013/022984. Gap-breaker oligonucleotides retain sufficient region of DNA nucleosides within the gap region to allow for RnaseH recruitment. The ability of gap-breaker oligonucleotide design to recruit RnaseH is typically sequence or even compound specific - see Rukov et al. 2015 Nucl. Acids Res. Vol. 43 pp. 8476-8487, which discloses "gap-breaker" oligonucleotides, which recruit RnaseH which in some instances provide a more specific cleavage of the target RNA. Modified nucleosides used within the gap region of gap-breaker oligonucleotides may for example be modified nucleosides which confer a 3'-endo conformation, such 2'-O-methyl (Ome) or 2'-O-MOE (MOE) nucleosides, or beta-D LNA nucleosides (the bridge between C2' and C4' of the ribose sugar ring of a nucleoside is in the beta conformation), such as beta-D-oxy LNA or ScET nucleosides.

TNA(PO) nucleosides may also be contemplated as gap-breakers.

As with gapmers containing region G described above, the gap region of gap-breaker or gap-disrupted gapmers, have a DNA nucleoside at the 5' end of the gap (adjacent to the 3' nucleoside of region F), and a DNA nucleoside at the 3' end of the gap (adjacent to the 5' nucleoside of region F'). Gapmers which comprise a disrupted gap typically retain a region of at least 3 or 4 contiguous DNA nucleosides at either the 5' end, the 3' end, or both, of the gap region.

Exemplary designs for gap-breaker gapmers as described herein include

$$F_{1-15}-[D_{3-4}-E_{1}-D_{3-4}]-F'_{1-15}$$

$$F_{1-15}-[D_{1-4}-E_{1}-D_{3-4}]-F'_{1-15}$$

$$F_{1-15}-[D_{3-4}-E_{1}-D_{1-4}]-F'_{1-15}$$

wherein region G is within the brackets [$D_n$-$E_r$- $D_m$], D is a contiguous sequence of DNA nucleosides, E is a modified nucleoside (a gap-breaker or gap-disrupting nucleoside), and F and F' are the flanking regions as defined herein, and with the proviso that the overall length of the gapmer regions F-G-F' is at least 12, such as at least 14 nucleotides in length.

Region G of a gap disrupted gapmer as described herein may comprise at least 4 DNA nucleosides, such as 4, 5, 6, 7, 8, 9, 10, 11, 12, 13, 14, 15 or 16 DNA nucleosides. As described above, the DNA nucleosides may be contiguous or may optionally be interspersed with one or more modified nucleosides, with the proviso that the gap region G is capable of mediating RNase H recruitment.

*Gapmer - flanking regions, F and F'*

Region F is positioned immediately adjacent to the 5' DNA nucleoside of region G. The 3' most nucleoside of

region F is a sugar modified nucleoside. Advantageously the one or two 5' most nucleosides of region F are also sugar modified nucleosides. In a gapmer as described herein, particularly a gapmer comprising one or more TNA(PO) nucleosides, Region F is at least one, such as at least 2, such as at least 3 contiguous nucleotides in length. Typically, region F is up to 15 contiguous nucleotides in length. For example, Region F can be 1, 2, 3, 4, 5, 6, 7, 8, 9, 10, 11, 12, 13, 14 or 15 contiguous nucleotides in length, such as 3, 4, 5, 6, 7, 8, 9, 10, 11 or 12 contiguous nucleotides in length.

Region F' is positioned immediately adjacent to the 3' DNA nucleoside of region G. The 5' most nucleoside of region F' is a sugar modified nucleoside. Advantageously the one or two 3' most nucleosides of region F' are also sugar modified nucleosides. In a gapmer as described herein, particularly a gapmer comprising one or more TNA(PO) nucleosides, Region F' is at least one, such as at least 2, such as at least 3 contiguous nucleotides in length. Typically, region F' is up to 15 contiguous nucleotides in length. For example, Region F' can be 2, 3, 4, 5, 6, 7, 8, 9, 10, 11, 12, 13, 14 or 15 contiguous nucleotides in length, such as 3, 4, 5, 6, 7, 8, 9, 10, 11 or 12 contiguous nucleotides in length.

Any sugar-modified nucleotide can be used in region F and/or F' of an antisense oligonucleotide as described herein provided that the antisense oligonucleotide retains the capability to recruit RNase H and any other desired properties. Examples of sugar-modified nucleosides for use in F, F', or both of F and F', are described herein and include, without limitation, those disclosed in the sections entitled "Sugar-modified nucleosides," including those more particularly described in the sections entitled "Threose nucleic acids (TNA), "2'-Sugar-modified nucleosides" and "Locked nucleic acids." A TNA(PO) gapmer as described herein may, for example, comprise one or more TNA nucleosides, LNA nucleosides, MOE nucleosides, or mixtures thereof.

*LNA gapmer*

An LNA gapmer is a gapmer wherein one or both of region F and F' comprise or consist of LNA nucleosides. A beta-D-oxy gapmer is a gapmer wherein either one or both of region F and F' comprises or consists of beta-D-oxy LNA nucleosides. An LNA gapmer can, for example, have the formula: $[LNA]_{1-5}$-[region G] -$[LNA]_{1-5}$, wherein region G is as described in the section entitled "Gapmer - Region G." An example of a specific LNA gapmer design is 3-10-3 (LNA-DNA-LNA).

*MOE gapmer*

A MOE gapmer is a gapmer wherein one or both of regions F and F' comprise or consist of MOE nucleosides, e.g., 2'-O-MOE nucleosides. A MOE gapmer can, for example, have the formula $[MOE]_{1-8}$-[Region G]-$[MOE]_{1-8}$, such as $[MOE]_{2-7}$-$[Region G]_{5-16}$-$[MOE]_{2-7}$, such as $[MOE]_{3-6}$-[Region G]-$[MOE]_{3-6}$, wherein region G is as described in the section entitled "Gapmer - Region G". MOE gapmers with a 5-10-5 design (MOE-DNA-MOE) have been widely used in the art.

*TNA Gapmer*

A "TNA gapmer" or "TNA modified gapmer" is a gapmer wherein the linked nucleosides of one or more of regions F, F' and G comprise at least one TNA nucleoside.

A TNA gapmer can, for example, have a formula in which the nucleosides of F, F', or both F and F', consist of TNA nucleosides.

*TNA(PO) Gapmer*

A "TNA(PO) gapmer" or "TNA(PO) modified gapmer" is a gapmer wherein the linked nucleosides of one or more of regions F, F' and G comprise at least one TNA nucleoside linked to at least one adjacent nucleoside by a phosphodiester (PO) internucleoside linkage.

A TNA(PO) gapmer can, for example, have a formula in which the nucleosides of F, F', or both F and F', consist of TNA nucleosides linked by PO internucleoside linkages. These and other examples of specific designs of TNA(PO) gapmers are described elsewhere herein.

*Mixed Wing Gapmer*

A mixed wing gapmer is a gapmer wherein one or both of region F and region F' comprise more than one type of sugar-modified nucleosides. Many sugar-modified nucleosides are known in the art and contemplated for this purpose. The two or more different sugar-modified nucleosides in a flank region can, for example, be selected from the those disclosed in the sections entitled "Sugar-modified nucleosides," including but limited to those disclosed in the sections entitled "Threose nucleic acids (TNA)", "2'-Sugar-modified nucleosides" and "Locked nucleic acids."

Mixed-wing gapmers contemplated include, for example, those where at least one of region F and region F' comprises a TNA or TNA(PO) nucleoside. The other sugar modified nucleoside(s) may then be selected from, for example, a 2' substituted nucleoside, such as a 2' substituted nucleoside independently selected from the group consisting of 2'-O-alkyl-RNA units, 2'-O-methyl-RNA, 2'-amino-DNA units, 2'-fluoro-DNA units, 2'-alkoxy-RNA, MOE units, arabino nucleic acid (ANA) units and 2'-fluoro-ANA units, such as MOE nucleosides.
Also contemplated are mixed wing gapmers wherein, when at least one of region F and F', or both region F and F' comprise at least one TNA or TNA(PO) nucleoside, the remaining nucleosides of region F and F' are independently selected from the group consisting of MOE and LNA. When at least one of region F and F', or both region F and F' comprise at least two LNA nucleosides, the remaining nucleosides of region F and F' can, for example, be independently selected from the group consisting of MOE and LNA. In some mixed wing gapmers, one or both of region F and F' may further comprise one or more DNA nucleosides.
Mixed wing gapmer designs are disclosed in WO2008/049085 and WO2012/109395, both of which are hereby incorporated by reference.

*Alternating Flank Gapmer*

Oligonucleotides with alternating flanks are gapmer oligonucleotides where at least one of the flanks (F or F') comprises DNA in addition to a sugar modified nucleoside, *e.g.*, a sugar-modified nucleoside selected from those described herein in the sections entitled "Sugar-modified nucleosides," including but limited to those described in the sections entitled "Threose nucleic acids", "2'-Sugar-modified nucleosides" and "Locked nucleic acids." For example, apart from DNA, an alternating flank gapmer may comprise TNA, TNA(PO), LNA and/or MOE nucleoside(s).
For example, at least one of region F or F', or both region F and F', may comprise both sugar modified nucleosides and DNA nucleosides. The flanking region For F', or both F and F', typically then comprises at least three nucleosides, wherein the 5' and 3' most nucleosides of the F and/or F' region are sugar-modified nucleosides.

*Region D' or D"*

Antisense oligonucleotides as described herein may comprise further 5' and/or 3' nucleosides which are not fully complementary to the target nucleic acid. The further 5' and/or 3' nucleosides may be referred to as region D' and D" herein.
The addition of region D' or D" may be used for the purpose of joining the contiguous nucleotide sequence, such as the gapmer, to a conjugate moiety or another functional group. When used for joining the contiguous nucleotide sequence with a conjugate moiety can serve as a cleavable linker. It may also or alternatively be used to provide exonuclease protection or for ease of synthesis or manufacture.
Region D' and D" can be attached to the 5' end of region F or the 3' end of region F', respectively to generate designs of the following formulas D'-F-G-F', F-G-F'-D" or D'-F-G-F'-D". In this instance the F-G-F' is the gapmer portion of the oligonucleotide and region D' or D" constitute a separate part of the oligonucleotide.
Region D' or D" may independently comprise or consist of 1, 2, 3, 4 or 5 additional nucleotides, which may be complementary or non-complementary to the target nucleic acid. The nucleotide adjacent to the F or F' region is not a sugar-modified nucleotide, such as a DNA or RNA or base modified versions of these. The D' or D' region may serve as a nuclease susceptible bio-cleavable linker. For example, the additional 5' and/or 3' end nucleotides can be DNA or RNA nucleotides and can be linked by phosphodiester linkages.
Nucleotide based bio-cleavable linkers suitable for use as region D' or D" are disclosed in WO2014/076195, which include by way of example a phosphodiester linked DNA dinucleotide. The use of bio-cleavable linkers in poly-oligonucleotide constructs is disclosed in WO2015/113922, where they are used to link multiple antisense constructs (e.g., gapmer regions) within a single oligonucleotide.

*Conjugates*

The term "conjugate" as used herein refers to an oligonucleotide, which is covalently linked to a non-nucleotide moiety (conjugate moiety or region C or third region).
Conjugation of antisense oligonucleotides described herein to one or more non-nucleotide moieties may improve the pharmacology of the oligonucleotide, e.g., by affecting the activity, cellular distribution, cellular uptake or stability of the oligonucleotide. Conjugation may, for example, modify or enhance the pharmacokinetic properties of the oligonucleotide by improving cellular distribution, bioavailability, metabolism, excretion, permeability, and/or cellular uptake of the oligonucleotide. In particular the conjugate may target the oligonucleotide to a specific organ, tissue or cell type and thereby enhance the effectiveness of the oligonucleotide in that organ, tissue or cell type. At the same time the conjugate may serve to reduce activity of the oligonucleotide in non-target cell types, tissues or organs, *e.g.*, off target activity or

activity in non-target cell types, tissues or organs.

The non-nucleotide moiety (conjugate moiety) can, for example, be selected from the group consisting of carbohydrates, cell surface receptor ligands, drug substances, hormones, lipophilic substances, polymers, proteins, peptides, toxins (*e.g.*, bacterial toxins), vitamins, viral proteins (*e.g.*, capsids) or combinations thereof.

*Linkers*

A linkage or linker is a connection between two atoms that links one chemical group or segment of interest to another chemical group or segment of interest via one or more covalent bonds. Conjugate moieties can be attached to the oligonucleotide directly or through a linking moiety (*e.g.*, linker or tether). Linkers serve to covalently connect a third region, *e.g.,* a conjugate moiety (Region C), to a first region, *e.g.*, an oligonucleotide or contiguous nucleotide sequence or gapmer region F-G-F' (region A).

A conjugate or oligonucleotide conjugate may optionally comprise a linker region (second region or region B and/or region Y) which is positioned between the oligonucleotide or contiguous nucleotide sequence complementary to the target nucleic acid (region A or first region) and the conjugate moiety (region C or third region).

Region B refers to bio-cleavable linkers comprising or consisting of a physiologically labile bond that is cleavable under conditions normally encountered or analogous to those encountered within a mammalian body. Conditions under which physiologically labile linkers undergo chemical transformation (*e.g.*, cleavage) include chemical conditions such as pH, temperature, oxidative or reductive conditions or agents, and salt concentration found in or analogous to those encountered in mammalian cells. Mammalian intracellular conditions also include the presence of enzymatic activity normally present in a mammalian cell such as from proteolytic enzymes or hydrolytic enzymes or nucleases. The bio-cleavable linker may, for example, be susceptible to S1 nuclease cleavage. DNA phosphodiester containing bio-cleavable linkers are described in more detail in WO 2014/076195 (hereby incorporated by reference) - see also region D' or D" herein.

Region Y refers to linkers that are not necessarily bio-cleavable but primarily serve to covalently connect a conjugate moiety (region C or third region), to an oligonucleotide (region A or first region). The region Y linkers may comprise a chain structure or an oligomer of repeating units such as ethylene glycol, amino acid units or amino alkyl groups. The oligonucleotide conjugates can be constructed of the following regional elements A-C, A-B-C, A-B-Y-C, A-Y-B-C or A-Y-C. The linker (region Y) can, for example, be an amino alkyl, such as a $C_2$ - $C_{36}$ amino alkyl group, including, for example $C_6$ to $C_{12}$ amino alkyl groups. Preferably, the linker (region Y) is a $C_6$ amino alkyl group.

*Treatment*

The term 'treatment' as used herein refers to both treatment of an existing disease (*e.g.*, a disease or disorder as herein referred to), or prevention of a disease, *i.e.,* prophylaxis. It will therefore be recognized that treatment as referred to herein may be prophylactic.

<u>TNA-Modified Antisense Oligonucleotides</u>

Despite being non-natural, Threose Nucleic Acids (TNAs) can form stable Watson-Crick duplexes and show strong affinity and specificity toward complementary RNA targets. As shown herein, TNA(PO) modified gapmers provide new design strategies for antisense oligonucleotide applications, particularly as an alternative to traditional gapmers employing only PS linkages. Using Caspase 3/7 activation, *in vitro* target knockdown and thermal melting assays, it was demonstrated that TNA(PO) modification can be used to mitigate toxicity while still maintaining target knockdown efficacy and affinity for the target nucleic acid. For example, in a gapmer of a state-of-the-art design such as an LNA or MOE gapmer, TNA(PO) units can replace one or more or all LNA or MOE units in the flank regions. TNA(PO) units can also replace one or more or up to all but three or four consecutive DNA units in the gap region of a gapmer of a state-of-the-art design, for example, which may effectively result in an extended 5' or 3' flank and a reduced gap. Moreover, TNAs are hardly recognized by nucleases. Therefore, when designed into gapmers, TNA(PO) units can lead to maintained or increased metabolic stability, maintained or longer duration of action, or both, while reducing the sulfur content as compared to gapmer designs employing only PS linkages. TNA(PO) modified gapmers can therefore yield long-acting therapeutic agents with an increased therapeutic index compared to classical gapmer designs.

Accordingly, the invention provides an antisense oligonucleotide, such as an antisense gapmer oligonucleotide, comprising a TNA nucleoside linked to an adjacent nucleoside by a PO internucleoside linkage. The TNA nucleoside can be linked to an adjacent nucleoside by a 2'-PO internucleoside linkage or a 3'-PO internucleoside linkage. The TNA nucleoside can also be linked to *any* adjacent nucleoside by a 2'-PO or 3'-PO internucleoside linkage. Non-limiting examples of adjacent nucleosides include sugar-modified nucleosides and DNA nucleosides. The TNA nucleoside can, for example, be linked to a first and a second adjacent nucleoside by a 2'-PO internucleoside linkage and a 3'-modified

internucleoside linkage, respectively; a 2'-modified internucleoside linkage and a 3'-PO internucleoside linkage, respectively; or a 2'-PO internucleoside linkage and a 3'-PO internucleoside linkage, respectively, optionally wherein the 3'-modified internucleoside linkage is a PS internucleoside linkage.

The invention also provides an antisense gapmer oligonucleotide, such as an antisense gapmer oligonucleotide, comprising one or more TNA(PO) nucleotides, typically selected from the group consisting of 2'-PO linked, 3'-PO linked and 2',3'-PO linked TNA nucleosides.

The antisense gapmer oligonucleotide may particularly comprise a contiguous nucleotide sequence of formula 5'-F-G-F'-3' (I) which is capable of recruiting ribonuclease (RNase) H. A contiguous nucleotide sequence of formula 5'-F-G-F'-3' (I) which comprises at least one TNA(PO) nucleoside can be referred to herein as a "TNA(PO) gapmer". Contemplated designs for a TNA(PO) gapmer include those wherein

G is a gap region of up to 18 linked nucleosides which comprises at least 3 contiguous DNA nucleosides,
each of F and F' is a flanking region of up to 15 linked nucleosides which independently comprises or consists of 1 to 15 sugar-modified nucleosides, and
at least one of F, F' and G comprises a sugar-modified nucleoside which is a TNA(PO) nucleoside.

While the following sections provide more details on TNA(PO) gapmers, it should be understood that, unless otherwise indicated or contradicted by context, they apply equally to an antisense gapmer oligonucleotide, or a conjugate thereof, which comprises or consist of a TNA(PO) gapmer.

Advantageously, a TNA(PO) gapmer can modulate the expression of a target gene by reducing or inhibiting its expression into mRNA and/or a protein, typically by hybridizing to a target nucleic acid. When the target nucleic acid is an RNA, *e.g.,* a pre-mRNA, mRNA, viral RNA, microRNA or lncRNA target nucleic acid, the TNA(PO) gapmer is capable of reducing or inhibiting the expression of the target RNA. This is achieved by the complementarity between the TNA(PO) gapmer and the target RNA, and, suitably, the recruitment of a cellular RNase such as RNase H. The TNA(PO) gapmer may additionally be able to reduce or inhibit the expression of a target RNA by non-RNase H mediated mechanisms, such as a steric blocking mechanism resulting in microRNA inhibition, reduced splice modulation of pre-mRNAs, or blocking of the interaction between an lncRNA and chromatin.

Preferably, the TNA(PO) gapmer can reduce the expression level of the target by at least about 20% compared to the normal expression level of the target, more preferably by at least about 30%, at least about 40%, at least about 50%, at least about 60%, at least about 70%, at least about 80%, or at least about 90% compared to the normal expression level of the target. The TNA(PO) gapmer can preferably also or alternatively inhibit the expression of the target by at least about 20% compared to the normal expression level of the target, more preferably by at least about 30%, at least about 40%, at least about 50%, at least about 60%, at least about 70%, at least about 80%, or at least about 90% compared to the normal expression level of the target.

Assays for evaluating the reduction of the expression level or inhibition of expression of a particular target are known to the skilled person. Suitable assays include *in vitro* assays using target cells which comprise at least one copy of the target gene in the genome and express the target, *e.g.,* the target RNA. For example, in an *in vitro* assay where target cells are incubated with about 25 μM TNA(PO) gapmer, a TNA(PO) gapmer can be capable of reducing the expression levels of an RNA target by at least about 50%, such as at least about 60%, as compared to the normal expression level of the RNA target. In such an assay, a TNA(PO) gapmer can also or alternatively be capable of inhibiting the expression of an RNA target by at least about 50%, such as at least about 60%, as compared to the normal expression of the RNA target. At a concentration of about 25 μM, a TNA(PO) gapmer may also be able to reduce or inhibit the expression level of an RNA target by at least about 70%, such as at least about 80%, such as at least about 90% as compared to the normal expression level of the target.

The normal expression level of the RNA target can be determined using a control where the target cells are incubated without TNA(PO) gapmer (*e.g.*, in the presence of vehicle only) or with an irrelevant control oligonucleotide. Target cells for *in vitro* assays can be obtained from commercial sources (*e.g.*, in the form of cell lines) or isolated from blood or other tissues of a human or an experimental animal. The target cells may, for example, be incubated with the TNA(PO) gapmer or control for about 1 to 5 days, such as about 2, 3 or 4 days, such as about 3 days. The RNA can then be extracted and the level of remaining target RNA in test and control samples determined by gene expression analysis. Alternatively, instead of determining the remaining target RNA, the level of an RNA species (*e.g.*, mRNA) or protein derived from the target RNA can be determined in test and control samples. An example of a typical assay for evaluating reduction of the expression level or inhibition of expression of a target RNA by a TNA(PO) gapmer, which can be adapted to other targets and target cells, is provided in Example 2.

The ability of a TNA(PO) gapmer to reduce the expression level of the target or inhibit the expression of the target can also be evaluated by determining the IC50 value, *i.e.,* the concentration of the TNA(PO) gapmer where the expression level of the target nucleic acid is reduced by half. In an *in vitro* assay using target cells which comprise at least one copy of the target gene in the genome and express the target, *e.g.*, the target RNA, the IC50 is preferably no

more than about 20 μM, such as no more than about 10 μM, such as no more than about 5 μM. Typically, the IC50 value is determined in a cell assay similar to that already described except that target cells are incubated with a dilution series of the TNA(PO) gapmer which spans the IC50 value. An example of a typical assay for evaluating the IC50 reduction of the expression level or inhibition of expression of a target RNA by a TNA(PO) gapmer, which can be adapted to other targets and target cells, is provided in Example 3.

The ability of a TNA(PO) gapmer can also be evaluated in relation to a control or "parent" gapmer from which the TNA(PO) gapmer is derived and which does not comprise any TNA nucleoside. The IC50 value of a TNA(PO) gapmer is preferably no more than about 10, no more than about 8, no more than about 6, no more than about 4, or no more than about 2 times that of the control or "parent" gapmer.

A TNA(PO) gapmer can also be characterized by having a low toxicity. For example, the TNA(PO) gapmer may have a lower toxicity than a corresponding control gapmer, such as a state-of-the-art reference gapmer or "parent" gapmer which differs from the TNA(PO) gapmer in that its nucleosides do not include any TNA nucleoside. Suitable assays for evaluating the toxicity of a gapmer or antisense nucleotide are known in the art and include, for example, *in vitro* assays such as Caspase 3/7 assays. Caspase 3/7 assays reflect the level of apoptosis induced by a compound and are suitable for, *e.g.*, evaluating the risk for hepatotoxicity of a compound based on tests on liver cells or cell lines. Briefly, HepG2 cells from a commercial source can be transfected with 100 nM TNA(PO) or control gapmers in a suitable vehicle and Caspase 3/7 activation determined at about 24 hours post-transfection. Preferably, the Caspase 3/7 activation from transfection with a TNA(PO) gapmer is at most about 70%, such as at most about 60%, such as at most about 50%, such as at most about 40%, such as at most about 30%, such as at most about 20% of the corresponding control gapmer. Alternatively, using the Caspase 3/7 assay described in Example 4, the percentage determined (% assay window, reflecting apoptotic cells over the total cells), for a TNA(PO) gapmer is preferably at most about 250%, more preferably at most about 200%, more preferably at most about 150%, at most about 100%, at most about 80%, at most about 60%, at most about 50%, at most about 40%, at most about 30%, at most about 20%, or at most about 10%. Preferably, using the using the Caspase 3/7 assay described in Example 4, the percentage determined (% assay window) for a TNA(PO) gapmer is at most about 80%, such as at most about 60%.

A TNA(PO) gapmer is capable of hybridizing to the target nucleic acid, *e.g.*, a target RNA, particularly to the target sequence to which its nucleobase sequence is complementary. The ability of a TNA(PO) gapmer to hybridize to its target nucleic acid can be evaluated according to any assay known in the art. Advantageously, thermal melting (Tm) analysis can be used, determining at which temperature a duplex between a TNA(PO) gapmer and its RNA target sequence denatures, which can be denoted the melting temperature or simply Tm. A typical assay for determining the Tm of a TNA(PO) gapmer (*i.e.*, in the form of a duplex with a complementary RNA target sequence) is described in Example 5. Briefly, TNA(PO) gapmer and RNA target sequence can be added to 20mM disodium phosphate buffer, 200 mM NaCl and 0.2 mM EDTA (pH 7) resulting in a final concentration of 1.5 μM. Samples can be heated to 95°C for 5 min and then slowly cooled to room temperature over a period of 1 hour, and thermal melting curves recorded at 260 nm using a temperature gradient, *e.g.*, with an increase by 5°C/min from 25°C to 95°C and then decreased to 25°C. From the derivative of both curves, the melting temperature (Tm) can be determined. Preferably, a TNA(PO) gapmer has a Tm of at least about 50°C, such as at least about 52°C, such as at least about 54°C, such as at least about 56°C, such as at least about 58°C, such as at least about 60°C, such as at least about 65°C, such as at least about 70°C.

In some cases, there may be mismatches between the oligonucleotide and the target nucleic acid, such as 1 or 2 mismatches. Despite mismatches, the hybridization to the target nucleic acid may still be sufficient to show a desired ability to modulate the target.

Preferably, a TNA(PO) gapmer

(a) can reduce the expression level of a target nucleic acid by at least about 50%, such as at least about 60%, such as at least about 70%, such as at least about 80%, such as at least about 90%, as compared to the normal expression level of the target;

(b) has an IC50 of no more than about 20 μM, such as no more than about 10 μM, such as no more than about 5 μM, for reducing the expression level of a target nucleic acid;

(c) using the Caspase 3/7 assay described in Example 4, the percentage assay window (%AW) is preferably at most about 60%, such as at most about 40%, such as at most about 20%, such as at most about 10%;

(d) has, in the form of a duplex of the antisense gapmer oligonucleotide with an RNA target sequence, a melting temperature (Tm) of at least about 50°C, such as at least about 52°C, such as at least about 54°C, such as at least about 56°C, such as at least about 58°C, such as at least about 60°C; or

(e) a combination of any two or more of (a) to (d).

For example, a preferred TNA(PO) gapmer can be characterized by both features (a) and (b). Another preferred TNA(PO) gapmer can be characterized by both features (a) and (c). Another preferred TNA(PO) gapmer can be characterized by both features (a) and (d). Another preferred TNA(PO) gapmer can be characterized by both features (b)

and (c). Another preferred TNA(PO) gapmer can be characterized by both features (b) and (d). Another preferred TNA(PO) gapmer can be characterized by both features (c) and (d). Another preferred TNA(PO) gapmer can be characterized by features (a), (b) and (c). Another preferred TNA(PO) gapmer can be characterized by features (a), (b) and (d). Another preferred TNA(PO) gapmer can be characterized by features (a), (c) and (c). Another preferred TNA(PO) gapmer can be characterized by features (b), (c) and (d). A preferred TNA(PO) gapmer can also be characterized by all of features (a) to (d).

Preferably, the target nucleic acid in (a) and (b) is an RNA, and the RNA target sequence in (c) has a nucleobase sequence complementary to the contiguous nucleotide sequence of the TNA(PO) gapmer. The reduction in expression level of the and (a) and (b) can, for example, be determined in a target cell expressing the target nucleic acid and incubated with the antisense gapmer oligonucleotide at a concentration of about 25 μM for about 3 days, as already described above.

In some TNA(PO) gapmers, region F comprises at least one TNA nucleoside. Region F of a TNA gapmer may, for example, comprise up to 15 TNA nucleosides.

In some TNA(PO) gapmers, region F comprises at least one TNA(PO) nucleoside. Region F may also comprise at least two, at least three, at least four, at least five, at least six, at least seven, at least eight, at least nine, at least ten, at least eleven or at least twelve TNA(PO) nucleosides, such as two, three, four, five, six, seven, eight, nine, ten, eleven, twelve, thirteen, fourteen or fifteen TNA(PO) nucleosides. TNA(PO) gapmers wherein region F comprises or consists of one TNA(PO) nucleoside are also contemplated.

In some TNA(PO) gapmers, region F comprises at least two, three, four, five, six, seven, eight, nine, ten, eleven or twelve adjacent TNA(PO) nucleosides, such as two, three, four, five, six, seven, eight, nine, ten, eleven, twelve, thirteen, fourteen or fifteen adjacent TNA(PO) nucleosides. Region F may also consist of two, three, four, five, six, seven, eight, nine, ten, eleven, twelve, thirteen, fourteen or fifteen adjacent TNA(PO) nucleosides.

In some TNA(PO) gapmers, at least the 3'-most nucleoside in F is a TNA nucleoside. Suitably, at least the two, at least the three, at least the four, at least the five, at least the six, at least the seven, at least the eight, at least the nine, at least the ten, at least the eleven, or at least the twelve 3'-most nucleosides in F can be TNA nucleosides. For example, the two, three, four, five, six, seven, eight, nine, ten, eleven, twelve, thirteen or fourteen 3'-most nucleosides in F can be TNA nucleosides, such as TNA(PO) nucleosides.

In some TNA(PO) gapmers, at least the 5'-most nucleoside in F is a TNA nucleoside. Suitably, at least the two, at least the three, at least the four, at least the five, at least the six, at least the seven, at least the eight, at least the nine, at least the ten, at least the eleven, or at least the twelve 5'-most nucleosides in F can be TNA nucleosides. For example, the two, three, four, five, six, seven, eight, nine, ten, eleven, twelve, thirteen or fourteen 5'-most nucleosides in F can be TNA nucleosides, such as TNA(PO) nucleosides.

In some TNA(PO) gapmers, both the 3'-most and the 5'-most nucleosides in F are TNA nucleosides. For example, the 3'-most and/or 5'-most nucleosides in F can be independently selected from one, two, three, four, five, six, seven, eight, nine, ten, eleven, or twelve TNA nucleosides, such as TNA(PO) nucleosides.

Suitably, in TNA(PO) gapmers, each TNA nucleoside in F can be a TNA(PO) nucleoside. However, also contemplated are TNA(PO) gapmers, wherein, except for the 5'-most TNA nucleoside in F, each TNA nucleoside in F is a TNA(PO) nucleoside. The 5'-most TNA nucleoside can, for example, be linked to the adjacent nucleoside via a PS internucleoside linkage.

Any remaining nucleoside(s) in F can be one or more other sugar-modified nucleosides than a TNA(PO) nucleoside (*e.g.*, in the form of a mixed-wing gapmer) or one or more DNA nucleosides (*e.g.*, in the form of an alternating flank gapmer). For example, F may further comprise 1 to 8 sugar-modified nucleosides other than TNA(PO) nucleosides, such as two, three, four or five sugar-modified nucleosides other than TNA(PO) nucleosides. Non-limiting examples of sugar-modified nucleosides include those described in the section entitled "Sugar-modified nucleosides," such as, *e.g.*, LNA, 2'-O-MOE, and TNA nucleosides, optionally linked by PS internucleoside linkages.

Contemplated are also TNA(PO) gapmers wherein all sugar-modified nucleosides of F are TNA(PO) nucleosides. A TNA(PO) gapmer may, for example, be an alternating flank gapmer where the nucleosides of F consist of DNA and TNA(PO) with, *e.g.*, 1, 2 or 3 DNA nucleosides. Suitably, at least the 5'-most and 3'-most nucleosides of F are then TNA(PO) nucleosides. However,

The nucleosides of F may also consist of a TNA(PO) nucleoside. Alternatively, region F may consist of more than one TNA(PO) nucleoside, such as two, three, four, five, six, seven, eight, nine, ten, eleven or twelve TNA(PO) nucleosides. TNA(PO) nucleosides where the nucleosides of F consist of thirteen, fourteen or fifteen TNA(PO) nucleosides are also contemplated. Typically, when the nucleosides of F consist of two or more TNA(PO) nucleosides, F is a contiguous sequence of linked TNA(PO) nucleosides.

In some TNA gapmers, F does not comprise any TNA(PO) nucleoside.

In some TNA(PO) gapmers, region F' comprises at least one TNA nucleoside. Region F' of a TNA gapmer may, for example, comprise up to 15 TNA nucleosides.

In some TNA(PO) gapmers, region F' comprises at least one TNA(PO) nucleoside. Region F' may also comprise

at least two, at least three, at least four, at least five, at least six, at least seven, at least eight, at least nine, at least ten, at least eleven or at least twelve TNA(PO) nucleosides, such as two, three, four, five, six, seven, eight, nine, ten, eleven, twelve, thirteen, fourteen or fifteen TNA(PO) nucleosides. TNA(PO) gapmers wherein region F' comprises or consists of one TNA(PO) nucleoside are also contemplated.

In some TNA(PO) gapmers, region F' comprises at least two, three, four, five, six, seven, eight, nine, ten, eleven or twelve adjacent TNA(PO) nucleosides, such as two, three, four, five, six, seven, eight, nine, ten, eleven, twelve, thirteen, fourteen or fifteen adjacent TNA(PO) nucleosides. Region F' may also consist of two, three, four, five, six, seven, eight, nine, ten, eleven, twelve, thirteen, fourteen or fifteen adjacent TNA(PO) nucleosides.

In some TNA(PO) gapmers, at least the 5'-most nucleoside in F' is a TNA nucleoside. Suitably, at least the two, at least the three, at least the four, at least the five, at least the six, at least the seven, at least the eight, at least the nine, at least the ten, at least the eleven, or at least the twelve 5'-most nucleosides in F' can be TNA nucleosides. For example, the two, three, four, five, six, seven, eight, nine, ten, eleven, twelve, thirteen or fourteen 5'-most nucleosides in F' can be TNA nucleosides, such as TNA(PO) nucleosides.

In some TNA(PO) gapmers, at least the 3'-most nucleoside in F' is a TNA nucleoside. Suitably, at least the two, at least the three, at least the four, at least the five, at least the six, at least the seven, at least the eight, at least the nine, at least the ten, at least the eleven, or at least the twelve 3'-most nucleosides in F' can be TNA nucleosides. For example, the two, three, four, five, six, seven, eight, nine, ten, eleven, twelve, thirteen or fourteen 3'-most nucleosides in F' can be TNA nucleosides, such as TNA(PO) nucleosides.

In some TNA(PO) gapmers, both the 3'-most and the 5'-most nucleosides in F' are TNA nucleosides. For example, the 3'-most and/or 5'-most nucleosides in F' can be independently selected from one, two, three, four, five, six, seven, eight, nine, ten, eleven, or twelve TNA nucleosides, such as TNA(PO) nucleosides.

Suitably, in TNA(PO) gapmers, each TNA nucleoside in F' can be a TNA(PO) nucleoside. However, also contemplated are TNA(PO) gapmers, wherein, except for the 3'-most TNA nucleoside in F', each TNA nucleoside in F is a TNA(PO) nucleoside. The 3'-most TNA nucleoside can, for example, be linked to the adjacent nucleoside via a PS internucleoside linkage.

Any remaining nucleoside(s) in F' can be one or more other sugar-modified nucleosides than a TNA(PO) nucleoside (*e.g.*, in the form of a mixed-wing gapmer) or one or more DNA nucleosides (*e.g.*, in the form of an alternating flank gapmer). For example, F' may further comprise 1 to 8 sugar-modified nucleosides other than TNA(PO) nucleosides, such as two, three, four or five sugar-modified nucleosides other than TNA(PO) nucleosides. Non-limiting examples of sugar-modified nucleosides include those described in the section entitled "Sugar-modified nucleosides," such as, *e.g.*, LNA, 2'-O-MOE, and TNA nucleosides, optionally linked by PS internucleoside linkages.

Contemplated are also TNA(PO) gapmers wherein all sugar-modified nucleosides of F' are TNA(PO) nucleosides. A TNA(PO) gapmer may, for example, be an alternating flank gapmer where the nucleosides of F' consist of DNA and TNA(PO) with, *e.g.*, 1, 2 or 3 DNA nucleosides. Suitably, at least the 5'-most and 3'-most nucleosides of F' are then TNA(PO) nucleosides. However,

The nucleosides of F' may also consist of a TNA(PO) nucleoside. Alternatively, region F' may consist of more than one TNA(PO) nucleoside, such as two, three, four, five, six, seven, eight, nine, ten, eleven or twelve TNA(PO) nucleosides. TNA(PO) nucleosides where the nucleosides of F' consist of thirteen, fourteen or fifteen TNA(PO) nucleosides are also contemplated. Typically, when the nucleosides of F' consist of two or more TNA(PO) nucleosides, F' is a contiguous sequence of linked TNA(PO) nucleosides.

In some TNA gapmers, F' does not comprise any TNA nucleoside.

G comprises a stretch of contiguous DNA nucleosides which enable the antisense oligonucleotide to recruit RNase H. Suitably, G may comprise up to eighteen nucleosides, such as DNA nucleosides. Typically, at least the 5'-most nucleoside in G and the 3'-most nucleoside in G are DNA nucleosides.

In some TNA(PO) gapmers, G does not comprise any TNA nucleoside. For example, G may comprise at least four DNA nucleosides, such as 4, 5, 6, 7, 8, 9, 10, 11, 12, 13, 14, 15 or 16 contiguous DNA nucleosides.

In some TNA(PO) gapmers according to the present invention, G comprises at least one TNA nucleoside. For example, depending on the overall length of region G, the second, third, fourth, fifth, sixth, seventh-most, or eight-most 5' nucleoside in G can be a TNA nucleoside. Alternatively, depending on the overall length of region G, the second, third, fourth, fifth, sixth, seventh-most, or eight-most 3' nucleoside in G can be a TNA nucleoside.

Also contemplated are TNA(PO) gapmers where G comprises at least two or at least three TNA nucleosides. The at least two or at least three TNA nucleosides may be consecutive or non-consecutive. Region G may, for example, comprise two, three, four, five, six, seven, eight, nine, ten, eleven, twelve, thirteen, or fourteen TNA nucleosides, optionally consecutive.

In some TNA(PO) gapmers according to the present invention, G comprises at least one TNA(PO) nucleoside. For example, depending on the overall length of region G, the second, third, fourth, fifth, sixth, seventh-most, or eight-most 5' nucleoside in G can be a TNA(PO) nucleoside. Alternatively, depending on the overall length of region G, the second, third, fourth, fifth, sixth, seventh-most, or eight-most 3' nucleoside in G can be a TNA nucleoside.

Also contemplated are TNA(PO) gapmers where G comprises at least two or at least three TNA(PO) nucleosides. The at least two or at least three TNA(PO) nucleosides may be consecutive or non-consecutive. Region G may, for example, comprise two, three, four, five, six, seven, eight, nine, ten, eleven, twelve, thirteen, or fourteen TNA(PO) nucleosides, optionally consecutive.

Furthermore, as described herein, other modified nucleosides have been reported as being capable of recruiting RNase H when included within a gap region and may also or alternatively be included in the gap region of a TNA(PO) gapmer. Preferably, in TNA(PO) gapmers comprising at least one, such as one, two or three, TNA(PO) nucleosides or other modified nucleosides in gap region G, gap region G still comprises at least three contiguous DNA nucleosides, such as at least four contiguous DNA nucleosides, such as at least five contiguous DNA nucleosides, such as 4, 5, 6, 7, 8, 9, 10, 11, 12, 13, 14, 15 or 16 contiguous DNA nucleosides. In some TNA(PO) gapmers, except for TNA(PO) nucleoside in gap region G, all nucleosides of G are DNA nucleosides.

In some TNA(PO) gapmers, any TNA(PO) nucleosides, particularly any contiguous stretch of two or more TNA or TNA(PO) nucleosides, are located closer to the 5'-end of region G than to the 3'-end of region G. Any contiguous stretch of two or more TNA or TNA(PO) nucleosides in region G may, for example, be located adjacent to the 5'-most nucleoside in region G; typically a DNA nucleoside.

As shown herein, a TNA(PO) gapmer may comprise a stretch of only three or four contiguous DNA nucleosides while still enabling recruitment of RNase H. Without being limited to theory, it is contemplated that a TNA gapmer with a shorter gap region than that of traditional gapmer designs and/or with one or more TNA residues in the gap region may provide for an increased resistance to endonuclease-mediated degradation and/or reduce off-target binding as compared to traditional gapmer designs. Consequently, in some TNA gapmers according to the invention, G comprises at most 10 DNA nucleosides, such as 9, 8, 7, 6, 5, or 4 DNA nucleosides. Optionally, G comprises at most 10 contiguous DNA nucleosides, such as 9, 8, 7, 6, 5, or 4 contiguous DNA nucleosides. Furthermore, as described in more detail further below, when G in a TNA(PO) gapmer comprises 9, 8, 7, 6, 5, or 4 contiguous DNA nucleosides, F' and F may optionally be of different lengths, *e.g.*, so that F comprises more linked nucleosides than F'.

Details on the number and placement of at least one TNA(PO) nucleoside in regions F, G or F' have been set out above and can be incorporated into the general gapmer formula 5'-F-G-F'-3' (I). Specifically contemplated TNA(PO) gapmers include those where

(i) region F comprises at least one TNA(PO) nucleoside but regions F' and G do not;
(ii) region F' comprises at least one TNA(PO) nucleoside but regions F and G do not;
(iii) region G comprises at least one TNA(PO) nucleoside but regions F and F' do not;
(iv) region F and F' each comprises at least one TNA(PO) nucleoside but region G does not;
(v) regions F and G each comprises at least one TNA(PO) nucleoside but region F' does not;
(vi) regions G and F' each comprises at least one TNA(PO) nucleoside but region F does not; and
(vii) regions F, G and F' each comprises at least one TNA(PO) nucleoside.

For example, in a TNA(PO) gapmer according to item (iv) or (vii), F and F' may each independently comprise one, two, three, four, five, six, seven, eight, nine, ten, eleven, twelve, thirteen, fourteen or fifteen TNA(PO) nucleosides. Also contemplated are TNA(PO) gapmers according to item (iv) or (vii) where F and F' each independently comprise one, two, three, four, five, six, seven, eight, nine, ten, eleven, twelve, thirteen, fourteen or fifteen contiguous TNA(PO) nucleosides.

Alternatively, in a TNA(PO) gapmer according to any item but (iii), F and F' may together comprise at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen or at least twenty TNA(PO) nucleosides, such as two, three, four, five, six, seven, eight, nine, ten, eleven, twelve, thirteen, fourteen, fifteen, sixteen, seventeen, eighteen, nineteen, twenty, twenty-one, twenty-two, twenty-three, twenty-four, twenty-five, twenty-six, twenty-seven, twenty-eight, twenty-nine or thirty TNA(PO) nucleosides.

For example, F and F' together may together comprise two, three, four, five, six, seven, eight, nine, ten, eleven, twelve, thirteen, fourteen, fifteen, sixteen, seventeen, eighteen, nineteen, twenty, twenty-one, twenty-two, twenty-three, twenty-four, twenty-five, twenty-six, twenty-seven, twenty-eight, twenty-nine or thirty TNA nucleosides which are linked to an adjacent TNA nucleoside by a PO internucleoside linkage

For example, in a TNA(PO) gapmer according to item (iv), F and F' may together comprise 1, 2, 3, 4, 5, 6, 7, 8, 9, 10 or 12 TNA(PO) nucleosides.

In some TNA(PO) gapmers, each TNA nucleoside in F and F' is linked to an adjacent nucleoside by a PO internucleoside linkage. However, also contemplated are TNA(PO) gapmers wherein, except for the 5'-most TNA nucleoside in F and the 3'-most TNA nucleoside in F', each TNA nucleoside in F and F' is a TNA(PO) nucleoside.

In some TNA(PO) gapmers, each of region F and F' may independently comprise or consist of a contiguous sequence of linked sugar-modified nucleosides.

In some TNA(PO) gapmers, at least one of region F and F' may consist of only one type of sugar modified nucleosides. For example, the sugar modified nucleoside can be a high-affinity nucleoside or a TNA(PO) nucleoside.

In some TNA(PO) gapmers, regions F and F' may both consist of only one type of sugar modified nucleosides (uniform flanks or uniform gapmer design). For example, the sugar modified nucleoside can be a high-affinity nucleoside or a TNA(PO) nucleoside. In some TNA gapmers, all the nucleosides of regions F and F' are TNA(PO) nucleosides. In some TNA(PO) gapmers, all the nucleosides of regions F and F' are sugar-modified nucleosides other than TNA nucleosides.

In some TNA(PO) gapmers, one or both of regions F and F' may independently comprise two different sugar-modified nucleosides (mixed wing design). One of the two different sugar-modified nucleosides can be a TNA(PO) nucleoside and the other sugar-modified nucleoside can be, for example, a high-affinity nucleoside or a TNA nucleoside linked by a modified internucleoside linkage.

In some TNA(PO) gapmers, all the nucleosides of region F can be TNA(PO) nucleosides. The nucleosides of region F' may then, for example, comprise or consist of sugar modified nucleosides other than TNA(PO) nucleosides, such as 2'-sugar modified nucleosides, such as high-affinity nucleosides. Optionally, region F' may comprise two different sugar-modified nucleosides, one of which may be TNA(PO). For example, F' may comprise 1 to 8 sugar-modified nucleosides other than TNA(PO) nucleosides, such as three, four or five sugar-modified nucleosides other than TNA(PO) nucleosides. Alternatively, F' may consist of 1 to 8 sugar-modified nucleosides other than TNA(PO) nucleosides, such as three, four or five sugar-modified nucleosides other than TNA(PO) nucleosides.

In some TNA(PO) gapmers, all the nucleosides of region F' can be TNA(PO) nucleosides. The nucleosides of region F may then, for example, comprise or consist of sugar modified nucleosides other than TNA(PO) nucleosides, such as 2'-sugar modified nucleosides, such as high-affinity nucleosides. Optionally, region F may comprise two different sugar-modified nucleosides, one of which may be TNA(PO). For example, F may comprise 1 to 8 sugar-modified nucleosides other than TNA(PO) nucleosides, such as three, four or five sugar-modified nucleosides other than TNA(PO) nucleosides. Alternatively, F may consist of 1 to 8 sugar-modified nucleosides other than TNA(PO) nucleosides, such as three, four or five sugar-modified nucleosides other than TNA(PO) nucleosides.

In TNA(PO) gapmers where F, F' or both F and F', comprise or consist of one or more sugar-modified nucleosides other than TNA nucleosides, non-limiting examples of sugar-modified nucleosides include those which have modified sugar moiety selected from the group consisting of:

2'-methoxy-ribose (2'-OMe),
2'-O-methoxyethyl-ribose (2'-O-MOE),
5'-methyl-2'-O-methoxyethyl ribose (5'-Me-2'-O-MOE),
2'-O-[2-(methylthio)ethyl]-ribose (2'-O-MTE),
2-(N-methylcarbamoyl)-ethyl]-ribose (2'-O-MCE),
2'-O-[2-(methylamino)-2-oxoethyl]-ribose (2'-O-NMA),
2'-deoxy-2'-fluoro-ribose (as in 2'-deoxy-2'-fluororibo-nucleic acid; 2'-F-RNA),
2'-fluoro-2'-arabinose (as in 2'-fluoro-2'-arabinose nucleic acid; 2'-F-ANA),
2'-O-benzyl-ribose,
oxy, amino or thio β-D-locked ribose (as in β-D-LNA),
oxy, amino or thio o-L-locked ribose (as in o-L-LNA),
2',4'-constrained 2'-O-ethyl ribose (as in constrained ethyl locked nucleic acid; cEt),
tricyclo-deoxyribose (as in tricyclo-deoxyribose DNA; TcDNA),
3'-deoxy-ribose (as in 3'-deoxy-ribose DNA; 3'-DNA),
unlocked ribose (as in unlocked nucleic acid; UNA),
glycol (as in glycol nucleic acid; GNA),
hexitol (as in hexitol nucleic acid; HNA),
3'-fluoro hexitol (as in 3'-fluoro hexitol nucleic acid; FHNA),
3'-arabino-fluoro hexitol (as in 3'-arabino-fluoro hexitol nucleic acid; Ara-FHNA),
cyclohexene (as in cyclohexene nucleic acid; CeNA), and
fluoro-cyclohexenenyl (as in 2'-fluoro-cyclohexenyl nucleic acid; F-CeNA).

Particularly contemplated are TNA(PO) gapmers wherein the sugar-modified nucleosides of F, F' or both F and F', comprise or consist of one or more 2'-sugar modified nucleosides, such as high-affinity 2'-sugar-modified nucleosides.

In some TNA(PO) gapmers, the sugar-modified nucleosides of F, F' or both F and F', comprise one or more LNA nucleosides. For example, except for any at least one TNA(PO) nucleoside, 1, 2, 3, 4, 5, 6, 7, 8 or all nucleosides in F', F, or both F and F', may be LNA nucleosides. In a TNA(PO) gapmer where only region G comprises one or more TNA(PO) nucleosides, regions F and F' may, for example, each comprise or consist of 1, 2, 3, 4, 5, 6, 7, 8 LNA nucleosides,

such as 3, 4 or 5 LNA nucleosides. Suitable LNA nucleosides include those which have a modified sugar moiety selected from oxy, amino or thio β-D-locked ribose (β-D-LNA) or from oxy, amino or thio o-L-locked ribose (a-L-LNA), such as β-D-oxy-LNA, 6'-methyl-β-D-oxy LNA such as (S)-6'-methyl-β-D-oxy-LNA (ScET) and ENA as well as the LNA nucleosides disclosed in Scheme 2. A particularly contemplated LNA nucleoside is β-D-oxy-LNA.

In some TNA(PO) gapmers, the sugar-modified nucleosides of F, F' or both F and F', comprise one or more MOE nucleosides. For example, except for any at least one TNA(PO) nucleoside, 1, 2, 3, 4, 5, 6, 7, 8 or all nucleosides in F', F, or both F and F', may be MOE nucleosides. In a TNA(PO) gapmer where only region G comprises one or more TNA(PO) nucleosides, regions F and F' may, for example, each comprise or consist of 1, 2, 3, 4, 5, 6, 7, 8 MOE nucleosides, such as 3, 4 or 5 MOE nucleosides. Suitable MOE nucleosides include 2'-O-MOE and 5'-Me-2'-O-MOE. A particularly contemplated MOE nucleoside is 2'-O-MOE.

In some TNA(PO) gapmers, the sugar-modified nucleosides of F, F' or both F and F', comprise one or more 2'-OMe nucleosides. For example, except for any at least one TNA(PO) nucleoside, 1, 2, 3, 4, 5, 6, 7, 8 or all nucleosides in F', F, or both F and F', may be 2'-OMe nucleosides. In a TNA(PO) gapmer where only region G comprises one or more TNA(PO) nucleosides, regions F and F' may, for example, each comprise or consist of 1, 2, 3, 4, 5, 6, 7, 8 2'-OMe nucleosides, such as 3, 4 or 5 2'-OMe nucleosides.

In some TNA(PO) gapmers, the sugar-modified nucleosides of F, F' or both F and F', comprise one or more 2'-O-MTE nucleosides. For example, except for any at least one TNA(PO) nucleoside, 1, 2, 3, 4, 5, 6, 7, 8 or all nucleosides in F', F, or both F and F', may be 2'-O-MTE nucleosides. In a TNA(PO) gapmer where only region G comprises one or more TNA(PO) nucleosides, regions F and F' may, for example, each comprise or consist of 1, 2, 3, 4, 5, 6, 7, 8 2'-O-MTE nucleosides, such as 3, 4 or 5 2'-O-MTE nucleosides.

In some TNA(PO) gapmers, the sugar-modified nucleosides of F, F' or both F and F', comprise one or more 2'-O-MCE nucleosides. For example, except for any at least one TNA(PO) nucleoside, 1, 2, 3, 4, 5, 6, 7, 8 or all nucleosides in F', F, or both F and F', may be 2'-O-MCE nucleosides. In a TNA(PO) gapmer where only region G comprises one or more TNA(PO) nucleosides, regions F and F' may, for example, each comprise or consist of 1, 2, 3, 4, 5, 6, 7, 8 2'-O-MCE nucleosides, such as 3, 4 or 5 2'-O-MCE nucleosides.

In some TNA(PO) gapmers, the sugar-modified nucleosides of F, F' or both F and F', comprise one or more 2'-O-NMA nucleosides. For example, except for any at least one TNA(PO) nucleoside, 1, 2, 3, 4, 5, 6, 7, 8 or all nucleosides in F', F, or both F and F', may be 2'-O-NMA nucleosides. In a TNA(PO) gapmer where only region G comprises one or more TNA(PO) nucleosides, regions F and F' may, for example, each comprise or consist of 1, 2, 3, 4, 5, 6, 7, 8 2'-O-NMA nucleosides, such as 3, 4 or 5 2'-O-NMA nucleosides.

In some TNA(PO) gapmers, the sugar-modified nucleosides of F, F' or both F and F', comprise one or more 2'-deoxy-2'-fluoro-ribose nucleosides. For example, except for any at least one TNA(PO) nucleoside, 1, 2, 3, 4, 5, 6, 7, 8 or all nucleosides in F', F, or both F and F', may be 2'-deoxy-2'-fluoro-ribose nucleosides. In a TNA(PO) gapmer where only region G comprises one or more TNA(PO) nucleosides, regions F and F' may, for example, each comprise or consist of 1, 2, 3, 4, 5, 6, 7, 8 2'-deoxy-2'-fluoro-ribose nucleosides, such as 3, 4 or 5 2'-deoxy-2'-fluoro-ribose nucleosides.

In some TNA(PO) gapmers, the sugar-modified nucleosides of F, F' or both F and F', comprise one or more 2'-fluoro-2'-arabinose nucleosides. For example, except for any at least one TNA(PO) nucleoside, 1, 2, 3, 4, 5, 6, 7, 8 or all nucleosides in F', F, or both F and F', may be 2'-fluoro-2'-arabinose nucleosides. In a TNA(PO) gapmer where only region G comprises one or more TNA(PO) nucleosides, regions F and F' may, for example, each comprise or consist of 1, 2, 3, 4, 5, 6, 7, 8 2'-fluoro-2'-arabinose nucleosides, such as 3, 4 or 5 2'-fluoro-2'-arabinose nucleosides.

In some TNA(PO) gapmers, the sugar-modified nucleosides of F, F' or both F and F', comprise one or more 2'-O-benzyl-ribose nucleosides. For example, except for any at least one TNA(PO) nucleoside, 1, 2, 3, 4, 5, 6, 7, 8 or all nucleosides in F', F, or both F and F', may be 2'-O-benzyl-ribose nucleosides. In a TNA(PO) gapmer where only region G comprises one or more TNA(PO) nucleosides, regions F and F' may, for example, each comprise or consist of 1, 2, 3, 4, 5, 6, 7, 8 2'-O-benzyl-ribose nucleosides, such as 3, 4 or 5 2'-O-benzyl-ribose nucleosides.

In some TNA(PO) gapmers, the sugar-modified nucleosides of F, F' or both F and F', comprise one or more cEt nucleosides. For example, except for any at least one TNA(PO) nucleoside, 1, 2, 3, 4, 5, 6, 7, 8 or all nucleosides in F', F, or both F and F', may be cEt nucleosides. In a TNA(PO) gapmer where only region G comprises one or more TNA(PO) nucleosides, regions F and F' may, for example, each comprise or consist of 1, 2, 3, 4, 5, 6, 7, 8 cEt nucleosides, such as 3, 4 or 5 cEt nucleosides.

In some TNA(PO) gapmers, the sugar-modified nucleosides of F, F' or both F and F', comprise one or more TcDNA nucleosides. For example, except for any at least one TNA(PO) nucleoside, 1, 2, 3, 4, 5, 6, 7, 8 or all nucleosides in F', F, or both F and F', may be TcDNA nucleosides. In a TNA(PO) gapmer where only region G comprises one or more TNA(PO) nucleosides, regions F and F' may, for example, each comprise or consist of 1, 2, 3, 4, 5, 6, 7, 8 TcDNA nucleosides, such as 3, 4 or 5 TcDNA nucleosides.

In some TNA(PO) gapmers, the sugar-modified nucleosides of F, F' or both F and F', comprise one or more 3'-DNA nucleosides. For example, except for any at least one TNA(PO) nucleoside, 1, 2, 3, 4, 5, 6, 7, 8 or all nucleosides in F', F, or both F and F', may be 3'-DNA nucleosides. In a TNA(PO) gapmer where only region G comprises one or

more TNA(PO) nucleosides, regions F and F' may, for example, each comprise or consist of 1, 2, 3, 4, 5, 6, 7, 8 3'-DNA nucleosides, such as 3, 4 or 5 3'-DNA nucleosides.

In some TNA(PO) gapmers, the sugar-modified nucleosides of F, F' or both F and F', comprise one or more UNA nucleosides. For example, except for any at least one TNA(PO) nucleoside, 1, 2, 3, 4, 5, 6, 7, 8 or all nucleosides in F', F, or both F and F', may be UNA nucleosides. In a TNA(PO) gapmer where only region G comprises one or more TNA(PO) nucleosides, regions F and F' may, for example, each comprise or consist of 1, 2, 3, 4, 5, 6, 7, 8 UNA nucleosides, such as 3, 4 or 5 UNA nucleosides.

In some TNA(PO) gapmers, the sugar-modified nucleosides of F, F' or both F and F', comprise one or more GNA nucleosides. For example, except for any at least one TNA(PO) nucleoside, 1, 2, 3, 4, 5, 6, 7, 8 or all nucleosides in F', F, or both F and F', may be GNA nucleosides. In a TNA(PO) gapmer where only region G comprises one or more TNA(PO) nucleosides, regions F and F' may, for example, each comprise or consist of 1, 2, 3, 4, 5, 6, 7, 8 GNA nucleosides, such as 3, 4 or 5 GNA nucleosides.

In some TNA(PO) gapmers, the sugar-modified nucleosides of F, F' or both F and F', comprise one or more HNA nucleosides. For example, except for any at least one TNA(PO) nucleoside, 1, 2, 3, 4, 5, 6, 7, 8 or all nucleosides in F', F, or both F and F', may be HNA nucleosides. In a TNA(PO) gapmer where only region G comprises one or more TNA(PO) nucleosides, regions F and F' may, for example, each comprise or consist of 1, 2, 3, 4, 5, 6, 7, 8 HNA nucleosides, such as 3, 4 or 5 HNA nucleosides. Suitable HNA nucleosides include HNA, FHNA, and Ara-FHNA.

In some TNA(PO) gapmers, the sugar-modified nucleosides of F, F' or both F and F', comprise one or more CeNA nucleosides. For example, except for any at least one TNA(PO) nucleoside, 1, 2, 3, 4, 5, 6, 7, 8 or all nucleosides in F', F, or both F and F', may be CeNA nucleosides. In a TNA(PO) gapmer where only region G comprises one or more TNA(PO) nucleosides, regions F and F' may, for example, each comprise or consist of 1, 2, 3, 4, 5, 6, 7, 8 CeNA nucleosides, such as 3, 4 or 5 CeNA nucleosides. Suitable CeNA nucleosides include CeNA and F-CeNA.

Particularly contemplated are TNA(PO) gapmers wherein the contiguous nucleotide sequence of formula 5'-F-G-F'-3' (I) has a length of from 12 to 32 nucleosides, such as from 12 to 28 nucleosides, such as from 12 to 26 nucleosides, such as from 14 to 26 nucleosides, such as from 14 to 24 nucleosides, such as from 14 to 22 nucleosides, such as from 16 to 22 nucleosides, such as from 16 to 20 nucleosides. However, any suitable length can be used for the F-G-F' design, including, but limited to, 12, 13, 14, 15, 16,17, 18, 19,20, 21, 22, 23, 24, 25, 26, 27, 28, 29, 30, 31, and 32 linked nucleosides.

By way of example, a TNA(PO) gapmer according to the present invention can be represented by one or more of the following formulae for regions F-G-F' (Formula I), with the proviso that the overall length of regions F-G-F' is at least 12, such as at least 14 nucleotides in length:

$F_{1-15}$-$G_{3-18}$-$F'_{1-15}$ (IV), such as $F_{1-15}$-$G_{3-18}$-$F'_{1-12}$ (IVa), or $F_{1-12}$-$G_{3-18}$-$F'_{1-15}$ (IVb);
$F_{1-12}$-$G_{3-18}$-$F'_{1-12}$ (V), such as $F_{1-12}$-$G_{3-18}$-$F'_{1-9}$ (Va), or $F_{1-9}$-$G_{3-18}$-$F'_{1-12}$ (Vb);
$F_{1-12}$-$G_{4-16}$-$F'_{1-12}$ (VI), such as $F_{1-12}$-$G_{4-16}$-$F'_{1-9}$ (VIa), or $F_{1-9}$-$G_{4-16}$-$F'_{1-12}$ (VIb); and
$F_{3-12}$-$G_{4-10}$-$F'_{3-12}$ (VII), such as $F_{3-12}$-$G_{4-10}$-$F'_{3-9}$ (VIIa), or $F_{3-9}$-$G_{4-10}$-$F'_{3-12}$ (VIIb).

Each of region F, G and F' as described herein can be incorporated into any of the F-G-F' formulae.

In some TNA(PO) gapmers, the contiguous nucleotide sequence of formula IVa has a length of at least 16 nucleosides and

(a) the 5'-most nucleosides in F and the nucleosides in F' are independently 3, 4 or 5 high-affinity sugar-modified nucleosides,
(b) the remaining nucleosides in F are TNA(PO) nucleosides, and
(c) all nucleosides in G are DNA nucleosides.

For example, G may comprise at most 10 contiguous DNA nucleosides, such as 9, 8, 7, 6, 5, or 4 contiguous DNA nucleosides, such as 4, 5 or 6 contiguous DNA nucleosides. F may, for example, comprise 1, 2, 3, 4, 5, 6, 7, 8, 9, 10, 11 or 12 contiguous TNA(PO) nucleosides.

In some TNA(PO) gapmers, the contiguous nucleotide sequence of formula IV has a length of at least 16 nucleosides and

(a) F and F' each independently consists of 3, 4, or 5 nucleosides, wherein at least one of the nucleosides in F and/or F' is a TNA(PO) nucleoside and any remaining nucleosides are high-affinity sugar-modified nucleosides, and
(b) all nucleosides in G are DNA nucleosides.

For example, all nucleosides in either F or F' may be TNA(PO) nucleosides. Alternatively, all nucleosides in F and F' may be TNA(PO) nucleosides. Alternatively, except for the 5'-most TNA nucleoside in F and the 3'-most TNA

nucleoside in F’, each TNA nucleoside in F and F’ may be linked to an adjacent nucleoside, particularly to any adjacent TNA nucleoside, by a PO internucleoside linkage.

In some TNA(PO) gapmers, the contiguous nucleotide sequence of formula IV has a length of at least 16 nucleosides and

(a) F and F’ each independently comprises or consists of 3, 4, or 5 linked high-affinity sugar-modified nucleosides and does not comprise any TNA nucleoside, and
(b) the second, third, fourth or fifth 5’-most nucleoside in G is a TNA(PO) nucleoside and the remaining nucleosides in G are DNA nucleosides.

The TNA(PO) nucleoside may, for example, be located closer to the 5’-end of region G than to the 3’-end of G.

Particularly contemplated designs of TNA(PO) gapmers include also those referred to as Design A and Design B in Example 1:

TTTTTddddddddddTTTTT (Design A), where T is TNA and d is DNA, and with the backbone (internucleoside linkage) pattern SOOOSSSSSSSSSSSSOOOS, where S corresponds to phosphorothioate (PS), and O to phosphodiester (PO).

MMMMMTTTTTddddMMMMM (Design B), where M is MOE (e.g., 2’-O-MOE), T is TNA and d is DNA, and with the backbone (internucleoside linkage) pattern SSSSOOOOOOSSSSSSSSS, where S corresponds to phosphorothioate (PS), and O to phosphodiester (PO).

Also particularly contemplated is the following design:
TTTTTTTTTTdddddMMMMM (Design C), where M is 2’-O-MOE, T is TNA and d is DNA, and with the backbone (internucleoside linkage) pattern OOOOOOOOOSSSSSSSSSS, where S corresponds to phosphorothioate (PS), and O to phosphodiester (PO).

An antisense gapmer oligonucleotide comprising a TNA(PO) gapmer, *i.e.,* contiguous nucleotide sequence of formula 5’-F-G-F’-3’ (I), may comprise additional linked nucleosides, e.g., from 1 to 100, 1 to 40, 40, 1 to 30, 1 to 20, 1 to 10, or 1 to 5 linked nucleosides, at the 3’- and/or 5’-end of the TNA(PO) gapmer. The additional linked nucleosides may, for example, facilitate delivery of the antisense gapmer oligonucleotide to the intended site or target a second molecule. The antisense gapmer oligonucleotide can also be, or can be part of, a longer nucleic acid construct. However, it is also contemplated that the antisense gapmer oligonucleotide may consist of the TNA(PO) gapmer.

Particularly contemplated for the present invention are TNA(PO) gapmers and antisense gapmer oligonucleotides, which are single-stranded antisense oligonucleotides. In a preparation of TNA(PO) gapmers or antisense gapmer oligonucleotides according to the invention, the TNA(PO) gapmers or antisense gapmer oligonucleotides are essentially single stranded, such that the majority of the TNA(PO) gapmer molecules or antisense gapmer oligonucleotide molecules are in single-stranded form.

*Method of manufacture*

Provided are also methods for manufacturing the oligonucleotides of the invention comprising reacting nucleotide units and thereby forming covalently linked contiguous nucleotide units comprised in the oligonucleotide. Preferably, the method uses phosphoramidite chemistry (see for example Caruthers et al, 1987, Methods in Enzymology vol. 154, pages 287-313).

The synthesis of TNA monomers and their incorporation into oligonucleotides are disclosed in, e.g., Zhang and Chaput, "Synthesis of Threose Nucleic Acid (TNA) Phosphoramidite Monomers and Oligonucleotide Polymers, Current Protocols in Nucleic Acid Chemistry, 4.51.1-4.51.26, 2012", WO 2012/078536, WO 2012/118911 and WO 2013/179292 A1.

Particularly provided is a method of preparing a modified version of a parent antisense gapmer oligonucleotide, wherein the parent antisense gapmer comprises a contiguous nucleotide sequence of formula 5’ F-G-F’ 3’ (I) which is capable of recruiting RNase H, wherein G is a gap region of 5 to 18 linked DNA nucleosides and each of F and F’ is a flanking region of up to 8 linked nucleosides which independently comprises or consists of 1 to 8 sugar-modified nucleosides other than TNA nucleosides, and wherein, in the modified version, at least one nucleoside in F, F’, and/or G of the parent antisense gapmer oligonucleotide has been replaced with a TNA nucleoside linked to an adjacent nucleoside by a PO internucleoside linkage,

the method comprising the step of manufacturing the modified antisense gapmer oligonucleotide by reacting nucleotide units to form covalently linked contiguous nucleotide units comprised in the oligonucleotide, wherein at least

one of the nucleotide units comprises a TNA nucleoside which is linked to an adjacent nucleoside by a PO internucleoside bond, and,
optionally purifying or isolating the modified antisense gapmer oligonucleotide.

In one embodiment, the modified antisense gapmer oligonucleotide has a reduced toxicity, optionally hepatotoxicity, as compared to the parent antisense gapmer oligonucleotide. In one embodiment, the modified antisense gapmer oligonucleotide is less toxic to HepG2 cells than the parent antisense gapmer oligonucleotide, optionally as determined by a Caspase 3/7 assay. In one embodiment, the modified antisense gapmer oligonucleotide has an increased exonuclease resistance as compared to the parent antisense gapmer oligonucleotide. In one embodiment, the modified antisense gapmer oligonucleotide has an increased endonuclease resistance as compared to the parent antisense gapmer oligonucleotide.
Optionally, the parent antisense gapmer oligonucleotide can be an LNA gapmer or MOE gapmer, such as an LNA gapmer or MOE gapmer wherein all internucleoside linkages are phosphorothioate linkages. Furthermore, the nucleotide units employed in the manufacturing step are advantageously nucleoside phosphoramidites.
The modified antisense gapmer oligonucleotide may comprise the features of any TNA(PO) gapmer described herein, *e.g.*, as to regions F, G and F' and the F-G-F' design.
Also provided by the present invention is an antisense gapmer oligonucleotide obtained or obtainable by the method.
The method may further comprise reacting the contiguous nucleotide sequence with a conjugating moiety (ligand) to covalently attach the conjugate moiety to the oligonucleotide.
In a further aspect, a method is provided for manufacturing a composition, comprising mixing the oligonucleotide or conjugated oligonucleotide with a pharmaceutically acceptable diluent, solvent, carrier, salt and/or adjuvant.

*Pharmaceutical Composition*

In a further aspect, the invention provides pharmaceutical compositions comprising any of the aforementioned oligonucleotides and/or oligonucleotide conjugates or salts thereof and a pharmaceutically acceptable diluent, carrier, salt and/or adjuvant.
In a further aspect, the invention provides pharmaceutical compositions comprising any of the aforementioned oligonucleotides and/or oligonucleotide conjugates or salts thereof and a pharmaceutically acceptable diluent, carrier, salt or adjuvant.
A pharmaceutically acceptable diluent includes phosphate-buffered saline (PBS) and pharmaceutically acceptable salts include, but are not limited to, sodium and potassium salts. In some embodiments the pharmaceutically acceptable diluent is sterile phosphate buffered saline. In some embodiments, the oligonucleotide is used in the pharmaceutically acceptable diluent at a concentration of 50 - 300$\mu$M solution.
The oligonucleotides or oligonucleotide conjugates according to the present invention may exist in the form of their pharmaceutically acceptable salts. The term "pharmaceutically acceptable salt" refers to conventional acid-addition salts or base-addition salts that retain the biological effectiveness and properties of the compounds of the present invention and are formed from suitable non-toxic organic or inorganic acids or organic or inorganic bases. Acid-addition salts include for example those derived from inorganic acids such as hydrochloric acid, hydrobromic acid, hydroiodic acid, sulfuric acid, sulfamic acid, phosphoric acid and nitric acid, and those derived from organic acids such as p-toluenesulfonic acid, salicylic acid, methanesulfonic acid, oxalic acid, succinic acid, citric acid, malic acid, lactic acid, fumaric acid, and the like. Base-addition salts include those derived from ammonium, potassium, sodium and quaternary ammonium hydroxides, such as for example, tetramethyl ammonium hydroxide. The chemical modification of a pharmaceutical compound into a salt is a technique well known to pharmaceutical chemists in order to obtain improved physical and chemical stability, hygroscopicity, flowability and solubility of compounds. It is for example described in Bastin, Organic Process Research & Development 2000, 4, 427-435 or in Ansel, In: Pharmaceutical Dosage Forms and Drug Delivery Systems, 6th ed. (1995), pp. 196 and 1456-1457. For example, the pharmaceutically acceptable salt of the compounds provided herein may be a sodium salt.
Suitable formulations for use in the present invention are found in Remington's Pharmaceutical Sciences, Mack Publishing Company, Philadelphia, Pa., 17th ed., 1985. For a brief review of methods for drug delivery, see, *e.g.*, Langer (Science 249: 1527-1533, 1990). WO 2007/031091 provides further suitable and preferred examples of pharmaceutically acceptable diluents, carriers and adjuvants (hereby incorporated by reference). Suitable dosages, formulations, administration routes, compositions, dosage forms, combinations with other therapeutic agents, pro-drug formulations are also provided in WO2007/031091.
Oligonucleotides or oligonucleotide conjugates of the invention may be mixed with pharmaceutically acceptable active or inert substances for the preparation of pharmaceutical compositions or formulations. Compositions and methods for the formulation of pharmaceutical compositions are dependent upon a number of criteria, including, but not limited

to, route of administration, extent of disease, or dose to be administered.

These compositions may be sterilized by conventional sterilization techniques or may be sterile filtered. The resulting aqueous solutions may be packaged for use as is, or lyophilized, the lyophilized preparation being combined with a sterile aqueous carrier prior to administration. The pH of the preparations typically will be between 3 and 11, more preferably between 5 and 9 or between 6 and 8, and most preferably between 7 and 8, such as 7 to 7.5. The resulting compositions in solid form may be packaged in multiple single dose units, each containing a fixed amount of the above-mentioned agent or agents, such as in a sealed package of tablets or capsules. The composition in solid form can also be packaged in a container for a flexible quantity, such as in a squeezable tube designed for a topically applicable cream or ointment.

In some embodiments, the oligonucleotide or oligonucleotide conjugate of the invention is a prodrug. In particular with respect to oligonucleotide conjugates the conjugate moiety is cleaved of the oligonucleotide once the prodrug is delivered to the site of action, e.g., the target cell.

*Applications*

The oligonucleotides or oligonucleotide conjugates described herein may be utilized as research reagents or as diagnostic, therapeutic and prophylactic agents.

In research, the oligonucleotides or oligonucleotide conjugates may be used to specifically modulate the expression of a target nucleic acid in cells (*e.g.*, in *in vitro* cell cultures) and experimental animals, thereby facilitating functional analysis of the target or an appraisal of its usefulness as a target for therapeutic intervention. Typically, the target modulation is achieved by degrading or inhibiting the mRNA producing the protein, thereby preventing protein formation or by degrading or inhibiting a modulator of the gene or mRNA producing the protein.

If employing the oligonucleotide or oligonucleotide conjugate in research or diagnostics, the target nucleic acid may be a cDNA or a synthetic nucleic acid derived from DNA or RNA.

Also provided are *in vivo* or *in vitro* methods for modulating the expression of a target gene in a target cell, which comprises a target nucleic acid, the method comprising administering an oligonucleotide or oligonucleotide conjugate of the invention in an effective amount to said cell.

In some embodiments, the target cell is a mammalian cell, in particular a human cell. The target cell may be an *in vitro* cell culture or an *in vivo* cell forming part of a tissue in a mammal, such as a human.

Also provided are diagnostic applications in which the oligonucleotides or oligonucleotide conjugates may be used to detect and quantitate the expression of a target gene in cells and tissues by northern blotting, in-situ hybridisation or similar techniques.

Also provided is an oligonucleotide, an oligonucleotide conjugate, or a pharmaceutical composition as described herein for use as a medicament.

The disease or disorder in which the oligonucleotide, oligonucleotide conjugate, or pharmaceutical composition is used is typically associated with expression of the target gene. Preferably, the disease or disorder is one, which may be treated by modulating the expression of the target gene.

The oligonucleotide, an oligonucleotide conjugate, or a pharmaceutical composition may, for example, be employed for treatment or prophylaxis against diseases or disorders caused by abnormal levels and/or activity of the target gene or an expression product from the target gene, *e.g.*, an RNA or protein. The disease or disorder may also or alternatively be associated with a mutation in the target gene. Therefore, in some embodiments, the target nucleic acid is a mutated form of the target gene. Non-limiting examples of target genes include genes associated with one or more cancers, infectious diseases, neurological diseases or disorders, ophthalmic diseases or disorders, or cardiovascular diseases or disorders.

The oligonucleotide, oligonucleotide conjugate or a pharmaceutical composition according to the invention is typically administered in an effective amount.

Also provided are therapeutic applications wherein the oligonucleotide, oligonucleotide conjugate, or pharmaceutical composition is for use in treating or preventing a disease or disorder in an animal or a human suffering from or suspected of having the disease or disorder. Typically, the disease or disorder is one, which can be treated by modulating the expression of the target gene.

Also provided are uses of the oligonucleotide or oligonucleotide conjugate in the manufacture of a medicament for treating or preventing a disease or disorder in an animal or a human suffering from or suspected of having the disease or disorder. Typically, the disease or disorder is one, which can be treated by modulating the expression of the target gene.

Also provided are methods for treating or preventing a disease or disorder, comprising administering a therapeutically or prophylactically effective amount of the oligonucleotide, oligonucleotide conjugate or pharmaceutical composition to a subject suffering from or susceptible to the disease or disorder.

*Administration*

In some embodiments, the oligonucleotides or pharmaceutical compositions of the present invention may be administered orally. In further embodiments, the oligonucleotides or pharmaceutical compositions of the present invention may be administered topically or enterally or parenterally (such as, intravenously, subcutaneously, intra-muscularly, intracerebrally, intracerebroventricularly or intrathecally).

In a preferred embodiment the oligonucleotide or pharmaceutical compositions of the present invention are administered by a parenteral route, such as, for example, by intravenous, intraarterial, subcutaneous, intraperitoneal or intramuscular injection or infusion, or intrathecal or intracranial administration, *e.g.*, intracerebral or intraventricular administration, or intravitreal administration. In one embodiment, the active oligonucleotide or oligonucleotide conjugate is administered intravenously. In another embodiment, the active oligonucleotide or oligonucleotide conjugate is administered subcutaneously.

In some embodiments, the oligonucleotide, oligonucleotide conjugate or pharmaceutical composition of the invention is administered at a dose of 0.1 - 15 mg/kg, such as from 0.2 - 10 mg/kg, such as from 0.25 - 5 mg/kg. The administration can be once a week, every 2nd week, every third week or even once a month.

*Combination therapies*

In some embodiments the oligonucleotide, oligonucleotide conjugate or pharmaceutical composition of the invention is for use in a combination treatment with another therapeutic agent. The therapeutic agent can for example be the standard of care for the diseases or disorders to be treated with the oligonucleotide, oligonucleotide conjugate or pharmaceutical composition of the invention.

EXAMPLES

*Example 1: Oligonucleotide synthesis using TNA modification*

Oligonucleotides were synthesized either on a MerMade 12 or on a Mermade 192 automated DNA synthesizer by BioAutomation. Syntheses were conducted on a 1 µmol scale using a controlled pore glass support (500A) bearing a universal linker.

*For the Mermade 12 synthesis*: standard cycle procedures for the coupling of DNA, LNA and MOE phosphoramidites: DMT deprotection was performed with 3% (w/v) trichloroacetic acid in $CH_2Cl_2$ in six applications of 230 µL for 70 sec. The respective phosphoramidites were coupled three times with 95 µL of 0.1M solutions in acetonitrile (or acetonitrile/$CH_2Cl_2$ 1:1 for the LNA-$^{m}$C building block) and 110 µL of a 0.3 M solution of 5-Benzylthio-1-H-tetrazole in anhydrous acetonitrile as an activator and a coupling time of 180 sec. Freshly prepared TNA phosphoramidites were coupled three times with 95 µL of 0.1M solution in acetonitrile and 110 µL of a 0.3 M solution of 5-Benzylthio-1-H-tetrazole in anhydrous acetonitrile as an activator and a coupling time of 360 sec. Sulfurization was performed using a 0.1M solution of 3-amino-1,2,4-dithiazole-5-thione in acetonitrile/pyridine: 1/1 in two applications of 200 µL for 80 sec. Oxidation was performed using a 0.02M $I_2$ in THF/pyr/$H_2O$:88/10/2 in two applications for 80 sec. Capping was performed using THF/lutidine/$Ac_2O$:8/1/1 (CapA, 125 µL) and THF/N-methylimidazole:84/16 (CapB, 125 µL) two times for 85 sec. After the synthesis, the CPG was then transferred carefully into a 4mL vial where 1mL of 25% $NH_4OH$ was added and left for 16 hours at 55°C. Crude DMT-on oligonucleotides were purified either using a solid-phase extraction cartridge (Oasis HLB 6 cc extraction cartridges from Waters) or by preparative reversed-phase HPLC (RP-HPLC) purification (C18 column, $NH_4OAc/CH_3CN$ buffer system) followed by DMT removal with 80% aqueous acetic acid. Following HPLC purification, oligonucleotides were desalted using HiPrep 26/10 desalting column on ÄKTA pure 25 and lyophilized. Oligonucleotides were characterized by reversed phase liquid chromatography coupled to high-resolution electrospray mass spectrometry.

*For the Mermade 192 synthesis:* standard cycle procedures for the coupling of DNA, LNA, MOE and TNA phosphoramidites: DMT deprotection was performed with 3% (w/v) trichloroacetic acid in $CH_2Cl_2$ in three applications of 180 µL for 75sec. The respective DNA and LNA phosphoramidites were coupled three times with 95 µL of 0.1M solutions in acetonitrile (or acetonitrile/$CH_2Cl_2$ 1:1 for the LNA-MeC building block) and 110 µL of a 0.285M solution of 5-Benzylthio-1-H-tetrazole in anhydrous acetonitrile as an activator and a coupling time of 205sec. Freshly prepared TNA phosphoramidites were coupled two times with 95 µL of 0.1M solution in acetonitrile/DMF 95:5 and 110 µL of a 0.285M solution of 5-Benzylthio-1-H-tetrazole in anhydrous acetonitrile/DMF 95:5 as an activator and a coupling time of 385sec. Sulfurization was performed using a 0.1M solution of 3-amino-1,2,4-dithiazole-5-thione in acetonitrile/pyridine: 1/1 in two applications of 190 µL for 385sec. Oxidation was performed using a 0.02M I2 in THF/pyr/H2O:88/10/2 in two applications of 160 µL for 78sec. Capping was performed using THF/Lutidine/Ac2O 8:1:1 (CapA, 125 µL) and THF/N-methylimidazole:84/16 (CapB, 125 µL) two times for 70 sec. After the synthesis, the oligonucleotides were cleaved off

the CPG in 3 portions of 200 µl using 25% $NH_4OH$. After every step, the CPG was allowed to stand at RT for 10min. After the DWP was sealed and the solutions were left for 15hr at 55°C. The crude DMT-on oligonucleotides were then purified and final DMT cleaved by cartridge (Agilent TOP-DNA Tubes, 150mg Resin/tube) using aqueous $NH_4Cl$ Buffers, acetonitrile and 5% TFA in Water.

The TNA phosphoramidites were synthesized as described: "Synthesis of Threose Nucleic Acid (TNA) Phosphoramidite Monomers and Oligonucleotide Polymers, Current Protocols in Nucleic Acid Chemistry, 4.51.1-4.51.26, 2012". All other reagents were purchased from Sigma Aldrich.

Tables 1-3 show molecules prepared following the above procedure.

**A, G, $^{m}$C, T (in bold):** represent TNA modification
A, G, $^{m}$C, T (underline): represent MOE modification
A, G, $^{m}$C, T represent LNA nucleotides
a, g, c, $^{m}$c, t represent DNA nucleotides
**o** represents phosphodiester linkages
all other linkages were prepared as phosphorothioates.

Table 1: Synthesized molecules containing TNA(PO) moieties (targeting Metastasis-associated lung adenocarcinoma transcript 1 (Malat-1)). CMP ID NO = Compound ID number.

| CMP ID NO | Sequence | Calculated mass | Found mass |
|---|---|---|---|
| CONTROL #1 | GAGttacttgccaA$^{m}$CT | 5279.24 | 5280.9 |
| #1 | GAG**oT**tacttgccaA$^{m}$CT | 5249.15 | 5250.6 |
| #2 | GAG**oToT**acttgccaA$^{m}$CT | 5219.06 | 5219.1 |
| #3 | GAG**oToToA**cttgccaA$^{m}$CT | 5188.97 | 5189.2 |
| #4 | GAG**oToToAo$^{m}$C**ttgccaA$^{m}$CT | 5172.9 | 5173.8 |
| #5 | GAG**oToToAo$^{m}$CoT**tgccaA$^{m}$CT | 5142.81 | 5142.6 |
| #6 | **GAoG**ttacttgccaA$^{m}$CT | 5137.07 | 5136.3 |
| #7 | **GoAoG**ttacttgccaA$^{m}$CT | 5121 | 5121 |
| #8 | **GoAoGoT**tacttgccaA$^{m}$CT | 5090.91 | 5090.7 |
| #9 | **GoAoGoToT**acttgccaA$^{m}$CT | 5060.82 | 5060.7 |
| #10 | **GoAoGoToToA**cttgccaA$^{m}$CT | 5030.73 | 5030.7 |
| #11 | **GoAoGoToToAo$^{m}$C**ttgccaA$^{m}$CT | 5014.66 | 5013.9 |
| #12 | **GoAoGoToToAo$^{m}$CoT**tgccaA$^{m}$CT | 4984.57 | 4984.2 |
| #13 | **GoAoGoToToA**cttgccaa$^{m}$CT | 5002.72 | 5002.1 |
| #14 | **GoAoGoToToA**cttgccaacT | 4960.68 | 4959.9 |
| #15 | **GoAoGoToToAo$^{m}$C**tt$^{g}$ccaa$^{m}$CT | 4986.65 | 4986.6 |
| #16 | **GoAoGoToToAo$^{m}$C**ttgccaacT | 4944.61 | 4944.3 |
| #17 | **GoAoGoToToAo$^{m}$CoT**tgccaa$^{m}$CT | 4956.56 | 4956 |
| #18 | **GoAoGoToToAo$^{m}$CoT**tgccaacT | 4914.52 | 4913.7 |
| #19 | **Go**AGttacttgccaA$^{m}$C**oT** | 5163.04 | 5163 |
| #20 | **Go**AGttacttgccaA**o$^{m}$CoT** | 5104.94 | 5105.7 |
| #21 | **GoAo**GttacttgccaA**o$^{m}$CoT** | 5046.84 | 5047.2 |
| #22 | GA**oG**ttacttgcca**oAo**$^{m}$CT | 5146.97 | 5147.4 |
| #23 | GA**oGo**ttacttgcca**oAo**$^{m}$CT | 5130.91 | 5130.9 |

(continued)

| CMP ID NO | Sequence | Calculated mass | Found mass |
|---|---|---|---|
| #24 | GA**oG**ttacttgccaA**o$^{m}$C**T | 5163.04 | 5162.7 |
| #25 | GA**oGo**ttacttgccaA**o$^{m}$C**T | 5146.97 | 5146.8 |
| #26 | GA**oGo**ttacttgccaA**o$^{m}$Co**T | 5130.91 | 5130.3 |
| #27 | G**oA**GttacttgccaA**o$^{m}$CT** | 5163.04 | 5162.7 |
| #28 | G**oAo**GttacttgccaA**o$^{m}$CT** | 5146.97 | 5147.7 |
| #29 | G**oAo**GttacttgccaA$^{m}$CT | 5205.08 | 5205.6 |
| #30 | GA**oGo**ttacttgccaA$^{m}$CT | 5205.08 | 5206.2 |
| #31 | GAG**oTo**tacttgccaA$^{m}$CT | 5233.09 | 5233.2 |
| #32 | GAGt**oTo**acttgccaA$^{m}$CT | 5233.09 | 5233.8 |
| #33 | GAGtt**oAo**cttgccaA$^{m}$CT | 5233.09 | 5232.9 |
| #34 | GAGtta**o$^{m}$Co**ttgccaA$^{m}$CT | 5247.11 | 5248.2 |
| #35 | GAGttac**oTo**tgccaA$^{m}$CT | 5233.09 | 5234.4 |
| #36 | GAGttact**oTo**gccaA$^{m}$CT | 5233.09 | 5234.4 |
| #37 | GAGttactt**oGo**ccaA$^{m}$CT | 5233.09 | 5232.6 |
| #38 | GAGttacttg**o$^{m}$Co**caA$^{m}$CT | 5247.11 | 5248.2 |
| #39 | GAGttacttgc**o$^{m}$Co**aA$^{m}$CT | 5247.11 | 5247 |
| #40 | GAGttacttgcc**oAo**A$^{m}$CT | 5233.09 | 5233.5 |
| #41 | GAGttacttgcca**oAo**$^{m}$CT | 5205.08 | 5207.1 |
| #42 | GAGttacttgccaA**o$^{m}$CoT** | 5205.08 | 5205.6 |
| #43 | GAGttacttgccaA$^{m}$C**oT** | 5221.14 | 5221.8 |
| #44 | G**oA**GttacttgccaA$^{m}$CT | 5221.14 | 5220.9 |
| #45 | GA**oG**ttacttgccaA$^{m}$CT | 5221.14 | 5221.5 |
| #46 | GAG**oT**tacttgccaA$^{m}$CT | 5249.15 | 5249.7 |
| #47 | GAGt**oT**acttgccaA$^{m}$CT | 5249.15 | 5249.4 |
| #48 | GAGtt**oA**cttgccaA$^{m}$CT | 5249.15 | 5250.6 |
| #49 | GAGtta**o$^{m}$C**ttgccaA$^{m}$CT | 5263.18 | 5263.8 |
| #50 | GAGttac**oT**tgccaA$^{m}$CT | 5249.15 | 5249.7 |
| #51 | GAGttact**oT**gccaA$^{m}$CT | 5249.15 | 5251.2 |
| #52 | GAGttactt**oG**ccaA$^{m}$CT | 5249.15 | 5249.7 |
| #53 | GAGttacttg**o$^{m}$C**caA$^{m}$CT | 5263.18 | 5264.1 |
| #54 | GAGttacttgc**o$^{m}$C**aA$^{m}$CT | 5263.18 | 5264.4 |
| #55 | GAGttacttgcc**oA**A$^{m}$CT | 5249.15 | 5250.6 |
| #56 | GAGttacttgcca**oA**$^{m}$CT | 5221.14 | 5222.4 |
| #57 | G**Ao**GttacttgccaA$^{m}$CT | 5221.14 | 5221.5 |
| #58 | GA**Go**ttacttgccaA$^{m}$CT | 5221.14 | 5221.2 |

(continued)

| CMP ID NO | Sequence | Calculated mass | Found mass |
|---|---|---|---|
| #59 | GAG**To**tacttgccaA$^{m}$CT | 5249.15 | 5249.1 |
| #60 | GAGt**To**acttgccaA$^{m}$CT | 5249.15 | 5249.7 |
| #61 | GAGtt**Ao**cttgccaA$^{m}$CT | 5249.15 | 5249.4 |
| #62 | GAGtta$^{m}$**Co**ttgccaA$^{m}$CT | 5263.18 | 5263.8 |
| #63 | GAGttac**To**tgccaA$^{m}$CT | 5249.15 | 5250 |
| #64 | GAGttact**To**gccaA$^{m}$CT | 5249.15 | 5249.7 |
| #65 | GAGttactt**Go**ccaA$^{m}$CT | 5249.15 | 5250.6 |
| #66 | GAGttacttg$^{m}$**Co**caA$^{m}$CT | 5263.18 | 5264.4 |
| #67 | GAGttacttgc$^{m}$**Co**aA$^{m}$CT | 5263.18 | 5264.7 |
| #68 | GAGttacttgcc**Ao**A$^{m}$CT | 5249.15 | 5249.1 |
| #69 | GAGttacttgcca**Ao**$^{m}$CT | 5221.14 | 5220.9 |
| #70 | GAGttacttgccaA$^{m}$**Co**T | 5221.14 | 5222.7 |
| CONTROL #2 | G$^{m}$C$^{m}$CAGgctggttatgA$^{m}$CT$^{m}$CA | 7235.11 | 7235.6 |
| #71 | **G$^{m}$C$^{m}$CAoG**gctggttatgA$^{m}$CT$^{m}$CA | 6778.52 | 6780 |
| #72 | **G$^{m}$C$^{m}$CoAoG**gctggttatgA$^{m}$CT$^{m}$CA | 6762.45 | 6763.2 |
| #73 | **G$^{m}$Co$^{m}$CoAoG**gctggttatgA$^{m}$CT$^{m}$CA | 6746.39 | 6747.2 |
| #74 | **Go$^{m}$Co$^{m}$CoAoG**gctggttatgA$^{m}$CT$^{m}$CA | 6730.32 | 6730.8 |
| #75 | **Go$^{m}$Co$^{m}$CoAoGoG**ctggttatgA$^{m}$CT$^{m}$CA | 6700.23 | 6700.4 |
| #76 | **Go$^{m}$Co$^{m}$CoAoGoGo$^{m}$C**tggttatgA$^{m}$CT$^{m}$CA | 6684.17 | 6685.6 |
| #77 | **Go$^{m}$Co$^{m}$CoAoGoGo$^{m}$CoT**ggttatgA$^{m}$CT$^{m}$CA | 6654.07 | 6654.8 |
| #78 | **Go$^{m}$Co$^{m}$CoAoGoGo$^{m}$CoToG**gttatgA$^{m}$CT$^{m}$CA | 6623.98 | 6624 |
| #79 | **Go$^{m}$Co$^{m}$CoAoGoGo$^{m}$CoToG**oGttatgA$^{m}$CT$^{m}$CA | 6593.89 | 6594 |
| #80 | G$^{m}$C$^{m}$CAG**oG**ctggttatgA$^{m}$CT$^{m}$CA | 7205.02 | 7205.5 |
| #81 | G$^{m}$C$^{m}$CAG**oGo**ctggttatgA$^{m}$CT$^{m}$CA | 7188.95 | 7188.9 |
| #82 | G$^{m}$C$^{m}$CAG**oGo$^{m}$C**tggttatgA$^{m}$CT$^{m}$CA | 7188.95 | 7189.6 |
| #83 | G$^{m}$C$^{m}$CAGo**Go$^{m}$Co**tggttatgA$^{m}$CT$^{m}$CA | 7172.89 | 7173.1 |
| #84 | G$^{m}$C$^{m}$CAG**oGo$^{m}$CoT**ggttatgA$^{m}$CT$^{m}$CA | 7158.86 | 7158.6 |
| #85 | G$^{m}$C$^{m}$CAG**oGo$^{m}$CoTo**ggttatgA$^{m}$CT$^{m}$CA | 7142.8 | 7143 |
| #86 | G$^{m}$C$^{m}$CAG**oGo$^{m}$CoToG**gttatgA$^{m}$CT$^{m}$CA | 7128.77 | 7129 |
| #87 | G$^{m}$C$^{m}$CAG**oGo$^{m}$CoToGo**gttatgA$^{m}$CT$^{m}$CA | 7112.7 | 7112.5 |
| #88 | G$^{m}$C$^{m}$CAG**oGo$^{m}$CoToGoG**ttatgA$^{m}$CT$^{m}$CA | 7098.68 | 7099.1 |
| #89 | G$^{m}$C$^{m}$CAG**oGo$^{m}$CoToGoGo**ttatgA$^{m}$CT$^{m}$CA | 7082.61 | 7082.4 |
| #90 | G$^{m}$C$^{m}$CAG**oGo$^{m}$CoToGoGoT**tatgA$^{m}$CT$^{m}$CA | 7068.58 | 7068.9 |
| #91 | **Go**$^{m}$C$^{m}$CAGgctggttatgA$^{m}$CT$^{m}$C**oA** | 7026.77 | 7028.4 |
| #92 | **Go**$^{m}$C$^{m}$CAGgctggttatgA$^{m}$CT**o$^{m}$CoA** | 6922.6 | 6922 |

(continued)

| CMP ID NO | Sequence | Calculated mass | Found mass |
|---|---|---|---|
| #93 | **Go$^{m}$Co**$^{m}$CAGgctggttatgA$^{m}$CT**o$^{m}$CoA** | 6818.43 | 6819.2 |
| #94 | **Go$^{m}$Co**$^{m}$CAG**oG**ctggttatgA$^{m}$CT**o$^{m}$CoA** | 6788.34 | 6788.4 |
| #95 | **Go$^{m}$Co**$^{m}$CAG**oGo$^{m}$C**tggttatgA$^{m}$CT**o$^{m}$CoA** | 6772.27 | 6772.8 |
| #96 | **Go$^{m}$Co**$^{m}$CAG**oGo$^{m}$CoT**ggttatgA$^{m}$CT**o$^{m}$CoA** | 6742.18 | 6742.8 |
| #97 | **Go$^{m}$Co**$^{m}$CAG**oGo$^{m}$CoToG**gttatgA$^{m}$CT**o$^{m}$CoA** | 6712.09 | 6712 |
| #98 | **Go$^{m}$Co**$^{m}$CAG**oGo$^{m}$CoToGoG**ttatgA$^{m}$CT**o$^{m}$CoA** | 6681.99 | 6682.4 |
| #99 | **Go$^{m}$Co$^{m}$CoAoG**gctggttatg**Ao$^{m}$CoTo$^{m}$CoA** | 6625.53 | 6225.6 |
| #100 | **G$^{m}$Co$^{m}$CoAoG**gctggttatg**Ao$^{m}$CoTo$^{m}$CA** | 6257.67 | 6258.8 |
| #101 | G$^{m}$C$^{m}$CAGgctggttatg**Ao$^{m}$CoTo$^{m}$CoA** | 6730.32 | 6731.1 |
| #102 | G$^{m}$C$^{m}$CAGgctggttat**GoAo$^{m}$CoTo$^{m}$CoA** | 6700.23 | 6700.4 |
| #103 | G$^{m}$C$^{m}$CAGgctggtta**ToGoAo$^{m}$CoTo$^{m}$CoA** | 6670.14 | 6670.5 |
| #104 | G$^{m}$C$^{m}$CAGgctggtt**AoToGoAo$^{m}$CoTo$^{m}$CoA** | 6640.05 | 6640.7 |
| #105 | G$^{m}$C$^{m}$CAGgctggttatgA$^{m}$CT**$^{m}$CoA** | 7042.84 | 7042.4 |
| #106 | G$^{m}$C$^{m}$CAGgctggttatgA$^{m}$CT**o$^{m}$CoA** | 7026.77 | 7026 |
| #107 | **Go**$^{m}$C$^{m}$CAGgctggttatgA$^{m}$CTo$^{m}$C**oA** | 7026.77 | 7026.8 |
| #108 | G$^{m}$C**o$^{m}$Co**AGgctggttatgA$^{m}$C**oTo**$^{m}$CA | 6994.64 | 6996 |
| #109 | G$^{m}$C**o$^{m}$Co**AGgctggttatgA**o$^{m}$Co**T$^{m}$CA | 6994.64 | 6994 |
| #110 | G$^{m}$C$^{m}$CA**GoGo**ctggttat**oGo**A$^{m}$CT$^{m}$CA | 7142.8 | 7142 |
| #111 | G$^{m}$C**o$^{m}$Co**AGgctggttatgA**o$^{m}$CoTo**$^{m}$CA | 6890.47 | 6891.2 |
| #112 | G$^{m}$C**o$^{m}$Co**AoGgctggttatgA**o$^{m}$CoTo**$^{m}$CA | 6786.3 | 6786.4 |
| #113 | Go**$^{m}$Co**$^{m}$CAGgctggttatgA$^{m}$CT$^{m}$CA | 7114.87 | 7114.8 |
| #114 | G$^{m}$C**o$^{m}$Co**AGgctggttatgA$^{m}$CT$^{m}$CA | 7114.87 | 7114.4 |
| #115 | G$^{m}$C$^{m}$C**oAo**GgctggttatgA$^{m}$CT$^{m}$CA | 7114.87 | 7116.4 |
| #116 | G$^{m}$C$^{m}$CA**oGo**gctggttatgA$^{m}$CT$^{m}$CA | 7114.87 | 7114.8 |
| #117 | G$^{m}$C$^{m}$CAG**oGo**ctggttatgA$^{m}$CT$^{m}$CA | 7188.95 | 7189.6 |
| #118 | G$^{m}$C$^{m}$CAGg**o$^{m}$Co**tggttatgA$^{m}$CT$^{m}$CA | 7202.98 | 7204.4 |
| #119 | G$^{m}$C$^{m}$CAGgc**oTo**ggttatgA$^{m}$CT$^{m}$CA | 7188.95 | 7188.8 |
| #120 | G$^{m}$C$^{m}$CAGgct**oG**ogttatgA$^{m}$CT$^{m}$CA | 7188.95 | 7188.8 |
| #121 | G$^{m}$C$^{m}$CAGgctg**oGo**ttatgA$^{m}$CT$^{m}$CA | 7188.95 | 7189.2 |
| #122 | G$^{m}$C$^{m}$CAGgctgg**oTo**tatgA$^{m}$CT$^{m}$CA | 7188.95 | 7190 |
| #123 | G$^{m}$C$^{m}$CAGgctggt**oTo**atgA$^{m}$CT$^{m}$CA | 7188.95 | 7188.8 |
| #124 | G$^{m}$C$^{m}$CAGgctggtt**oAo**tgA$^{m}$CT$^{m}$CA | 7188.95 | 7189.2 |
| #125 | G$^{m}$C$^{m}$CAGgctggtta**oTo**gA$^{m}$CT$^{m}$CA | 7188.95 | 7189.2 |
| #126 | G$^{m}$C$^{m}$CAGgctggttat**oGo**A$^{m}$CT$^{m}$CA | 7188.95 | 7190 |
| #127 | G$^{m}$C$^{m}$CAGgctggttatg**oAo**$^{m}$CT$^{m}$CA | 7114.87 | 7114.8 |

(continued)

| CMP ID NO | Sequence | Calculated mass | Found mass |
|---|---|---|---|
| #128 | G$^{m}$C$^{m}$CAGgctggttatgA**o**$^{m}$**Co**T$^{m}$CA | 7114.87 | 7114.8 |
| #129 | G$^{m}$C$^{m}$CAGgctggttatgA$^{m}$C**oTo**$^{m}$CA | 7114.87 | 7116.4 |
| #130 | G$^{m}$C$^{m}$CAGgctggttatgA$^{m}$CT**o**$^{m}$**Co**A | 7114.87 | 7115.6 |
| #131 | G$^{m}$C$^{m}$CAGgctggttatgA$^{m}$CT$^{m}$C**oA** | 7130.94 | 7131.2 |
| #132 | **Go**$^{m}$C$^{m}$CAGgctggttatgA$^{m}$CT$^{m}$CA | 7130.94 | 7130.4 |
| #133 | G**o**$^{m}$**C**$^{m}$CAGgctggttatgA$^{m}$CT$^{m}$CA | 7130.94 | 7131.6 |
| #134 | G$^{m}$C**o**$^{m}$**C**AGgctggttatgA$^{m}$CT$^{m}$CA | 7130.94 | 7131.2 |
| #135 | G$^{m}$C$^{m}$C**oA**GgctggttatgA$^{m}$CT$^{m}$CA | 7130.94 | 7131.2 |
| #136 | G$^{m}$C$^{m}$CA**oGg**ctggttatgA$^{m}$CT$^{m}$CA | 7130.94 | 7132 |
| #137 | G$^{m}$C$^{m}$CAG**oG**ctggttatgA$^{m}$CT$^{m}$CA | 7205.02 | 7205.2 |
| #138 | G$^{m}$C$^{m}$CAGg**o**$^{m}$**C**tggttatgA$^{m}$CT$^{m}$CA | 7219.05 | 7219.2 |
| #139 | G$^{m}$C$^{m}$CAGgc**oT**ggttatgA$^{m}$CT$^{m}$CA | 7205.02 | 7204.8 |
| #140 | G$^{m}$C$^{m}$CAGgct**oG**gttatgA$^{m}$CT$^{m}$CA | 7205.02 | 7204.4 |
| #141 | G$^{m}$C$^{m}$CAGgctg**oG**ttatgA$^{m}$CT$^{m}$CA | 7205.02 | 7206 |
| #142 | G$^{m}$C$^{m}$CAGgctgg**oT**tatgA$^{m}$CT$^{m}$CA | 7205.02 | 7204.8 |
| #143 | G$^{m}$C$^{m}$CAGgctggt**oT**atgA$^{m}$CT$^{m}$CA | 7205.02 | 7204 |
| #144 | G$^{m}$C$^{m}$CAGgctggtt**oA**tgA$^{m}$CT$^{m}$CA | 7205.02 | 7205.2 |
| #145 | G$^{m}$C$^{m}$CAGgctggtta**oT**gA$^{m}$CT$^{m}$CA | 7205.02 | 7205.2 |
| #146 | G$^{m}$C$^{m}$CAGgctggttat**oG**A$^{m}$CT$^{m}$CA | 7205.02 | 7206.4 |
| #147 | G$^{m}$C$^{m}$CAGgctggttatg**oA**$^{m}$CT$^{m}$CA | 7130.94 | 7132 |
| #148 | G$^{m}$C$^{m}$CAGgctggttatgA**o**$^{m}$**C**T$^{m}$CA | 7130.94 | 7132 |
| #149 | G$^{m}$C$^{m}$CAGgctggttatgA$^{m}$C**oT**$^{m}$CA | 7130.94 | 7131.2 |
| #150 | G$^{m}$C$^{m}$CAGgctggttatgA$^{m}$CT**o**$^{m}$**C**A | 7130.94 | 7131.2 |
| CONTROL #3 | GG$^{m}$CATatgcagataaTGTT$^{m}$C | 7230.09 | 7230 |
| #151 | GG$^{m}$CAT**oA**tgcagataaTGTT$^{m}$C | 7200 | 7200.8 |
| #152 | GG$^{m}$CAT**oAo**TgcagataaTGTT$^{m}$C | 7169.91 | 7170.2 |
| #153 | GG$^{m}$CAT**oAoToG**cagataaTGTT$^{m}$C | 7139.82 | 7140.5 |
| #154 | GG$^{m}$CAT**oAoToGo**$^{m}$**C**agataaTGTT$^{m}$C | 7123.75 | 7123.9 |
| #155 | GG$^{m}$CAT**oAoToGo**$^{m}$**CoA**gataaTGTT$^{m}$C | 7093.66 | 7093.5 |
| #156 | **GG**$^{m}$**CAoT**atgcagataaTGTT$^{m}$C | 6773.5 | 6774.4 |
| #157 | **GG**$^{m}$**CoAoT**atgcagataaTGTT$^{m}$C | 6757.44 | 6760.4 |
| #158 | **GGo**$^{m}$**CoAoT**atgcagataaTGTT$^{m}$C | 6741.37 | 6744 |
| #159 | **GoGo**$^{m}$**CoAoT**atgcagataaTGTT$^{m}$C | 6725.31 | 6726.2 |
| #160 | **GoGo**$^{m}$**CoAoToA**tgcagataaTGTT$^{m}$C | 6695.21 | 6696.4 |
| #161 | **GoGo**$^{m}$**CoAoToAoT**gcagataaTGTT$^{m}$C | 6665.12 | 6664.4 |

(continued)

| CMP ID NO | Sequence | Calculated mass | Found mass |
|---|---|---|---|
| #162 | **GoGo**$^{m}$**CoAoToAoToG**cagataaTGTT$^{m}$C | 6635.03 | 6634.4 |
| #163 | **GoGo**$^{m}$**CoAoToAoToGo**$^{m}$**C**agataaTGTT$^{m}$C | 6618.96 | 6618.4 |
| #164 | **GoGo**$^{m}$**CoAoToAoToGo**$^{m}$**CoA**gataaTGTT$^{m}$C | 6588.87 | 6590.4 |
| #165 | **GoGo**$^{m}$**CoAoToAoToGo**$^{m}$**CoA**gataatGTT$^{m}$C | 6514.79 | 6514.4 |
| #166 | **GoGo**$^{m}$**CoAoToAoToGo**$^{m}$**CoA**gataatgTT$^{m}$c | 6440.72 | 6440 |
| #167 | **GoGo**$^{m}$**CoAoToAoToGo**$^{m}$**CoAoG**ataatGTT$^{m}$C | 6484.7 | 6484 |
| #168 | **GoGo**$^{m}$**CoAoToAoToGo**$^{m}$**CoAoG**ataatgTT$^{m}$C | 6410.62 | 6411.2 |
| #169 | **Go**G$^{m}$CATatgcagataaTGTT**o**$^{m}$**C** | 7021.75 | 7023.1 |
| #170 | **Go**G$^{m}$CATatgcagataaTGT**oTo**$^{m}$**C** | 6917.58 | 6917.2 |
| #171 | **GoGo**$^{m}$CATatgcagataaTGT**oTo**$^{m}$**C** | 6813.41 | 6812.8 |
| #172 | **GoGo**$^{m}$CAT**oA**tgcagataaTGT**oTo**$^{m}$**C** | 6783.32 | 6782.8 |
| #173 | **GoGo**$^{m}$CAT**oAoT**gcagataaTGT**oTo**$^{m}$**C** | 6753.23 | 6752.3 |
| #174 | **GoGo**$^{m}$CAT**oAoToG**cagataaTGT**oTo**$^{m}$**C** | 6723.13 | 6723.3 |
| #175 | **GoGo**$^{m}$CAT**oAoToGo**$^{m}$**C**agataaTGT**oTo**$^{m}$**C** | 6707.07 | 6707.6 |
| #176 | **GoGo**$^{m}$CAT**oAoToGo**$^{m}$**CoA**gataaTGT**oTo**$^{m}$**C** | 6676.98 | 6676.4 |
| #177 | **GoGo**$^{m}$**CoAoT**atgcagataa**ToGoToTo**$^{m}$**C** | 6220.53 | 6220.4 |
| #178 | **GGo**$^{m}$**CoAoT**atgcagataa**ToGoToT**$^{m}$**C** | 6252.65 | 6253.2 |
| #179 | GG$^{m}$CATatgcagataa**ToGoToTo**$^{m}$**C** | 6725.31 | 6725.6 |
| #180 | GG$^{m}$CATatgcagata**AoToGoToTo**$^{m}$**C** | 6695.21 | 6694.9 |
| #181 | GG$^{m}$CATatgcagat**AoAoToGoToTo**$^{m}$**C** | 6665.12 | 6665.6 |
| #182 | GG$^{m}$CATatgcaga**ToAoAoToGoToTo**$^{m}$**C** | 6635.03 | 6635.6 |
| #183 | GG$^{m}$CATatgcagataaTGT**To**$^{m}$**C** | 7037.82 | 7037.6 |
| #184 | GG$^{m}$CATatgcagataaTGT**oTo**$^{m}$**C** | 7021.75 | 7021.6 |
| #185 | GG**o**$^{m}$**C**ATatgcagataaTG**oT**T$^{m}$C | 7021.75 | 7023.6 |
| #186 | GG**o**$^{m}$**Co**ATatacaaataaTG**oTo**T$^{m}$C | 6989.62 | 6990.4 |
| #187 | GG**o**$^{m}$**Co**ATatgcagataaT**GoTo**T$^{m}$C | 6901.56 | 6902.4 |
| #188 | GG**o**$^{m}$**Co**ATatgcagataaT**oGoTo**T$^{m}$C | 6885.45 | 6885.6 |
| #189 | GG**o**$^{m}$**CoAo**TatgcagataaT**oGoTo**T$^{m}$C | 6781.28 | 6780.4 |
| #190 | GG$^{m}$CAT**oA**tgcagata**oA**TGTT$^{m}$C | 7169.91 | 7171.2 |
| #191 | GG$^{m}$CAT**oAo**tgcagata**oAo**TGTT$^{m}$C | 7137.78 | 7137.6 |
| CONTROL #4 | GGGAGttacttgccaA$^{m}$CTTG | 7208.04 | 7208.1 |
| #192 | GGGAG**oT**tacttgccaA$^{m}$CTTG | 7177.95 | 7178.8 |
| #193 | GGGAG**oToT**acttgccaA$^{m}$CTTG | 7147.86 | 7148.3 |
| #194 | GGGAG**oToToA**cttgccaA$^{m}$CTTG | 7117.77 | 7118 |
| #195 | GGGAG**oToToAo**$^{m}$**C**ttgccaA$^{m}$CTTG | 7101.7 | 7102 |

(continued)

| CMP ID NO | Sequence | Calculated mass | Found mass |
|---|---|---|---|
| #196 | GGGAG**oToToAo$^{m}$CoT**tgccaA$^{m}$CTTG | 7071.61 | 7071.4 |
| #197 | GGGAG**oToToAo$^{m}$CoToT**gccaA$^{m}$CTTG | 7041.52 | 7041.5 |
| #198 | **GoGo**GAGttacttgccaA$^{m}$CT**oToG** | 6791.36 | 6790.8 |
| #199 | **GoGo**GAG**oT**tacttgccaA$^{m}$CT**oToG** | 6761.27 | 6760.8 |
| #200 | **GoGo**GAG**oToT**acttgccaA$^{m}$CT**oToG** | 6731.18 | 6730.4 |
| #201 | **GoGo**GAG**oToToA**cttgccaA$^{m}$CT**oToG** | 6701.08 | 6701.2 |
| #202 | **GoGo**GAG**oToToAo$^{m}$C**ttgccaA$^{m}$CT**oToG** | 6685.02 | 6685.2 |
| #203 | **GoGo**GAG**oToToAo$^{m}$CoT**tgccaA$^{m}$CT**oToG** | 6654.93 | 6655.6 |
| #204 | **GoGoGoAoG**ttacttgcca**Ao$^{m}$CoToToG** | 6198.47 | 6198 |
| #205 | **GGoGoAoG**ttacttgcca**Ao$^{m}$CoToTG** | 6230.6 | 6229.6 |
| #206 | **GGGAoG**ttacttgccaA$^{m}$CTTG | 6751.45 | 6751.7 |
| #207 | **GGGoAoG**ttacttgccaA$^{m}$CTTG | 6735.39 | 6735.7 |
| #208 | **GGoGoAoG**ttacttgccaA$^{m}$CTTG | 6719.32 | 6719.5 |
| #209 | **GoGoGoAoG**ttacttgccaA$^{m}$CTTG | 6703.25 | 6703.6 |
| #210 | **GoGoGoAoGoT**tacttgccaA$^{m}$CTTG | 6673.16 | 6673 |
| #211 | **GoGoGoAoGoToT**acttgccaA$^{m}$CTTG | 6643.07 | 6642.4 |
| #212 | **GoGoGoAoGoToToA**cttgccaA$^{m}$CTTG | 6612.98 | 6612.8 |
| #213 | **GoGoGoAoGoToToAo$^{m}$C**ttgccaA$^{m}$CTTG | 6596.91 | 6596.1 |
| #214 | **GoGoGoAoGoToToAo$^{m}$CoT**ttgccaA$^{m}$CTTG | 6566.82 | 6566.9 |
| #215 | **GoGoGoAoGoToToAo$^{m}$CoT**ttgccaa$^{m}$CTTG | 6492.74 | 6492.4 |
| #216 | **GoGoGoAoGoToToAo$^{m}$CoT**ttgccaacTTG | 6404.64 | 6403.6 |
| #217 | GGGAGttacttgcca**Ao$^{m}$CoToToG** | 6703.25 | 6704.7 |
| #218 | GGGAGttattgc**$^{m}$CoAoAo$^{m}$CoToToG** | 6657.1 | 6658.1 |
| CONTROL #5 | TG$^{m}$C$^{m}$CTttaggattctAGA$^{m}$CA | 7195.08 | 7195.4 |
| #219 | TG$^{m}$C$^{m}$CT**oT**taggattctAGA$^{m}$CA | 7164.99 | 7165.7 |
| #220 | TG$^{m}$C$^{m}$CT**oToT**aggattctAGA$^{m}$CA | 7134.9 | 7135.5 |
| #221 | TG$^{m}$C$^{m}$CT**oToToA**ggattctAGA$^{m}$CA | 7104.81 | 7105.4 |
| #222 | TG$^{m}$C$^{m}$CT**oToToAoG**gattctAGA$^{m}$CA | 7074.71 | 7075.7 |
| #223 | TG$^{m}$C$^{m}$CT**oToToAoGoG**attctAGA$^{m}$CA | 7044.62 | 7045.7 |
| #224 | TG$^{m}$C$^{m}$CT**oToToAoGoGoA**ttctAGA$^{m}$CA | 7014.53 | 7015.1 |
| #225 | **ToGo$^{m}$Co$^{m}$CoT**ttaggattct**AoGoAo$^{m}$CoA** | 6185.51 | 6184.4 |
| #226 | **TGo$^{m}$Co$^{m}$CoT**ttaggattct**AoGoAo$^{m}$CA** | 6217.64 | 6217.6 |
| #227 | **TG$^{m}$C$^{m}$CoT**ttaggattctAGA$^{m}$CA | 6738.49 | 6738.3 |
| #228 | **TG$^{m}$Co$^{m}$CoT**ttaggattctAGA$^{m}$CA | 6722.43 | 6722.6 |
| #229 | **TGo$^{m}$Co$^{m}$CoT**ttaggattctAGA$^{m}$CA | 6706.36 | 6705.7 |

(continued)

| CMP ID NO | Sequence | Calculated mass | Found mass |
|---|---|---|---|
| #230 | **ToGo$^{m}$Co$^{m}$CoT**ttaggattctAGA$^{m}$CA | 6690.3 | 6690.5 |
| #231 | **ToGo$^{m}$Co$^{m}$CoToT**taggattctAGA$^{m}$CA | 6660.2 | 6660 |
| #232 | **ToGo$^{m}$Co$^{m}$CoToToT**aggattctAGA$^{m}$CA | 6630.11 | 6630 |
| #233 | **ToGo$^{m}$Co$^{m}$CoToToToA**aaattctAGA$^{m}$CA | 6600.02 | 6599.8 |
| #234 | **ToGo$^{m}$Co$^{m}$CoToToToAoG**gattctAGA$^{m}$CA | 6569.93 | 6569 |
| #235 | **ToGo$^{m}$Co$^{m}$CoToToToAoGoG**attctAGA$^{m}$CA | 6539.83 | 6539.1 |
| #236 | **ToGo$^{m}$Co$^{m}$CoToToToAoGoG**attctaGA$^{m}$CA | 6465.76 | 6466.4 |
| #237 | **ToGo$^{m}$Co$^{m}$CoToToToAoGoG**attctagA$^{m}$CA | 6391.69 | 6392.4 |
| #238 | TG$^{m}$C$^{m}$CTttaggattct**AoGoAo$^{m}$CoA** | 6690.3 | 6690.5 |
| #239 | TG$^{m}$C$^{m}$CTttaggattc**ToAoGoAo$^{m}$CoA** | 6660.2 | 6660.7 |
| #240 | TG$^{m}$C$^{m}$CTttaggatt**$^{m}$CoToAoGoAo$^{m}$CoA** | 6644.14 | 6642.8 |
| CONTROL #6 | $^{m}$C$^{m}$CAGGctggttatga$^{m}$CT$^{m}$CAG | 7235.11 | 7234 |
| #241 | **$^{m}$C$^{m}$CAGoG**ctggttatga$^{m}$CT$^{m}$CAG | 6778.52 | 6778 |
| #242 | **$^{m}$C$^{m}$CAoGoG**ctggttatga$^{m}$CT$^{m}$CAG | 6762.45 | 6762 |
| #243 | **$^{m}$C$^{m}$CoAoGoG**ctggttatga$^{m}$CT$^{m}$CAG | 6746.39 | 6746.4 |
| #244 | **$^{m}$Co$^{m}$CoAoGoG**ctggttatga$^{m}$CT$^{m}$CAG | 6730.32 | 6729.2 |
| #245 | **$^{m}$Co$^{m}$CoAoGoGo$^{m}$C**tggttatga$^{m}$CT$^{m}$CAG | 6714.26 | 6714 |
| #246 | **$^{m}$Co$^{m}$CoAoGoGo$^{m}$CoT**ggttatga$^{m}$CT$^{m}$CAG | 6684.17 | 6683.2 |
| #247 | **$^{m}$Co$^{m}$CoAoGoGo$^{m}$CoToG**gttatga$^{m}$CT$^{m}$CAG | 6654.04 | 6654 |
| #248 | **$^{m}$Co$^{m}$CoAoGoGo$^{m}$CoToGoG**ttatga$^{m}$CT$^{m}$CAG | 6623.98 | 6624 |
| CONTROL #7 | TTAT$^{m}$CaattcaccaaGGAG$^{m}$C | 7159.06 | 7158 |
| #249 | **TTATo$^{m}$C**aattcaccaaGGAG$^{m}$C | 6702.47 | 6702.4 |
| #250 | **TTAoTo$^{m}$C**aattcaccaaGGAG$^{m}$C | 6686.4 | 6685.6 |
| #251 | **TToAoTo$^{m}$C**aattcaccaaGGAG$^{m}$C | 6670.34 | 6672.4 |
| #252 | **ToToAoTo$^{m}$C**aattcaccaaGGAG$^{m}$C | 6654.27 | 6654 |
| #253 | **ToToAoTo$^{m}$CoA**attcaccaaGGAG$^{m}$C | 6624.18 | 6624 |
| #254 | **ToToAoTo$^{m}$CoAoA**ttcaccaaGGAG$^{m}$C | 6594.09 | 6593.2 |
| #255 | **ToToAoTo$^{m}$CoAoAoT**tcaccaaGGAG$^{m}$C | 6564 | 6563.6 |
| #256 | **ToToAoTo$^{m}$CoAoAoToT**caccaaGGAG$^{m}$C | 6533.9 | 6533.2 |
| #257 | **ToToAoTo$^{m}$CoAoAoToTo$^{m}$C**accaaGGAG$^{m}$C | 6517.84 | 6518 |
| CONTROL #8 | ATGGAqqtatgacatATAAT | 7250.09 | 7250.4 |
| #258 | **ATGGoA**ggtatgacatATAAT | 6793.5 | 6792.4 |
| #259 | **ATGoGoA**ggtatgacatATAAT | 6777.43 | 6776.4 |
| #260 | **AToGoGoA**ggtatgacatATAAT | 6761.37 | 6761.2 |
| #261 | **AoToGoGoA**ggtatgacatATAAT | 6745.3 | 6744.4 |
| #262 | **AoToGoGoAoG**gtatgacatATAAT | 6715.21 | 6713.6 |

(continued)

| CMP ID NO | Sequence | Calculated mass | Found mass |
|---|---|---|---|
| #263 | **AoToGoGoAoGoG**tatgacatATAAT | 6685.12 | 6684.8 |
| #264 | **AoToGoGoAoGoGoT**atgacatATAAT | 6655.03 | 6654 |
| #265 | **AoToGoGoAoGoGoToA**tgacatATAAT | 6624.93 | 6624 |
| #266 | **AoToGoGoAoGoGoToAoT**gacatATAAT | 6594.84 | 6594.4 |

We have designed two libraries of antisense oligonucleotides directed against human MALAT1 (Gencode: ENSG00000251562). Compounds were 20-nucleotide long, with two different sugar patterns: Design A: TTTTTd-ddddddddTTTTT, where T is TNA and d is DNA, and with the backbone pattern SOOOSSSSSSSSSSSOOOS, where S corresponds to phosphorothioate, and O to phosphodiester. Design B: MMMMMTTTTTddddMMMMM, where M is MOE, T is TNA and d is DNA, and with the backbone pattern SSSSOOOOOOSSSSSSSS, where S corresponds to phosphorothioate, and O to phosphodiester. The compounds were tested at two different concentrations (5 & 25μM) and the 18 most potent oligonucleotides for Design A (#267-284) and the 19 most potent oligonucleotides for Design B (#285-303) were selected for full dose response determinations.

Table 2: Selected compounds from the two libraries synthesized containing TNA PO (targeting Malat-1)

| CMP ID NO | Sequence | Calculated mass | Found mass |
|---|---|---|---|
| #267 | **GToToAoT**gcttattccc**$^{m}$CoAoAoTG** | 6140.55 | 6140 |
| #268 | **$^{m}$CTo$^{m}$CoAoG**cctttatcac**ToCoAoG$^{m}$C** | 6112.58 | 6112 |
| #269 | **AAo$^{m}$CoAoA**atttccttag**ToToGoG$^{m}$C** | 6187.61 | 6186.9 |
| #270 | **TAo$^{m}$CoAo$^{m}$C**cagtcctttt**AoGoToAG** | 6163.59 | 6163 |
| #271 | **AToGo$^{m}$CoT**catcacttta**ToGoAoAG** | 6173.59 | 6173 |
| #272 | **AAoAoAoG**gcttag$^{m}$cgcc**$^{m}$CoAo$^{m}$Co$^{m}$CT** | 6195.64 | 6195.2 |
| #273 | **AAoGoAoG**aaccacacac**ToAo$^{m}$Co$^{m}$CA** | 6153.62 | 6153.2 |
| #274 | **$^{m}$CAoTo$^{m}$CoT**caacctc$^{m}$cgt**$^{m}$CoAoToGT** | 6112.58 | 6112 |
| #275 | **AAoGoGoT**ctcatacact**$^{m}$CoAo$^{m}$CoTA** | 6141.59 | 6141 |
| #276 | **GToAo$^{m}$CoT**atcccatcac**ToGoAoAG** | 6143.56 | 6142.9 |
| #277 | **GAo$^{m}$Co$^{m}$Co$^{m}$C**ctgactttct**GoGoAoAA** | 6187.62 | 6187.1 |
| #278 | **$^{m}$CAoGo$^{m}$CoG**gtacactcct**To$^{m}$CoToCT** | 6128.58 | 6128 |
| #279 | **G$^{m}$Co$^{m}$CoAoA**tatttgcccc**To$^{m}$Co$^{m}$Co$^{m}$C$^{m}$C** | 6101.6 | 6101.1 |
| #280 | **TGo$^{m}$CoAoT**ttacttgcca**Ao$^{m}$CoAoGA** | 6172.6 | 6172.1 |
| #281 | **AAoAoGoA**gtaactacca**Go$^{m}$Co$^{m}$CoAT** | 6199.64 | 6199.1 |
| #282 | **AAo$^{m}$CoAoG**gtcatctatt**$^{m}$CoAo$^{m}$CoAA** | 6179.64 | 6179.1 |
| #283 | **TGoToAo$^{m}$C**attttgccct**ToAoGo$^{m}$CT** | 6145.56 | 6145.1 |
| #284 | **G$^{m}$Co$^{m}$CoAoA**gcactcatat**Go$^{m}$CoAoAT** | 6180.63 | 6180.1 |
| #285 | GTTAT**oGo$^{m}$CoToToAoT**tccc$^{m}$CAATG | 6951.47 | 6950.8 |
| #286 | GTGTA**oAoToToAo$^{m}$Co$^{m}$C**ttttA$^{m}$CT$^{m}$CT | 6969.52 | 6968.9 |
| #287 | $^{m}$C$^{m}$CTT$^{m}$C**oAoGoAoGoAoT**tcaaTG$^{m}$CTA | 6997.55 | 6996.8 |
| #288 | G$^{m}$C$^{m}$CAA**oToAoToToToG**ccccT$^{m}$C$^{m}$C$^{m}$C$^{m}$C | 6898.49 | 6897.8 |
| #289 | TG$^{m}$CTA**oAoAo$^{m}$CoAoAoA**tttc$^{m}$CTTAG | 6982.53 | 6981.9 |

(continued)

| CMP ID NO | Sequence | Calculated mass | Found mass |
|---|---|---|---|
| #290 | AA$^m$CAA**oAoToToTo$^m$Co$^m$C**ttagTTGG$^m$C | 7012.56 | 7011.8 |
| #291 | T$^m$CAGT**oGo$^m$CoToAoToT**ttat$^m$C$^m$CAAT | 6978.53 | 6977.8 |
| #292 | TGTA$^m$C**oAoToToToToG**ccctTAG$^m$CT | 6942.45 | 6941.8 |
| #293 | TTG$^m$CT**oGoAoAoAoToT**gtct$^m$CAATT | 6990.5 | 6989.9 |
| #294 | TTGAA**oGo$^m$CoAoToAo$^m$C**cttaA$^m$CAT$^m$C | 6981.55 | 6980.9 |
| #295 | $^m$CT$^m$C$^m$CA**oAoToAo$^m$CoToT**gtctTAG$^m$CT | 6953.52 | 6952.8 |
| #296 | G$^m$C$^m$CTT**oAoAoAoGoToT**acatT$^m$CGTT | 6989.52 | 6988.8 |
| #297 | AAGGT**o$^m$CoTo$^m$CoAoToA**cact$^m$CA$^m$CTA | 6966.54 | 6965.9 |
| #298 | GTA$^m$CT**oAoTo$^m$Co$^m$Co$^m$CoA**tcacTGAAG | 6982.53 | 6981.8 |
| #299 | ATG$^m$CA**oAoGoToToAoA**acttAT$^m$CTG | 7008.53 | 7007.8 |
| #300 | TG$^m$C$^m$CT**o$^m$CoToTo$^m$CoAoT**tgtaTTT$^m$CT | 6950.51 | 6949.8 |
| #301 | $^m$CAT$^m$CA**oAoGoGo$^m$CoAo$^m$C**tgat$^m$CA$^m$CTT | 7010.59 | 7009.9 |
| #302 | G$^m$C$^m$CAA**oGo$^m$CoAo$^m$CoTo$^m$C**atatG$^m$CAAT | 7019.6 | 7018.8 |
| #303 | GGGAG**oToToAo$^m$CoToT**gccaA$^m$CTTG | 7041.52 | 7040.8 |

Table 3: TNA(PO) vs DNA(PO)

| CMP ID NO | Sequence | Calculated mass | Found mass |
|---|---|---|---|
| CONTROL #1 | GAGttacttgccaA$^m$CT | 5279.24 | 5280.9 |
| #1 | GAG**oT**tacttgccaA$^m$CT | 5249.15 | 5250.6 |
| #2 | GAG**oToT**acttgccaA$^m$CT | 5219.06 | 5219.1 |
| #3 | GAG**oToToA**cttgccaA$^m$CT | 5188.97 | 5189.2 |
| #4 | GAG**oToToAo$^m$C**ttgccaA$^m$CT | 5172.9 | 5173.8 |
| #5 | GAG**oToToAo$^m$CoT**tgccaA$^m$CT | 5142.81 | 5142.6 |
| #304 | GAG**o**ttacttgccaA$^m$CT | 5263.18 | 5263.9 |
| #305 | GAG**o**t**o**tacttgccaA$^m$CT | 5247.11 | 5247.9 |
| #306 | GAG**o**t**o**t**o**acttgccaA$^m$CT | 5231.05 | 5231.1 |
| #307 | GAG**o**t**o**t**o**a**o**cttgccaA$^m$CT | 5214.98 | 5215.5 |
| #308 | GAG**o**t**o**t**o**a**o**c**o**ttgccaA$^m$CT | 5198.92 | 5199.3 |

Further details on the molecules in Tables 1-3 are set out in Table 4, in which the structure of each synthesized molecule is defined by the hierarchical editing language for macromolecules (HELM) (for details, see Zhang et al., Chem. Inf. Model. 2012;52(10):2796-2806). In addition, the SEQ ID NO of the nucleobase sequence upon which each respective synthesized molecule is based is indicated. The following HELM annotation keys are used:

[LR](G) is a beta-D-oxy-LNA guanine nucleoside,
[LR](T) is a beta-D-oxy-LNA thymine nucleoside,
[LR](A) is a beta-D-oxy-LNA adenine nucleoside,
[LR]([5meC] is a beta-D-oxy-LNA 5-methyl cytosine nucleoside,
[dR](G) is a DNA guanine nucleoside,

[dR](T) is a DNA thymine nucleoside,
[dR](A) is a DNA adenine nucleoside,
[dR](C) is a DNA cytosine nucleoside,
[dR]([5meC]) is a DNA 5-methyl cytosine nucleoside,
[MOE]([5meC]) is a 2'O-MOE [2'O-(2-methoxyethyl)] 5-methyl cytidine nucleoside
[MOE](A) is a 2'O-MOE [2'O-(2-methoxyethyl)] adenine nucleoside
[MOE](T) is a 2'-O-MOE [2'O-(2-methoxyethyl)] thymine nucleoside
[MOE](G) is a 2'-O-MOE [2'O-(2-methoxyethyl)] guanine nucleoside
[TNA]([5meC]) is a α-L-threose nucleic acid 5-methyl cytidine nucleoside
[TNA](A) is a α-L-threose nucleic acid adenine nucleoside
[TNA](T) is a α-L-threose nucleic acid thymine nucleoside
[TNA](G) is a α-L-threose nucleic acid guanine nucleoside
[sP] is a phosphorothioate internucleoside linkage
P is a phosphodiester internucleoside linkage

Further information as well as open-source tools for HELM can be found at the internet addresses www.pistoiaalliance.org/helm-tools/ (accessed on 26 August 2022).

Table 4: Synthesized molecules in HELM annotations

| CMP ID NO | SEQ ID NO | Base sequence 5'-3' | HELM |
|---|---|---|---|
| CONTROL #1 | 1 | GAGTTACTTGCCAACT | [LR](G)[sP].[LR](A)[sP].[LR](G)[sP].[dR](T)[sP].[ dR](T)[sP].[dR](A)[sP].[dR](C)[sP].[dR](T)[sP].[ dR](T)[sP].[dR](G)[sP].[dR](C)[sP].[dR](C)[sP].[ dR](A)[sP].[LR](A)[sP].[LR]([5meC])[sP].[LR](T) |
| #1 | 1 | GAGTTACTTGCCAACT | [LR](G)[sP].[LR](A)[sP].[LR](G)P.[TNA](T)[sP].[d R] (T) [sP].[d R] (A) [sP]. [dR] (C) [sP]. [d R] (T) [sP]. [d R](T)[sP].[dR](G)[sP].[dR](C)[sP].[dR](C)[sP].[d R](A)[sP].[LR](A)[sP].[LR]([5meC])[sP].[LR](T) |
| #2 | 1 | GAGTTACTTGCCAACT | [LR](G)[sP].[LR](A)[sP].[LR](G)P.[TNA](T)P.[TNA ](T)[sP].[dR](A)[sP].[dR](C)[sP].[dR](T)[sP].[dR] (T)[sP].[dR](G)[sP].[dR](C)[sP].[dR](C)[sP].[dR] (A)[sP].[LR](A)[sP].[LR]([5meC])[sP].[LR](T) |
| #3 | 1 | GAGTTACTTGCCAACT | [LR](G)[sP].[LR](A)[sP].[LR](G)P.[TNA](T)P.[TNA ](T)P.[TNA](A)[sP].[dR](C)[sP].[dR](T)[sP].[dR]( T)[sP].[dR](G)[sP].[dR](C)[sP].[dR](C)[sP].[dR]( A)[sP].[LR](A)[sP].[LR]([5meC])[sP].[LR](T) |
| #4 | 1 | GAGTTACTTGCCAACT | [LR](G)[sP].[LR](A)[sP].[LR](G)P.[TNA](T)P.[TNA ](T)P.[TNA](A)P.[TNA]([5meC])[sP].[dR](T)[sP].[ dR](T)[sP].[dR](G)[sP].[dR](C)[sP].[dR](C)[sP].[ dR](A)[sP].[LR](A)[sP].[LR]([5meC])[sP].[LR](T) |
| #5 | 1 | GAGTTACTTGCCAACT | [LR](G)[sP].[LR](A)[sP].[LR](G)P.[TNA](T)P.[TNA ](T)P.[TNA](A)P.[TNA]([5meC])P.[TNA](T)[sP].[d R](T)[sP].[dR](G)[sP].[dR](C)[sP].[dR](C)[sP].[d R](A)[sP].[LR](A)[sP].[LR]([5meC])[sP].[LR](T) |
| #6 | 1 | GAGTTACTTGCCAACT | [TNA](G)[sP].[TNA](A)P.[TNA](G)[sP].[dR](T)[sP ].[dR](T)[sP].[dR](A)[sP].[dR](C)[sP].[dR](T)[sP] .[dR](T)[sP].[dR](G)[sP].[dR](C)[sP].[dR](C)[sP]. [dR](A)[sP].[LR](A)[sP].[LR]([5meC])[sP].[LR](T) |

(continued)

| CMP ID NO | SEQ ID NO | Base sequence 5'-3' | HELM |
|---|---|---|---|
| #7 | 1 | GAGTTACTTGCCAACT | [TNA](G)P.[TNA](A)P.[TNA](G)[sP].[dR](T)[sP].[ dR](T)[sP].[dR](A)[sP].[dR](C)[sP].[dR](T)[sP].[ dR](T)[sP].[dR](G)[sP].[dR](C)[sP].[dR](C)[sP].[ dR](A)[sP].[LR](A)[sP].[LR]( [5meC])[sP].[LR](T) |
| #8 | 1 | GAGTTACTTGCCAACT | [TNA](G)P.[TNA](A)P.[TNA](G)P.[TNA](T)[sP].[d R](T)[sP].[dR](A)[sP].[dR](C)[sP].[dR](T)[sP].[d R](T)[sP].[dR](G)[sP].[dR](C)[sP].[dR](C)[sP].[d R] (A) [sP]. [LR] (A) [sP]. [LR] ([5meC]) [sP]. [LR] (T) |
| #9 | 1 | GAGTTACTTGCCAACT | [TNA](G)P.[TNA](A)P.[TNA](G)P.[TNA](T)P.[TNA] (T)[sP].[dR](A)[sP].[dR](C)[sP].[dR](T)[sP].[dR]( T)[sP].[dR](G)[sP].[dR](C)[sP].[dR](C)[sP].[dR]( A)[sP].[LR](A)[sP].[LR]( [5meC])[sP].[LR](T) |
| #10 | 1 | GAGTTACTTGCCAACT | [TNA](G)P.[TNA](A)P.[TNA](G)P.[TNA](T)P.[TNA] (T)P.[TNA](A)[sP].[dR](C)[sP].[dR](T)[sP].[dR](T )[sP].[dR](G)[sP].[dR](C)[sP].[dR](C)[sP].[dR](A )[sP].[LR](A)[sP].[LR]( [5meC])[sP].[LR](T) |
| #11 | 1 | GAGTTACTTGCCAACT | [TNA](G)P.[TNA](A)P.[TNA](G)P.[TNA](T)P.[TNA] (T)P.[TNA](A)P.[TNA]([5meC])[sP].[dR](T)[sP].[d R] (T) [sP]. [d R] (G) [sP]. [dR] (C) [sP]. [dR] (C) [sP]. [d R] (A) [sP]. [LR] (A) [sP]. [LR] ([5meC]) [sP]. [LR] (T) |
| #12 | 1 | GAGTTACTTGCCAACT | [TNA](G)P.[TNA](A)P.[TNA](G)P.[TNA](T)P.[TNA] (T)P.[TNA](A)P.[TNA]([5meC])P.[TNA](T)[sP].[dR ](T)[sP].[dR](G)[sP].[dR](C)[sP].[dR](C)[sP].[dR ](A)[sP].[LR](A)[sP].[LR]([5meC])[sP].[LR](T) |
| #13 | 1 | GAGTTACTTGCCAACT | [TNA] (G) P. [TNA] (A) P. [TNA] (G) P. [TNA] (T) P. [TNA] (T) P.[TNA](A)[sP].[dR](C)[sP].[dR](T)[sP].[dR](T )[sP].[dR](G)[sP].[dR](C)[sP].[dR](C)[sP].[dR](A ) [sP]. [dR] (A) [sP]. [LR] ([5meC]) [sP]. [LR] (T) |
| #14 | 1 | GAGTTACTTGCCAACT | [TNA] (G) P. [TNA] (A) P. [TNA] (G) P. [TNA] (T) P. [TNA] (T) P.[TNA](A)[sP].[dR](C)[sP].[dR](T)[sP].[dR](T )[sP].[dR](G)[sP].[dR](C)[sP].[dR](C)[sP].[dR](A ) [sP]. [dR] (A) [sP]. [dR] (C) [sP]. [LR] (T) |
| #15 | 1 | GAGTTACTTGCCAACT | [TNA] (G) P. [TNA] (A) P. [TNA] (G) P. [TNA] (T) P. [TNA] (T) P.[TNA](A)P.[TNA]([5meC])[sP].[dR](T)[sP].[d R] (T) [sP]. [d R] (G) [sP]. [dR] (C) [sP]. [dR] (C) [sP]. [d R] (A) [sP]. [dR] (A) [sP]. [LR] ([5meC]) [sP]. [LR] (T) |
| #16 | 1 | GAGTTACTTGCCAACT | [TNA] (G) P. [TNA] (A) P. [TNA] (G) P. [TNA] (T) P. [TNA] (T) P.[TNA](A)P.[TNA]([5meC])[sP].[dR](T)[sP].[d R] (T) [sP]. [d R] (G) [sP]. [dR] (C) [sP]. [dR] (C) [sP]. [d R] (A) [sP]. [dR] (A) [sP]. [dR] (C) [sP]. [LR] (T) |
| #17 | 1 | GAGTTACTTGCCAACT | [TNA] (G) P. [TNA] (A) P. [TNA] (G) P. [TNA] (T) P. [TNA] (T) P.[TNA](A)P.[TNA]([5meC])P.[TNA](T)[sP].[dR ](T)[sP].[dR](G)[sP].[dR](C)[sP].[dR](C)[sP].[dR ] (A) [sP]. [dR] (A) [sP]. [LR] ([5meC]) [sP]. [LR] (T) |

(continued)

| CMP ID NO | SEQ ID NO | Base sequence 5'-3' | HELM |
|---|---|---|---|
| #18 | 1 | GAGTTACTTGCCAACT | [TNA] (G) P. [TNA] (A) P. [TNA] (G) P. [TNA] (T) P. [TNA] (T) P.[TNA](A)P.[TNA]([5meC])P.[TNA](T)[sP].[dR ](T)[sP].[dR](G)[sP].[dR](C)[sP].[dR](C)[sP].[dR ] (A) [sP]. [dR] (A) [sP]. [dR] (C) [sP]. [LR] (T) |
| #19 | 1 | GAGTTACTTGCCAACT | [TNA](G)P.[LR](A)[sP].[LR](G)[sP].[dR](T)[sP].[d R] (T) [sP]. [d R] (A) [sP]. [dR] (C) [sP]. [d R] (T) [sP]. [d R](T)[sP].[dR](G)[sP].[dR](C)[sP].[dR](C)[sP].[d R] (A) [sP]. [LR] (A) [sP]. [LR] ([5meC]) P. [TNA] (T) |
| #20 | 1 | GAGTTACTTGCCAACT | [TNA](G)P.[LR](A)[sP].[LR](G)[sP].[dR](T)[sP].[d R] (T) [sP]. [d R] (A) [sP]. [dR] (C) [sP]. [d R] (T) [sP]. [d R](T)[sP].[dR](G)[sP].[dR](C)[sP].[dR](C)[sP].[d R](A)[sP].[LR](A)P.[TNA]([5meC])P.[TNA] (T) |
| #21 | 1 | GAGTTACTTGCCAACT | [TNA](G)P.[TNA](A)P.[LR](G)[sP].[dR](T)[sP].[dR ](T)[sP].[dR](A)[sP].[dR](C)[sP].[dR](T)[sP].[dR] (T)[sP].[dR](G)[sP].[dR](C)[sP].[dR](C)[sP].[dR] (A)[sP].[LR](A)P.[TNA]([5meC])P.[TNA] (T) |
| #22 | 1 | GAGTTACTTGCCAACT | [LR](G)[sP].[LR](A)P.[TNA](G)[sP].[dR](T)[sP].[d R](T)[sP].[dR](A)[sP].[dR](C)[sP].[dR](T)[sP].[d R](T)[sP].[dR](G)[sP].[dR](C)[sP].[dR](C)[sP].[d R](A)P.[TNA](A)P.[LR]([5meC])[sP].[LR](T) |
| #23 | 1 | GAGTTACTTGCCAACT | [LR](G)[sP].[LR](A)P.[TNA](G)P.[dR](T)[sP].[dR] (T)[sP].[dR](A)[sP].[dR](C)[sP].[dR](T)[sP].[dR]( T)[sP].[dR](G)[sP].[dR](C)[sP].[dR](C)[sP].[dR]( A)P.[TNA](A)P.[LR]([5meC])[sP].[LR](T) |
| #24 | 1 | GAGTTACTTGCCAACT | [LR](G)[sP].[LR](A)P.[TNA](G)[sP].[dR](T)[sP].[d R] (T) [sP]. [d R] (A) [sP]. [dR] (C) [sP]. [d R] (T) [sP]. [d R](T)[sP].[dR](G)[sP].[dR](C)[sP].[dR](C)[sP].[d R] (A) [sP]. [LR] (A) P. [TNA] ([5meC]) [sP]. [LR] (T) |
| #25 | 1 | GAGTTACTTGCCAACT | [LR](G)[sP].[LR](A)P.[TNA](G)P.[dR](T)[sP].[dR] (T)[sP].[dR](A)[sP].[dR](C)[sP].[dR](T)[sP].[dR]( T)[sP].[dR](G)[sP].[dR](C)[sP].[dR](C)[sP].[dR]( A)[sP].[LR](A)P.[TNA]( [5meC])[sP].[LR](T) |
| #26 | 1 | GAGTTACTTGCCAACT | [LR](G)[sP].[LR](A)P.[TNA](G)P.[dR](T)[sP].[dR] (T)[sP].[dR](A)[sP].[dR](C)[sP].[dR](T)[sP].[dR]( T)[sP].[dR](G)[sP].[dR](C)[sP].[dR](C)[sP].[dR]( A) [sP]. [LR] (A) P. [TNA] ([5meC]) P. [LR] (T) |
| #27 | 1 | GAGTTACTTGCCAACT | [LR](G)P.[TNA](A)[sP].[LR](G)[sP].[dR](T)[sP].[d R](T)[sP].[dR](A)[sP].[dR](C)[sP].[dR](T)[sP].[d R](T)[sP].[dR](G)[sP].[dR](C)[sP].[dR](C)[sP].[d R](A)[sP].[LR](A)P.[TNA]([5meC])[sP].[LR](T) |
| #28 | 1 | GAGTTACTTGCCAACT | [LR](G)P.[TNA](A)P.[LR](G)[sP].[dR](T)[sP].[dR] (T)[sP].[dR](A)[sP].[dR](C)[sP].[dR](T)[sP].[dR]( T)[sP].[dR](G)[sP].[dR](C)[sP].[dR](C)[sP].[dR]( A)[sP].[LR](A)P.[TNA]( [5meC])[sP].[LR](T) |

(continued)

| CMP ID NO | SEQ ID NO | Base sequence 5'-3' | HELM |
|---|---|---|---|
| #29 | 1 | GAGTTACTTGCCAACT | [LR](G)P.[TNA](A)P.[LR](G)[sP].[dR](T)[sP].[dR] (T)[sP].[dR](A)[sP].[dR](C)[sP].[dR](T)[sP].[dR]( T)[sP].[dR](G)[sP].[dR](C)[sP].[dR](C)[sP].[dR]( A)[sP].[LR](A)[sP].[LR]( [5meC])[sP].[LR](T) |
| #30 | 1 | GAGTTACTTGCCAACT | [LR](G)[sP].[LR](A)P.[TNA](G)P.[dR](T)[sP].[dR] (T)[sP].[dR](A)[sP].[dR](C)[sP].[dR](T)[sP].[dR]( T)[sP].[dR](G)[sP].[dR](C)[sP].[dR](C)[sP].[dR]( A)[sP].[LR](A)[sP].[LR]( [5meC])[sP].[LR](T) |
| #31 | 1 | GAGTTACTTGCCAACT | [LR](G)[sP].[LR](A)[sP].[LR](G)P.[TNA](T)P.[dR]( T)[sP].[dR](A)[sP].[dR](C)[sP].[dR](T)[sP].[dR](T )[sP].[dR](G)[sP].[dR](C)[sP].[dR](C)[sP].[dR](A ) [sP]. [LR] (A) [sP]. [LR] ([5meC]) [sP]. [LR] (T) |
| #32 | 1 | GAGTTACTTGCCAACT | [LR] (G) [sP]. [LR] (A) [sP]. [LR] (G) [sP]. [dR] (T) P. [TN A] (T)P.[dR](A)[sP].[dR](C)[sP].[dR](T)[sP].[dR]( T)[sP].[dR] (G)[sP].[dR](C)[sP].[dR](C)[sP].[dR]( A) [sP]. [LR] (A) [sP]. [LR] ([5meC]) [sP]. [LR] (T) |
| #33 | 1 | GAGTTACTTGCCAACT | [LR](G)[sP].[LR](A)[sP].[LR](G)[sP].[dR](T)[sP].[ dR](T)P.[TNA](A)P.[dR](C)[sP].[dR](T)[sP].[dR]( T)[sP].[dR](G)[sP].[dR](C)[sP].[dR](C)[sP].[dR]( A)[sP].[LR](A)[sP].[LR]( [5meC])[sP].[LR](T) |
| #34 | 1 | GAGTTACTTGCCAACT | [LR](G)[sP].[LR](A)[sP].[LR](G)[sP].[dR](T)[sP].[ dR](T)[sP].[dR](A)P.[TNA]([5meC])P.[dR](T)[sP]. [dR](T)[sP].[dR](G) [sP].[dR](C)[sP].[dR](C)[sP]. [d R] (A) [sP]. [LR] (A) [sP]. [LR] ([5meC]) [sP]. [LR] (T) |
| #35 | 1 | GAGTTACTTGCCAACT | [LR](G)[sP].[LR](A)[sP].[LR](G)[sP].[dR](T)[sP].[ dR](T)[sP].[dR](A)[sP].[dR](C)P.[TNA](T)P.[dR]( T)[sP].[dR](G)[sP].[dR](C)[sP].[dR](C)[sP].[dR]( A)[sP].[LR](A)[sP].[LR]( [5meC])[sP].[LR](T) |
| #36 | 1 | GAGTTACTTGCCAACT | [LR](G)[sP].[LR](A)[sP].[LR](G)[sP].[dR](T)[sP].[ dR](T)[sP].[dR](A)[sP].[dR](C)[sP].[dR](T)P.[TN A](T)P.[dR](G)[sP].[dR](C)[sP].[dR](C)[sP].[dR]( A)[sP].[LR](A)[sP].[LR]( [5meC])[sP].[LR](T) |
| #37 | 1 | GAGTTACTTGCCAACT | [LR](G)[sP].[LR](A)[sP].[LR](G)[sP].[dR](T)[sP].[ dR](T)[sP].[dR](A)[sP].[dR](C)[sP].[dR](T)[sP].[ dR](T)P.[TNA](G)P.[dR](C)[sP].[dR](C)[sP].[dR]( A)[sP].[LR](A)[sP].[LR]( [5meC])[sP].[LR](T) |
| #38 | 1 | GAGTTACTTGCCAACT | [LR](G)[sP].[LR](A)[sP].[LR](G)[sP].[dR](T)[sP].[ dR](T)[sP].[dR](A)[sP].[dR](C)[sP].[dR](T)[sP].[ dR](T)[sP].[dR](G)P.[TNA]([5meC])P.[dR](C)[sP] .[dR](A)[sP].[LR](A)[sP].[LR]( [5meC])[sP].[LR](T ) |
| #39 | 1 | GAGTTACTTGCCAACT | [LR](G)[sP].[LR](A)[sP].[LR](G)[sP].[dR](T)[sP].[ dR](T)[sP].[dR](A)[sP].[dR](C)[sP].[dR](T)[sP].[ dR](T)[sP].[dR](G)[sP].[dR](C)P.[TNA]([5meC])P .[dR](A)[sP].[LR](A)[sP].[LR]( [5meC])[sP].[LR](T ) |

(continued)

| CMP ID NO | SEQ ID NO | Base sequence 5'-3' | HELM |
|---|---|---|---|
| #40 | 1 | GAGTTACTTGCCAACT | [LR](G)[sP].[LR](A)[sP].[LR](G)[sP].[dR](T)[sP].[ dR](T)[sP].[dR](A)[sP].[dR](C)[sP].[dR](T)[sP].[ d R] (T) [sP]. [d R] (G) [sP]. [dR] (C) [sP]. [dR] (C) P. [TN A] (A) P. [LR] (A) [sP]. [LR] ([5meC]) [sP]. [LR] (T) |
| #41 | 1 | GAGTTACTTGCCAACT | [LR](G)[sP].[LR](A)[sP].[LR](G)[sP].[dR](T)[sP].[ dR](T)[sP].[dR](A)[sP].[dR](C)[sP].[dR](T)[sP].[ dR](T)[sP].[dR](G)[sP].[dR](C)[sP].[dR](C)[sP].[ dR](A)P.[TNA](A)P.[LR]([5meC])[sP].[LR](T) |
| #42 | 1 | GAGTTACTTGCCAACT | [LR](G)[sP].[LR](A)[sP].[LR](G)[sP].[dR](T)[sP].[ dR](T)[sP].[dR](A)[sP].[dR](C)[sP].[dR](T)[sP].[ dR](T)[sP].[dR](G)[sP].[dR](C)[sP].[dR](C)[sP].[ dR](A)[sP].[LR](A)P.[TNA]([5meC])P.[LR](T) |
| #43 | 1 | GAGTTACTTGCCAACT | [LR](G)[sP].[LR](A)[sP].[LR](G)[sP].[dR](T)[sP].[ dR](T)[sP].[dR](A)[sP].[dR](C)[sP].[dR](T)[sP].[dR](T)[sP].[dR](G)[sP].[dR](C)[sP].[dR](C)[sP].[ dR](A)[sP].[LR](A)[sP].[LR]([5meC])P.[TNA](T) |
| #44 | 1 | GAGTTACTTGCCAACT | [LR](G)P.[TNA](A)[sP].[LR](G)[sP].[dR](T)[sP].[d R](T)[sP].[dR](A)[sP].[dR](C)[sP].[dR](T)[sP].[d R] (T) [sP]. [d R] (G) [sP]. [dR] (C) [sP]. [dR] (C) [sP]. [d R] (A) [sP]. [LR] (A) [sP]. [LR] ([5meC]) [sP]. [LR] (T)} |
| #45 | 1 | GAGTTACTTGCCAACT | [LR](G)[sP].[LR](A)P.[TNA](G)[sP].[dR](T)[sP].[d R](T)[sP].[dR](A)[sP].[dR](C)[sP].[dR](T)[sP].[d R](T)[sP].[dR](G)[sP].[dR](C)[sP].[dR](C)[sP].[d R] (A) [sP]. [LR] (A) [sP]. [LR] ([5meC]) [sP]. [LR] (T)} |
| #46 | 1 | GAGTTACTTGCCAACT | [LR](G)[sP].[LR](A)[sP].[LR](G)P.[TNA](T)[sP].[d R] (T) [sP]. [d R] (A) [sP]. [dR] (C) [sP]. [d R] (T) [sP]. [d R](T)[sP].[dR](G)[sP].[dR](C)[sP].[dR](C)[sP].[d R] (A) [sP]. [LR] (A) [sP]. [LR] ([5meC]) [sP]. [LR] (T) |
| #47 | 1 | GAGTTACTTGCCAACT | [LR](G)[sP].[LR](A)[sP].[LR](G)[sP].[dR](T)P.[TN A](T)[sP].[dR](A)[sP].[dR](C)[sP].[dR](T)[sP].[d R](T)[sP].[dR](G)[sP].[dR](C)[sP].[dR](C)[sP].[d R] (A) [sP]. [LR] (A) [sP]. [LR] ([5meC]) [sP]. [LR] (T) |
| #48 | 1 | GAGTTACTTGCCAACT | [LR](G)[sP].[LR](A)[sP].[LR](G)[sP].[dR](T)[sP].[ dR](T)P.[TNA](A)[sP].[dR](C)[sP].[dR](T)[sP].[d R](T)[sP].[dR](G)[sP].[dR](C)[sP].[dR](C)[sP].[d R] (A) [sP]. [LR] (A) [sP]. [LR] ([5meC]) [sP]. [LR] (T) |
| #49 | 1 | GAGTTACTTGCCAACT | [LR](G)[sP].[LR](A)[sP].[LR](G)[sP].[dR](T)[sP].[ dR](T)[sP].[dR](A)P.[TNA]([5meC])[sP].[dR](T)[s P]. [d R] (T) [sP]. [dR] (G) [sP]. [dR] (C) [sP]. [d R] (C) [s P]. [dR] (A) [sP]. [LR] (A) [sP]. [LR] ([5meC]) [sP]. [LR] (T) |
| #50 | 1 | GAGTTACTTGCCAACT | [LR](G)[sP].[LR](A)[sP].[LR](G)[sP].[dR](T)[sP].[ d R] (T) [sP]. [dR] (A) [sP]. [dR] (C) P. [TNA] (T) [sP]. [d R] (T) [sP]. [d R] (G) [sP]. [dR] (C) [sP]. [dR] (C) [sP]. [d R] (A) [sP]. [LR] (A) [sP]. [LR] ([5meC]) [sP]. [LR] (T) |

(continued)

| CMP ID NO | SEQ ID NO | Base sequence 5'-3' | HELM |
|---|---|---|---|
| #51 | 1 | GAGTTACTTGCCAACT | [LR](G)[sP].[LR](A)[sP].[LR](G)[sP].[dR](T)[sP].[ dR](T)[sP].[dR](A)[sP].[dR](C)[sP].[dR](T)P.[TN A](T)[sP].[dR](G)[sP].[dR](C)[sP].[dR](C)[sP].[d R](A)[sP].[LR](A)[sP].[LR]([5meC])[sP].[LR](T) |
| #52 | 1 | GAGTTACTTGCCAACT | [LR](G)[sP].[LR](A)[sP].[LR](G)[sP].[dR](T)[sP].[ dR](T)[sP].[dR](A)[sP].[dR](C)[sP].[dR](T)[sP].[dR](T)P.[TNA](G)[sP].[dR](C)[sP].[dR](C)[sP].[d R] (A) [sP]. [LR] (A) [sP]. [LR] ([5meC]) [sP]. [LR] (T) |
| #53 | 1 | GAGTTACTTGCCAACT | [LR](G)[sP].[LR](A)[sP].[LR](G)[sP].[dR](T)[sP].[ dR](T)[sP].[dR](A)[sP].[dR](C)[sP].[dR](T)[sP].[ dR](T)[sP].[dR](G)P.[TNA]([5meC])[sP].[dR](C)[ sP].[dR](A)[sP].[LR](A)[sP].[LR]([5meC])[sP].[LR ](T) |
| #54 | 1 | GAGTTACTTGCCAACT | [LR](G)[sP].[LR](A)[sP].[LR](G)[sP].[dR](T)[sP].[ dR](T)[sP].[dR](A)[sP].[dR](C)[sP].[dR](T)[sP].[ dR](T)[sP].[dR](G)[sP].[dR](C)P.[TNA]([5meC])[ sP].[dR](A)[sP].[LR](A)[sP].[LR]([5meC])[sP].[LR ](T) |
| #55 | 1 | GAGTTACTTGCCAACT | [LR](G)[sP].[LR](A)[sP].[LR](G)[sP].[dR](T)[sP].[ dR](T)[sP].[dR](A)[sP].[dR](C)[sP].[dR](T)[sP].[ dR](T)[sP].[dR](G)[sP].[dR](C)[sP].[dR](C)P.[TN A] (A) [sP]. [LR] (A) [sP]. [LR] ([5meC]) [sP]. [LR] (T) |
| #56 | 1 | GAGTTACTTGCCAACT | [LR](G)[sP].[LR](A)[sP].[LR](G)[sP].[dR](T)[sP].[ dR](T)[sP].[dR](A)[sP].[dR](C)[sP].[dR](T)[sP].[ dR](T)[sP].[dR](G)[sP].[dR](C)[sP].[dR](C)[sP].[ d R] (A) P. [TNA] (A) [sP]. [LR] ([5meC]) [sP]. [LR] (T) |
| #57 | 1 | GAGTTACTTGCCAACT | [LR] (G) [sP]. [TNA] (A) P. [LR] (G) [sP]. [dR] (T) [sP]. [d R] (T) [sP]. [d R] (A) [sP]. [dR] (C) [sP]. [d R] (T) [sP]. [d R] (T) [sP]. [d R] (G) [sP]. [dR] (C) [sP]. [dR] (C) [sP]. [d R] (A) [sP]. [LR] (A) [sP]. [LR] ([5meC]) [sP]. [LR] (T) |
| #58 | 1 | GAGTTACTTGCCAACT | [LR] (G) [sP]. [LR] (A) [sP]. [TNA] (G) P. [dR] (T) [sP]. [d R] (T) [sP]. [d R] (A) [sP]. [dR] (C) [sP]. [d R] (T) [sP]. [d R](T) [sP].[dR](G)[sP].[dR](C)[sP].[dR](C)[sP].[d R] (A) [sP]. [LR] (A) [sP]. [LR] ([5meC]) [sP]. [LR] (T) |
| #59 | 1 | GAGTTACTTGCCAACT | [LR](G)[sP].[LR](A)[sP].[LR](G)[sP].[TNA](T)P.[d R] (T) [sP]. [d R] (A) [sP]. [dR] (C) [sP]. [d R] (T) [sP]. [d R](T)[sP].[dR](G)[sP].[dR](C)[sP].[dR](C)[sP].[d R](A)[sP].[LR](A)[sP].[LR]([5meC])[sP].[LR](T) |
| #60 | 1 | GAGTTACTTGCCAACT | [LR](G)[sP].[LR](A)[sP].[LR](G)[sP].[dR](T)[sP].[ TNA](T)P.[dR](A)[sP].[dR](C)[sP].[dR](T)[sP].[d R](T)[sP].[dR](G)[sP].[dR](C)[sP].[dR](C)[sP].[d R] (A) [sP]. [LR] (A) [sP]. [LR] ([5meC]) [sP]. [LR] (T) |
| #61 | 1 | GAGTTACTTGCCAACT | [LR](G)[sP].[LR](A)[sP].[LR](G)[sP].[dR](T)[sP].[ d R] (T) [sP]. [TNA] (A) P. [dR] (C) [sP]. [dR] (T) [sP]. [d R](T)[sP].[dR](G)[sP].[dR](C)[sP].[dR](C)[sP].[d R] (A) [sP]. [LR] (A) [sP]. [LR] ([5meC]) [sP]. [LR] (T) |

(continued)

| CMP ID NO | SEQ ID NO | Base sequence 5'-3' | HELM |
|---|---|---|---|
| #62 | 1 | GAGTTACTTGCCAACT | [LR](G)[sP].[LR](A)[sP].[LR](G)[sP].[dR](T)[sP].[ d R] (T) [sP]. [dR] (A) [sP]. [TNA] ([5meC]) P. [d R] (T) [s P]. [d R] (T) [sP]. [dR] (G) [sP]. [dR] (C) [sP]. [d R] (C) [s P]. [dR] (A) [sP]. [LR] (A) [sP]. [LR] ([5meC]) [sP]. [LR] (T) |
| #63 | 1 | GAGTTACTTGCCAACT | [LR](G)[sP].[LR](A)[sP].[LR](G)[sP].[dR](T)[sP].[ dR](T)[sP]. [dR](A)[sP].[dR](C)[sP].[TNA](T)P.[d R] (T) [sP]. [d R] (G) [sP]. [dR] (C) [sP]. [dR] (C) [sP]. [d R] (A) [sP]. [LR] (A) [sP]. [LR] ([5meC]) [sP]. [LR] (T) |
| #64 | 1 | GAGTTACTTGCCAACT | [LR](G)[sP].[LR](A)[sP].[LR](G)[sP].[dR](T)[sP].[ dR](T)[sP]. [dR](A)[sP].[dR](C)[sP].[dR](T)[sP].[T NA](T)P.[dR](G)[sP]. [dR](C)[sP].[dR](C)[sP].[dR ](A)[sP].[LR](A)[sP].[LR]( [5meC])[sP].[LR](T) |
| #65 | 1 | GAGTTACTTGCCAACT | [LR](G)[sP].[LR](A)[sP].[LR](G)[sP].[dR](T)[sP].[ dR](T)[sP]. [dR](A)[sP].[dR](C)[sP].[dR](T)[sP].[ d R] (T) [sP]. [TNA] (G) P. [d R] (C) [sP]. [dR] (C) [sP]. [d R] (A) [sP]. [LR] (A) [sP]. [LR] ([5meC]) [sP]. [LR] (T) |
| #66 | 1 | GAGTTACTTGCCAACT | [LR](G)[sP].[LR](A)[sP].[LR](G)[sP].[dR](T)[sP].[ dR](T)[sP]. [dR](A)[sP].[dR](C)[sP].[dR](T)[sP].[ dR](T)[sP].[dR](G)[sP]. [TNA]([5meC])P.[dR](C)[ sP].[dR](A)[sP].[LR](A)[sP].[LR]( [5meC])[sP].[LR ](T) |
| #67 | 1 | GAGTTACTTGCCAACT | [LR](G)[sP].[LR](A)[sP].[LR](G)[sP].dR](T)[sP].[ dR](T)[sP]. [dR](A)[sP].[dR](C)[sP].[dR](T)[sP].[ dR](T)[sP].[dR](G)[sP]. [dR](C)[sP].[TNA]([5meC ])P.[dR](A)[sP].[LR](A)[sP].[LR]( [5meC])[sP].[LR ](T) |
| #68 | 1 | GAGTTACTTGCCAACT | [LR](G)[sP].[LR](A)[sP].[LR](G)[sP].[dR](T)[sP].[ dR](T)[sP]. [dR](A)[sP].[dR](C)[sP].[dR](T)[sP].[ dR](T)[sP].[dR](G)[sP]. [dR](C)[sP].[dR](C)[sP].[ TNA](A)P.[LR](A)[sP].[LR]( [5meC])[sP].[LR](T) |
| #69 | 1 | GAGTTACTTGCCAACT | [LR](G)[sP].[LR](A)[sP].[LR](G)[sP].[dR](T)[sP].[ dR](T)[sP]. [dR](A)[sP].[dR](C)[sP].[dR](T)[sP].[dR](T)[sP].[dR](G)[sP]. [dR](C)[sP].[dR](C)[sP].[ dR](A)[sP].[TNA](A)P.[LR]( [5meC])[sP].[LR](T) |
| #70 | 1 | GAGTTACTTGCCAACT | [LR](G)[sP].[LR](A)[sP].[LR](G)[sP].[dR](T)[sP].[ dR](T)[sP]. [dR](A)[sP].[dR](C)[sP].[dR](T)[sP].[ dR](T)[sP].[dR](G)[sP]. [dR](C)[sP].[dR](C)[sP].[ dR](A)[sP].[LR](A)[sP].[TNA]( [5meC])P.[LR](T) |
| CONTROL #2 | 2 | GCCAGGCTGGTTATGAC TCA | [MOE](G)[sP].[MOE]([5meC])[sP].[MOE]([5meC] )[sP]. [MOE](A)[sP].[MOE](G)[sP].[dR](G)[sP].[dR ](C)[sP].[dR] (T)[sP].[dR](G)[sP].[dR](G)[sP].[dR ](T)[sP].[dR](T)[sP].[dR] (A)[sP].[dR](T)[sP].[dR] (G)[sP].[MOE](A)[sP].[MOE]( [5meC])[sP].[MOE]( T) [sP]. [MOE] ([5meC]) [sP]. [MOE] (A) |
| #71 | 2 | GCCAGGCTGGTTATGAC TCA | [TNA](G)[sP].[TNA]([5meC])[sP].[TNA]([5meC])[ sP].[TNA] (A)P.[TNA](G)[sP].[dR](G)[sP].[dR](C)[ sP].[dR](T)[sP].[dR] (G)[sP].[dR](G)[sP].[dR](T)[ sP].[dR](T)[sP].[dR](A)[sP].[dR] (T)[sP].[dR](G)[s P].[MOE](A)[sP].[MOE]([5meC])[sP]. [MOE](T)[sP ]. [MOE] ([5meC]) [sP]. [MOE] (A) |

(continued)

| CMP ID NO | SEQ ID NO | Base sequence 5'-3' | HELM |
|---|---|---|---|
| #72 | 2 | GCCAGGCTGGTTATGACTCA | [TNA](G)[sP].[TNA]([5meC])[sP].[TNA]([5meC]) P.[TNA](A) P.[TNA](G)[sP].[dR](G)[sP].[dR](C)[sP ].[dR](T)[sP].[dR](G) [sP].[dR](G)[sP].[dR](T)[sP ]. [dR] (T) [sP]. [d R] (A) [sP]. [dR] (T) [sP]. [dR] (G) [sP] [MOE] (A) [sP]. [MOE] ([5meC]) [sP]. [MOE] (T) [sP]. MOE]([5meC])[sP].[MOE](A) |
| #73 | 2 | GCCAGGCTGGTTATGACTCA | [TNA](G)[sP].[TNA]([5meC])P.[TNA]([5meC])P.[ TNA](A)P. [TNA](G)[sP].[dR](G)[sP].[dR](C)[sP].[ dR](T)[sP].[dR](G) [sP].[dR](G)[sP].[dR](T)[sP].[ dR](T)[sP].[dR](A)[sP].[dR](T) [sP].[dR](G)[sP].[ MOE](A)[sP].[MOE]([5meC])[sP].[MOE] (T)[sP].[ MOE] ([5meC]) [sP]. [MOE] (A) |
| #74 | 2 | GCCAGGCTGGTTATGACTCA | [TNA](G)P.[TNA]([5meC])P.[TNA]([5meC])P.[TN A](A)P. [TNA](G)[sP].[dR](G)[sP].[dR](C)[sP].[dR ](T)[sP].[dR](G) [sP].[dR](G)[sP].[dR](T)[sP].[dR ](T)[sP].[dR](A)[sP].[dR](T) [sP].[dR](G)[sP].[MO E](A)[sP].[MOE]([5meC])[sP].[MOE] (T)[sP].[MOE ] ([5meC]) [sP]. [MOE] (A) |
| #75 | 2 | GCCAGGCTGGTTATGACTCA | [TNA] (G) P. [TNA] ([5meC]) P. [TNA] ([5meC]) P. [TN A](A) P.[TNA](G)P.[TNA](G)[sP].[dR](C)[sP].[dR](T)[sP].[dR](G) [sP].[dR](G)[sP].[dR](T)[sP].[dR]( T) [sP]. [dR] (A) [sP]. [dR] (T) [sP]. [d R](G) [sP]. [MOE] (A)[sP].[MOE]([5meC])[sP]. [MOE](T)[sP].[MOE]( [5meC])[sP].[MOE](A) |
| #76 | 2 | GCCAGGCTGGTTATGACTCA | [TNA] (G) P. [TNA] ([5meC]) P. [TNA] ([5meC]) P. [TN A] (A) P. [TNA] (G) P. [TNA] (G) P. [TNA] ([5meC]) [sP]. [dR](T)[sP]. [dR](G)[sP].[dR](G)[sP].[dR](T)[sP]. [dR](T)[sP].[dR](A)[sP]. [dR](T)[sP].[dR](G)[sP].[ MOE](A)[sP].[MOE]([5meC])[sP]. [MOE](T)[sP].[ MOE] ([5meC]) [sP]. [MOE] (A) |
| #77 | 2 | GCCAGGCTGGTTATGACTCA | [TNA](G)P.[TNA]([5meC])P.[TNA]([5meC])P.[TN A](A)P. [TNA](G)P.[TNA](G)P.[TNA]([5meC])P.[T NA](T)[sP].[dR] (G)[sP].[dR](G)[sP].[dR](T)[sP].[ dR](T)[sP].[dR](A)[sP].[dR] (T)[sP].[dR](G)[sP].[ MOE](A)[sP].[MOE]([5meC])[sP]. [MOE](T)[sP]. MOE]([5meC])[sP].[MOE](A) |
| #78 | 2 | GCCAGGCTGGTTATGACTCA | [TNA](G)P.[TNA]([5meC])P.[TNA]([5meC])P.[TN A](A)P. [TNA](G)P.[TNA](G)P.[TNA]([5meC])P.[T NA](T)P.[TNA](G) [sP].[dR](G)[sP].[dR](T)[sP].[d R](T)[sP].[dR](A)[sP].[dR](T) [sP].[dR](G)[sP].[M OE](A)[sP].[MOE]([5meC])[sP].[MOE] (T)[sP].[MO E]([5meC])[sP].[MOE](A) |
| #79 | 2 | GCCAGGCTGGTTATGACTCA | [TNA](G)P.[TNA]([5meC])P.[TNA]([5meC])P.[TN A](A)P. [TNA](G)P.[TNA](G)P.[TNA]([5meC])P.[T NA](T)P.[TNA](G) P.[TNA](G)[sP].[dR](T)[sP].[dR ](T)[sP].[dR](A)[sP].[dR](T) [sP].[dR](G)[sP].[MO E](A)[sP].[MOE]([5meC])[sP].[MOE] (T)[sP].[MOE ]([5meC])[sP].[MOE](A) |
| #80 | 2 | GCCAGGCTGGTTATGACTCA | [MOE](G)[sP].[MOE]([5meC])[sP].[MOE]([5meC] )[sP]. [MOE](A)[sP].[MOE](G)P.[TNA](G)[sP].[dR] (C)[sP].[dR](T) [sP].[dR](G)[sP].[dR](G)[sP].[dR] (T)[sP].[dR](T)[sP].[dR](A) [sP].[dR](T)[sP].[dR]( G)[sP].[MOE](A)[sP].[MOE]([5meC]) [sP].[MOE](T ) [sP]. [MOE] ([5meC]) [sP]. [MOE] (A) |

(continued)

| CMP ID NO | SEQ ID NO | Base sequence 5'-3' | HELM |
|---|---|---|---|
| #81 | 2 | GCCAGGCTGGTTATGAC TCA | [MOE](G)[sP].[MOE]([5meC])[sP].[MOE]([5meC] )[sP].[MOE](A)[sP].[MOE](G)P.[TNA](G)P.[dR](C) [sP].[dR](T)[sP].[dR](G)[sP].[dR](G)[sP].[dR](T) [sP].[dR](T)[sP].[dR](A)[sP].[dR](T)[sP].[dR](G)[sP]. [MOE] (A) [sP]. [MOE] ([5meC])[sP]. [MOE] (T) [s P]. [MOE] ([5meC]) [sP]. [MOE] (A) |
| #82 | 2 | GCCAGGCTGGTTATGAC TCA | [MOE](G)[sP].[MOE]([5meC])[sP].[MOE]([5meC] )[sP].[MOE](A)[sP].[MOE](G)P.[TNA](G)P.[TNA]([ 5meC])[sP].[dR](T)[sP].[dR](G)[sP].[dR](G)[sP]. [dR](T)[sP].[dR](T)[sP].[dR](A)[sP].[dR](T)[sP].[ d R] (G) [sP]. [MOE] (A) [sP]. [MOE]([5meC]) [sP]. [M OE] (T) [sP]. [MOE] ([5meC]) [sP]. [MOE] (A) |
| #83 | 2 | GCCAGGCTGGTTATGAC TCA | [MOE](G)[sP].[MOE]([5meC])[sP].[MOE]([5meC] )[sP].[MOE](A)[SP].[MOE](G)P.[TNA](G)P.[TNA]([ 5meC])P.[dR](T)[sP].[dR](G)[sP].[dR](G)[sP].[dR ](T)[sP].[dR](T)[sP].[dR](A)[sP].[dR](T)[sP].[dR] (G)[sP].[MOE](A)[sP].[MOE]([5meC])[sP].[MOE]( T) [sP]. [MOE] ([5meC]) [sP]. [MOE] (A) |
| #84 | 2 | GCCAGGCTGGTTATGAC TCA | [MOE](G)[sP].[MOE]([5meC])[sP].[MOE]([5meC] )[sP].[MOE](A)[SP].[MOE](G)P.[TNA](G)P.[TNA]([ 5meC])P.[TNA](T)[sP].[dR](G)[sP].[dR](G)[sP].[d R](T)[sP].[dR](T)[sP].[dR](A)[sP].[dR](T)[sP].[dR ] (G) [sP]. [MOE] (A) [sP]. [MOE] ([5meC]) [sP]. [MOE] (T) [sP]. [MOE] ([5meC]) [sP]. [MOE] (A) |
| #85 | 2 | GCCAGGCTGGTTATGAC TCA | [MOE](G)[sP].[MOE]([5meC])[sP].[MOE]([5meC] )[sP].[MOE](A)[sP].[MOE](G)P.[TNA](G)P.[TNA]([ 5meC])P.[TNA](T)P.[dR](G)[sP].[dR](G)[sP].[dR] (T)[sP].[dR](T)[sP].[dR](A)[sP].[dR](T)[sP].[dR]( G)[sP].[MOE](A)[sP].[MOE]([5meC])[sP].[MOE](T ) [sP]. [MOE] ([5meC]) [sP]. [MOE] (A) |
| #86 | 2 | GCCAGGCTGGTTATGAC TCA | [MOE](G)[sP].[MOE]([5meC])[sP].[MOE]([5meC] )[sP].[MOE](A)[sP].[MOE](G)P.[TNA](G)P.[TNA]([ 5meC])P.[TNA](T)P.[TNA](G)[sP].[dR](G)[sP].[dR ](T)[sP].[dR](T)[sP].[dR](A)[sP].[dR](T)[sP].[dR] (G)[sP].[MOE](A)[sP].[MOE]([5meC])[sP].[MOE]( T)[sP].[MOE]([5meC])[sP].[MOE](A) |
| #87 | 2 | GCCAGGCTGGTTATGAC TCA | [MOE](G)[sP].[MOE]([5meC])[sP].[MOE]([5meC] )[sP].[MOE](A)[SP].[MOE](G)P.[TNA](G)P.[TNA]([ 5meC])P.[TNA](T)P.[TNA](G)P.[dR](G)[sP].[dR]( T)[sP].[dR](T)[sP].[dR](A)[sP].[dR](T)[sP].[dR]( G)[sP].[MOE](A)[sP].[MOE]([5meC])[sP].[MOE](T ) [sP]. [MOE] ([5meC]) [sP]. [MOE] (A) |
| #88 | 2 | GCCAGGCTGGTTATGAC TCA | [MOE](G)[sP].[MOE]([5meC])[sP].[MOE]([5meC] )[sP].[MOE](A)[sP].[MOE](G)P.[TNA](G)P.[TNA]([ 5meC])P.[TNA](T)P.[TNA](G)P.[TNA](G)[sP].[dR] (T)[sP].[dR](T)[sP].[dR](A)[sP].[dR](T)[sP].[dR]( G)[sP].[MOE](A)[sP].[MOE]([5meC])[sP].[MOE](T ) [sP]. [MOE] ([5meC]) [sP]. [MOE] (A) |
| #89 | 2 | GCCAGGCTGGTTATGAC TCA | [MOE](G)[sP].[MOE]([5meC])[sP].[MOE]([5meC] )[sP].[MOE](A)[SP].[MOE](G)P.[TNA](G)P.[TNA]([ 5meC])P.[TNA](T)P.[TNA](G)P.[TNA](G)P.[dR](T) [sP].[dR](T)[sP].[dR](A)[sP].[dR](T)[sP].[dR](G)[ sP].[MOE](A)[sP].[MOE]([5meC])[sP].[MOE](T)[s P]. [MOE] ([5meC]) [sP]. [MOE] (A) |

(continued)

| CMP ID NO | SEQ ID NO | Base sequence 5'-3' | HELM |
|---|---|---|---|
| #90 | 2 | GCCAGGCTGGTTATGAC TCA | [MOE](G)[sP].[MOE]([5meC])[sP].[MOE]([5meC] )[sP].[MOE](A)[SP].[MOE](G)P.[TNA](G)P.[TNA]([ 5meC])P.[TNA](T)P.[TNA](G)P.[TNA](G)P.[TNA]( T)[sP].[dR](T)[sP].[dR](A)[sP].[dR](T)[sP].[dR]( G)[sP].[MOE](A)[sP].[MOE]([5meC])[sP].[MOE](T )[sP].[MOE]([5meC])[sP].[MOE](A) |
| #91 | 2 | GCCAGGCTGGTTATGAC TCA | [TNA] (G) P. [MOE] ([5meC]) [sP]. [MOE] ([5meC]) [s P].[MOE](A)[sP].[MOE](G)[sP].[dR](G)[sP].[dR]( C)[sP].[dR](T)[sP].[dR](G)[sP].[dR](G)[sP].[dR]( T)[sP].[dR](T)[sP].[dR](A)[sP].[dR](T)[sP].[dR]( G)[sP].[MOE](A)[sP].[MOE]([5meC])[sP].[MOE](T ) [sP]. [MOE] ([5meC]) P. [TNA] (A) |
| #92 | 2 | GCCAGGCTGGTTATGAC TCA | [TNA] (G) P. [MOE] ([5meC]) [sP]. [MOE] ([5meC]) [s P].[MOE](A)[sP].[MOE](G)[sP].[dR](G)[sP].[dR]( C)[sP].[dR](T)[sP].[dR](G)[sP].[dR](G)[sP].[dR]( T)[sP].[dR](T)[sP].[dR](A)[sP].[dR](T)[sP].[dR]( G)[sP].[MOE](A)[sP].[MOE]([5meC])[sP].[MOE](T ) P. [TNA] ([5meC]) P. [TNA] (A) |
| #93 | 2 | GCCAGGCTGGTTATGAC TCA | [TNA](G)P.[TNA]([5meC])P.[MOE]([5meC])[sP].[ MOE] (A)[sP]. [MOE] (G) [sP]. [d R] (G) [sP]. [dR] (C) [s P].[dR](T)[sP].[dR](G)[sP].[dR](G)[sP].[dR](T)[s P].[dR](T)[sP].[dR](A)[sP].[dR](T)[sP].[dR](G)[s P].[MOE](A)[sP].[MOE]([5meC])[sP].[MOE](T)P.[ TNA]([5meC])P.[TNA](A) |
| #94 | 2 | GCCAGGCTGGTTATGAC TCA | [TNA](G)P.[TNA]([5meC])P.[MOE]([5meC])[sP].[ MOE](A)[sP].[MOE](G)P.[TNA](G)[sP].[dR](C)[sP].[dR](T)[sP].[dR](G)[sP].[dR](G)[sP].[dR](T)[sP ].[dR](T)[sP].[dR](A)[sP].[dR](T)[sP].[dR](G)[sP] .[MOE](A)[sP].[MOE]([5meC])[sP].[MOE](T)P.[TN A]([5meC])P.[TNA](A) |
| #95 | 2 | GCCAGGCTGGTTATGAC TCA | [TNA](G)P.[TNA]([5meC])P.[MOE]([5meC])[sP].[ MOE](A)[sP].[MOE](G)P.[TNA](G)P.[TNA]([5meC] )[sP].[dR](T)[sP].[dR](G)[sP].[dR](G)[sP].[dR](T )[sP].[dR](T)[sP].[dR](A)[sP].[dR](T)[sP].[dR](G) [sP].[MOE](A)[sP].[MOE]([5meC])[sP].[MOE](T)P .[TNA]([5meC])P.[TNA](A) |
| #96 | 2 | GCCAGGCTGGTTATGAC TCA | [TNA](G)P.[TNA]([5meC])P.[MOE]([5meC])[sP].[ MOE](A)[sP].[MOE](G)P.[TNA](G)P.[TNA]([5meC] )P.[TNA](T)[sP].[dR](G)[sP].[dR](G)[sP].[dR](T)[ sP]. [dR] (T) [sP]. [d R] (A)[sP]. [d R] (T) [sP]. [dR] (G) [s P].[MOE](A)[sP].[MOE]([5meC])[sP].[MOE](T)P.[ TNA]([5meC])P.[TNA](A) |
| #97 | 2 | GCCAGGCTGGTTATGAC TCA | [TNA](G)P.[TNA]([5meC])P.[MOE]([5meC])[sP].[ MOE](A)[sP].[MOE](G)P.[TNA](G)P.[TNA]([5meC] ) P. [TNA] (T) P.[TNA] (G) [sP]. [dR] (G) [sP]. [d R] (T) [s P].[dR](T)[sP].[dR](A)[sP].[dR](T)[sP].[dR](G)[s P].[MOE](A)[sP].[MOE]([5meC])[sP].[MOE](T)P.[ TNA]([5meC])P.[TNA](A) |
| #98 | 2 | GCCAGGCTGGTTATGAC TCA | [TNA](G)P.[TNA]([5meC])P.[MOE]([5meC])[sP].[ MOE](A)[sP].[MOE](G)P.[TNA](G)P.[TNA]([5meC] )P.[TNA](T)P.[TNA](G)P.[TNA](G)[sP].[dR](T)[sP] .[dR](T)[sP].[dR](A)[sP].[dR](T)[sP].[dR](G)[sP]. [MOE](A)[sP].[MOE]([5meC])[sP].[MOE](T)P.[TN A]([5meC])P.[TNA](A) |

(continued)

| CMP ID NO | SEQ ID NO | Base sequence 5'-3' | HELM |
|---|---|---|---|
| #99 | 2 | GCCAGGCTGGTTATGACTCA | [TNA] (G) P. [TNA] ([5meC]) P. [TNA] ([5meC]) P. [TN A](A) P.[TNA](G)[sP].[dR](G)[sP].[dR](C)[sP].[dR ](T)[sP].[dR](G)[sP].[dR](G)[sP].[dR](T)[sP].[dR ](T)[sP].[dR](A)[sP].[dR](T)[sP].[dR](G)[sP].[TN A](A)P.[TNA]([5meC])P.[TNA](T)P.[TNA]([5meC] ) P. [TNA] (A) |
| #100 | 2 | GCCAGGCTGGTTATGACTCA | [TNA](G)[sP].[TNA]([5meC])P.[TNA]([5meC])P.[ TNA](A)P.[TNA](G)[sP].[dR](G)[sP].[dR](C)[sP].[ dR](T)[sP].[dR](G)[sP].[dR](G)[sP].[dR](T)[sP].[ dR](T)[sP].[dR](A)[sP].[dR](T)[sP].[dR](G)[sP].[TNA] (A) P. [TNA] ([5meC]) P. [TNA] (T) P. [TNA] ([5me C])[sP].[TNA](A) |
| #101 | 2 | GCCAGGCTGGTTATGACTCA | [MOE](G)[sP].[MOE]([5meC])[sP].[MOE]([5meC] )[sP].[MOE](A)[sP].[MOE](G)[sP].[dR](G)[sP].[dR ](C)[sP].[dR](T)[sP].[dR](G)[sP].[dR](G)[sP].[dR ](T)[sP].[dR](T)[sP].[dR](A)[sP].[dR](T)[sP].[dR] (G)[sP].[TNA](A)P.[TNA]([5meC])P.[TNA](T)P.[T NA]([5meC])P.[TNA](A) |
| #102 | 2 | GCCAGGCTGGTTATGACTCA | [MOE](G)[sP].[MOE]([5meC])[sP].[MOE]([5meC] )[sP].[MOE](A)[sP].[MOE](G)[sP].[dR](G)[sP].[dR ](C)[sP].[dR](T)[sP].[dR](G)[sP].[dR](G)[sP].[dR ] (T) [sP]. [dR] (T) [sP]. [dR] (A) [sP]. [dR] (T) [sP]. [TN A] (G) P. [TNA] (A) P. [TNA] ([5meC]) P. [TNA] (T) P. [TN A]([5meC])P.[TNA](A) |
| #103 | 2 | GCCAGGCTGGTTATGACTCA | [MOE](G)[sP].[MOE]([5meC])[sP].[MOE]([5meC] )[sP].[MOE](A)[sP].[MOE](G)[sP].[dR](G)[sP].[dR ](C)[sP].[dR](T)[sP].[dR](G)[sP].[dR](G)[sP].[dR ](T)[sP].[dR](T)[sP].[dR](A)[sP].[TNA](T)P.[TNA] (G) P. [TNA] (A) P. [TNA] ([5meC]) P. [TNA] (T) P. [TNA] ([5meC])P.[TNA](A) |
| #104 | 2 | GCCAGGCTGGTTATGACTCA | [MOE](G)[sP].[MOE]([5meC])[sP].[MOE]([5meC] )[sP].[MOE](A)[sP].[MOE](G)[sP].[dR](G)[sP].[dR ](C)[sP].[dR](T)[sP].[dR](G)[sP].[dR](G)[sP].[dR ](T)[sP].[dR](T)[sP].[TNA](A)P.[TNA](T)P.[TNA]( G)P.[TNA](A)P.[TNA]([5meC])P.[TNA](T)P.[TNA]( [5meC])P.[TNA](A) |
| #105 | 2 | GCCAGGCTGGTTATGACTCA | [MOE](G)[sP].[MOE]([5meC])[sP].[MOE]([5meC] )[sP].[MOE](A)[sP].[MOE](G)[sP].[dR](G)[sP].[dR ](C)[sP].[dR](T)[sP].[dR](G)[sP].[dR](G)[sP].[dR ](T)[sP].[dR](T)[sP].[dR](A)[sP].[dR](T)[sP].[dR] (G)[sP].[MOE](A)[sP].[MOE]([5meC])[sP].[MOE]( T)[sP].[TNA]([5meC])P.[TNA](A) |
| #106 | 2 | GCCAGGCTGGTTATGACTCA | [MOE](G)[sP].[MOE]([5meC])[sP].[MOE]([5meC] )[sP].[MOE](A)[sP].[MOE](G)[sP].[dR](G)[sP].[dR ](C)[sP].[dR](T)[sP].[dR](G)[sP].[dR](G)[sP].[dR ](T)[sP].[dR](T)[sP].[dR](A)[sP].[dR](T)[sP].[dR] (G)[sP].[MOE](A)[sP].[MOE]([5meC])[sP].[MOE]( T)P.[TNA]([5meC])P.[TNA](A) |
| #107 | 2 | GCCAGGCTGGTTATGACTCA | [TNA](G)P.[MOE]([5meC])[sP].[MOE]([5meC])[s P]. [MOE] (A) [sP]. [MOE] (G) [sP]. [d R] (G) [sP]. [dR] ( C)[sP].[dR](T)[sP].[dR](G)[sP].[dR](G)[sP].[dR]( T)[sP].[dR](T)[sP].[dR](A)[sP].[dR](T)[sP].[dR]( G)[sP].[MOE](A)[sP].[MOE]([5meC])[sP].[MOE](T ) [sP]. [MOE] ([5meC]) P. [TNA] (A) |

(continued)

| CMP ID NO | SEQ ID NO | Base sequence 5'-3' | HELM |
|---|---|---|---|
| #108 | 2 | GCCAGGCTGGTTATGAC TCA | [MOE](G)[sP].[MOE]([5meC])P.[TNA]([5meC])P.[ MOE] (A) [sP]. [MOE] (G) [sP]. [d R] (G) [sP]. [dR] (C) [s P].[dR](T)[sP].[dR](G)[sP].[dR](G)[sP].[dR](T)[s P].[dR](T)[sP].[dR](A)[sP].[dR](T)[sP].[dR](G)[s P]. [MOE] (A) [sP]. [MOE] ([5meC]) P. [TNA] (T) P. [MO E]([5meC])[sP].[MOE](A) |
| #109 | 2 | GCCAGGCTGGTTATGAC TCA | [MOE](G)[sP].[MOE]([5meC])P.[TNA]([5meC])P.[ MOE] (A) [sP]. [MOE] (G) [sP]. [d R] (G) [sP]. [dR] (C) [s P].[dR](T)[sP].[dR](G)[sP].[dR](G)[sP].[dR](T)[s P].[dR](T)[sP].[dR](A)[sP].[dR](T)[sP].[dR](G)[s P]. [MOE] (A) P. [TNA] ([5meC]) P. [MOE] (T) [sP]. [MO E]([5meC])[sP].[MOE](A) |
| #110 | 2 | GCCAGGCTGGTTATGAC TCA | [MOE](G)[sP].[MOE]([5meC])[sP].[MOE]([5meC] ) [sP]. [MOE] (A) [sP]. [MOE] (G) P. [TNA] (G) P. [dR] (C) [sP]. [dR] (T) [sP]. [d R](G) [sP]. [dR] (G) [sP]. [dR] (T) [sP]. [dR] (T) [sP]. [d R] (A) [sP]. [dR] (T) P. [TNA] (G) P. [MOE](A)[sP].[MOE]([5meC])[sP].[MOE](T)[sP].[ MOE] ([5meC]) [sP]. [MOE] (A) |
| #111 | 2 | GCCAGGCTGGTTATGAC TCA | [MOE](G)[sP].[MOE]([5meC])P.[TNA]([5meC])P.[ MOE] (A) [sP]. [MOE] (G) [sP]. [d R] (G) [sP]. [dR] (C) [s P].[dR](T)[sP].[dR](G)[sP].[dR](G)[sP].[dR](T)[s P].[dR](T)[sP].[dR](A)[sP].[dR](T)[sP].[dR](G)[s P].[MOE](A)P.[TNA]([5meC])P.[TNA](T)P.[MOE]([ 5meC])[sP].[MOE](A) |
| #112 | 2 | GCCAGGCTGGTTATGAC TCA | [MOE](G)[sP].[MOE]([5meC])P.[TNA]([5meC])P.[ TNA](A) P.[MOE](G)[sP].[dR](G)[sP].[dR](C)[sP].[ dR](T)[sP].[dR](G)[sP].[dR](G)[sP].[dR](T)[sP].[ dR](T)[sP].[dR](A)[sP].[dR](T)[sP].[dR](G)[sP].[ MOE] (A) P. [TNA] ([5meC]) P. [TNA] (T) P. [MOE] ([5m eC])[sP].[MOE](A) |
| #113 | 2 | GCCAGGCTGGTTATGAC TCA | [MOE](G)P.[TNA]([5meC])P.[MOE]([5meC])[sP].[ MOE] (A) [sP]. [MOE] (G) [sP]. [d R] (G) [sP]. [dR] (C) [sP]. [d R] (T) [sP]. [dR] (G) [sP]. [dR] (G) [sP]. [dR] (T) [s P].[dR](T)[sP].[dR](A)[sP].[dR](T)[sP].[dR](G)[s P].[MOE](A)[sP].[MOE]([5meC])[sP].[MOE](T)[sP ].[MOE]([5meC])[sP].[MOE](A) |
| #114 | 2 | GCCAGGCTGGTTATGAC TCA | [MOE](G)[sP].[MOE]([5meC])P.[TNA]([5meC])P.[ MOE] (A) [sP]. [MOE] (G) [sP]. [d R] (G) [sP]. [dR] (C) [s P].[dR](T)[sP].[dR](G)[sP].[dR](G)[sP].[dR](T)[s P].[dR](T)[sP].[dR](A)[sP].[dR](T)[sP].[dR](G)[s P].[MOE](A)[sP].[MOE]([5meC])[sP].[MOE](T)[sP ].[MOE]([5meC])[sP].[MOE](A) |
| #115 | 2 | GCCAGGCTGGTTATGAC TCA | [MOE](G)[sP].[MOE]([5meC])[sP].[MOE]([5meC] )P.[TNA](A)P.[MOE](G)[sP].[dR](G)[sP].[dR](C)[s P]. [d R] (T) [sP]. [dR] (G) [sP]. [dR] (G) [sP]. [dR] (T) [s P].[dR](T)[sP].[dR](A)[sP].[dR](T)[sP].[dR](G)[s P].[MOE](A)[sP].[MOE]([5meC])[sP].[MOE](T)[sP ]. [MOE] ([5meC]) [sP]. [MOE] (A) |
| #116 | 2 | GCCAGGCTGGTTATGAC TCA | [MOE](G)[sP].[MOE]([5meC])[sP].[MOE]([5meC] ) [sP]. [MOE] (A) P. [TNA] (G) P. [dR] (G) [sP]. [d R] (C) [s P]. [d R] (T) [sP]. [dR] (G) [sP]. [dR] (G) [sP]. [dR] (T) [s P].[dR](T)[sP].[dR](A)[sP].[dR](T)[sP].[dR](G)[s P].[MOE](A)[sP].[MOE]([5meC])[sP].[MOE](T)[sP ]. [MOE] ([5meC]) [sP]. [MOE] (A) |

(continued)

| CMP ID NO | SEQ ID NO | Base sequence 5'-3' | HELM |
|---|---|---|---|
| #117 | 2 | GCCAGGCTGGTTATGAC TCA | [MOE](G)[sP].[MOE]([5meC])[sP].[MOE]([5meC] ) [sP]. [MOE] (A) [sP]. [MOE] (G) P. [TNA] (G) P. [dR] (C) [sP]. [dR] (T) [sP]. [d R](G) [sP]. [dR] (G) [sP]. [dR] (T) [sP].[dR](T)[sP]. [dR](A)[sP].[dR](T)[sP].[dR](G)[ sP]. [MOE] (A) [sP]. [MOE] ([5meC]) [sP]. [MOE] (T) [s P]. [MOE] ([5meC]) [sP]. [MOE] (A) |
| #118 | 2 | GCCAGGCTGGTTATGAC TCA | [MOE](G)[sP].[MOE]([5meC])[sP].[MOE]([5meC] ) [sP]. [MOE] (A) [sP]. [MOE] (G) [sP]. [dR] (G) P. [TNA] ([5meC]) P.[dR](T)[sP].[dR](G)[sP].[dR](G)[sP].[ d R] (T) [sP]. [d R] (T) [sP]. [dR] (A) [sP]. [d R] (T) [sP]. [d R] (G) [sP]. [MOE] (A) [sP]. [MOE] ([5meC]) [sP]. [MO E] (T) [sP]. [MOE] ([5meC]) [sP]. [MOE] (A) |
| #119 | 2 | GCCAGGCTGGTTATGAC TCA | [MOE](G)[sP].[MOE]([5meC])[sP].[MOE]([5meC] )[sP]. [MOE](A)[sP].[MOE](G)[sP].[dR](G)[sP].[dR ](C)P.[TNA](T) P.[dR](G)[sP].[dR](G)[sP].[dR](T)[ sP]. [dR] (T) [sP]. [d R] (A) [sP]. [d R] (T) [sP]. [dR] (G) [sP].[MOE](A)[sP].[MOE]([5meC]) [sP].[MOE](T)[sP ].[MOE]([5meC])[sP].[MOE](A) |
| #120 | 2 | GCCAGGCTGGTTATGAC TCA | [MOE](G)[sP].[MOE]([5meC])[sP].[MOE]([5meC] )[sP]. [MOE](A)[sP].[MOE](G)[sP].[dR](G)[sP].[dR ](C)[sP].[dR] (T)P.[TNA](G)P.[dR](G)[sP].[dR](T)[ sP]. [dR] (T) [sP]. [d R] (A) [sP]. [d R] (T) [sP]. [dR] (G) [s P].[MOE](A)[sP].[MOE]( [5meC])[sP].[MOE](T)[sP ].[MOE]([5meC])[sP].[MOE](A) |
| #121 | 2 | GCCAGGCTGGTTATGAC TCA | [MOE](G)[sP].[MOE]([5meC])[sP].[MOE]([5meC] )[sP]. [MOE](A)[sP].[MOE](G)[sP].[dR](G)[sP].[dR ](C)[sP].[dR] (T)[sP].[dR](G)P.[TNA](G)P.[dR](T)[ sP]. [dR] (T) [sP]. [d R] (A) [sP]. [d R] (T) [sP]. [dR] (G) [s P].[MOE](A)[sP].[MOE]( [5meC])[sP].[MOE](T)[sP ].[MOE]([5meC])[sP].[MOE](A) |
| #122 | 2 | GCCAGGCTGGTTATGAC TCA | [MOE](G)[sP].[MOE]([5meC])[sP].[MOE]([5meC] )[sP]. [MOE](A)[sP].[MOE](G)[sP].[dR](G)[sP].[dR ](C)[sP].[dR] (T)[sP].[dR](G)[sP].[dR](G)P.[TNA]( T)P.[dR](T)[sP].[dR](A) [sP].[dR](T)[sP].[dR](G)[ sP]. [MOE] (A) [sP]. [MOE] ([5meC]) [sP]. [MOE] (T) [s P]. [MOE] ([5meC]) [sP]. [MOE] (A) |
| #123 | 2 | GCCAGGCTGGTTATGAC TCA | [MOE](G)[sP].[MOE]([5meC])[sP].[MOE]([5meC] )[sP]. [MOE](A)[sP].[MOE](G)[sP].[dR](G)[sP].[dR ](C)[sP].[dR] (T)[sP].[dR](G)[sP].[dR](G)[sP].[dR ](T)P.[TNA](T)P.[dR](A) [sP].[dR](T)[sP].[dR](G)[ sP]. [MOE] (A) [sP]. [MOE] ([5meC]) [sP]. [MOE] (T) [s P]. [MOE] ([5meC]) [sP]. [MOE] (A) |
| #124 | 2 | GCCAGGCTGGTTATGAC TCA | [MOE](G)[sP].[MOE]([5meC])[sP].[MOE]([5meC] )[sP]. [MOE](A)[sP].[MOE](G)[sP].[dR](G)[sP].[dR ](C)[sP].[dR] (T)[sP].[dR](G)[sP].[dR](G)[sP].[dR ](T)[sP].[dR](T)P.[TNA] (A)P.[dR](T)[sP].[dR](G)[ sP]. [MOE] (A) [sP]. [MOE] ([5meC]) [sP]. [MOE] (T) [s P]. [MOE] ([5meC]) [sP]. [MOE] (A) |
| #125 | 2 | GCCAGGCTGGTTATGAC TCA | [MOE](G)[sP].[MOE]([5meC])[sP].[MOE]([5meC] )[sP]. [MOE](A)[sP].[MOE](G)[sP].[dR](G)[sP].[dR ](C)[sP].[dR] (T)[sP].[dR](G)[sP].[dR](G)[sP].[dR ](T)[sP].[dR](T)[sP].[dR] (A)P.[TNA](T)P.[dR](G)[ sP].[MOE](A)[sP].[MOE]([5meC]) [sP].[MOE](T)[s P]. [MOE] ([5meC]) [sP]. [MOE] (A) |

(continued)

| CMP ID NO | SEQ ID NO | Base sequence 5'-3' | HELM |
|---|---|---|---|
| #126 | 2 | GCCAGGCTGGTTATGAC TCA | [MOE](G)[sP].[MOE]([5meC])[sP].[MOE]([5meC] )[sP].[MOE](A)[sP].[MOE](G)[sP].[dR](G)[sP].[dR ](C)[sP].[dR](T)[sP].[dR](G)[sP].[dR](G)[sP].[dR ](T)[sP].[dR](T)[sP].[dR](A)[sP].[dR](T)P.[TNA]( G)P.[MOE](A)[sP].[MOE]([5meC])[sP].[MOE](T)[s P].[MOE]([5meC])[sP].[MOE](A) |
| #127 | 2 | GCCAGGCTGGTTATGAC TCA | [MOE](G)[sP].[MOE]([5meC])[sP].[MOE]([5meC] )[sP].[MOE](A)[sP].[MOE](G)[sP].[dR](G)[sP].[dR ](C)[sP].[dR](T)[sP].[dR](G)[sP].[dR](G)[sP].[dR ](T)[sP].[dR](T)[sP].[dR](A)[sP].[dR](T)[sP].[dR] (G) P. [TNA] (A) P. [MOE] ([5meC])[sP]. [MOE] (T) [sP] .[MOE]([5meC])[sP].[MOE](A) |
| #128 | 2 | GCCAGGCTGGTTATGAC TCA | [MOE](G)[sP].[MOE]([5meC])[sP].[MOE]([5meC] )[sP].[MOE](A)[sP].[MOE](G)[sP].[dR](G)[sP].[dR ](C)[sP].[dR](T)[sP].[dR](G)[sP].[dR](G)[sP].[dR ](T)[sP].[dR](T)[sP].[dR](A)[sP].[dR](T)[sP].[dR] (G) [sP]. [MOE] (A) P. [TNA] ([5meC]) P. [MOE] (T) [sP] .[MOE]([5meC])[sP].[MOE](A) |
| #129 | 2 | GCCAGGCTGGTTATGAC TCA | [MOE](G)[sP].[MOE]([5meC])[sP].[MOE]([5meC] )[sP].[MOE](A)[sP].[MOE](G)[sP].[dR](G)[sP].[dR ](C)[sP].[dR](T)[sP].[dR](G)[sP].[dR](G)[sP].[dR ](T)[sP].[dR](T)[sP].[dR](A)[sP].[dR](T)[sP].[dR] (G)[sP].[MOE](A)[sP].[MOE]( [5meC])P.[TNA](T)P .[MOE]([5meC])[sP].[MOE](A) |
| #130 | 2 | GCCAGGCTGGTTATGAC TCA | [MOE](G)[sP].[MOE]([5meC])[sP].[MOE]([5meC] )[sP].[MOE](A)[sP].[MOE](G)[sP].[dR](G)[sP].[dR ](C)[sP].[dR](T)[sP].[dR](G)[sP].[dR](G)[sP].[dR ](T)[sP].[dR](T)[sP].[dR](A)[sP].[dR](T)[sP].[dR] (G)[sP].[MOE](A)[sP].[MOE]( [5meC])[sP].[MOE]( T)P.[TNA]([5meC])P.[MOE](A) |
| #131 | 2 | GCCAGGCTGGTTATGAC TCA | [MOE](G)[sP].[MOE]([5meC])[sP].[MOE]([5meC] )[sP].[MOE](A)[sP].[MOE](G)[sP].[dR](G)[sP].[dR ](C)[sP].[dR](T)[sP].[dR](G)[sP].[dR](G)[sP].[dR ](T)[sP].[dR](T)[sP].[dR](A)[sP].[dR](T)[sP].[dR] (G)[sP].[MOE](A)[sP].[MOE]( [5meC])[sP].[MOE]( T) [sP]. [MOE] ([5meC]) P. [TNA] (A) |
| #132 | 2 | GCCAGGCTGGTTATGAC TCA | [TNA](G)P.[MOE]([5meC])[sP].[MOE]([5meC])[s P].[MOE](A)[sP].[MOE](G)[sP].[dR](G)[sP].[dR](C)[sP].[dR](T)[sP].[dR](G)[sP].[dR](G)[sP].[dR]( T)[sP].[dR](T)[sP].[dR](A)[sP].[dR](T)[sP].[dR]( G)[sP].[MOE](A)[sP].[MOE]([5meC])[sP].[MOE](T ) [sP]. [MOE] ([5meC]) [sP]. [MOE] (A) |
| #133 | 2 | GCCAGGCTGGTTATGAC TCA | [MOE](G)P.[TNA]([5meC])[sP].[MOE]([5meC])[s P].[MOE](A)[sP].[MOE](G)[sP].[dR](G)[sP].[dR]( C)[sP].[dR](T)[sP].[dR](G)[sP].[dR](G)[sP].[dR]( T)[sP].[dR](T)[sP].[dR](A)[sP].[dR](T)[sP].[dR]( G)[sP].[MOE](A)[sP].[MOE]([5meC])[sP].[MOE](T )[sP].[MOE] ([5meC])[sP].[MOE](A) |
| #134 | 2 | GCCAGGCTGGTTATGAC TCA | [MOE](G)[sP]. [MOE] ([5meC])P.[TNA]([5meC])[s P].[MOE](A)[sP].[MOE](G)[sP].[dR](G)[sP].[dR]( C)[sP].[dR](T)[sP].[dR](G)[sP].[dR](G)[sP].[dR]( T)[sP].[dR](T)[sP].[dR](A)[sP].[dR](T)[sP].[dR]( G)[sP].[MOE](A)[sP].[MOE]([5meC])[sP].[MOE](T )[sP].[MOE]([5meC])[sP].[MOE](A) |

(continued)

| CMP ID NO | SEQ ID NO | Base sequence 5'-3' | HELM |
|---|---|---|---|
| #135 | 2 | GCCAGGCTGGTTATGAC TCA | [MOE](G)[sP].[MOE]([5meC])[sP].[MOE]([5meC] )P.[TNA](A)[sP].[MOE](G)[sP].[dR](G)[sP].[dR](C )[sP].[dR](T)[sP].[dR](G)[sP].[dR](G)[sP].[dR](T )[sP].[dR](T)[sP].[dR](A)[sP].[dR](T)[sP].[dR](G) [sP].[MOE](A)[sP].[MOE]([5meC])[sP].[MOE](T)[ sP]. [MOE] ([5meC]) [sP]. [MOE] (A) |
| #136 | 2 | GCCAGGCTGGTTATGAC TCA | [MOE](G)[sP].[MOE]([5meC])[sP].[MOE]([5meC] )[sP].[MOE](A)P.[TNA](G)[sP].[dR](G)[sP].[dR](C )[sP].[dR](T)[sP].[dR](G)[sP].[dR](G)[sP].[dR](T )[sP].[dR](T)[sP].[dR](A)[sP].[dR](T)[sP].[dR](G) [sP].[MOE](A)[sP].[MOE]([5meC])[sP].[MOE](T)[ sP]. [MOE] ([5meC]) [sP]. [MOE] (A) |
| #137 | 2 | GCCAGGCTGGTTATGAC TCA | [MOE](G)[sP].[MOE]([5meC])[sP].[MOE]([5meC] )[sP].[MOE](A)[sP].[MOE](G)P.[TNA](G)[sP].[dR] (C)[sP].[dR](T)[sP].[dR](G)[sP].[dR](G)[sP].[dR] (T)[sP].[dR](T)[sP].[dR](A)[sP].[dR](T)[sP].[dR]( G)[sP].[MOE](A)[sP].[MOE]([5meC])[sP].[MOE](T ) [sP]. [MOE] ([5meC]) [sP]. [MOE] (A) |
| #138 | 2 | GCCAGGCTGGTTATGAC TCA | [MOE](G)[sP].[MOE]([5meC])[sP].[MOE]([5meC] ) [sP].[MOE] (A) [sP]. [MOE] (G) [sP]. [dR] (G) P. [TNA] ([5meC])[sP].[dR](T)[sP].[dR](G)[sP].[dR](G)[sP ]. [dR] (T) [sP]. [d R](T) [sP]. [dR] (A) [sP]. [d R] (T) [sP] .[dR](G)[sP].[MOE](A)[sP].[MOE]([5meC])[sP].[ MOE] (T) [sP]. [MOE] ([5meC])[sP]. [MOE] (A) |
| #139 | 2 | GCCAGGCTGGTTATGAC TCA | [MOE](G)[sP].[MOE]([5meC])[sP].[MOE]([5meC] )[sP].[MOE](A)[sP].[MOE](G)[sP].[dR](G)[sP].[dR ](C)P.[TNA](T)[sP].[dR](G)[sP].[dR](G)[sP].[dR]( T)[sP].[dR](T)[sP].[dR](A)[sP].[dR](T)[sP].[dR]( G)[sP].[MOE](A)[sP].[MOE]([5meC])[sP].[MOE](T ) [sP]. [MOE] ([5meC]) [sP]. [MOE] (A) |
| #140 | 2 | GCCAGGCTGGTTATGAC TCA | [MOE](G)[sP].[MOE]([5meC])[sP].[MOE]([5meC] )[sP].[MOE](A)[sP].[MOE](G)[sP].[dR](G)[sP].[dR ](C)[sP].[dR](T)P.[TNA](G)[sP].[dR](G)[sP].[dR]( T)[sP].[dR](T)[sP].[dR](A)[sP].[dR](T)[sP].[dR]( G)[sP].[MOE](A)[sP].[MOE]( [5meC])[sP].[MOE](T ) [sP]. [MOE] ([5meC]) [sP]. [MOE] (A) |
| #141 | 2 | GCCAGGCTGGTTATGAC TCA | [MOE](G)[sP].[MOE]([5meC])[sP].[MOE]([5meC] )[sP].[MOE](A)[sP].[MOE](G)[sP].[dR](G)[sP].[dR ](C)[sP].[dR](T)[sP].[dR](G)P.[TNA](G)[sP].[dR]( T)[sP].[dR](T)[sP].[dR](A)[sP].[dR](T)[sP].[dR]( G)[sP].[MOE](A)[sP].[MOE]( [5meC])[sP].[MOE](T ) [sP]. [MOE] ([5meC]) [sP]. [MOE] (A) |
| #142 | 2 | GCCAGGCTGGTTATGAC TCA | [MOE](G)[sP].[MOE]([5meC])[sP].[MOE]([5meC] )[sP].[MOE](A)[sP].[MOE](G)[sP].[dR](G)[sP].[dR ](C)[sP].[dR](T)[sP].[dR](G)[sP].[dR](G)P.[TNA]( T)[sP].[dR](T)[sP].[dR](A)[sP].[dR](T)[sP].[dR]( G)[sP].[MOE](A)[sP].[MOE]( [5meC])[sP].[MOE](T ) [sP]. [MOE] ([5meC]) [sP]. [MOE] (A) |
| #143 | 2 | GCCAGGCTGGTTATGAC TCA | [MOE](G)[sP].[MOE]([5meC])[sP].[MOE]([5meC] )[sP].[MOE](A)[sP].[MOE](G)[sP].[dR](G)[sP].[dR ](C)[sP].[dR](T)[sP].[dR](G)[sP].[dR](G)[sP].[dR ](T)P.[TNA](T)[sP].[dR](A)[sP].[dR](T)[sP].[dR]( G)[sP].[MOE](A)[sP].[MOE]( [5meC])[sP].[MOE](T ) [sP]. [MOE] ([5meC]) [sP]. [MOE] (A) |

(continued)

| CMP ID NO | SEQ ID NO | Base sequence 5'-3' | HELM |
|---|---|---|---|
| #144 | 2 | GCCAGGCTGGTTATGAC TCA | [MOE](G)[sP].[MOE]([5meC])[sP].[MOE]([5meC] )[sP]. [MOE](A)[sP].[MOE](G)[sP].[dR](G)[sP].[dR ](C)[sP].[dR] (T)[sP].[dR](G)[sP].[dR](G)[sP].[dR ](T)[sP].[dR](T)P.[TNA] (A)[sP].[dR](T)[sP].[dR]( G)[sP].[MOE](A)[sP].[MOE]( [5meC])[sP].[MOE](T ) [sP]. [MOE] ([5meC]) [sP]. [MOE] (A) |
| #145 | 2 | GCCAGGCTGGTTATGAC TCA | [MOE](G)[sP].[MOE]([5meC])[sP].[MOE]([5meC] )[sP]. [MOE](A)[sP].[MOE](G)[sP].[dR](G)[sP].[dR ](C)[sP].[dR] (T)[sP].[dR](G)[sP].[dR](G)[sP].[dR ](T)[sP].[dR](T)[sP].[dR] (A)P.[TNA](T)[sP].[dR]( G)[sP].[MOE](A)[sP].[MOE]( [5meC])[sP].[MOE](T ) [sP]. [MOE] ([5meC]) [sP]. [MOE] (A) |
| #146 | 2 | GCCAGGCTGGTTATGAC TCA | [MOE](G)[sP].[MOE]([5meC])[sP].[MOE]([5meC] )[sP]. [MOE](A)[sP].[MOE](G)[sP].[dR](G)[sP].[dR ](C)[sP].[dR] (T)[sP].[dR](G)[sP].[dR](G)[sP].[dR ](T)[sP].[dR](T)[sP].[dR] (A)[sP].[dR](T)P.[TNA]( G)[sP].[MOE](A)[sP].[MOE]( [5meC])[sP].[MOE](T ) [sP]. [MOE] ([5meC]) [sP]. [MOE] (A) |
| #147 | 2 | GCCAGGCTGGTTATGAC TCA | [MOE](G)[sP].[MOE]([5meC])[sP].[MOE]([5meC] )[sP]. [MOE](A)[sP].[MOE](G)[sP].[dR](G)[sP].[dR ](C)[sP].[dR] (T)[sP].[dR](G)[sP].[dR](G)[sP].[dR ](T)[sP].[dR](T)[sP].[dR] (A)[sP].[dR](T)[sP].[dR] (G)P.[TNA](A)[sP].[MOE]([5meC]) [sP].[MOE](T)[ sP]. [MOE] ([5meC]) [sP]. [MOE] (A) |
| #148 | 2 | GCCAGGCTGGTTATGAC TCA | [MOE](G)[sP].[MOE]([5meC])[sP].[MOE]([5meC] )[sP]. [MOE](A)[sP].[MOE](G)[sP].[dR](G)[sP].[dR ](C)[sP].[dR] (T)[sP].[dR](G)[sP].[dR](G)[sP].[dR ](T)[sP].[dR](T)[sP].[dR] (A)[sP].[dR](T)[sP].[dR] (G)[sP].[MOE](A)P.[TNA]([5meC]) [sP].[MOE](T)[ sP]. [MOE] ([5meC]) [sP]. [MOE] (A) |
| #149 | 2 | GCCAGGCTGGTTATGAC TCA | [MOE](G)[sP].[MOE]([5meC])[sP].[MOE]([5meC] )[sP]. [MOE](A)[sP].[MOE](G)[sP].[dR](G)[sP].[dR ](C)[sP].[dR] (T)[sP].[dR](G)[sP].[dR](G)[sP].[dR ](T)[sP].[dR](T)[sP].[dR] (A)[sP].[dR](T)[sP].[dR] (G)[sP].[MOE](A)[sP].[MOE]( [5meC])P.[TNA](T)[ sP].[MOE]([5meC])[sP].[MOE](A) |
| #150 | 2 | GCCAGGCTGGTTATGAC TCA | [MOE](G)[sP].[MOE]([5meC])[sP].[MOE]([5meC] )[sP]. [MOE](A)[sP].[MOE](G)[sP].[dR](G)[sP].[dR ](C)[sP].[dR] (T)[sP].[dR](G)[sP].[dR](G)[sP].[dR ](T)[sP].[dR](T)[sP].[dR] (A)[sP].[dR](T)[sP].[dR] (G)[sP].[MOE](A)[sP].[MOE]( [5meC])[sP].[MOE]( T)P.[TNA]([5meC])[sP].[MOE](A) |
| CONTROL #3 | 3 | GGCATATGCAGATAATG TTC | [MOE](G)[sP].[MOE](G)[sP].[MOE]([5meC])[sP].[ MOE](A) [sP].[MOE](T)[sP].[dR](A)[sP].[dR](T)[sP].[dR](G)[sP].[dR] (C)[sP].[dR](A)[sP].[dR](G)[sP ]. [dR] (A) [sP]. [dR] (T) [sP]. [d R] (A) [sP]. [dR] (A) [sP] .[MOE](T)[sP].[MOE](G)[sP]. [MOE](T)[sP].[MOE] (T) [sP]. [MOE] ([5meC]) |
| #151 | 3 | GGCATATGCAGATAATG TTC | [MOE](G)[sP].[MOE](G)[sP].[MOE]([5meC])[sP].[ MOE](A) [sP].[MOE](T)P.[TNA](A)[sP].[dR](T)[sP] .[dR](G)[sP].[dR] (C)[sP].[dR](A)[sP].[dR](G)[sP] .[dR](A)[sP].[dR](T)[sP].[dR] (A)[sP].[dR](A)[sP]. [MOE](T)[sP].[MOE](G)[sP].[MOE](T) [sP].[MOE]( T)[sP].[MOE]([5meC] |

(continued)

| CMP ID NO | SEQ ID NO | Base sequence 5'-3' | HELM |
|---|---|---|---|
| #152 | 3 | GGCATATGCAGATAATG TTC | [MOE](G)[sP].[MOE](G)[sP].[MOE]([5meC])[sP].[ MOE](A)[sP].[MOE](T)P.[TNA](A)P.[TNA](T)[sP].[ dR](G)[sP].[dR](C)[sP].[dR](A)[sP].[dR](G)[sP].[ dR](A)[sP].[dR](T)[sP].[dR](A)[sP].[dR](A)[sP].[ MOE](T)[sP].[MOE](G)[sP].[MOE](T)[sP].[MOE](T )[sP].[MOE]([5meC]) |
| #153 | 3 | GGCATATGCAGATAATG TTC | [MOE](G)[sP].[MOE](G)[sP].[MOE]([5meC])[sP].[ MOE](A)[sP].[MOE](T)P.[TNA](A)P.[TNA](T)P.[TN A] (G) [sP]. [dR](C) [sP]. [dR] (A) [sP]. [dR] (G) [sP]. [d R](A)[sP].[dR](T)[sP].[dR](A)[sP].[dR](A)[sP].[M OE](T)[sP].[MOE](G)[sP].[MOE](T)[sP].[MOE](T)[ sP].[MOE]([5meC]) |
| #154 | 3 | GGCATATGCAGATAATG TTC | [MOE](G)[sP].[MOE](G)[sP].[MOE]([5meC])[sP].[ MOE](A)[sP].[MOE](T)P.[TNA](A)P.[TNA](T)P.[TN A] (G) P. [TNA]([5meC]) [sP]. [dR] (A) [sP]. [dR] (G) [s P].[dR](A)[sP].[dR](T)[sP].[dR](A)[sP].[dR](A)[s P]. [MOE] (T) [sP]. [MOE] (G) [sP]. [MOE] (T) [sP]. [MO E] (T) [sP]. [MOE] ([5meC]) |
| #155 | 3 | GGCATATGCAGATAATG TTC | [MOE] (G) [sP]. [MOE] (G) [sP]. [MOE] ([5meC]) [sP]. MOE](A)[sP].[MOE](T)P.[TNA](A)P.[TNA](T)P.[TN A](G)P.[TNA]([5meC])P.[TNA](A)[sP].[dR](G)[sP] .[dR](A)[sP].[dR](T)[sP].[dR](A)[sP].[dR](A)[sP]. [MOE](T)[sP].[MOE](G)[sP].[MOE](T)[sP].[MOE]( T)[sP].[MOE]([5meC]) |
| #156 | 3 | GGCATATGCAGATAATG TTC | [TNA] (G) [sP]. [TNA] (G) [sP]. [TNA] ([5meC]) [sP]. [T NA](A) P. [TNA] (T) [sP]. [d R] (A) [sP]. [d R] (T) [sP]. [d R] (G)[sP]. [d R] (C) [sP]. [dR] (A) [sP]. [d R] (G) [sP]. [d R](A)[sP].[dR](T)[sP].[dR](A)[sP].[dR](A)[sP].[MOE](T)[sP].[MOE](G)[sP].[MOE](T)[sP].[MOE](T)[ sP].[MOE]([5meC]) |
| #157 | 3 | GGCATATGCAGATAATG TTC | [TNA](G)[sP].[TNA](G)[sP].[TNA]([5meC])P.[TNA ](A)P.[TNA](T)[sP].[dR](A)[sP].[dR](T)[sP].[dR]( G)[sP].[dR](C)[sP].[dR](A)[sP].[dR](G)[sP].[dR]( A)[sP].[dR](T)[sP].[dR](A)[sP].[dR](A)[sP].[MOE] (T)[sP].[MOE](G)[sP].[MOE](T)[sP].[MOE](T)[sP]. [MOE]([5meC]) |
| #158 | 3 | GGCATATGCAGATAATG TTC | [TNA](G)[sP].[TNA](G)P.[TNA]([5meC])P.[TNA]( A)P.[TNA](T) [sP]. [dR] (A) [sP]. [d R] (T) [sP]. [dR] (G) [sP].[dR](C)[sP].[dR](A)[sP].[dR](G)[sP].[dR](A) [sP].[dR](T)[sP].[dR](A)[sP].[dR](A)[sP].[MOE](T )[sP].[MOE](G)[sP].[MOE](T)[sP].[MOE](T)[sP].[ MOE]([5meC]) |
| #159 | 3 | GGCATATGCAGATAATG TTC | [TNA](G)P.[TNA](G)P.[TNA]([5meC])P.[TNA](A)P .[TNA](T)[sP].[dR](A)[sP].[dR](T)[sP].[dR](G)[sP ].[dR](C)[sP].[dR](A)[sP].[dR](G)[sP].[dR](A)[sP ]. [dR] (T) [sP]. [d R] (A) [sP]. [d R] (A) [sP]. [MOE] (T) [s P]. [MOE] (G) [sP]. [MOE] (T) [sP]. [MOE] (T) [sP]. [MO E]([5meC]) |
| #160 | 3 | GGCATATGCAGATAATG TTC | [TNA](G)P.[TNA](G)P.[TNA]([5meC])P.[TNA](A)P .[TNA](T)P.[TNA](A)[sP].[dR](T)[sP].[dR](G)[sP]. [dR](C)[sP].[dR](A)[sP].[dR](G)[sP].[dR](A)[sP]. [dR](T)[sP].[dR](A)[sP].[dR](A)[sP].[MOE](T)[sP] .[MOE](G)[sP].[MOE](T)[sP].[MOE](T)[sP].[MOE] ([5meC]) |

(continued)

| CMP ID NO | SEQ ID NO | Base sequence 5'-3' | HELM |
|---|---|---|---|
| #161 | 3 | GGCATATGCAGATAATG TTC | [TNA](G)P.[TNA](G)P.[TNA]([5meC])P.[TNA](A)P .[TNA](T) P.[TNA](A)P.[TNA](T)[sP].[dR](G)[sP].[ dR](C)[sP].[dR](A) [sP].[dR](G)[sP].[dR](A)[sP].[ d R] (T) [sP]. [dR] (A) [sP]. [d R] (A) [sP]. [MOE] (T) [sP]. [MOE](G)[sP].[MOE](T)[sP]. [MOE](T)[sP].[MOE]( [5meC]) |
| #162 | 3 | GGCATATGCAGATAATG TTC | [TNA](G)P.[TNA](G)P.[TNA]([5meC])P.[TNA](A)P .[TNA](T) P.[TNA](A)P.[TNA](T)P.[TNA](G)[sP].[d R] (C) [sP]. [d R] (A) [sP]. [dR] (G) [sP]. [d R] (A) [sP]. [d R](T)[sP].[dR](A)[sP].[dR] (A)[sP].[MOE](T)[sP].[ MOE](G)[sP].[MOE](T)[sP].[MOE](T) [sP].[MOE]([ 5meC]) |
| #163 | 3 | GGCATATGCAGATAATG TTC | [TNA](G)P.[TNA](G)P.[TNA]([5meC])P.[TNA](A)P .[TNA](T) P.[TNA](A)P.[TNA](T)P.[TNA](G)P.[TNA] ([5meC])[sP].[dR] (A)[sP].[dR](G)[sP].[dR](A)[sP ].[dR](T)[sP].[dR](A)[sP].[dR] (A)[sP].[MOE](T)[s P].[MOE](G)[sP].[MOE](T)[sP].[MOE](T) [sP].[MO E]([5meC]) |
| #164 | 3 | GGCATATGCAGATAATG TTC | [TNA](G)P.[TNA](G)P.[TNA]([5meC])P.[TNA](A)P .[TNA](T) P.[TNA](A)P.[TNA](T)P.[TNA](G)P.[TNA] ([5meC])P.[TNA] (A)[sP].[dR](G)[sP].[dR](A)[sP]. [dR](T)[sP].[dR](A)[sP].[dR] (A)[sP].[MOE](T)[sP] .[MOE](G)[sP].[MOE](T)[sP].[MOE](T) [sP].[MOE] ([5meC]) |
| #165 | 3 | GGCATATGCAGATAATG TTC | [TNA](G)P.[TNA](G)P.[TNA]([5meC])P.[TNA](A)P .[TNA](T) P.[TNA](A)P.[TNA](T)P.[TNA](G)P.[TNA] ([5meC])P.[TNA] (A)[sP].[dR](G)[sP].[dR](A)[sP]. [dR](T)[sP].[dR](A)[sP].[dR] (A)[sP].[dR](T)[sP].[ MOE](G)[sP].[MOE](T)[sP].[MOE](T) [sP].[MOE]([ 5meC]) |
| #166 | 3 | GGCATATGCAGATAATG TTC | [TNA](G)P.[TNA](G)P.[TNA]([5meC])P.[TNA](A)P .[TNA](T) P.[TNA](A)P.[TNA](T)P.[TNA](G)P.[TNA] ([5meC])P.[TNA] (A)[sP].[dR](G)[sP].[dR](A)[sP]. [dR](T)[sP].[dR](A)[sP].[dR] (A)[sP].[dR](T)[sP].[ dR](G)[sP].[MOE](T)[sP].[MOE](T)[sP]. [MOE]([5 meC]) |
| #167 | 3 | GGCATATGCAGATAATG TTC | [TNA](G)P.[TNA](G)P.[TNA]([5meC])P.[TNA](A)P .[TNA](T) P.[TNA](A)P.[TNA](T)P.[TNA](G)P.[TNA] ([5meC])P.[TNA] (A)P.[TNA](G)[sP].[dR](A)[sP].[ dR](T)[sP].[dR](A)[sP].[dR] (A)[sP].[dR](T)[sP].[ MOE](G)[sP].[MOE](T)[sP].[MOE](T) [sP].[MOE]([ 5meC]) |
| #168 | 3 | GGCATATGCAGATAATG TTC | [TNA](G)P.[TNA](G)P.[TNA]([5meC])P.[TNA](A)P .[TNA](T) P.[TNA](A)P.[TNA](T)P.[TNA](G)P.[TNA] ([5meC])P.[TNA] (A)P.[TNA](G)[sP].[dR](A)[sP].[ d R] (T) [sP]. [dR] (A) [sP]. [d R] (A) [sP]. [dR] (T) [sP]. [d R](G)[sP].[MOE](T)[sP].[MOE](T) [sP].[MOE]([5m eC]) |
| #169 | 3 | GGCATATGCAGATAATG TTC | [TNA] (G) P. [MOE] (G) [sP]. [MOE] ([5meC]) [sP]. [MO E](A) [sP].[MOE](T)[sP].[dR](A)[sP].[dR](T)[sP].[dR](G)[sP].[dR] (C)[sP].[dR](A)[sP].[dR](G)[sP].[ dR](A)[sP].[dR](T)[sP].[dR] (A)[sP].[dR](A)[sP].[ MOE](T)[sP].[MOE](G)[sP].[MOE](T) [sP].[MOE](T )P.[TNA]([5meC]) |

(continued)

| CMP ID NO | SEQ ID NO | Base sequence 5'-3' | HELM |
|---|---|---|---|
| #170 | 3 | GGCATATGCAGATAATG TTC | [TNA] (G) P. [MOE] (G) [sP]. [MOE] ([5meC]) [sP]. [MO E](A) [sP].[MOE](T)[sP].[dR](A)[sP].[dR](T)[sP].[ dR](G)[sP].[dR] (C)[sP].[dR](A)[sP].[dR](G)[sP].[ dR](A)[sP].[dR](T)[sP].[dR] (A)[sP].[dR](A)[sP].[ MOE] (T) [sP]. [MOE] (G) [sP]. [MOE] (T) P. [TNA] (T) P. [TNA]([5meC]) |
| #171 | 3 | GGCATATGCAGATAATG TTC | [TNA](G)P.[TNA](G)P.[MOE]([5meC])[sP].[MOE]( A) [sP]. [MOE] (T) [sP]. [dR] (A) [sP]. [dR] (T) [sP]. [dR] (G)[sP].[dR] (C)[sP].[dR](A)[sP].[dR](G)[sP].[dR] (A)[sP].[dR](T)[sP].[dR] (A)[sP].[dR](A)[sP].[MOE ](T)[sP].[MOE](G)[sP].[MOE](T)P. [TNA](T)P.[TNA ]([5meC]) |
| #172 | 3 | GGCATATGCAGATAATG TTC | [TNA](G)P.[TNA](G)P.[MOE]([5meC])[sP].[MOE]( A)[sP]. [MOE](T)P.[TNA](A)[sP].[dR](T)[sP].[dR]( G)[sP].[dR](C) [sP].[dR](A)[sP].[dR](G)[sP].[dR]( A) [sP]. [dR] (T) [sP]. [dR] (A) [sP]. [dR] (A) [sP]. [MOE] (T)[sP].[MOE](G)[sP].[MOE](T) P.[TNA](T)P.[TNA] ([5meC]) |
| #173 | 3 | GGCATATGCAGATAATG TTC | [TNA](G)P.[TNA](G)P.[MOE]([5meC])[sP].[MOE]( A)[sP]. [MOE](T)P.[TNA](A)P.[TNA](T)[sP].[dR](G )[sP].[dR](C) [sP].[dR](A)[sP].[dR](G)[sP].[dR](A )[sP].[dR](T)[sP].[dR](A) [sP].[dR](A)[sP].[MOE]( T)[sP].[MOE](G)[sP].[MOE](T)P. [TNA](T)P.[TNA]( [5meC]) |
| #174 | 3 | GGCATATGCAGATAATG TTC | [TNA](G)P.[TNA](G)P.[MOE]([5meC])[sP].[MOE]( A)[sP]. [MOE](T)P.[TNA](A)P.[TNA](T)P.[TNA](G)[ sP].[dR](C)[sP]. [dR](A)[sP].[dR](G)[sP].[dR](A)[ sP]. [dR] (T) [sP]. [dR] (A) [sP]. [d R] (A) [sP]. [MOE] (T) [sP]. [MOE] (G) [sP]. [MOE] (T) P. [TNA] (T) P. [TNA] ([5 meC]) |
| #175 | 3 | GGCATATGCAGATAATG TTC | [TNA](G)P.[TNA](G)P.[MOE]([5meC])[sP].[MOE]( A)[sP]. [MOE](T)P.[TNA](A)P.[TNA](T)P.[TNA](G) P.[TNA]([5meC]) [sP].[dR](A)[sP].[dR](G)[sP].[dR ](A)[sP].[dR](T)[sP].[dR](A) [sP].[dR](A)[sP].[MOE](T)[sP].[MOE](G)[sP].[MOE](T)P. [TNA](T)P.[TN A]([5meC]) |
| #176 | 3 | GGCATATGCAGATAATG TTC | [TNA](G)P.[TNA](G)P.[MOE]([5meC])[sP].[MOE]( A)[sP]. [MOE](T)P.[TNA](A)P.[TNA](T)P.[TNA](G) P.[TNA]([5meC]) P.[TNA](A)[sP].[dR](G)[sP].[dR] (A)[sP].[dR](T)[sP].[dR](A) [sP].[dR](A)[sP].[MOE ](T)[sP].[MOE](G)[sP].[MOE](T)P. [TNA](T)P.[TNA ]([5meC]) |
| #177 | 3 | GGCATATGCAGATAATG TTC | [TNA](G)P.[TNA](G)P.[TNA]([5meC])P.[TNA](A)P .[TNA](T) [sP].[dR](A)[sP].[dR](T)[sP].[dR](G)[sP ].[dR](C)[sP].[dR](A) [sP].[dR](G)[sP].[dR](A)[sP ].[dR](T)[sP].[dR](A)[sP].[dR](A) [sP].[TNA](T)P.[ TNA](G)P.[TNA](T)P.[TNA](T)P.[TNA]( [5meC]) |
| #178 | 3 | GGCATATGCAGATAATG TTC | [TNA](G)[sP].[TNA](G)P.[TNA]([5meC])P.[TNA]( A) P. [TNA] (T) [sP]. [dR] (A) [sP]. [d R] (T) [sP]. [dR] (G) [sP].[dR](C)[sP]. [dR](A)[sP].[dR](G)[sP].[dR](A) [sP].[dR](T)[sP].[dR](A)[sP]. [dR](A)[sP].[TNA](T )P.[TNA](G)P.[TNA](T)P.[TNA](T)[sP]. [TNA]([5m eC]) |

(continued)

| CMP ID NO | SEQ ID NO | Base sequence 5'-3' | HELM |
|---|---|---|---|
| #179 | 3 | GGCATATGCAGATAATG TTC | [MOE](G)[sP].[MOE](G)[sP].[MOE]([5meC])[sP].[ MOE](A) [sP].[MOE](T)[sP].[dR](A)[sP].[dR](T)[sP ].[dR](G)[sP].[dR] (C)[sP].[dR](A)[sP].[dR](G)[sP ].[dR](A)[sP].[dR](T)[sP].[dR] (A)[sP].[dR](A)[sP] .[TNA](T)P.[TNA](G)P.[TNA](T)P.[TNA] (T)P.[TNA] ([5meC]) |
| #180 | 3 | GGCATATGCAGATAATG TTC | [MOE](G)[sP].[MOE](G)[sP].[MOE]([5meC])[sP].[ MOE](A) [sP].[MOE](T)[sP].[dR](A)[sP].[dR](T)[sP ].[dR](G)[sP].[dR] (C)[sP].[dR](A)[sP].[dR](G)[sP ]. [dR] (A) [sP]. [dR] (T) [sP]. [dR] (A) [sP]. [TNA] (A) P. [TNA](T)P.[TNA](G)P.[TNA](T)P. [TNA](T)P.[TNA] ([5meC]) |
| #181 | 3 | GGCATATGCAGATAATG TTC | [MOE](G)[sP].[MOE](G)[sP].[MOE]([5meC])[sP].[ MOE](A) [sP].[MOE](T)[sP].[dR](A)[sP].[dR](T)[sP ].[dR](G)[sP].[dR] (C)[sP].[dR](A)[sP].[dR](G)[sP ].[dR](A)[sP].[dR](T)[sP]. [TNA](A)P.[TNA](A)P.[T NA](T)P.[TNA](G)P.[TNA](T)P. [TNA](T)P.[TNA]([ 5meC]) |
| #182 | 3 | GGCATATGCAGATAATG TTC | [MOE](G)[sP].[MOE](G)[sP].[MOE]([5meC])[sP].[ MOE](A) [sP].[MOE](T)[sP].[dR](A)[sP].[dR](T)[sP ].[dR](G)[sP].[dR] (C)[sP].[dR](A)[sP].[dR](G)[sP ].[dR](A)[sP].[TNA](T)P. [TNA](A)P.[TNA](A)P.[TN A](T)P.[TNA](G)P.[TNA](T)P. [TNA](T)P.[TNA]([5 meC]) |
| #183 | 3 | GGCATATGCAGATAATG TTC | [MOE](G)[sP].[MOE](G)[sP].[MOE]([5meC])[sP].[ MOE](A) [sP].[MOE](T)[sP].[dR](A)[sP].[dR](T)[sP ].[dR](G)[sP].[dR] (C)[sP].[dR](A)[sP].[dR](G)[sP ]. [dR] (A) [sP]. [dR] (T) [sP]. [d R] (A) [sP]. [dR] (A) [sP] .[MOE](T)[sP].[MOE](G)[sP]. [MOE](T)[sP].[TNA]( T)P.[TNA]([5meC]) |
| #184 | 3 | GGCATATGCAGATAATG TTC | [MOE](G)[sP].[MOE](G)[sP].[MOE]([5meC])[sP].[ MOE](A) [sP].[MOE](T)[sP].[dR](A)[sP].[dR](T)[sP ].[dR](G)[sP].[dR] (C)[sP].[dR](A)[sP].[dR](G)[sP ]. [dR] (A) [sP]. [dR] (T) [sP]. [d R] (A) [sP]. [dR] (A) [sP] .[MOE](T)[sP].[MOE](G)[sP]. [MOE](T)P.[TNA](T) P.[TNA]([5meC]) |
| #185 | 3 | GGCATATGCAGATAATG TTC | [MOE] (G) [sP]. [MOE] (G) P. [TNA] ([5meC]) [sP]. [MO E](A) [sP].[MOE](T)[sP].[dR](A)[sP].[dR](T)[sP].[ dR](G)[sP].[dR] (C)[sP].[dR](A)[sP].[dR](G)[sP].[ dR](A)[sP].[dR](T)[sP].[dR] (A)[sP].[dR](A)[sP].[ MOE] (T) [sP]. [MOE] (G) P. [TNA] (T) [sP]. [MOE] (T) [s P].[MOE]([5meC]) |
| #186 | 3 | GGCATATGCAGATAATG TTC | [MOE](G)[sP].[MOE](G)P.[TNA]([5meC])P.[MOE]( A) [sP]. [MOE] (T) [sP]. [dR] (A) [sP]. [d R] (T) [sP]. [dR] (G)[sP].[dR] (C)[sP].[dR](A)[sP].[dR](G)[sP].[dR] (A)[sP].[dR](T)[sP].[dR] (A)[sP].[dR](A)[sP].[MOE ](T)[sP].[MOE](G)P.[TNA](T)P. [MOE](T)[sP].[MO E]([5meC]) |
| #187 | 3 | GGCATATGCAGATAATG TTC | [MOE](G)[sP].[MOE](G)P.[TNA]([5meC])P.[MOE]( A) [sP]. [MOE] (T) [sP]. [dR] (A) [sP]. [d R] (T) [sP]. [dR] (G)[sP].[dR] (C)[sP].[dR](A)[sP].[dR](G)[sP].[dR] (A)[sP].[dR](T)[sP].[dR] (A)[sP].[dR](A)[sP].[MOE ](T)[sP].[TNA](G)P.[TNA](T)P. [MOE](T)[sP].[MOE ]([5meC]) |

(continued)

| CMP ID NO | SEQ ID NO | Base sequence 5'-3' | HELM |
|---|---|---|---|
| #188 | 3 | GGCATATGCAGATAATG TTC | [MOE](G)[sP].[MOE](G)P.[TNA]([5meC])P.[MOE]( A)[sP].[MOE](T)[sP].[dR](A)[sP].[dR](T)[sP].[dR] (G)[sP].[dR](C)[sP].[dR](A)[sP].[dR](G)[sP].[dR] (A)[sP].[dR](T)[sP].[dR](A)[sP].[dR](A)[sP].[MOE ](T)P.[TNA](G)P.[TNA](T)P.[MOE](T)[sP].[MOE]([ 5meC]) |
| #189 | 3 | GGCATATGCAGATAATG TTC | [MOE](G)[sP].[MOE](G)P.[TNA]([5meC])P.[TNA]( A)P.[MOE](T)[sP].[dR](A)[sP].[dR](T)[sP].[dR](G )[sP].[dR](C)[sP].[dR](A)[sP].[dR](G)[sP].[dR](A )[sP].[dR](T)[sP].[dR](A)[sP].[dR](A)[sP].[MOE]( T) P. [TNA] (G) P. [TNA] (T) P. [MOE](T)[sP]. [MOE] ([5 meC]) |
| #190 | 3 | GGCATATGCAGATAATG TTC | [MOE](G)[sP].[MOE](G)[sP].[MOE]([5meC])[sP].[ MOE](A)[sP].[MOE](T)P.[TNA](A)[sP].[dR](T)[sP] .[dR](G)[sP].[dR](C)[sP].[dR](A)[sP].[dR](G)[sP] .[dR](A)[sP].[dR](T)[sP].[dR](A)P.[TNA](A)[sP].[ MOE](T)[sP].[MOE](G)[sP].[MOE](T)[sP].[MOE](T )[sP].[MOE]([5meC]) |
| #191 | 3 | GGCATATGCAGATAATG TTC | [MOE](G)[sP].[MOE](G)[sP].[MOE]([5meC])[sP].[ MOE] (A)[sP]. [MOE] (T) P. [TNA] (A) P. [dR] (T) [sP]. [d R] (G) [sP].[d R] (C) [sP]. [dR] (A) [sP]. [d R] (G) [sP]. [d R](A)[sP].[dR](T)[sP].[dR](A)P.[TNA](A)P.[MOE]( T)[sP].[MOE](G)[sP].[MOE](T)[sP].[MOE](T)[sP].[ MOE]([5meC]) |
| CONTROL #4 | 4 | GGGAGTTACTTGCCAAC TTG | [MOE](G)[sP].[MOE](G)[sP].[MOE](G)[sP].[MOE] (A)[sP].[MOE](G)[sP].[dR](T)[sP].[dR](T)[sP].[dR ] (A) [sP]. [dR] (C)[sP]. [dR] (T) [sP]. [d R] (T) [sP]. [d R] (G) [sP]. [dR] (C) [sP].[d R] (C) [sP]. [dR] (A) [sP]. [MO E](A)[sP].[MOE]([5meC])[sP].[MOE](T)[sP].[MOE ](T)[sP].[MOE](G) |
| #192 | 4 | GGGAGTTACTTGCCAAC TTG | [MOE](G)[sP].[MOE](G)[sP].[MOE](G)[sP].[MOE] (A)[sP].[MOE](G)P.[TNA](T)[sP].[dR](T)[sP].[dR] (A)[sP].[dR](C)[sP].[dR](T)[sP].[dR](T)[sP].[dR]( G)[sP].[dR](C)[sP].[dR](C)[sP].[dR](A)[sP].[MOE ] (A) [sP]. [MOE] ([5meC]) [sP]. [MOE](T) [sP]. [MOE] (T)[sP].[MOE](G) |
| #193 | 4 | GGGAGTTACTTGCCAAC TTG | [MOE](G)[sP].[MOE](G)[sP].[MOE](G)[sP].[MOE] (A)[sP].[MOE](G)P.[TNA](T)P.[TNA](T)[sP].[dR]( A)[sP].[dR](C)[sP].[dR](T)[sP].[dR](T)[sP].[dR]( G)[sP].[dR](C)[sP].[dR](C)[sP].[dR](A)[sP].[MOE](A)[sP].[MOE]([5meC])[sP].[MOE](T)[sP].[MOE] (T)[sP].[MOE](G) |
| #194 | 4 | GGGAGTTACTTGCCAAC TTG | [MOE](G)[sP].[MOE](G)[sP].[MOE](G)[sP].[MOE] (A)[sP].[MOE](G)P.[TNA](T)P.[TNA](T)P.[TNA](A) [sP].[dR](C)[sP].[dR](T)[sP].[dR](T)[sP].[dR](G)[ sP].[dR](C)[sP].[dR](C)[sP].[dR](A)[sP].[MOE](A )[sP].[MOE]([5meC])[sP].[MOE](T)[sP].[MOE](T) [sP].[MOE](G) |
| #195 | 4 | GGGAGTTACTTGCCAAC TTG | [MOE](G)[sP].[MOE](G)[sP].[MOE](G)[sP].[MOE] (A)[sP].[MOE](G)P.[TNA](T)P.[TNA](T)P.[TNA](A) P.[TNA]([5meC])[sP].[dR](T)[sP].[dR](T)[sP].[dR ](G)[sP].[dR](C)[sP].[dR](C)[sP].[dR](A)[sP].[MO E](A)[sP].[MOE]([5meC])[sP].[MOE](T)[sP].[MOE ](T)[sP].[MOE](G) |

(continued)

| CMP ID NO | SEQ ID NO | Base sequence 5'-3' | HELM |
|---|---|---|---|
| #196 | 4 | GGGAGTTACTTGCCAAC TTG | [MOE](G)[sP].[MOE](G)[sP].[MOE](G)[sP].[MOE] (A)[sP]. [MOE](G)P.[TNA](T)P.[TNA](T)P.[TNA](A) P.[TNA]([5meC]) P.[TNA](T)[sP].[dR](T)[sP].[dR]( G)[sP].[dR](C)[sP].[dR](C) [sP].[dR](A)[sP].[MOE ](A)[sP].[MOE]([5meC])[sP].[MOE] (T)[sP].[MOE] (T)[sP].[MOE](G) |
| #197 | 4 | GGGAGTTACTTGCCAAC TTG | [MOE](G)[sP].[MOE](G)[sP].[MOE](G)[sP].[MOE] (A)[sP]. [MOE](G)P.[TNA](T)P.[TNA](T)P.[TNA](A) P.[TNA]([5meC]) P.[TNA](T)P.[TNA](T)[sP].[dR]( G)[sP].[dR](C)[sP].[dR](C) [sP].[dR](A)[sP].[MOE ](A)[sP].[MOE]([5meC])[sP].[MOE] (T)[sP].[MOE] (T)[sP].[MOE](G) |
| #198 | 4 | GGGAGTTACTTGCCAAC TTG | [TNA] (G) P. [TNA] (G) P. [MOE] (G) [sP]. [MOE] (A) [sP] . [MOE](G)[sP].[dR](T)[sP].[dR](T)[sP].[dR](A)[sP ]. [dR] (C) [sP]. [dR] (T) [sP]. [dR] (T) [sP]. [dR] (G) [sP] .[dR](C)[sP]. [dR](C)[sP].[dR](A)[sP].[MOE](A)[s P].[MOE]([5meC])[sP]. [MOE](T)P.[TNA](T)P.[TNA ](G) |
| #199 | 4 | GGGAGTTACTTGCCAAC TTG | [TNA] (G) P. [TNA] (G) P. [MOE] (G) [sP]. [MOE] (A) [sP] . [MOE](G)P.[TNA](T)[sP].[dR](T)[sP].[dR](A)[sP] .[dR](C) [sP].[dR](T)[sP].[dR](T)[sP].[dR](G)[sP]. [dR](C)[sP].[dR](C) [sP].[dR](A)[sP].[MOE](A)[sP ].[MOE]([5meC])[sP].[MOE] (T)P.[TNA](T)P.[TNA] (G) |
| #200 | 4 | GGGAGTTACTTGCCAAC TTG | [TNA] (G) P. [TNA] (G) P. [MOE] (G) [sP]. [MOE] (A) [sP] . [MOE](G)P.[TNA](T)P.[TNA](T)[sP].[dR](A)[sP].[ dR](C) [sP].[dR](T)[sP].[dR](T)[sP].[dR](G)[sP].[ dR](C)[sP].[dR](C) [sP].[dR](A)[sP].[MOE](A)[sP] .[MOE]([5meC])[sP].[MOE] (T)P.[TNA](T)P.[TNA]( G) |
| #201 | 4 | GGGAGTTACTTGCCAAC TTG | [TNA](G)P.[TNA](G)P.[MOE](G)[sP].[MOE](A)[sP] .[MOE] (G)P.[TNA](T)P.[TNA](T)P.[TNA](A)[sP].[d R](C)[sP].[dR](T) [sP].[dR](T)[sP].[dR](G)[sP].[d R](C)[sP].[dR](C)[sP].[dR](A) [sP].[MOE](A)[sP].[ MOE]([5meC])[sP].[MOE](T)P.[TNA](T) P.[TNA](G ) |
| #202 | 4 | GGGAGTTACTTGCCAAC TTG | [TNA](G)P.[TNA](G)P.[MOE](G)[sP].[MOE](A)[sP] .[MOE] (G)P.[TNA](T)P.[TNA](T)P.[TNA](A)P.[TNA ]([5meC])[sP]. [dR](T)[sP].[dR](T)[sP].[dR](G)[sP ].[dR](C)[sP].[dR](C)[sP]. [dR](A)[sP].[MOE](A)[s P].[MOE]([5meC])[sP].[MOE](T)P. [TNA](T)P.[TNA ](G) |
| #203 | 4 | GGGAGTTACTTGCCAAC TTG | [TNA](G)P.[TNA](G)P.[MOE](G)[sP].[MOE](A)[sP] .[MOE] (G)P.[TNA](T)P.[TNA](T)P.[TNA](A)P.[TNA ]([5meC])P. [TNA](T)[sP].[dR](T)[sP].[dR](G)[sP] .[dR](C)[sP].[dR](C) [sP].[dR](A)[sP].[MOE](A)[s P].[MOE]([5meC])[sP].[MOE](T) P.[TNA](T)P.[TNA ](G) |
| #204 | 4 | GGGAGTTACTTGCCAAC TTG | [TNA](G)P.[TNA](G)P.[TNA](G)P.[TNA](A)P.[TNA] (G)[sP]. [dR](T)[sP].[dR](T)[sP].[dR](A)[sP].[dR]( C)[sP].[dR](T)[sP]. [dR](T)[sP].[dR](G)[sP].[dR]( C)[sP].[dR](C)[sP].[dR](A)[sP]. [TNA](A)P.[TNA]( [5meC])P.[TNA](T)P.[TNA](T)P.[TNA](G) |

(continued)

| CMP ID NO | SEQ ID NO | Base sequence 5'-3' | HELM |
|---|---|---|---|
| #205 | 4 | GGGAGTTACTTGCCAACTTG | [TNA](G)[sP].[TNA](G)P.[TNA](G)P.[TNA](A)P.[T NA](G)[sP].[dR](T)[sP].[dR](T)[sP].[dR](A)[sP].[ dR](C)[sP].[dR](T)[sP].[dR](T)[sP].[dR](G)[sP].[ dR](C)[sP].[dR](C)[sP].[dR](A)[sP].[TNA](A)P.[T NA]([5meC])P. [TNA](T)P. [TNA](T)[sP]. [TNA] (G) |
| #206 | 4 | GGGAGTTACTTGCCAACTTG | [TNA](G)[sP].[TNA](G)[sP].[TNA](G)[sP].[TNA](A )P.[TNA](G)[sP].[dR](T)[sP].[dR](T)[sP].[dR](A)[ sP]. [dR] (C) [sP]. [d R] (T) [sP]. [d R] (T) [sP]. [dR] (G) [s P].[dR](C)[sP].[dR](C)[sP].[dR](A)[sP].[MOE](A)[sP].[MOE]([5meC])[sP].[MOE](T)[sP].[MOE](T)[s P].[MOE](G) |
| #207 | 4 | GGGAGTTACTTGCCAACTTG | [TNA](G)[sP].[TNA](G)[sP].[TNA](G)P.[TNA](A)P. [TNA](G)[sP].[dR](T)[sP].[dR](T)[sP].[dR](A)[sP] .[dR](C)[sP].[dR](T)[sP].[dR](T)[sP].[dR](G)[sP]. [dR](C)[sP].[dR](C)[sP].[dR](A)[sP].[MOE](A)[sP ].[MOE]([5meC])[sP].[MOE](T)[sP].[MOE](T)[sP]. [MOE](G) |
| #208 | 4 | GGGAGTTACTTGCCAACTTG | [TNA](G)[sP].[TNA](G)P.[TNA](G)P.[TNA](A)P.[T NA](G)[sP].[dR](T)[sP].[dR](T)[sP].[dR](A)[sP].[ dR](C)[sP].[dR](T)[sP].[dR](T)[sP].[dR](G)[sP].[ dR](C)[sP].[dR](C)[sP].[dR](A)[sP].[MOE](A)[sP] .[MOE]([5meC])[sP].[MOE](T)[sP].[MOE](T)[sP].[ MOE](G) |
| #209 | 4 | GGGAGTTACTTGCCAACTTG | [TNA](G)P.[TNA](G)P.[TNA](G)P.[TNA](A)P.[TNA] (G)[sP].[dR](T)[sP].[dR](T)[sP].[dR](A)[sP].[dR]( C)[sP].[dR](T)[sP].[dR](T)[sP].[dR](G)[sP].[dR]( C)[sP].[dR](C)[sP].[dR](A)[sP].[MOE](A)[sP].[MO E]([5meC])[sP].[MOE](T)[sP].[MOE](T)[sP].[MOE ](G) |
| #210 | 4 | GGGAGTTACTTGCCAACTTG | [TNA](G)P.[TNA](G)P.[TNA](G)P.[TNA](A)P.[TNA] (G)P.[TNA](T)[sP].[dR](T)[sP].[dR](A)[sP].[dR](C )[sP].[dR](T)[sP].[dR](T)[sP].[dR](G)[sP].[dR](C) [sP].[dR](C)[sP].[dR](A)[sP].[MOE](A)[sP].[MOE] ([5meC])[sP].[MOE](T)[sP].[MOE](T)[sP].[MOE]( G) |
| #211 | 4 | GGGAGTTACTTGCCAACTTG | [TNA](G)P.[TNA](G)P.[TNA](G)P.[TNA](A)P.[TNA] (G)P.[TNA](T)P.[TNA](T)[sP].[dR](A)[sP].[dR](C) [sP].[dR](T)[sP].[dR](T)[sP].[dR](G)[sP].[dR](C)[ sP].[dR](C)[sP].[dR](A)[sP].[MOE](A)[sP].[MOE]( [5meC])[sP].[MOE](T)[sP].[MOE](T)[sP].[MOE](G ) |
| #212 | 4 | GGGAGTTACTTGCCAACTTG | [TNA](G)P.[TNA](G)P.[TNA](G)P.[TNA](A)P.[TNA] (G)P.[TNA](T)P.[TNA](T)P.[TNA](A)[sP].[dR](C)[s P].[dR](T)[sP].[dR](T)[sP].[dR](G)[sP].[dR](C)[s P].[dR](C)[sP].[dR](A)[sP].[MOE](A)[sP].[MOE]([ 5meC])[sP].[MOE](T)[sP].[MOE](T)[sP].[MOE](G) |
| #213 | 4 | GGGAGTTACTTGCCAACTTG | [TNA](G)P.[TNA](G)P.[TNA](G)P.[TNA](A)P.[TNA] (G)P.[TNA](T)P.[TNA](T)P.[TNA](A)P.[TNA]([5me C])[sP].[dR](T)[sP].[dR](T)[sP].[dR](G)[sP].[dR]( C)[sP].[dR](C)[sP].[dR](A)[sP].[MOE](A)[sP].[MO E]([5meC])[sP].[MOE](T)[sP].[MOE](T)[sP].[MOE ](G) |

(continued)

| CMP ID NO | SEQ ID NO | Base sequence 5'-3' | HELM |
|---|---|---|---|
| #214 | 4 | GGGAGTTACTTGCCAAC TTG | [TNA](G)P.[TNA](G)P.[TNA](G)P.[TNA](A)P.[TNA] (G)P. [TNA](T)P.[TNA](T)P.[TNA](A)P.[TNA]([5me C])P.[TNA](T) [sP].[dR](T)[sP].[dR](G)[sP].[dR](C )[sP].[dR](C)[sP].[dR](A) [sP].[MOE](A)[sP].[MOE ]([5meC])[sP].[MOE](T)[sP].[MOE] (T)[sP].[MOE] (G) |
| #215 | 4 | GGGAGTTACTTGCCAAC TTG | [TNA](G)P.[TNA](G)P.[TNA](G)P.[TNA](A)P.[TNA] (G)P. [TNA](T)P.[TNA](T)P.[TNA](A)P.[TNA]([5me C])P.[TNA](T) [sP].[dR](T)[sP].[dR](G)[sP].[dR](C )[sP].[dR](C)[sP].[dR](A) [sP].[dR](A)[sP].[MOE]( [5meC])[sP].[MOE](T)[sP].[MOE] (T)[sP].[MOE](G ) |
| #216 | 4 | GGGAGTTACTTGCCAAC TTG | [TNA](G)P.[TNA](G)P.[TNA](G)P.[TNA](A)P.[TNA] (G)P. [TNA](T)P.[TNA](T)P.[TNA](A)P.[TNA]([5me C])P.[TNA](T) [sP].[dR](T)[sP].[dR](G)[sP].[dR](C )[sP].[dR](C)[sP].[dR](A) [sP].[dR](A)[sP].[dR](C) [sP]. [MOE] (T) [sP]. [MOE] (T) [sP]. [MOE] (G) |
| #217 | 4 | GGGAGTTACTTGCCAAC TTG | [MOE](G)[sP].[MOE](G)[sP].[MOE](G)[sP].[MOE] (A)[sP]. [MOE](G)[sP].[dR](T)[sP].[dR](T)[sP].[dR ](A)[sP].[dR](C) [sP].[dR](T)[sP].[dR](T)[sP].[dR] (G)[sP].[dR](C)[sP].[dR](C) [sP].[dR](A)[sP].[TNA ](A)P.[TNA]([5meC])P.[TNA](T)P. [TNA](T)P.[TNA ](G) |
| #218 | 4 | GGGAGTTACTTGCCAAC TTG | [MOE](G)[sP].[MOE](G)[sP].[MOE](G)[sP].[MOE] (A)[sP]. [MOE](G)[sP].[dR](T)[sP].[dR](T)[sP].[dR ](A)[sP].[dR](C) [sP].[dR](T)[sP].[dR](T)[sP].[dR] (G)[sP].[dR](C)[sP].[TNA]( [5meC])P.[TNA](A)P.[ TNA](A)P.[TNA]([5meC])P.[TNA](T) P.[TNA](T)P.[ TNA](G) |
| CONTROL #5 | 5 | TGCCTTTAGGATTCTAG ACA | [MOE](T)[sP].[MOE](G)[sP].[MOE]([5meC])[sP].[ MOE] ([5meC]) [sP]. [MOE] (T) [sP]. [dR] (T) [sP]. [dR] (T)[sP].[dR] (A)[sP].[dR](G)[sP].[dR](G)[sP].[dR](A)[sP].[dR](T)[sP].[dR] (T)[sP].[dR](C)[sP].[dR]( T)[sP].[MOE](A)[sP].[MOE](G)[sP]. [MOE](A)[sP]. [MOE]([5meC])[sP].[MOE](A) |
| #219 | 5 | TGCCTTTAGGATTCTAG ACA | [MOE](T)[sP].[MOE](G)[sP].[MOE]([5meC])[sP].[ MOE]( [5meC])[sP].[MOE](T)P.[TNA](T)[sP].[dR]( T)[sP].[dR](A) [sP].[dR](G)[sP].[dR](G)[sP].[dR]( A)[sP].[dR](T)[sP].[dR](T) [sP].[dR](C)[sP].[dR](T )[sP].[MOE](A)[sP].[MOE](G)[sP]. [MOE](A)[sP].[ MOE]([5meC])[sP].[MOE](A) |
| #220 | 5 | TGCCTTTAGGATTCTAG ACA | [MOE](T)[sP].[MOE](G)[sP].[MOE]([5meC])[sP].[ MOE]( [5meC])[sP].[MOE](T)P.[TNA](T)P.[TNA](T )[sP].[dR](A) [sP].[dR](G)[sP].[dR](G)[sP].[dR](A ) [sP]. [dR] (T) [sP]. [d R] (T) [sP]. [dR] (C) [sP]. [dR] (T) [sP]. [MOE] (A) [sP]. [MOE] (G) [sP]. [MOE] (A) [sP]. [M OE]([5meC])[sP].[MOE](A) |
| #221 | 5 | TGCCTTTAGGATTCTAG ACA | [MOE](T)[sP].[MOE](G)[sP].[MOE]([5meC])[sP].[ MOE]( [5meC])[sP].[MOE](T)P.[TNA](T)P.[TNA](T )P.[TNA](A) [sP].[dR](G)[sP].[dR](G)[sP].[dR](A)[ sP].[dR](T)[sP].[dR](T) [sP].[dR](C)[sP].[dR](T)[s P].[MOE](A)[sP].[MOE](G)[sP]. [MOE](A)[sP].[MO E]([5meC])[sP].[MOE](A) |

(continued)

| CMP ID NO | SEQ ID NO | Base sequence 5'-3' | HELM |
|---|---|---|---|
| #222 | 5 | TGCCTTTAGGATTCTAG ACA | [MOE](T)[sP].[MOE](G)[sP].[MOE]([5meC])[sP].[ MOE]( [5meC])[sP].[MOE](T)P.[TNA](T)P.[TNA](T )P.[TNA](A)P. [TNA](G)[sP].[dR](G)[sP].[dR](A)[s P].[dR](T)[sP].[dR](T) [sP].[dR](C)[sP].[dR](T)[sP ].[MOE](A)[sP].[MOE](G)[sP]. [MOE](A)[sP].[MOE ] ([5meC]) [sP]. [MOE] (A) |
| #223 | 5 | TGCCTTTAGGATTCTAG ACA | [MOE](T)[sP].[MOE](G)[sP].[MOE]([5meC])[sP].[ MOE]( [5meC])[sP].[MOE](T)P.[TNA](T)P.[TNA](T )P.[TNA](A)P. [TNA](G)P.[TNA](G)[sP].[dR](A)[sP ].[dR](T)[sP].[dR](T) [sP].[dR](C)[sP].[dR](T)[sP] .[MOE](A)[sP].[MOE](G)[sP]. [MOE](A)[sP].[MOE] ([5meC])[sP].[MOE](A) |
| #224 | 5 | TGCCTTTAGGATTCTAG ACA | [MOE](T)[sP].[MOE](G)[sP].[MOE]([5meC])[sP].[ MOE]( [5meC])[sP].[MOE](T)P.[TNA](T)P.[TNA](T )P.[TNA](A)P. [TNA](G)P.[TNA](G)P.[TNA](A)[sP]. [dR](T)[sP].[dR](T)[sP]. [dR](C)[sP].[dR](T)[sP].[MOE](A)[sP].[MOE](G)[sP].[MOE] (A)[sP].[MOE]([ 5meC])[sP].[MOE](A) |
| #225 | 5 | TGCCTTTAGGATTCTAG ACA | [TNA](T)P.[TNA](G)P.[TNA]([5meC])P.[TNA]([5m eC])P. [TNA](T)[sP].[dR](T)[sP].[dR](T)[sP].[dR]( A)[sP].[dR](G) [sP].[dR](G)[sP].[dR](A)[sP].[dR]( T)[sP].[dR](T)[sP].[dR](C) [sP].[dR](T)[sP].[TNA] (A)P.[TNA](G)P.[TNA](A)P.[TNA]( [5meC])P.[TNA] (A) |
| #226 | 5 | TGCCTTTAGGATTCTAG ACA | [TNA](T)[sP].[TNA](G)P.[TNA]([5meC])P.[TNA]([ 5meC])P. [TNA](T)[sP].[dR](T)[sP].[dR](T)[sP].[d R](A)[sP].[dR](G) [sP].[dR](G)[sP].[dR](A)[sP].[d R](T)[sP].[dR](T)[sP].[dR](C) [sP].[dR](T)[sP].[T NA](A)P.[TNA](G)P.[TNA](A)P.[TNA]( [5meC])[sP] .[TNA](A) |
| #227 | 5 | TGCCTTTAGGATTCTAG ACA | [TNA](T)[sP].[TNA](G)[sP].[TNA]([5meC])[sP].[T NA]( [5meC])P.[TNA](T)[sP].[dR](T)[sP].[dR](T)[s P].[dR](A)[sP]. [dR](G)[sP].[dR](G)[sP].[dR](A)[s P].[dR](T)[sP].[dR](T)[sP]. [dR](C)[sP].[dR](T)[sP ].[MOE](A)[sP].[MOE](G)[sP].[MOE] (A)[sP].[MOE ]([5meC])[sP].[MOE](A) |
| #228 | 5 | TGCCTTTAGGATTCTAG ACA | [TNA](T)[sP].[TNA](G)[sP].[TNA]([5meC])P.[TNA ]([5meC]) P.[TNA](T)[sP].[dR](T)[sP].[dR](T)[sP]. [dR](A)[sP].[dR](G) [sP].[dR](G)[sP].[dR](A)[sP]. [dR](T)[sP].[dR](T)[sP].[dR](C) [sP].[dR](T)[sP].[ MOE](A)[sP].[MOE](G)[sP].[MOE](A)[sP]. [MOE]([ 5meC])[sP].[MOE](A) |
| #229 | 5 | TGCCTTTAGGATTCTAG ACA | [TNA](T)[sP].[TNA](G)P.[TNA]([5meC])P.[TNA]([ 5meC])P. [TNA](T)[sP].[dR](T)[sP].[dR](T)[sP].[d R](A)[sP].[dR](G) [sP].[dR](G)[sP].[dR](A)[sP].[d R](T)[sP].[dR](T)[sP].[dR](C) [sP].[dR](T)[sP].[M OE](A)[sP].[MOE](G)[sP].[MOE](A)[sP]. [MOE]([5 meC])[sP].[MOE](A) |
| #230 | 5 | TGCCTTTAGGATTCTAG ACA | [TNA](T)P.[TNA](G)P.[TNA]([5meC])P.[TNA]([5m eC])P. [TNA](T)[sP].[dR](T)[sP].[dR](T)[sP].[dR]( A)[sP].[dR](G) [sP].[dR](G)[sP].[dR](A)[sP].[dR]( T)[sP].[dR](T)[sP].[dR](C) [sP].[dR](T)[sP].[MOE] (A)[sP].[MOE](G)[sP].[MOE](A)[sP]. [MOE]([5me C])[sP].[MOE](A) |

(continued)

| CMP ID NO | SEQ ID NO | Base sequence 5'-3' | HELM |
|---|---|---|---|
| #231 | 5 | TGCCTTTAGGATTCTAG ACA | [TNA](T)P.[TNA](G)P.[TNA]([5meC])P.[TNA]([5m eC])P. [TNA](T)P.[TNA](T)[sP].[dR](T)[sP].[dR](A )[sP].[dR](G) [sP].[dR](G)[sP].[dR](A)[sP].[dR](T )[sP].[dR](T)[sP].[dR](C) [sP].[dR](T)[sP].[MOE]( A)[sP].[MOE](G)[sP].[MOE](A)[sP]. [MOE]([5meC ])[sP].[MOE](A) |
| #232 | 5 | TGCCTTTAGGATTCTAG ACA | [TNA](T)P.[TNA](G)P.[TNA]([5meC])P.[TNA]([5m eC])P. [TNA](T)P.[TNA](T)P.[TNA](T)[sP].[dR](A)[ sP].[dR](G)[sP]. [dR](G)[sP].[dR](A)[sP].[dR](T)[ sP].[dR](T)[sP].[dR](C)[sP]. [dR](T)[sP].[MOE](A) [sP].[MOE](G)[sP].[MOE](A)[sP]. [MOE]([5meC])[ sP].[MOE](A) |
| #233 | 5 | TGCCTTTAGGATTCTAG ACA | [TNA](T)P.[TNA](G)P.[TNA]([5meC])P.[TNA]([5m eC])P. [TNA](T)P.[TNA](T)P.[TNA](T)P.[TNA](A)[s P].[dR](G)[sP]. [dR](G)[sP].[dR](A)[sP].[dR](T)[s P].[dR](T)[sP].[dR](C)[sP]. [dR](T)[sP].[MOE](A)[ sP].[MOE](G)[sP].[MOE](A)[sP]. [MOE]([5meC])[s P].[MOE](A) |
| #234 | 5 | TGCCTTTAGGATTCTAG ACA | [TNA](T)P.[TNA](G)P.[TNA]([5meC])P.[TNA]([5m eC])P. [TNA](T)P.[TNA](T)P.[TNA](T)P.[TNA](A)P. [TNA](G)[sP]. [dR](G)[sP].[dR](A)[sP].[dR](T)[sP] .[dR](T)[sP].[dR](C)[sP]. [dR](T)[sP].[MOE](A)[sP ].[MOE](G)[sP].[MOE](A)[sP]. [MOE]([5meC])[sP] .[MOE](A) |
| #235 | 5 | TGCCTTTAGGATTCTAG ACA | [TNA](T)P.[TNA](G)P.[TNA]([5meC])P.[TNA]([5m eC])P. [TNA](T)P.[TNA](T)P.[TNA](T)P.[TNA](A)P. [TNA](G)P. [TNA](G)[sP].[dR](A)[sP].[dR](T)[sP]. [dR](T)[sP].[dR](C) [sP].[dR](T)[sP].[MOE](A)[sP] .[MOE](G)[sP].[MOE](A)[sP]. [MOE]([5meC])[sP]. [MOE](A) |
| #236 | 5 | TGCCTTTAGGATTCTAG ACA | [TNA](T)P.[TNA](G)P.[TNA]([5meC])P.[TNA]([5m eC])P. [TNA](T)P.[TNA](T)P.[TNA](T)P.[TNA](A)P. [TNA](G)P. [TNA](G)[sP].[dR](A)[sP].[dR](T)[sP]. [dR](T)[sP].[dR](C) [sP].[dR](T)[sP].[dR](A)[sP].[ MOE](G)[sP].[MOE](A)[sP]. [MOE]([5meC])[sP].[ MOE](A) |
| #237 | 5 | TGCCTTTAGGATTCTAG ACA | [TNA](T)P.[TNA](G)P.[TNA]([5meC])P.[TNA]([5m eC])P. [TNA](T)P.[TNA](T)P.[TNA](T)P.[TNA](A)P. [TNA](G)P. [TNA](G)[sP].[dR](A)[sP].[dR](T)[sP]. [dR](T)[sP].[dR](C) [sP].[dR](T)[sP].[dR](A)[sP].[ dR](G)[sP].[MOE](A)[sP]. [MOE]([SmeC])[sP].[M OE](A) |
| #238 | 5 | TGCCTTTAGGATTCTAG ACA | [MOE](T)[sP].[MOE](G)[sP].[MOE]([5meC])[sP].[ MOE]( [5meC])[sP].[MOE](T)[sP].[dR](T)[sP].[dR] (T)[sP].[dR](A) [sP].[dR](G)[sP].[dR](G)[sP].[dR] (A)[sP].[dR](T)[sP].[dR](T) [sP].[dR](C)[sP].[dR]( T)[sP].[TNA](A)P.[TNA](G)P.[TNA](A) P.[TNA]([5 meC])P.[TNA](A) |
| #239 | 5 | TGCCTTTAGGATTCTAG ACA | [MOE](T)[sP].[MOE](G)[sP].[MOE]([5meC])[sP].[ MOE]( [5meC])[sP].[MOE](T)[sP].[dR](T)[sP].[dR] (T)[sP].[dR](A) [sP].[dR](G)[sP].[dR](G)[sP].[dR] (A)[sP].[dR](T)[sP].[dR](T) [sP].[dR](C)[sP].[TNA ](T)P.[TNA](A)P.[TNA](G)P.[TNA](A) P.[TNA]([5m eC])P.[TNA](A) |

(continued)

| CMP ID NO | SEQ ID NO | Base sequence 5'-3' | HELM |
|---|---|---|---|
| #240 | 5 | TGCCTTTAGGATTCTAG ACA | [MOE](T)[sP].[MOE](G)[sP].[MOE]([5meC])[sP].[ MOE] ([5meC]) [sP]. [MOE] (T) [sP]. [dR] (T) [sP]. [dR] (T)[sP].[dR] (A)[sP].[dR](G)[sP].[dR](G)[sP].[dR] (A)[sP].[dR](T)[sP].[dR] (T)[sP].[TNA]([5meC])P. [TNA](T)P.[TNA](A)P.[TNA](G)P. [TNA](A)P.[TNA] ([5meC])P.[TNA](A) |
| CONTROL #6 | 6 | CCAGGCTGGTTATGACT CAG | [MOE]([5meC])[sP].[MOE]([5meC])[sP].[MOE](A) [sP]. [MOE](G)[sP].[MOE](G)[sP].[dR](C)[sP].[dR] (T)[sP].[dR](G) [sP].[dR](G)[sP].[dR](T)[sP].[dR] (T)[sP].[dR](A)[sP].[dR](T) [sP].[dR](G)[sP].[dR]( A)[sP].[MOE]([5meC])[sP].[MOE](T) [sP].[MOE]([ 5meC])[sP].[MOE](A)[sP].[MOE](G) |
| #241 | 6 | CCAGGCTGGTTATGACT CAG | [TNA]([5meC])[sP].[TNA]([5meC])[sP].[TNA](A)[ sP].[TNA] (G)P.[TNA](G)[sP].[dR](C)[sP].[dR](T)[ sP].[dR](G)[sP].[dR] (G)[sP].[dR](T)[sP].[dR](T)[ sP].[dR](A)[sP].[dR](T)[sP].[dR] (G)[sP].[dR](A)[ sP].[MOE]([5meC])[sP].[MOE](T)[sP]. [MOE]([5m eC]) [sP]. [MOE] (A) [sP]. [MOE] (G) |
| #242 | 6 | CCAGGCTGGTTATGACT CAG | [TNA]([5meC])[sP].[TNA]([5meC])[sP].[TNA](A)P .[TNA](G) P.[TNA](G)[sP].[dR](C)[sP].[dR](T)[sP] .[dR](G)[sP].[dR](G) [sP].[dR](T)[sP].[dR](T)[sP]. [dR](A)[sP].[dR](T)[sP].[dR](G) [sP].[dR](A)[sP].[MOE]([5meC])[sP].[MOE](T)[sP].[MOE]( [5meC])[ sP].[MOE](A)[sP].[MOE](G) |
| #243 | 6 | CCAGGCTGGTTATGACT CAG | [TNA]([5meC])[sP].[TNA]([5meC])P.[TNA](A)P.[T NA](G)P. [TNA](G)[sP].[dR](C)[sP].[dR](T)[sP].[d R](G)[sP].[dR](G) [sP].[dR](T)[sP].[dR](T)[sP].[d R](A)[sP].[dR](T)[sP].[dR](G) [sP].[dR](A)[sP].[M OE]([5meC])[sP].[MOE](T)[sP].[MOE]( [5meC])[s P].[MOE](A)[sP].[MOE](G) |
| #244 | 6 | CCAGGCTGGTTATGACT CAG | [TNA]([5meC])P.[TNA]([5meC])P.[TNA](A)P.[TNA ](G)P. [TNA](G)[sP].[dR](C)[sP].[dR](T)[sP].[dR]( G)[sP].[dR](G) [sP].[dR](T)[sP].[dR](T)[sP].[dR]( A)[sP].[dR](T)[sP].[dR](G) [sP].[dR](A)[sP].[MOE ]([5meC])[sP].[MOE](T)[sP].[MOE]( [5meC])[sP]. [MOE](A)[sP].[MOE](G) |
| #245 | 6 | CCAGGCTGGTTATGACT CAG | [TNA]([5meC])P.[TNA]([5meC])P.[TNA](A)P.[TNA ](G)P. [TNA](G)P.[TNA]([5meC])[sP].[dR](T)[sP]. [dR](G)[sP].[dR] (G)[sP].[dR](T)[sP].[dR](T)[sP]. [dR](A)[sP].[dR](T)[sP].[dR] (G)[sP].[dR](A)[sP].[ MOE]([5meC])[sP].[MOE](T)[sP]. [MOE]([5meC])[ sP]. [MOE] (A) [sP]. [MOE] (G) |
| #246 | 6 | CCAGGCTGGTTATGACT CAG | [TNA]([5meC])P.[TNA]([5meC])P.[TNA](A)P.[TNA ](G)P. [TNA](G)P.[TNA]([5meC])P.[TNA](T)[sP].[d R](G)[sP].[dR] (G)[sP].[dR](T)[sP].[dR](T)[sP].[d R](A)[sP].[dR](T)[sP].[dR] (G)[sP].[dR](A)[sP].[M OE]([5meC])[sP].[MOE](T)[sP]. [MOE]([5meC])[s P]. [MOE] (A) [sP]. [MOE] (G) |
| #247 | 6 | CCAGGCTGGTTATGACT CAG | [TNA]([5meC])P.[TNA]([5meC])P.[TNA](A)P.[TNA ](G)P. [TNA](G)P.[TNA]([5meC])P.[TNA](T)P.[TNA ](G)[sP].[dR] (G)[sP].[dR](T)[sP].[dR](T)[sP].[dR ](A)[sP].[dR](T)[sP].[dR] (G)[sP].[dR](A)[sP].[MO E]([5meC])[sP].[MOE](T)[sP]. [MOE]([5meC])[sP] .[MOE](A)[sP].[MOE](G) |

(continued)

| CMP ID NO | SEQ ID NO | Base sequence 5'-3' | HELM |
|---|---|---|---|
| #248 | 6 | CCAGGCTGGTTATGACT CAG | [TNA]([5meC])P.[TNA]([5meC])P.[TNA](A)P.[TNA ](G)P. [TNA](G)P.[TNA]([5meC])P.[TNA](T)P.[TNA ](G)P.[TNA] (G)[sP].[dR](T)[sP].[dR](T)[sP].[dR]( A)[sP].[dR](T)[sP].[dR] (G)[sP].[dR](A)[sP].[MOE ]([5meC])[sP].[MOE](T)[sP]. [MOE]([5meC])[sP]. [MOE] (A) [sP]. [MOE] (G) |
| CONTROL #7 | 7 | TTATCAATTCACCAAGG AGC | [MOE](T)[sP].[MOE](T)[sP].[MOE](A)[sP].[MOE]( T)[sP]. [MOE]([5meC])[sP].[dR](A)[sP].[dR](A)[s P].[dR](T)[sP].[dR] (T)[sP].[dR](C)[sP].[dR](A)[sP ].[dR](C)[sP].[dR](C)[sP].[dR] (A)[sP].[dR](A)[sP] .[MOE](G)[sP].[MOE](G)[sP].[MOE](A) [sP].[MOE] (G)[sP].[MOE]([5meC]) |
| #249 | 7 | TTATCAATTCACCAAGG AGC | [TNA](T)[sP].[TNA](T)[sP].[TNA](A)[sP].[TNA](T) P.[TNA]( [5meC])[sP].[dR](A)[sP].[dR](A)[sP].[dR ](T)[sP].[dR](T) [sP].[dR](C)[sP].[dR](A)[sP].[dR] (C)[sP].[dR](C)[sP].[dR](A) [sP].[dR](A)[sP].[MO E](G)[sP].[MOE](G)[sP].[MOE](A)[sP]. [MOE](G)[ sP].[MOE]([5meC]) |
| #250 | 7 | TTATCAATTCACCAAGG AGC | [TNA](T)[sP].[TNA](T)[sP].[TNA](A)P.[TNA](T)P.[ TNA]( [5meC])[sP].[dR](A)[sP].[dR](A)[sP].[dR](T )[sP].[dR](T) [sP].[dR](C)[sP].[dR](A)[sP].[dR](C) [sP].[dR](C)[sP].[dR](A) [sP].[dR](A)[sP].[MOE]( G)[sP].[MOE](G)[sP].[MOE](A)[sP]. [MOE](G)[sP]. [MOE]([5meC]) |
| #251 | 7 | TTATCAATTCACCAAGG AGC | [TNA](T)[sP].[TNA](T)P.[TNA](A)P.[TNA](T)P.[TN A]( [5meC])[sP].[dR](A)[sP].[dR](A)[sP].[dR](T)[ sP].[dR](T) [sP].[dR](C)[sP].[dR](A)[sP].[dR](C)[s P].[dR](C)[sP].[dR](A) [sP].[dR](A)[sP].[MOE](G) [sP].[MOE](G)[sP].[MOE](A)[sP]. [MOE](G)[sP].[M OE]([5meC]) |
| #252 | 7 | TTATCAATTCACCAAGG AGC | [TNA](T)P.[TNA](T)P.[TNA](A)P.[TNA](T)P.[TNA]( [5meC]) [sP].[dR](A)[sP].[dR](A)[sP].[dR](T)[sP]. [dR](T)[sP].[dR](C) [sP].[dR](A)[sP].[dR](C)[sP].[ dR](C)[sP].[dR](A)[sP].[dR](A) [sP].[MOE](G)[sP] .[MOE](G)[sP].[MOE](A)[sP].[MOE](G) [sP].[MOE] ([5meC]) |
| #253 | 7 | TTATCAATTCACCAAGG AGC | [TNA](T)P.[TNA](T)P.[TNA](A)P.[TNA](T)P.[TNA]( [5meC]) P.[TNA](A)[sP].[dR](A)[sP].[dR](T)[sP].[ dR](T)[sP].[dR](C) [sP].[dR](A)[sP].[dR](C)[sP].[ dR](C)[sP].[dR](A)[sP].[dR](A) [sP].[MOE](G)[sP] .[MOE](G)[sP].[MOE](A)[sP].[MOE](G) [sP].[MOE] ([5meC]) |
| #254 | 7 | TTATCAATTCACCAAGG AGC | [TNA](T)P.[TNA](T)P.[TNA](A)P.[TNA](T)P.[TNA]( [5meC]) P.[TNA](A)P.[TNA](A)[sP].[dR](T)[sP].[dR](T)[sP].[dR](C) [sP].[dR](A)[sP].[dR](C)[sP].[d R](C)[sP].[dR](A)[sP].[dR](A) [sP].[MOE](G)[sP].[ MOE](G)[sP].[MOE](A)[sP].[MOE](G) [sP].[MOE]([ 5meC]) |
| #255 | 7 | TTATCAATTCACCAAGG AGC | [TNA](T)P.[TNA](T)P.[TNA](A)P.[TNA](T)P.[TNA]( [5meC]) P.[TNA](A)P.[TNA](A)P.[TNA](T)[sP].[dR ](T)[sP].[dR](C) [sP].[dR](A)[sP].[dR](C)[sP].[dR] (C)[sP].[dR](A)[sP].[dR](A) [sP].[MOE](G)[sP].[M OE](G)[sP].[MOE](A)[sP].[MOE](G) [sP].[MOE]([5 meC]) |

(continued)

| CMP ID NO | SEQ ID NO | Base sequence 5'-3' | HELM |
|---|---|---|---|
| #256 | 7 | TTATCAATTCACCAAGG AGC | [TNA](T)P.[TNA](T)P.[TNA](A)P.[TNA](T)P.[TNA]( [5meC]) P.[TNA](A)P.[TNA](A)P.[TNA](T)P.[TNA]( T)[sP].[dR](C) [sP].[dR](A)[sP].[dR](C)[sP].[dR]( C)[sP].[dR](A)[sP].[dR](A) [sP].[MOE](G)[sP].[MO E](G)[sP].[MOE](A)[sP].[MOE](G) [sP].[MOE]([5m eC]) |
| #257 | 7 | TTATCAATTCACCAAGG AGC | [TNA](T)P.[TNA](T)P.[TNA](A)P.[TNA](T)P.[TNA]( [5meC]) P.[TNA](A)P.[TNA](A)P.[TNA](T)P.[TNA]( T)P.[TNA]( [5meC]) [sP].[dR](A)[sP].[dR](C)[sP].[dR](C)[sP].[dR](A) [ sP].[dR](A)[sP].[MOE](G)[sP].[MOE](G)[sP].[MO E](A)[sP]. [MOE](G)[sP].[MOE]([5meC]) |
| CONTROL #8 | 8 | ATGGAGGTATGACATAT AAT | [MOE](A)[sP].[MOE](T)[sP].[MOE](G)[sP].[MOE]( G)[sP]. [MOE](A)[sP].[dR](G)[sP].[dR](G)[sP].[dR ](T)[sP].[dR](A) [sP].[dR](T)[sP].[dR](G)[sP].[dR] (A)[sP].[dR](C)[sP].[dR](A) [sP].[dR](T)[sP].[MOE ](A)[sP].[MOE](T)[sP].[MOE](A)[sP]. [MOE](A)[sP ].[MOE](T) |
| #258 | 8 | ATGGAGGTATGACATAT AAT | [TNA](A)[sP].[TNA](T)[sP].[TNA](G)[sP].[TNA](G )P.[TNA] (A)[sP].[dR](G)[sP].[dR](G)[sP].[dR](T)[ sP].[dR](A)[sP].[dR] (T)[sP].[dR](G)[sP].[dR](A)[ sP].[dR](C)[sP].[dR](A)[sP].[dR] (T)[sP].[MOE](A) [sP].[MOE](T)[sP].[MOE](A)[sP].[MOE](A) [sP].[M OE](T) |
| #259 | 8 | ATGGAGGTATGACATAT AAT | [TNA](A)[sP].[TNA](T)[sP].[TNA](G)P.[TNA](G)P. [TNA](A) [sP].[dR](G)[sP].[dR](G)[sP].[dR](T)[sP] .[dR](A)[sP].[dR](T) [sP].[dR](G)[sP].[dR](A)[sP]. [dR](C)[sP].[dR](A)[sP].[dR](T) [sP].[MOE](A)[sP].[MOE](T)[sP].[MOE](A)[sP].[MOE](A) [sP].[MOE ](T) |
| #261 | 8 | ATGGAGGTATGACATAT AAT | [TNA](A)P.[TNA](T)P.[TNA](G)P.[TNA](G)P.[TNA] (A)[sP]. [dR](G)[sP].[dR](G)[sP].[dR](T)[sP].[dR] (A)[sP].[dR](T)[sP]. [dR](G)[sP].[dR](A)[sP].[dR]( C)[sP].[dR](A)[sP].[dR](T)[sP]. [MOE](A)[sP].[MO E](T)[sP].[MOE](A)[sP].[MOE](A)[sP]. [MOE](T) |
| #262 | 8 | ATGGAGGTATGACATAT AAT | [TNA](A)P.[TNA](T)P.[TNA](G)P.[TNA](G)P.[TNA] (A)P. [TNA](G)[sP].[dR](G)[sP].[dR](T)[sP].[dR]( A)[sP].[dR](T) [sP].[dR](G)[sP].[dR](A)[sP].[dR]( C)[sP].[dR](A)[sP].[dR](T) [sP].[MOE](A)[sP].[MO E](T)[sP].[MOE](A)[sP].[MOE](A) [sP].[MOE](T) |
| #263 | 8 | ATGGAGGTATGACATAT AAT | [TNA](A)P.[TNA](T)P.[TNA](G)P.[TNA](G)P.[TNA] (A)P. [TNA](G)P.[TNA](G)[sP].[dR](T)[sP].[dR](A) [sP].[dR](T) [sP].[dR](G)[sP].[dR](A)[sP].[dR](C)[ sP].[dR](A)[sP].[dR](T) [sP].[MOE](A)[sP].[MOE]( T)[sP].[MOE](A)[sP].[MOE](A) [sP].[MOE](T) |
| #264 | 8 | ATGGAGGTATGACATAT AAT | [TNA](A)P.[TNA](T)P.[TNA](G)P.[TNA](G)P.[TNA] (A)P. [TNA](G)P.[TNA](G)P.[TNA](T)[sP].[dR](A)[ sP].[dR](T)[sP]. [dR](G)[sP].[dR](A)[sP].[dR](C)[ sP].[dR](A)[sP].[dR](T)[sP]. [MOE](A)[sP].[MOE]( T)[sP].[MOE](A)[sP].[MOE](A)[sP]. [MOE](T) |

(continued)

| CMP ID NO | SEQ ID NO | Base sequence 5'-3' | HELM |
|---|---|---|---|
| #265 | 8 | ATGGAGGTATGACATAT AAT | [TNA](A)P.[TNA](T)P.[TNA](G)P.[TNA](G)P.[TNA] (A)P.[TNA](G)P.[TNA](G)P.[TNA](T)P.[TNA](A)[sP ].[dR](T)[sP].[dR](G)[sP].[dR](A)[sP].[dR](C)[sP] .[dR](A)[sP].[dR](T)[sP].[MOE](A)[sP].[MOE](T)[ sP].[MOE](A)[sP].[MOE](A)[sP].[MOE](T) |
| #266 | 8 | ATGGAGGTATGACATAT AAT | [TNA](A)P.[TNA](T)P.[TNA](G)P.[TNA](G)P.[TNA] (A)P.[TNA](G)P.[TNA](G)P.[TNA](T)P.[TNA](A)P.[ TNA](T)[sP].[dR](G)[sP].[dR](A)[sP].[dR](C)[sP]. [dR](A)[sP].[dR](T)[sP].[MOE](A)[sP].[MOE](T)[s P].[MOE](A)[sP].[MOE](A)[sP].[MOE](T) |
| #267 | 9 | GTTATGCTTATTCCCCA ATG | [TNA](G)[sP].[TNA](T)P.[TNA](T)P.[TNA](A)P.[TN A](T)[sP].[dR](G)[sP].[dR](C)[sP].[dR](T)[sP].[d R](T)[sP].[dR](A)[sP].[dR](T)[sP].[dR](T)[sP].[dR ](C)[sP].[dR](C)[sP].[dR](C)[sP].[TNA]([5meC])P .[TNA](A)P.[TNA](A)P.[TNA](T)[sP].[TNA](G) |
| #268 | 10 | CTCAGCCTTTATCACTC AGC | [TNA]([5meC])[sP].[TNA](T)P.[TNA]([5meC])P.[T NA](A)P.[TNA](G)[sP].[dR](C)[sP].[dR](C)[sP].[d R](T)[sP].[dR](T)[sP].[dR](T)[sP].[dR](A)[sP].[dR ](T)[sP].[dR](C)[sP].[dR](A)[sP].[dR](C)[sP].[TN A](T)P.[TNA]([5meC])P.[TNA](A)P.[TNA](G)[sP].[ TNA]([5meC] |
| #269 | 11 | AACAAATTTCCTTAGTT GGC | [TNA](A)[sP].[TNA](A)P.[TNA]([5meC])P.[TNA](A )P.[TNA](A)[sP].[dR](A)[sP].[dR](T)[sP].[dR](T)[ sP].[dR](T)[sP].[dR](C)[sP].[dR](C)[sP].[dR](T)[s P].[dR](T)[sP].[dR](A)[sP].[dR](G)[sP].[TNA](T)P .[TNA](T)P.[TNA](G)P.[TNA](G)[sP].[TNA]([5meC ]) |
| #270 | 12 | TACACCAGTCCTTTTAG TAG | [TNA](T)[sP].[TNA](A)P.[TNA]([5meC])P.[TNA](A )P.[TNA]([5meC])[sP].[dR](C)[sP].[dR](A)[sP].[d R](G)[sP].[dR](T)[sP].[dR](C)[sP].[dR](C)[sP].[d R](T)[sP].[dR](T)[sP].[dR](T)[sP].[dR](T)[sP].[TN A](A)P.[TNA](G)P.[TNA](T)P.[TNA](A)[sP].[TNA]( G) |
| #271 | 13 | ATGCTCATCACTTTATG AAG | [TNA](A)[sP].[TNA](T)P.[TNA](G)P.[TNA]([5meC] )P.[TNA](T)[sP].[dR](C)[sP].[dR](A)[sP].[dR](T)[ sP].[dR](C)[sP].[dR](A)[sP].[dR](C)[sP].[dR](T)[s P].[dR](T)[sP].[dR](T)[sP].[dR](A)[sP].[TNA](T)P .[TNA](G)P.[TNA](A)P.[TNA](A)[sP].[TNA](G) |
| #272 | 14 | AAAAGGCTTAGCGCCCA CCT | [TNA](A)[sP].[TNA](A)P.[TNA](A)P.[TNA](A)P.[TN A](G)[sP].[dR](G)[sP].[dR](C)[sP].[dR](T)[sP].[d R](T)[sP].[dR](A)[sP].[dR](G)[sP].[dR]([5meC])[ sP].[dR](G)[sP].[dR](C)[sP].[dR](C)[sP].[TNA]([5 meC])P.[TNA](A)P.[TNA]([5meC])P.[TNA]([5meC ])[sP].[TNA](T) |
| #273 | 15 | AAGAGAACCACACACTA CCA | [TNA](A)[sP].[TNA](A)P.[TNA](G)P.[TNA](A)P.[T NA](G)[sP].[dR](A)[sP].[dR](A)[sP].[dR](C)[sP].[ dR](C)[sP].[dR](A)[sP].[dR](C)[sP].[dR](A)[sP].[ dR](C)[sP].[dR](A)[sP].[dR](C)[sP].[TNA](T)P.[TNA](A)P.[TNA]([5meC])P.[TNA]([5meC])[sP].[TN A](A) |

(continued)

| CMP ID NO | SEQ ID NO | Base sequence 5'-3' | HELM |
|---|---|---|---|
| #274 | 16 | CATCTCAACCTCCGTCA TGT | [TNA]([5meC])[sP].[TNA](A)P.[TNA](T)P.[TNA]([ 5meC])P. [TNA](T)[sP].[dR](C)[sP].[dR](A)[sP].[d R](A)[sP].[dR](C) [sP].[dR](C)[sP].[dR](T)[sP].[d R](C)[sP].[dR]([5meC])[sP]. [dR](G)[sP].[dR](T)[ sP].[TNA]([5meC])P.[TNA](A)P.[TNA] (T)P.[TNA]( G)[sP].[TNA](T) |
| #275 | 17 | AAGGTCTCATACACTCA CTA | [TNA](A)[sP].[TNA](A)P.[TNA](G)P.[TNA](G)P.[T NA](T) [sP].[dR](C)[sP].[dR](T)[sP].[dR](C)[sP].[ dR](A)[sP].[dR](T) [sP].[dR](A)[sP].[dR](C)[sP].[ dR](A)[sP].[dR](C)[sP].[dR](T) [sP].[TNA]([5meC ])P.[TNA](A)P.[TNA]([5meC])P.[TNA](T) [sP].[TN A](A) |
| #276 | 18 | GTACTATCCCATCACTG AAG | [TNA](G)[sP].[TNA](T)P.[TNA](A)P.[TNA]([5meC] )P.[TNA] (T)[sP].[dR](A)[sP].[dR](T)[sP].[dR](C)[ sP].[dR](C)[sP].[dR] (C)[sP].[dR](A)[sP].[dR](T)[s P].[dR](C)[sP].[dR](A)[sP].[dR] (C)[sP].[TNA](T)P .[TNA](G)P.[TNA](A)P.[TNA](A)[sP]. [TNA](G) |
| #277 | 19 | GACCCCTGACTTTCTGG AAA | [TNA](G)[sP].[TNA](A)P.[TNA]([5meC])P.[TNA]([ 5meC])P. [TNA]([5meC])[sP].[dR](C)[sP].[dR](T)[ sP].[dR](G)[sP].[dR] (A)[sP].[dR](C)[sP].[dR](T)[ sP].[dR](T)[sP].[dR](T)[sP].[dR] (C)[sP].[dR](T)[s P].[TNA](G)P.[TNA](G)P.[TNA](A)P.[TNA] (A)[sP]. [TNA](A) |
| #278 | 20 | CAGCGGTACACTCCTTC TCT | [TNA]([5meC])[sP].[TNA](A)P.[TNA](G)P.[TNA]([ 5meC])P. [TNA](G)[sP].[dR](G)[sP].[dR](T)[sP].[d R](A)[sP].[dR](C) [sP].[dR](A)[sP].[dR](C)[sP].[d R](T)[sP].[dR](C)[sP].[dR](C) [sP].[dR](T)[sP].[T NA](T)P.[TNA]([5meC])P.[TNA](T)P. [TNA]([5meC ])[sP].[TNA](T) |
| #279 | 21 | GCCAATATTTGCCCCTC CCC | [TNA](G)[sP].[TNA]([5meC])P.[TNA]([5meC])P.[ TNA](A)P. [TNA](A)[sP].[dR](T)[sP].[dR](A)[sP].[ dR](T)[sP].[dR](T) [sP].[dR](T)[sP].[dR](G)[sP].[ dR](C)[sP].[dR](C)[sP].[dR](C) [sP].[dR](C)[sP].[ TNA](T)P.[TNA]([5meC])P.[TNA]([5meC]) P.[TNA] ([5meC])[sP].[TNA]([5meC]) |
| #280 | 22 | TGCATTTACTTGCCAAC AGA | [TNA](T)[sP].[TNA](G)P.[TNA]([5meC])P.[TNA](A )P.[TNA] (T)[sP].[dR](T)[sP].[dR](T)[sP].[dR](A)[ sP].[dR](C)[sP].[dR] (T)[sP].[dR](T)[sP].[dR](G)[s P].[dR](C)[sP].[dR](C)[sP].[dR] (A)[sP].[TNA](A)P .[TNA]([5meC])P.[TNA](A)P.[TNA](G) [sP].[TNA]( A) |
| #281 | 23 | AAAGAGTAACTACCAGC CAT | [TNA](A)[sP].[TNA](A)P.[TNA](A)P.[TNA](G)P.[T NA](A) [sP].[dR](G)[sP].[dR](T)[sP].[dR](A)[sP].[ dR](A)[sP].[dR](C) [sP].[dR](T)[sP].[dR](A)[sP].[ dR](C)[sP].[dR](C)[sP].[dR](A) [sP].[TNA](G)P.[T NA]([5meC])P.[TNA]([5meC])P.[TNA](A) [sP].[TN A](T) |
| #282 | 24 | AACAGGTCATCTATTCA CAA | [TNA](A)[sP].[TNA](A)P.[TNA]([5meC])P.[TNA](A )P.[TNA] (G)[sP].[dR](G)[sP].[dR](T)[sP].[dR](C)[ sP].[dR](A)[sP].[dR] (T)[sP].[dR](C)[sP].[dR](T)[s P].[dR](A)[sP].[dR](T)[sP].[dR] (T)[sP].[TNA]([5 meC])P.[TNA](A)P.[TNA]([5meC])P.[TNA] (A)[sP]. [TNA](A) |

(continued)

| CMP ID NO | SEQ ID NO | Base sequence 5'-3' | HELM |
|---|---|---|---|
| #283 | 25 | TGTACATTTTGCCCTTAGCT | [TNA](T)[sP].[TNA](G)P.[TNA](T)P.[TNA](A)P.[TN A]([5meC])[sP].[dR](A)[sP].[dR](T)[sP].[dR](T)[s P].[dR](T)[sP].[dR](T)[sP].[dR](G)[sP].[dR](C)[s P].[dR](C)[sP].[dR](C)[sP].[dR](T)[sP].[TNA](T)P .[TNA](A)P.[TNA](G)P.[TNA]([5meC])[sP].[TNA]( T) |
| #284 | 26 | GCCAAGCACTCATATGCAAT | [TNA](G)[sP].[TNA]([5meC])P.[TNA]([5meC])P.[ TNA](A)P.[TNA](A)[sP].[dR](G)[sP].[dR](C)[sP].[ dR](A)[sP].[dR](C)[sP].[dR](T)[sP].[dR](C)[sP].[ dR](A)[sP].[dR](T)[sP].[dR](A)[sP].[dR](T)[sP].[T NA](G)P.[TNA]([5meC])P.[TNA](A)P.[TNA](A)[sP] .[TNA](T) |
| #285 | 27 | GTTATGCTTATTCCCCAATG | [MOE](G)[sP].[MOE](T)[sP].[MOE](T)[sP].[MOE]( A)[sP].[MOE](T)P.[TNA](G)P.[TNA]([5meC])P.[TN A](T)P.[TNA](T)P.[TNA](A)P.[TNA](T)[sP].[dR](T) [sP].[dR](C)[sP].[dR](C)[sP].[dR](C)[sP].[MOE]([ 5meC])[sP].[MOE](A)[sP].[MOE](A)[sP].[MOE](T) [sP].[MOE](G) |
| #286 | 28 | GTGTAATTACCTTTTACTCT | [MOE](G)[sP].[MOE](T)[sP].[MOE](G)[sP].[MOE]( T)[sP].[MOE](A)P.[TNA](A)P.[TNA](T)P.[TNA](T)P.[TNA](A)P.[TNA]([5meC])P.[TNA]([5meC])[sP].[ dR](T)[sP].[dR](T)[sP].[dR](T)[sP].[dR](T)[sP].[ MOE](A)[sP].[MOE]([5meC])[sP].[MOE](T)[sP].[ MOE]([5meC])[sP].[MOE](T) |
| #287 | 29 | CCTTCAGAGATTCAATGCTA | [MOE]([5meC])[sP].[MOE]([5meC])[sP].[MOE](T) [sP].[MOE](T)[sP].[MOE]([5meC])P.[TNA](A)P.[T NA](G)P.[TNA](A)P.[TNA](G)P.[TNA](A)P.[TNA](T )[sP].[dR](T)[sP].[dR](C)[sP].[dR](A)[sP].[dR](A) [sP].[MOE](T)[sP].[MOE](G)[sP].[MOE]([5meC])[ sP].[MOE](T)[sP].[MOE](A) |
| #288 | 30 | GCCAATATTTGCCCCTCCCC | [MOE](G)[sP].[MOE]([5meC])[sP].[MOE]([5meC] )[sP].[MOE](A)[sP].[MOE](A)P.[TNA](T)P.[TNA](A )P.[TNA](T)P.[TNA](T)P.[TNA](T)P.[TNA](G)[sP].[ dR](C)[sP].[dR](C)[sP].[dR](C)[sP].[dR](C)[sP].[ MOE](T)[sP].[MOE]([5meC])[sP].[MOE]([5meC])[ sP].[MOE]([5meC])[sP].[MOE]([5meC]) |
| #289 | 31 | TGCTAAACAAATTTCCTTAG | [MOE](T)[sP].[MOE](G)[sP].[MOE]([5meC])[sP].[ MOE](T)[sP].[MOE](A)P.[TNA](A)P.[TNA](A)P.[TN A]([5meC])P.[TNA](A)P.[TNA](A)P.[TNA](A)[sP].[ dR](T)[sP].[dR](T)[sP].[dR](T)[sP].[dR](C)[sP].[ MOE]([5meC])[sP].[MOE](T)[sP].[MOE](T)[sP].[M OE](A)[sP].[MOE](G) |
| #290 | 32 | AACAAATTTCCTTAGTTGGC | [MOE](A)[sP].[MOE](A)[sP].[MOE]([5meC])[sP].[ MOE](A)[sP].[MOE](A)P.[TNA](A)P.[TNA](T)P.[TN A](T)P.[TNA](T)P.[TNA]([5meC])P.[TNA]([5meC] )[sP].[dR](T)[sP].[dR](T)[sP].[dR](A)[sP].[dR](G) [sP].[MOE](T)[sP].[MOE](T)[sP].[MOE](G)[sP].[M OE](G)[sP].[MOE]([5meC]) |
| #291 | 33 | TCAGTGCTATTTTATCCAAT | [MOE](T)[sP].[MOE]([5meC])[sP].[MOE](A)[sP].[ MOE](G)[sP].[MOE](T)P.[TNA](G)P.[TNA]([5meC] )P.[TNA](T)P.[TNA](A)P.[TNA](T)P.[TNA](T)[sP].[ dR](T)[sP].[dR](T)[sP].[dR](A)[sP].[dR](T)[sP].[ MOE]([5meC])[sP].[MOE]([5meC])[sP].[MOE](A)[ sP].[MOE](A)[sP].[MOE](T) |

(continued)

| CMP ID NO | SEQ ID NO | Base sequence 5'-3' | HELM |
|---|---|---|---|
| #292 | 34 | TGTACATTTTGCCCTTA GCT | [MOE](T)[sP].[MOE](G)[sP].[MOE](T)[sP].[MOE]( A)[sP].[MOE]([5meC])P.[TNA](A)P.[TNA](T)P.[TN A](T)P.[TNA](T)P.[TNA](T)P.[TNA](G)[sP].[dR](C )[sP].[dR](C)[sP].[dR](C)[sP].[dR](T)[sP].[MOE](T)[sP].[MOE](A)[sP].[MOE](G)[sP].[MOE]([5meC] )[sP].[MOE](T) |
| #293 | 35 | TTGCTGAAATTGTCTCA ATT | [MOE](T)[sP].[MOE](T)[sP].[MOE](G)[sP].[MOE]( [5meC])[sP].[MOE](T)P.[TNA](G)P.[TNA](A)P.[TN A](A)P.[TNA](A)P.[TNA](T)P.[TNA](T)[sP].[dR](G )[sP].[dR](T)[sP].[dR](C)[sP].[dR](T)[sP].[MOE]( [5meC])[sP].[MOE](A)[sP].[MOE](A)[sP].[MOE](T )[sP].[MOE](T) |
| #294 | 36 | TTGAAGCATACCTTAAC ATC | [MOE](T)[sP].[MOE](T)[sP].[MOE](G)[sP].[MOE]( A)[sP].[MOE](A)P.[TNA](G)P.[TNA]([5meC])P.[TN A](A)P.[TNA](T)P.[TNA](A)P.[TNA]([5meC])[sP].[ dR](C)[sP].[dR](T)[sP].[dR](T)[sP].[dR](A)[sP].[ MOE](A)[sP].[MOE]([5meC])[sP].[MOE](A)[sP].[ MOE](T)[sP].[MOE]([5meC]) |
| #295 | 37 | CTCCAATACTTGTCTTA GCT | [MOE]([5meC])[sP].[MOE](T)[sP].[MOE]([5meC]) [sP].[MOE]([5meC])[sP].[MOE](A)P.[TNA](A)P.[T NA](T)P.[TNA](A)P.[TNA]([5meC])P.[TNA](T)P.[T NA](T)[sP].[dR](G)[sP].[dR](T)[sP].[dR](C)[sP].[ dR](T)[sP].[MOE](T)[sP].[MOE](A)[sP].[MOE](G)[ sP].[MOE]([5meC])[sP].[MOE](T) |
| #296 | 38 | GCCTTAAAGTTACATTC GTT | MOE](G)[sP].[MOE]([5meC])[sP].[MOE]([5meC]) [sP].[MOE](T)[sP].[MOE](T)P.[TNA](A)P.[TNA](A) P.[TNA](A)P.[TNA](G)P.[TNA](T)P.[TNA](T)[sP].[ dR](A)[sP].[dR](C)[sP].[dR](A)[sP].[dR](T)[sP].[ MOE](T)[sP].[MOE]([5meC])[sP].[MOE](G)[sP].[ MOE](T)[sP].[MOE](T) |
| #297 | 39 | AAGGTCTCATACACTCA CTA | [MOE](A)[sP].[MOE](A)[sP].[MOE](G)[sP].[MOE]( G)[sP].[MOE](T)P.[TNA]([5meC])P.[TNA](T)P.[TN A]([5meC])P.[TNA](A)P.[TNA](T)P.[TNA](A)[sP].[ dR](C)[sP].[dR](A)[sP].[dR](C)[sP].[dR](T)[sP].[ MOE]([5meC])[sP].[MOE](A)[sP].[MOE]([5meC])[ sP].[MOE](T)[sP].[MOE](A) |
| #298 | 40 | GTACTATCCCATCACTG AAG | [MOE](G)[sP].[MOE](T)[sP].[MOE](A)[sP].[MOE]( [5meC])[sP].[MOE](T)P.[TNA](A)P.[TNA](T)P.[TN A]([5meC])P.[TNA]([5meC])P.[TNA]([5meC])P.[T NA](A)[sP].[dR](T)[sP].[dR](C)[sP].[dR](A)[sP].[ dR](C)[sP].[MOE](T)[sP].[MOE](G)[sP].[MOE](A)[ sP].[MOE](A)[sP].[MOE](G) |
| #299 | 41 | ATGCAAGTTAAACTTAT CTG | [MOE](A)[sP].[MOE](T)[sP].[MOE](G)[sP].[MOE]( [5meC])[sP].[MOE](A)P.[TNA](A)P.[TNA](G)P.[TN A](T)P.[TNA](T)P.[TNA](A)P.[TNA](A)[sP].[dR](A) [sP].[dR](C)[sP].[dR](T)[sP].[dR](T)[sP].[MOE](A )[sP].[MOE](T)[sP].[MOE]( [5meC])[sP].[MOE](T) [sP].[MOE](G) |
| #300 | 42 | TGCCTCTTCATTGTATTT CT | [MOE](T)[sP].[MOE](G)[sP].[MOE]([5meC])[sP].[ MOE]( [5meC])[sP].[MOE](T)P.[TNA]([5meC])P.[T NA](T)P.[TNA](T)P.[TNA]([5meC])P.[TNA](A)P.[T NA](T)[sP].[dR](T)[sP].[dR](G)[sP].[dR](T)[sP].[ dR](A)[sP].[MOE](T)[sP].[MOE](T)[sP].[MOE](T)[ sP].[MOE]([5meC])[sP].[MOE](T) |

(continued)

| CMP ID NO | SEQ ID NO | Base sequence 5'-3' | HELM |
|---|---|---|---|
| #301 | 43 | CATCAAGGCACTGATCA CTT | [MOE]([5meC])[sP].[MOE](A)[sP].[MOE](T)[sP].[ MOE]( [5meC])[sP].[MOE](A)P.[TNA](A)P.[TNA](G )P.[TNA](G)P. [TNA]([5meC])P.[TNA](A)P.[TNA]([ 5meC])[sP].[dR](T)[sP]. [dR](G)[sP].[dR](A)[sP].[ dR](T)[sP].[MOE]([5meC])[sP]. [MOE](A)[sP].[MO E]([5meC])[sP].[MOE](T)[sP].[MOE](T) |
| #302 | 44 | GCCAAGCACTCATATGC AAT | [MOE](G)[sP].[MOE]([5meC])[sP].[MOE]([5meC] )[sP]. [MOE](A)[sP].[MOE](A)P.[TNA](G)P.[TNA]([ 5meC])P. [TNA](A)P.[TNA]([5meC])P.[TNA](T)P.[ TNA]([5meC])[sP]. [dR](A)[sP].[dR](T)[sP].[dR](A )[sP].[dR](T)[sP].[MOE](G) [sP].[MOE]([5meC])[s P].[MOE](A)[sP].[MOE](A)[sP].[MOE] (T) |
| #303 | 45 | GGGAGTTACTTGCCAAC TTG | [MOE](G)[sP].[MOE](G)[sP].[MOE](G)[sP].[MOE] (A)[sP]. [MOE](G)P.[TNA](T)P.[TNA](T)P.[TNA](A) P.[TNA]([5meC]) P.[TNA](T)P.[TNA](T)[sP].[dR]( G)[sP].[dR](C)[sP].[dR](C) [sP].[dR](A)[sP].[MOE ](A)[sP].[MOE]([5meC])[sP].[MOE] (T)[sP].[MOE] (T)[sP].[MOE](G) |
| #304 | 46 | GAGTTACTTGCCAACT | [LR](G)[sP].[LR](A)[sP].[LR](G)P.[dR](T)[sP].[dR ](T)[sP]. [dR](A)[sP].[dR](C)[sP].[dR](T)[sP].[dR] (T)[sP].[dR](G)[sP]. [dR](C)[sP].[dR](C)[sP].[dR] (A)[sP].[LR](A)[sP].[LR]( [5meC])[sP].[LR](T) |
| #305 | 46 | GAGTTACTTGCCAACT | [LR](G)[sP].[LR](A)[sP].[LR](G)P.[dR](T)P.[dR](T )[sP].[dR] (A)[sP].[dR](C)[sP].[dR](T)[sP].[dR](T) [sP].[dR](G)[sP].[dR] (C)[sP].[dR](C)[sP].[dR](A) [sP].[LR](A)[sP].[LR]([5meC]) [sP].[LR](T) |
| #306 | 46 | GAGTTACTTGCCAACT | [LR](G)[sP].[LR](A)[sP].[LR](G)P.[dR](T)P.[dR](T )P.[dR](A) [sP].[dR](C)[sP].[dR](T)[sP].[dR](T)[sP ].[dR](G)[sP].[dR](C) [sP].[dR](C)[sP].[dR](A)[sP ].[LR](A)[sP].[LR]([5meC])[sP]. [LR](T) |
| #307 | 46 | GAGTTACTTGCCAACT | [LR](G)[sP].[LR](A)[sP].[LR](G)P.[dR](T)P.[dR](T )P.[dR](A) P.[dR](C)[sP].[dR](T)[sP].[dR](T)[sP].[ dR](G)[sP].[dR](C) [sP].[dR](C)[sP].[dR](A)[sP].[ LR](A)[sP].[LR]([5meC])[sP]. [LR](T) |
| #308 | 46 | GAGTTACTTGCCAACT | [LR](G)[sP].[LR](A)[sP].[LR](G)P.[dR](T)P.[dR](T )P.[dR](A) P.[dR](C)P.[dR](T)[sP].[dR](T)[sP].[dR ](G)[sP].[dR](C)[sP]. [dR](C)[sP].[dR](A)[sP].[LR ](A)[sP].[LR]([5meC])[sP].[LR] (T) |

*Example 2:* In vitro *efficacy of oligonucleotides targeting Malat-1 mRNA in A549 cells at two different concentrations (5 and 25 $\mu$M)*

A549 cell line was purchased from ATCC and maintained as recommended by the supplier in a humidified incubator at 37°C with 5% CO2. For assays, 3000 cells/well were seeded in a 96 multi well plate in full culture media. Cells were incubated for 24 hours before addition of oligonucleotides dissolved in PBS for final concentrations as indicated. 3 days after addition of oligonucleotides, the cells were harvested. RNA was extracted using the RNeasy 96 RNA Purification kit (Qiagen) according to the manufacturer's instructions and eluated in 50 $\mu$L of water. The RNA was subsequently diluted 10 times with DNase/RNase free water and heated to 90°C for one minute.

For gene expression analysis, One Step RT-qPCR was performed using qScript™ XLT One-Step RT-qPCR ToughMix®, Low ROX™ (Quantabio) in a duplex setup. The following TaqMan primer assays were used for qRT-PCR: MALAT1, Hs00273907_s1 [FAM-MGB] and endogenous control GAPDH, Hs99999905_m1 [VIC-MGB-PL]. All primer

sets were purchased from Thermo Fisher Scientific. The relative MALAT1 mRNA expression level, also referred to as the knock-down (KD) value, was calculated as percent of control (PBS-treated cells).

Determination of KD values was performed using GraphPad Prism.

The results are shown in Tables 5 and 6. Values separated by "/" indicate the individual results when the compound or control was tested in more than one test vial.

Table 5: Knock-down (KD) values

| CMP ID NO | Sequence | KD (25μM) (%) | SD (%) | KD (5μM) (%) | SD (%) |
|---|---|---|---|---|---|
| CONTROL #1 | GAGttacttgccaA$^{m}$CT | 14/7 | 0/1 | 30/19 | 2/1 |
| #1 | GAG**oT**tacttgccaA$^{m}$CT | 10 | 0 | 24 | 1 |
| #2 | GAG**oToT**acttgccaA$^{m}$CT | 14 | 0 | 28 | 2 |
| #3 | GAG**oToToA**cttgccaA$^{m}$CT | 13 | 0 | 31 | 0 |
| #4 | GAG**oToToAo$^{m}$C**ttgccaA$^{m}$CT | 18 | 0 | 39 | 1 |
| #5 | GAG**oToToAo$^{m}$CoT**tgccaA$^{m}$CT | 35 | 4 | 72 | 2 |
| #6 | **GAoG**ttacttgccaA$^{m}$CT | 4 | 1 | 14 | 2 |
| #7 | **GoAoG**ttacttgccaA$^{m}$CT | 6 | 0 | 21 | 4 |
| #8 | **GoAoGoT**tacttgccaA$^{m}$CT | 10 | 2 | 28 | 5 |
| #9 | **GoAoGoToT**acttgccaA$^{m}$CT | 11 | 0 | 27 | 4 |
| #10 | **GoAoGoToToA**cttgccaA$^{m}$CT | 8 | 1 | 31 | 6 |
| #11 | **GoAoGoToToAo$^{m}$C**ttgccaA$^{m}$CT | 16 | 1 | 41 | 1 |
| #12 | **GoAoGoToToAo$^{m}$CoT**tgccaA$^{m}$CT | 32 | 2 | 63 | 0 |
| #13 | **GoAoGoToToA**cttgccaa$^{m}$CT | 4 | 0 | 13 | 2 |
| #14 | **GoAoGoToToA**cttgccaacT | 3 | 0 | 9 | 1 |
| #15 | **GoAoGoToToAo$^{m}$C**ttgccaa$^{m}$CT | 4 | 0 | 20 | 0 |
| #16 | **GoAoGoToToAo$^{m}$C**ttgccaacT | 2 | 0 | 12 | 1 |
| #17 | **GoAoGoToToAo$^{m}$CoT**tgccaa$^{m}$CT | 5 | 0 | 18 | 0 |
| #18 | **GoAoGoToToAo$^{m}$CoT**tgccaacT | 3 | 0 | 13 | 3 |
| #19 | **Go**AGttacttgccaA$^{m}$C**oT** | 10 | 0 | 20 | 0 |
| #20 | **Go**AGttacttgccaA**o$^{m}$CoT** | 8 | 1 | 21 | 1 |
| #21 | **GoAo**GttacttgccaA**o$^{m}$CoT** | 57 | 4 | 75 | 2 |
| #22 | GA**oG**ttacttgcca**oAo**$^{m}$CT | 21 | 3 | 36 | 1 |
| #23 | GA**oGo**ttacttgcca**oAo**$^{m}$CT | 17 | 1 | 43 | 6 |
| #24 | GA**oGo**ttacttgccaA**o$^{m}$C**T | 4 | 0 | 18 | 3 |
| #25 | GA**oGo**ttacttgccaA**o$^{m}$C**T | 5 | 0 | 18 | 0 |
| #26 | GA**oGo**ttacttgccaA**o$^{m}$Co**T | 7 | 0 | 30 | 1 |
| #27 | G**oA**GttacttgccaA**o$^{m}$C**T | 7 | 1 | 30 | 0 |
| #28 | G**oAo**GttacttgccaA**o$^{m}$C**T | 38 | 0 | 62 | 0 |
| #29 | G**oAo**GttacttgccaA$^{m}$CT | 11 | 2 | 29 | 2 |
| #30 | GA**oGo**ttacttgccaA$^{m}$CT | 7 | 1 | 18 | 1 |

(continued)

| CMP ID NO | Sequence | KD (25μM) (%) | SD (%) | KD (5μM) (%) | SD (%) |
|---|---|---|---|---|---|
| #31 | GAG**oTo**tacttgccaA$^{m}$CT | 7 | 1 | 19 | 0 |
| #32 | GAGt**oTo**acttgccaA$^{m}$CT | 10 | 1 | 27 | 0 |
| #33 | GAGtt**oAo**cttgccaA$^{m}$CT | 18 | 1 | 36 | 0 |
| #34 | GAGtta**o$^{m}$Co**ttgccaA$^{m}$CT | 19 | 0 | 43 | 0 |
| #35 | GAGttac**oTo**tgccaA$^{m}$CT | 23 | 4 | 40 | 1 |
| #36 | GAGttact**oTo**gccaA$^{m}$CT | 26 | 3 | 45 | 1 |
| #37 | GAGttactt**oGo**ccaA$^{m}$CT | 27 | 2 | 61 | 3 |
| #38 | GAGttacttg**o$^{m}$Co**caA$^{m}$CT | 51 | 5 | 78 | 2 |
| #39 | GAGttacttgc**o$^{m}$Co**aA$^{m}$CT | 44 | 3 | 66 | 4 |
| #40 | GAGttacttgcc**oAo**A$^{m}$CT | 25 | 2 | 49 | 3 |
| #41 | GAGttacttgcca**oAo**$^{m}$CT | 9 | 1 | 19 | 1 |
| #42 | GAGttacttgccaA**o$^{m}$Co**T | 3 | 0 | 6 | 0 |
| #43 | GAGttacttgccaA$^{m}$C**oT** | 4 | 0 | 16 | 2 |
| #44 | G**oA**GttacttgccaA$^{m}$CT | 8 | 0 | 29 | 2 |
| #45 | GA**oG**ttacttgccaA$^{m}$CT | 4 | 0 | 14 | 1 |
| #46 | GAG**oT**tacttgccaA$^{m}$CT | 9 | 0 | 31 | 0 |
| #47 | GAGt**oT**acttgccaA$^{m}$CT | 16 | 0 | 37 | 2 |
| #48 | GAGtt**oA**cttgccaA$^{m}$CT | 10 | 0 | 27 | 2 |
| #49 | GAGtta**o$^{m}$C**ttgccaA$^{m}$CT | 13 | 1 | 39 | 3 |
| #50 | GAGttac**oT**tgccaA$^{m}$CT | 18 | 0 | 48 | 1 |
| #51 | GAGttact**oT**gccaA$^{m}$CT | 18 | 3 | 42 | 4 |
| #52 | GAGttactt**oG**ccaA$^{m}$CT | 30 | 0 | 49 | 0 |
| #53 | GAGttacttg**o$^{m}$C**caA$^{m}$CT | 40 | 2 | 64 | 4 |
| #54 | GAGttacttgc**o$^{m}$C**aA$^{m}$CT | 25 | 0 | 39 | 3 |
| #55 | GAGttacttgcc**oA**A$^{m}$CT | 16 | 2 | 30 | 1 |
| #56 | GAGttacttgcca**oA**$^{m}$CT | 6 | 1 | 17 | 1 |
| #57 | G**Ao**GttacttgccaA$^{m}$CT | 8 | 0 | 20 | 1 |
| #58 | GA**Go**ttacttgccaA$^{m}$CT | 4 | 1 | 14 | 2 |
| #59 | GAG**To**tacttgccaA$^{m}$CT | 4 | 2 | 7 | 0 |
| #60 | GAGt**To**acttgccaA$^{m}$CT | 14 | 0 | 33 | 3 |
| #61 | GAGtt**Ao**cttgccaA$^{m}$CT | 17 | 1 | 33 | 3 |
| #62 | GAGtta**$^{m}$Co**ttgccaA$^{m}$CT | 16 | 4 | 43 | 5 |
| #63 | GAGttac**To**tgccaA$^{m}$CT | 23 | 0 | 60 | 8 |
| #64 | GAGttact**To**gccaA$^{m}$CT | 27 | 2 | 66 | 3 |
| #65 | GAGttactt**Go**ccaA$^{m}$CT | 29 | 0 | 67 | 3 |

(continued)

| CMP ID NO | Sequence | KD (25μM) (%) | SD (%) | KD (5μM) (%) | SD (%) |
|---|---|---|---|---|---|
| #66 | GAGttacttg**$^{m}$Co**caA$^{m}$CT | 64 | 4 | 87 | 20 |
| #67 | GAGttacttgc**$^{m}$Co**aA$^{m}$CT | 55 | 3 | 76 | 2 |
| #68 | GAGttacttgcc**Ao**A$^{m}$CT | 15 | 0 | 53 | 4 |
| #69 | GAGttacttgcca**Ao**$^{m}$CT | 7 | 0 | 24 | 7 |
| #70 | GAGttacttgccaA**$^{m}$Co**T | 3 | 0 | 6 | 0 |
| CONTROL #2 | G$^{m}$C$^{m}$CAGgctggttatgA$^{m}$CT$^{m}$CA | 12/8 | 1/1 | 17/14 | 0/0 |
| #71 | **G$^{m}$C$^{m}$CAoG**gctggttatgA$^{m}$CT$^{m}$CA | 5 | 0 | 10 | 1 |
| #72 | **G$^{m}$C$^{m}$CoAoG**gctggttatgA$^{m}$CT$^{m}$CA | 4 | 0 | 8 | 2 |
| #73 | **G$^{m}$Co$^{m}$CoAoG**gctggttatgA$^{m}$CT$^{m}$CA | 4 | 0 | 7 | 1 |
| #74 | **Go$^{m}$Co$^{m}$CoAoG**gctggttatgA$^{m}$CT$^{m}$CA | 3 | 0 | 6 | 1 |
| #75 | **Go$^{m}$Co$^{m}$CoAoGoG**ctggttatgA$^{m}$CT$^{m}$CA | 5 | 0 | 11 | 0 |
| #76 | **Go$^{m}$Co$^{m}$CoAoGoGo$^{m}$C**tggttatgA$^{m}$CT$^{m}$CA | 6 | 1 | 16 | 1 |
| #77 | **Go$^{m}$Co$^{m}$CoAoGoGo$^{m}$CoT**ggttatgA$^{m}$CT$^{m}$CA | 8 | 1 | 18 | 1 |
| #78 | **Go$^{m}$Co$^{m}$CoAoGoGo$^{m}$CoToG**gttatgA$^{m}$CT$^{m}$CA | 14 | 2 | 23 | 2 |
| #79 | **Go$^{m}$Co$^{m}$CoAoGoGo$^{m}$CoToGoG**ttatgA$^{m}$CT$^{m}$CA | 16 | 1 | 26 | 0 |
| #80 | G$^{m}$C$^{m}$CAG**oG**ctggttatgA$^{m}$CT$^{m}$CA | 8 | 1 | 14 | 2 |
| #81 | G$^{m}$C$^{m}$CAG**oGo**ctggttatgA$^{m}$CT$^{m}$CA | 8 | 2 | 15 | 1 |
| #82 | G$^{m}$C$^{m}$CAG**oGo$^{m}$C**tggttatgA$^{m}$CT$^{m}$CA | 9 | 0 | 13 | 2 |
| #83 | G$^{m}$c$^{m}$CAG**oGo$^{m}$Co**tggttatgA$^{m}$CT$^{m}$CA | 7 | 1 | 13 | 2 |
| #84 | G$^{m}$C$^{m}$CAG**oGo$^{m}$CoT**ggttatgA$^{m}$CT$^{m}$CA | 14 | 3 | 18 | 3 |
| #85 | G$^{m}$C$^{m}$CAG**oGo$^{m}$CoT**oggttatgA$^{m}$CT$^{m}$CA | 13 | 1 | 25 | 0 |
| #86 | G$^{m}$C$^{m}$CAG**oGo$^{m}$CoToG**gttatgA$^{m}$CT$^{m}$CA | 11 | 0 | 19 | 1 |
| #87 | G$^{m}$C$^{m}$CAG**oGo$^{m}$CoToGo**gttatgA$^{m}$CT$^{m}$CA | 17 | 3 | 39 | 1 |
| #88 | G$^{m}$C$^{m}$CAG**oGo$^{m}$CoToGoG**ttatgA$^{m}$CT$^{m}$CA | 16 | 1 | 24 | 1 |
| #89 | G$^{m}$C$^{m}$CAG**oGo$^{m}$CoToGoG**ottatgA$^{m}$CT$^{m}$CA | 18 | 2 | 38 | 5 |
| #90 | G$^{m}$C$^{m}$CAG**oGo$^{m}$CoToGoGoT**tatgA$^{m}$CT$^{m}$CA | 28 | 4 | 46 | 1 |
| #91 | **Go**$^{m}$C$^{m}$CAGgctggttatgA$^{m}$CT$^{m}$C**oA** | 11 | 1 | 29 | 0 |
| #92 | **Go**$^{m}$C$^{m}$CAGgctggttatgA$^{m}$CT**o$^{m}$CoA** | 17 | 1 | 28 | 1 |
| #93 | **Go$^{m}$Co**$^{m}$CAGgctggttatgA$^{m}$CT**o$^{m}$CoA** | 13 | 0 | 26 | 0 |
| #101 | G$^{m}$C$^{m}$CAGgctggttatg**Ao$^{m}$CoTo$^{m}$CoA** | 10 | 1 | 14 | 1 |
| #102 | G$^{m}$C$^{m}$CAGgctggttat**GoAo$^{m}$CoTo$^{m}$CoA** | 20 | 3 | 30 | 1 |
| #103 | G$^{m}$C$^{m}$CAGgctggtta**ToGoAo$^{m}$CoTo$^{m}$CoA** | 34 | 3 | 45 | 3 |
| #104 | G$^{m}$C$^{m}$CAGgctggtt**AoToGoAo$^{m}$CoTo$^{m}$CoA** | 33 | 4 | 37 | 2 |
| #105 | G$^{m}$C$^{m}$CAGgctggttatgA$^{m}$CT**$^{m}$CoA** | 10 | 0 | 14 | 1 |
| #106 | G$^{m}$C$^{m}$CAGgctggttatgA$^{m}$CT**o$^{m}$CoA** | 11 | 1 | 18 | 1 |

(continued)

| CMP ID NO | Sequence | KD (25μM) (%) | SD (%) | KD (5μM) (%) | SD (%) |
|---|---|---|---|---|---|
| #107 | **Go**$^{m}$C$^{m}$CAGgctggttatgA$^{m}$CTo$^{m}$C**oA** | 9 | 1 | 16 | 0 |
| #108 | G$^{m}$C**o$^{m}$Co**AGgctggttatgA$^{m}$C**oTo**$^{m}$CA | 16 | 1 | 27 | 6 |
| #109 | G$^{m}$C**o$^{m}$Co**AGgctggttatgA**o$^{m}$Co**T$^{m}$CA | 13 | 1 | 21 | 0 |
| #110 | G$^{m}$C$^{m}$CAG**oGo**ctggttat**oGo**A$^{m}$CT$^{m}$CA | 21 | 1 | 31 | 1 |
| #111 | G$^{m}$C**o$^{m}$Co**AGgctggttatgA**o$^{m}$CoTo**$^{m}$CA | 22 | 1 | 40 | 3 |
| #112 | G$^{m}$C**o$^{m}$CoAo**GgctggttatgA**o$^{m}$CoTo**$^{m}$CA | 27 | 2 | 52 | 5 |
| #113 | G**o$^{m}$Co**$^{m}$CAGgctggttatgA$^{m}$CT$^{m}$CA | 8 | 1 | 12 | 1 |
| #114 | G$^{m}$C**o$^{m}$Co**AGgctggttatgA$^{m}$CT$^{m}$CA | 6 | 1 | 13 | 0 |
| #115 | G$^{m}$C$^{m}$C**oAo**GgctggttatgA$^{m}$CT$^{m}$CA | 7 | 2 | 14 | 2 |
| #116 | G$^{m}$C$^{m}$CA**oGo**gctggttatgA$^{m}$CT$^{m}$CA | 7 | 0 | 12 | 0 |
| #117 | G$^{m}$C$^{m}$CAG**oGo**ctggttatgA$^{m}$CT$^{m}$CA | 9 | 0 | 12 | 2 |
| #118 | G$^{m}$c$^{m}$CAGg**o$^{m}$Co**tggttatgA$^{m}$CT$^{m}$CA | 9 | 1 | 13 | 1 |
| #119 | G$^{m}$C$^{m}$CAGgc**oTo**ggttatgA$^{m}$CT$^{m}$CA | 10 | 1 | 16 | 0 |
| #120 | G$^{m}$C$^{m}$CAGgct**oGo**gttatgA$^{m}$CT$^{m}$CA | 8 | 0 | 11 | 1 |
| #121 | G$^{m}$C$^{m}$CAGgctg**oGo**ttatgA$^{m}$CT$^{m}$CA | 13 | 1 | 24 | 0 |
| #122 | G$^{m}$C$^{m}$CAGgctgg**oTo**tatgA$^{m}$CT$^{m}$CA | 9 | 1 | 16 | 1 |
| #123 | G$^{m}$C$^{m}$CAGgctggt**oTo**atgA$^{m}$CT$^{m}$CA | 12 | 0 | 21 | 0 |
| #124 | G$^{m}$C$^{m}$CAGgctggtt**oAo**tgA$^{m}$CT$^{m}$CA | 21 | 3 | 25 | 2 |
| #125 | G$^{m}$C$^{m}$CAGgctggtta**oTo**gA$^{m}$CT$^{m}$CA | 29 | 2 | 25 | 4 |
| #126 | G$^{m}$c$^{m}$CAGgctggttat**oGo**A$^{m}$CT$^{m}$CA | 20 | 0 | 23 | 1 |
| #127 | G$^{m}$C$^{m}$CAGgctggttatg**oAo**$^{m}$CT$^{m}$CA | 20 | 1 | 25 | 3 |
| #128 | G$^{m}$C$^{m}$CAGgctggttatgA**o$^{m}$Co**T$^{m}$CA | 12 | 1 | 16 | 0 |
| #129 | G$^{m}$C$^{m}$CAGgctggttatgA$^{m}$C**oTo**$^{m}$CA | 13 | 1 | 15 | 0 |
| #130 | G$^{m}$C$^{m}$CAGgctggttatgA$^{m}$CT**o$^{m}$Co**A | 12 | 2 | 22 | 4 |
| #131 | G$^{m}$C$^{m}$CAGgctggttatgA$^{m}$CT$^{m}$C**oA** | 10 | 1 | 16 | 3 |
| #132 | **Go**$^{m}$C$^{m}$CAGgctggttatgA$^{m}$CT$^{m}$CA | 7 | 1 | 11 | 1 |
| #133 | G**o$^{m}$C**$^{m}$CAGgctggttatgA$^{m}$CT$^{m}$CA | 6 | 0 | 13 | 1 |
| #134 | G$^{m}$C**o$^{m}$C**AGgctggttatgA$^{m}$CT$^{m}$CA | 6 | 0 | 11 | 2 |
| #135 | G$^{m}$C$^{m}$C**oA**GgctggttatgA$^{m}$CT$^{m}$CA | 6 | 0 | 10 | 1 |
| #136 | G$^{m}$C$^{m}$CA**oG**gctggttatgA$^{m}$CT$^{m}$CA | 6 | 0 | 9 | 1 |
| #137 | G$^{m}$C$^{m}$CAG**oG**ctggttatgA$^{m}$CT$^{m}$CA | 13 | 1 | 19 | 3 |
| #138 | G$^{m}$C$^{m}$CAGg**o$^{m}$C**tggttatgA$^{m}$CT$^{m}$CA | 12 | 2 | 14 | 1 |
| #139 | G$^{m}$C$^{m}$CAGgc**oT**ggttatgA$^{m}$CT$^{m}$CA | 17 | 3 | 20 | 1 |
| #140 | G$^{m}$C$^{m}$CAGgct**oG**gttatgA$^{m}$CT$^{m}$CA | 13 | 1 | 15 | 1 |
| #141 | G$^{m}$C$^{m}$CAGgctg**oG**ttatgA$^{m}$CT$^{m}$CA | 12 | 1 | 12 | 1 |

(continued)

| CMP ID NO | Sequence | KD (25μM) (%) | SD (%) | KD (5μM) (%) | SD (%) |
|---|---|---|---|---|---|
| #142 | G$^{m}$C$^{m}$CAGgctgg**oT**tatgA$^{m}$CT$^{m}$CA | 14 | 1 | 14 | 1 |
| #143 | G$^{m}$C$^{m}$CAGgctggt**oT**atgA$^{m}$CT$^{m}$CA | 21 | 1 | 21 | 1 |
| #144 | G$^{m}$C$^{m}$CAGgctggtt**oA**tgA$^{m}$CT$^{m}$CA | 17 | 0 | 18 | 1 |
| #145 | G$^{m}$C$^{m}$CAGgctggtta**oT**gA$^{m}$CT$^{m}$CA | 30 | 4 | 34 | 6 |
| #146 | G$^{m}$C$^{m}$CAGgctggttat**oG**A$^{m}$CT$^{m}$CA | 31 | 5 | 28 | 0 |
| #147 | G$^{m}$C$^{m}$CAGgctggttatg**oA**$^{m}$CT$^{m}$CA | 16 | 1 | 29 | 5 |
| #148 | G$^{m}$C$^{m}$CAGgctggttatgA**o$^{m}$C**T$^{m}$CA | 17 | 4 | 21 | 1 |
| #149 | G$^{m}$c$^{m}$CAGgctggttatgA$^{m}$C**oT**$^{m}$CA | 18 | 6 | 26 | 2 |
| #150 | G$^{m}$C$^{m}$CAGgctggttatgA$^{m}$CT**o$^{m}$C**A | 12 | 3 | 17 | 2 |
| CONTROL #3 | GG$^{m}$CATatgcagataaTGTT$^{m}$C | 9 | 2 | 12 | 2 |
| #151 | GG$^{m}$CAT**oA**tgcagataaTGTT$^{m}$C | 12 | 1 | 17 | 6 |
| #152 | GG$^{m}$CAT**oAoT**gcagataaTGTT$^{m}$C | 12 | 1 | 15 | 2 |
| #153 | GG$^{m}$CAT**oAoToG**cagataaTGTT$^{m}$C | 18 | 1 | 24 | 5 |
| #154 | GG$^{m}$CAT**oAoToGo$^{m}$C**agataaTGTT$^{m}$C | 22 | 0 | 31 | 5 |
| #155 | GG$^{m}$CAT**oAoToGo$^{m}$CoA**gataaTGTT$^{m}$C | 26 | 3 | 36 | 6 |
| #156 | **GG$^{m}$CAoT**atgcagataaTGTT$^{m}$C | 5 | 1 | 9 | 2 |
| #157 | **GG$^{m}$CoAoT**atgcagataaTGTT$^{m}$C | 3 | 0 | 8 | 0 |
| #158 | **GGo$^{m}$CoAoT**atgcagataaTGTT$^{m}$C | 4 | 0 | 8 | 0 |
| #159 | **GoGo$^{m}$CoAoT**atgcagataaTGTT$^{m}$C | 3 | 0 | 8 | 2 |
| #160 | **GoGo$^{m}$CoAoToA**tgcagataaTGTT$^{m}$C | 5 | 0 | 13 | 1 |
| #161 | **GoGo$^{m}$CoAoToAoT**gcagataaTGTT$^{m}$C | 6 | 0 | 11 | 0 |
| #162 | **GoGo$^{m}$CoAoToAoToG**cagataaTGTT$^{m}$C | 11 | 0 | 23 | 0 |
| #163 | **GoGo$^{m}$CoAoToAoToGo$^{m}$C**agataaTGTT$^{m}$C | 13 | 4 | 19 | 0 |
| #164 | **GoGo$^{m}$CoAoToAoToGo$^{m}$CoA**gataaTGTT$^{m}$C | 19 | 3 | 29 | 0 |
| #165 | **GOGO$^{m}$COAOTOAOTOGO$^{m}$COA**gataatGTT$^{m}$C | 5 | 1 | 10 | 0 |
| #166 | **GoGo$^{m}$CoAoToAoToGo$^{m}$CoA**gataatgTT$^{m}$C | 4 | 1 | 9 | 1 |
| #167 | **GoGo$^{m}$CoAoToAoToGo$^{m}$CoAoG**ataatGTT$^{m}$C | 18 | 7 | 30 | 3 |
| #168 | **GoGo$^{m}$CoAoToAoToGo$^{m}$CoAoG**ataatgTT$^{m}$C | 12 | 1 | 27 | 2 |
| #169 | **Go**G$^{m}$CATatgcagataaTGTT**o$^{m}$C** | 5 | 0 | 10 | 0 |
| #170 | **Go**G$^{m}$CATatgcagataaTGT**oTo$^{m}$C** | 6 | 0 | 6 | 0 |
| #171 | **GoGo**$^{m}$CATatgcagataaTGT**oTo$^{m}$C** | 5 | 0 | 7 | 1 |
| #179 | GG$^{m}$CATatgcagataa**ToGoToTo$^{m}$C** | 8 | 2 | 13 | 0 |
| #180 | GG$^{m}$CATatgcagata**AoToGoToTo$^{m}$C** | 10 | 1 | 17 | 1 |
| #181 | GG$^{m}$CATatgcagat**AoAoToGoToTo$^{m}$C** | 12 | 0 | 28 | 1 |
| #182 | GG$^{m}$CATatgcaga**ToAoAoToGoToTo$^{m}$C** | 18 | 0 | 46 | 0 |

(continued)

| CMP ID NO | Sequence | KD (25μM) (%) | SD (%) | KD (5μM) (%) | SD (%) |
|---|---|---|---|---|---|
| CONTROL #4 | GGGAGttacttgccaA$^{m}$CTTG | 5/4/6 | 0/0/ 2 | 8/11/12 | 1/1/ 1 |
| #192 | GGGAG**oT**tacttgccaA$^{m}$CTTG | 7 | 2 | 14 | 4 |
| #193 | GGGAG**oToT**acttgccaA$^{m}$CTTG | 7 | 1 | 16 | 1 |
| #194 | GGGAG**oToToA**cttgccaA$^{m}$CTTG | 7 | 1 | 17 | 1 |
| #195 | GGGAG**oToToAo$^{m}$C**ttgccaA$^{m}$CTTG | 7 | 1 | 19 | 5 |
| #196 | GGGAG**oToToAo$^{m}$CoT**tgccaA$^{m}$CTTG | 11 | 2 | 26 | 1 |
| #197 | GGGAG**oToToAo$^{m}$CoToT**gccaA$^{m}$CTTG | 13 | 1 | 24 | 1 |
| #204 | **GoGoGoAoG**ttacttgcca**Ao$^{m}$CoToToG** | 4 | 0 | 8 | 1 |
| #205 | **GGoGoAoG**ttacttgcca**Ao$^{m}$CoToTG** | 4 | 1 | 8 | 1 |
| #206 | **GGGAoG**ttacttgccaA$^{m}$CTTG | 4 | 0 | 5 | 0 |
| #207 | **GGGoAoG**ttacttgccaA$^{m}$CTTG | 4 | 0 | 7 | 0 |
| #208 | **GGoGoAoG**ttacttgccaA$^{m}$CTTG | 3 | 0 | 9 | 0 |
| #209 | **GoGoGoAoG**ttacttgccaA$^{m}$CTTG | 4 | 0 | 7 | 1 |
| #210 | **GoGoGoAoGoT**tacttgccaA$^{m}$CTTG | 3 | 0 | 7 | 1 |
| #211 | **GoGoGoAoGoToT**acttgccaA$^{m}$CTTG | 2 | 0 | 4 | 0 |
| #212 | **GoGoGoAoGoToToA**cttgccaA$^{m}$CTTG | 3 | 1 | 5 | 0 |
| #213 | **GoGoGoAoGoToToAo$^{m}$C**ttgccaA$^{m}$CTTG | 6 | 0 | 10 | 0 |
| #214 | **GoGoGoAoGoToToAo$^{m}$CoT**tgccaA$^{m}$CTTG | 6/5 | 1/0 | 17/11 | 1/1 |
| #215 | **GoGoGoAoGoToToAo$^{m}$CoT**tgccaa$^{m}$CTTG | 3 | 0 | 7 | 0 |
| #216 | **GoGoGoAoGoToToAo$^{m}$CoT**tgccaacTTG | 1 | 0 | 3 | 0 |
| #217 | GGGAGttacttgcca**Ao$^{m}$CoToToG** | 5 | 0 | 16 | 3 |
| #218 | GGGAGttacttgc**$^{m}$CoAoAo$^{m}$CoToToG** | 27 | 3 | 66 | 3 |
| CONTROL #5 | TG$^{m}$C$^{m}$CTttagaattctAGA$^{m}$CA | 12/3 | 1/0 | 23/8 | 0/0 |
| #219 | TG$^{m}$C$^{m}$CT**oT**taggattctAGA$^{m}$CA | 16 | 2 | 32 | 4 |
| #220 | TG$^{m}$C$^{m}$CT**oToT**aggattctAGA$^{m}$CA | 9 | 1 | 14 | 2 |
| #221 | TG$^{m}$C$^{m}$CT**oToToA**ggattctAGA$^{m}$CA | 13 | 2 | 24 | 3 |
| #222 | TG$^{m}$C$^{m}$CT**oToToAoG**gattctAGA$^{m}$CA | 14 | 2 | 29 | 4 |
| #223 | TG$^{m}$C$^{m}$CT**oToToAoGoG**attctAGA$^{m}$CA | 38 | 2 | 59 | 2 |
| #224 | TG$^{m}$C$^{m}$CT**oToToAoGoGoA**ttctAGA$^{m}$CA | 29 | 2 | 56 | 4 |
| #227 | **TG$^{m}$C$^{m}$CoT**ttaggattctAGA$^{m}$CA | 23 | 1 | 43 | 13 |
| #228 | **TG$^{m}$Co$^{m}$CoT**ttaggattctAGA$^{m}$CA | 15 | 1 | 33 | 2 |
| #229 | **TGo$^{m}$Co$^{m}$CoT**ttaggattctAGA$^{m}$CA | 13 | 1 | 29 | 2 |
| #230 | **ToGo$^{m}$Co$^{m}$CoT**ttaggattctAGA$^{m}$CA | 10 | 0 | 28 | 3 |
| #231 | **ToGo$^{m}$Co$^{m}$CoToT**taggattctAGA$^{m}$CA | 10 | 0 | 28 | 4 |
| #232 | **ToGo$^{m}$Co$^{m}$CoToToT**aggattctAGA$^{m}$CA | 11 | 1 | 21 | 2 |

(continued)

| CMP ID NO | Sequence | KD (25μM) (%) | SD (%) | KD (5μM) (%) | SD (%) |
|---|---|---|---|---|---|
| #233 | **ToGo$^{m}$Co$^{m}$CoToToToA**ggattctAGA$^{m}$CA | 13 | 1 | 33 | 2 |
| #234 | **ToGo$^{m}$Co$^{m}$CoToToToAoG**gattctAGA$^{m}$CA | 7 | 0 | 13 | 1 |
| #235 | **ToGo$^{m}$Co$^{m}$CoToToToAoGoG**attctAGA$^{m}$CA | 17/18 | 1/2 | 24/31 | 1/7 |
| #236 | **ToGo$^{m}$Co$^{m}$CoToToToAoGoG**attctaGA$^{m}$CA | 20 | 1 | 35 | 2 |
| #237 | **ToGo$^{m}$Co$^{m}$CoToToToAoGoG**attctagA$^{m}$CA | 11 | 0 | 22 | 2 |
| #238 | TG$^{m}$C$^{m}$CTttaggattct**AoGoAo$^{m}$CoA** | 13 | 3 | 38 | 8 |
| #239 | TG$^{m}$C$^{m}$CTttaggattc**ToAoGoAo$^{m}$CoA** | 34 | 0 | 46 | 1 |
| #240 | TG$^{m}$C$^{m}$CTttaggatt**$^{m}$CoToAoGoAo$^{m}$CoA** | 41 | 0 | 52 | 0 |
| CONTROL #6 | $^{m}$C$^{m}$CAGGctggttatga$^{m}$CT$^{m}$CAG | 4 | 0 | 10 | 2 |
| #241 | **$^{m}$C$^{m}$CAGoG**ctggttatga$^{m}$CT$^{m}$CAG | 3 | 0 | 6 | 0 |
| #242 | **$^{m}$C$^{m}$CAoGoG**ctggttatga$^{m}$CT$^{m}$CAG | 2 | 0 | 6 | 1 |
| #243 | **$^{m}$C$^{m}$CoAoGoG**ctggttatga$^{m}$CT$^{m}$CAG | 3 | 1 | 6 | 0 |
| #244 | **$^{m}$Co$^{m}$CoAoGoG**ctggttatga$^{m}$CT$^{m}$CAG | 6 | 0 | 11 | 2 |
| #245 | **$^{m}$Co$^{m}$CoAoGoGo$^{m}$C**tggttatga$^{m}$CT$^{m}$CAG | 6 | 0 | 14 | 1 |
| #246 | **$^{m}$Co$^{m}$CoAoGoGo$^{m}$CoT**ggttatga$^{m}$CT$^{m}$CAG | 6 | 1 | 13 | 1 |
| #247 | **$^{m}$Co$^{m}$CoAoGoGo$^{m}$CoToG**gttatga$^{m}$CT$^{m}$CAG | 8 | 0 | 16 | 4 |
| #248 | **$^{m}$Co$^{m}$CoAoGoGo$^{m}$CoToGoG**ttatga$^{m}$CT$^{m}$CAG | 8 | 1 | 21 | 0 |
| #267 | **GToToAoT**gcttattccc**$^{m}$CoAoAoTG** | 15 | 1 | 27 | 0 |
| #268 | **$^{m}$CTo$^{m}$CoAoG**cctttatcac**ToCoAoG$^{m}$C** | 17 | 0 | 33 | 3 |
| #269 | **AAo$^{m}$CoAoA**atttccttag**ToToGoG$^{m}$C** | 10 | 0 | 23 | 1 |
| #270 | **TAo$^{m}$CoAo$^{m}$C**cagtcctttt**AoGoToAG** | 6 | 0 | 13 | 0 |
| #271 | **AToGo$^{m}$CoT**catcacttta**ToGoAoAG** | 7 | 0 | 16 | 2 |
| #272 | **AAoAoAoG**gcttag$^{m}$cgcc**$^{m}$CoAo$^{m}$Co$^{m}$CT** | 17 | 1 | 28 | 1 |
| #273 | **AAoGoAoG**aaccacacac**ToAo$^{m}$Co$^{m}$CA** | 11 | 0 | 26 | 0 |
| #274 | **$^{m}$CAoTo$^{m}$CoT**caacctc$^{m}$cgt**$^{m}$CoAoToGT** | 6 | 0 | 14 | 0 |
| #275 | **AAoGoGoT**ctcatacact**$^{m}$CoAo$^{m}$CoTA** | 7 | 0 | 22 | 1 |
| #276 | **GToAo$^{m}$CoT**atcccatcac**ToGoAoAG** | 9 | 0 | 16 | 1 |
| #277 | **GAo$^{m}$Co$^{m}$Co$^{m}$C**ctgactttct**GoGoAoAA** | 15 | 1 | 29 | 1 |
| #278 | **$^{m}$CAoGo$^{m}$CoG**gtacactcct**To$^{m}$CoToCT** | 3 | 0 | 6 | 0 |
| #279 | **G$^{m}$Co$^{m}$CoAoA**tatttgcccc**To$^{m}$Co$^{m}$Co$^{m}$C$^{m}$C** | 9 | 0 | 23 | 4 |
| #280 | **TGo$^{m}$CoAoT**ttacttgcca**Ao$^{m}$CoAoGA** | 8 | 0 | 15 | 0 |
| #281 | **AAoAoGoA**gtaactacca**Go$^{m}$Co$^{m}$CoAT** | 9 | 0 | 23 | 1 |
| #282 | **AAo$^{m}$CoAoG**gtcatctatt**$^{m}$CoAo$^{m}$CoAA** | 5 | 0 | 13 | 0 |
| #283 | **TGoToAo$^{m}$C**attttgccct**ToAoGo$^{m}$CT** | 2 | 0 | 6 | 0 |
| #284 | **G$^{m}$Co$^{m}$CoAoA**gcactcatat**Go$^{m}$CoAoAT** | 10 | 0 | 25 | 1 |

(continued)

| CMP ID NO | Sequence | KD (25μM) (%) | SD (%) | KD (5μM) (%) | SD (%) |
|---|---|---|---|---|---|
| #285 | GTTAT**oGo$^{m}$CoToToAoT**tccc$^{m}$CAATG | 11 | 0 | 19 | 0 |
| #286 | GTGTA**oAoToToAo$^{m}$Co$^{m}$C**ttttA$^{m}$CT$^{m}$CT | 6 | 1 | 14 | 1 |
| #287 | $^{m}$C$^{m}$CTT$^{m}$C**oAoGoAoGoAoT**tcaaTG$^{m}$CTA | 11 | 1 | 20 | 1 |
| #288 | G$^{m}$C$^{m}$CAA**oToAoToToToG**ccccT$^{m}$C$^{m}$C$^{m}$C$^{m}$C | 11 | 1 | 21 | 1 |
| #289 | TG$^{m}$CTA**oAoAo$^{m}$CoAoAoA**tttc$^{m}$CTTAG | 9 | 0 | 20 | 2 |
| #290 | AA$^{m}$CAA**oAoToToTo$^{m}$Co$^{m}$C**ttagTTGG$^{m}$C | 10 | 0 | 16 | 1 |
| #291 | T$^{m}$CAGT**oGo$^{m}$CoToAoToT**ttat$^{m}$C$^{m}$CAAT | 4 | 0 | 8 | 0 |
| #292 | TGTA$^{m}$C**oAoToToToToG**ccctTAG$^{m}$CT | 9 | 1 | 14 | 1 |
| #293 | TTG$^{m}$CT**oGoAoAoAoToT**gtct$^{m}$CAATT | 7 | 1 | 14 | 1 |
| #294 | TTGAA**oGo$^{m}$CoAoToAo$^{m}$C**cttaA$^{m}$CAT$^{m}$C | 10 | 0 | 19 | 1 |
| #295 | $^{m}$CT$^{m}$C$^{m}$CA**oAoToAo$^{m}$CoToT**gtctTAG$^{m}$CT | 9 | 1 | 15 | 1 |
| #296 | G$^{m}$c$^{m}$CTT**oAoAoAoGoToT**acatT$^{m}$CGTT | 10 | 1 | 21 | 2 |
| #297 | AAGGT**o$^{m}$CoTo$^{m}$CoAoToA**cact$^{m}$CA$^{m}$CTA | 14 | 0 | 30 | 1 |
| #298 | GTA$^{m}$CT**oAoTo$^{m}$Co$^{m}$Co$^{m}$CoA**tcacTGAAG | 12 | 0 | 24 | 1 |
| #299 | ATG$^{m}$CA**oAoGoToToAoA**acttAT$^{m}$CTG | 11 | 0 | 20 | 1 |
| #300 | TG$^{m}$C$^{m}$CT**o$^{m}$CoToTo$^{m}$CoAoT**tgtaTTT$^{m}$CT | 7 | 0 | 11 | 0 |
| #301 | $^{m}$CAT$^{m}$CA**oAoGoGo$^{m}$CoAo$^{m}$C**tgat$^{m}$CA$^{m}$CTT | 10 | 1 | 15 | 1 |
| #302 | G$^{m}$C$^{m}$CAA**oGo$^{m}$CoAo$^{m}$CoTo$^{m}$C**atatG$^{m}$CAAT | 14 | 1 | 24 | 1 |
| #303 | GGGAG**oToToAo$^{m}$CoToT**gccaA$^{m}$CTTG | 8 | 0 | 12 | 0 |

Table 6: Knock-down (KD) values - DNA PO vs TNA PO

| CMP ID NO | Sequence | KD (25μM) | SD | KD (5μM) | SD |
|---|---|---|---|---|---|
| CONTROL #1 | GAGttacttgccaA$^{m}$CT | 18 | 0 | 31 | 0 |
| #1 | GAG**oT**tacttgccaA$^{m}$CT | 10 | 0 | 24 | 1 |
| #2 | GAG**oToT**acttgccaA$^{m}$CT | 14 | 0 | 28 | 2 |
| #3 | GAG**oToToA**cttgccaA$^{m}$CT | 13 | 0 | 31 | 0 |
| #4 | GAG**oToToAo$^{m}$C**ttgccaA$^{m}$CT | 18 | 0 | 39 | 1 |
| #5 | GAG**oToToAo$^{m}$CoT**tgccaA$^{m}$CT | 35 | 4 | 72 | 2 |
| #304 | GAG**o**ttacttgccaA$^{m}$CT | 20 | 1 | 50 | 0 |
| #305 | GAG**o**t**o**tacttgccaA$^{m}$CT | 44 | 1 | 72 | 1 |
| #306 | GAG**o**t**o**t**o**acttgccaA$^{m}$CT | 57 | 0 | 78 | 3 |
| #307 | GAG**o**t**o**t**o**a**o**cttgccaA$^{m}$CT | 68 | 1 | 92 | 2 |
| #308 | GAG**o**t**o**t**o**a**o**c**o**ttgccaA$^{m}$CT | 88 | 1 | 95 | 2 |

**A, G, $^{m}$C, T (in bold):** represent TNA modification

A, G, $^{m}$C, T (underline): represent MOE modification
A, G, $^{m}$C, T represent LNA nucleotides
a, g, c, $^{m}$c, t represent DNA nucleotides
**o** represents phosphodiester linkages
all other linkages were prepared as phosphorothioates

*Example 3:* In vitro *potency and efficacy of oligonucleotides targeting MALAT1 mRNA in A549 cells at different concentrations for a dose response curve*

A549 cell lines were purchased from ATCC and maintained as recommended by the supplier in a humidified incubator at 37°C with 5% CO2. For assays, 3500 cells/well (A549) were seeded in a 96 multi well plate in culture media. Cells were incubated for 24 hours before addition of oligonucleotides dissolved in PBS. Concentration range of oligonucleotides: highest concentration 25 μM, 1:1 dilutions in 8 steps. Three days after addition of oligonucleotides, the cells were harvested. RNA was extracted using the PureLink Pro 96 RNA Purification kit (Thermo Fisher Scientific) according to the manufacturer's instructions and eluated in 50 μL of water. The RNA was subsequently diluted 10 times with DNase/RNase free Water (Gibco) and heated to 90°C for one minute.

For gene expression analysis, One Step RT-qPCR was performed using qScript™ XLT One-Step RT-qPCR ToughMix®, Low ROX™ (Quantabio) in a duplex setup. The following TaqMan primer assays were used for qPCR: MALAT1, Hs00273907_s1 (FAM-MGB) with endogenous control GAPDH. All primer sets were purchased from Thermo Fisher Scientific. The relative expression level of MALAT1 mRNA is shown as percent of control (PBS-treated cells) and IC50 values have been determined using GraphPad Prism7 on data from n=2 biological replicates.

The results are shown in Table 7 below. Values separated by "/" indicate the individual results when the compound or control was tested in more than one test vial.

Table 7: *In vitro* potency results (IC50)

| CMP ID NO | Sequence | $IC_{50}$ (N=2) [uM] |
|---|---|---|
| CONTROL #1 | GAGttacttgccaA$^{m}$CT | 0.58/1.31 |
| #1 | GAG**oT**tacttgccaA$^{m}$CT | 1.04 |
| #2 | GAG**oToT**acttgccaA$^{m}$CT | 1.98 |
| #3 | GAG**oToToA**cttgccaA$^{m}$CT | 1.36 |
| #4 | GAG**oToToAo$^{m}$C**ttgccaA$^{m}$CT | 2.33 |
| #5 | GAG**oToToAo$^{m}$CoT**tgccaA$^{m}$CT | 4.60 |
| #6 | **GAoG**ttacttgccaA$^{m}$CT | 0.37 |
| #7 | **GoAoG**ttacttgccaA$^{m}$CT | 0.53 |
| #8 | **GoAoGoT**tacttgccaA$^{m}$CT | 0.94 |
| #9 | **GoAoGoToT**acttgccaA$^{m}$CT | 0.78 |
| #10 | **GoAoGoToToA**cttgccaA$^{m}$CT | 0.74 |
| #11 | **GoAoGoToToAo$^{m}$C**ttgccaA$^{m}$CT | 1.49 |
| #12 | **GoAoGoToToAo$^{m}$CoT**tgccaA$^{m}$CT | 6.11 |
| #19 | **Go**AGttacttgccaA$^{m}$C**oT** | 0.34 |
| #20 | **Go**AGttacttgccaA**o$^{m}$CoT** | 0.41 |
| #21 | **GoAo**GttacttgccaA**o$^{m}$CoT** | 4.46 |
| #22 | GA**oG**ttacttgcca**oAo**$^{m}$CT | 2.98 |
| #23 | GA**oGo**ttacttgcca**oAo**$^{m}$CT | 3.51 |
| #24 | GA**oG**ttacttgccaA**o$^{m}$C**T | 1.01 |
| #25 | GA**oGo**ttacttgccaA**o$^{m}$C**T | 0.61 |

(continued)

| CMP ID NO | Sequence | $IC_{50}$ (N=2) [uM] |
|---|---|---|
| #26 | GA**oGo**ttacttgccaA**o$^{m}$Co**T | 0.81 |
| #27 | G**oA**GttacttgccaA**o$^{m}$C**T | 0.94 |
| #28 | G**oAo**GttacttgccaA**o$^{m}$C**T | 9.03 |
| #29 | G**oAo**GttacttgccaA$^{m}$CT | 1.6 |
| #30 | GA**oGo**ttacttgccaA$^{m}$CT | 0.5 |
| #31 | GAG**oTo**tacttgccaA$^{m}$CT | 0.5 |
| #32 | GAGt**oTo**acttgccaA$^{m}$CT | 1.1 |
| #33 | GAGtt**oAo**cttgccaA$^{m}$CT | 1.6 |
| #34 | GAGtta**o$^{m}$Co**ttgccaA$^{m}$CT | 2.7 |
| #35 | GAGttac**oTo**tgccaA$^{m}$CT | 1.4 |
| #36 | GAGttact**oTo**gccaA$^{m}$CT | 4.1 |
| #37 | GAGttactt**oGo**ccaA$^{m}$CT | 3.1 |
| #38 | GAGttacttg**o$^{m}$Co**caA$^{m}$CT | 4.7 |
| #39 | GAGttacttgc**o$^{m}$Co**aA$^{m}$CT | 3.2 |
| #40 | GAGttacttgcc**oAo**A$^{m}$CT | 0.8 |
| #41 | GAGttacttgcca**oAo**$^{m}$CT | 0.5 |
| #42 | GAGttacttgccaA**o$^{m}$Co**T | 0.1 |
| #43 | GAGttacttgccaA$^{m}$C**oT** | 0.3 |
| CONTROL #2 | G$^{m}$C$^{m}$CAGgctggttatgA$^{m}$CT$^{m}$CA | 0.46/0.26/0.74 |
| #71 | **G$^{m}$C$^{m}$CAoG**gctggttatgA$^{m}$CT$^{m}$CA | 0.16 |
| #72 | **G$^{m}$C$^{m}$CoAoG**gctggttatgA$^{m}$CT$^{m}$CA | 0.20 |
| #73 | **G$^{m}$Co$^{m}$CoAoG**gctggttatgA$^{m}$CT$^{m}$CA | 0.18 |
| #74 | **Go$^{m}$Co$^{m}$CoAoG**gctggttatgA$^{m}$CT$^{m}$CA | 0.13 |
| #75 | **Go$^{m}$Co$^{m}$CoAoGoG**ctggttatgA$^{m}$CT$^{m}$CA | 0.41 |
| #76 | **Go$^{m}$Co$^{m}$CoAoGoGo$^{m}$C**tggttatgA$^{m}$CT$^{m}$CA | 0.38 |
| #77 | **Go$^{m}$Co$^{m}$CoAoGoGo$^{m}$CoT**ggttatgA$^{m}$CT$^{m}$CA | 0.52 |
| #78 | **Go$^{m}$Co$^{m}$CoAoGoGo$^{m}$CoToG**gttatgA$^{m}$CT$^{m}$CA | 0.52 |
| #79 | **Go$^{m}$Co$^{m}$CoAoGoGo$^{m}$CoToGoG**ttatgA$^{m}$CT$^{m}$CA | 0.84 |
| #80 | G$^{m}$C$^{m}$CAG**oG**ctggttatgA$^{m}$CT$^{m}$CA | 0.78 |
| #81 | G$^{m}$C$^{m}$CAG**oGo**ctggttatgA$^{m}$CT$^{m}$CA | 0.54 |
| #82 | G$^{m}$C$^{m}$CAG**oGo$^{m}$C**tggttatgA$^{m}$CT$^{m}$CA | 0.53 |
| #83 | G$^{m}$C$^{m}$CAG**oGo$^{m}$Co**tggttatgA$^{m}$CT$^{m}$CA | 0.74 |
| #84 | G$^{m}$C$^{m}$CAG**oGo$^{m}$CoT**ggttatgA$^{m}$CT$^{m}$CA | 0.88 |
| #85 | G$^{m}$C$^{m}$CAG**oGo$^{m}$CoTo**ggttatgA$^{m}$CT$^{m}$CA | 1.91 |
| #86 | G$^{m}$C$^{m}$CAG**oGo$^{m}$CoToG**gttatgA$^{m}$CT$^{m}$CA | 1.07 |

(continued)

| CMP ID NO | Sequence | $IC_{50}$ (N=2) [uM] |
|---|---|---|
| #87 | G$^{m}$C$^{m}$CAG**oGo$^{m}$CoToGo**gttatgA$^{m}$CT$^{m}$CA | 1.71 |
| #88 | G$^{m}$C$^{m}$CAG**oGo$^{m}$CoToG**oGttatgA$^{m}$CT$^{m}$CA | 2.49 |
| #89 | G$^{m}$C$^{m}$CAG**oGo$^{m}$CoToGoGo**ttatgA$^{m}$CT$^{m}$CA | 4.80 |
| #90 | G$^{m}$C$^{m}$CAG**oGo$^{m}$CoToGoGoT**tatgA$^{m}$CT$^{m}$CA | 8.51 |
| #91 | **Go**$^{m}$C$^{m}$CAGgctggttatgA$^{m}$CT$^{m}$C**oA** | 0.28 |
| #92 | **Go**$^{m}$C$^{m}$CAGgctggttatgA$^{m}$CT**o$^{m}$CoA** | 0.26 |
| #93 | **Go$^{m}$Co**$^{m}$CAGgctggttatgA$^{m}$CT**o$^{m}$CoA** | 0.36/0.43 |
| #94 | **Go$^{m}$Co**$^{m}$CAG**oG**ctggttatgA$^{m}$CT**o$^{m}$CoA** | 1.17 |
| #95 | **Go$^{m}$Co**$^{m}$CAG**oGo$^{m}$C**tggttatgA$^{m}$CT**o$^{m}$CoA** | 0.67 |
| #96 | **Go$^{m}$Co**$^{m}$CAG**oGo$^{m}$CoT**ggttatgA$^{m}$CT**o$^{m}$CoA** | 0.89 |
| #97 | **Go$^{m}$Co**$^{m}$CAG**oGo$^{m}$CoToG**gttatgA$^{m}$CT**o$^{m}$CoA** | 0.45 |
| #98 | **Go$^{m}$Co**$^{m}$CAG**oGo$^{m}$CoToGoG**ttatgA$^{m}$CT**o$^{m}$CoA** | 0.99 |
| #99 | **Go$^{m}$Co$^{m}$CoAoG**gctggttatg**Ao$^{m}$CoTo$^{m}$CoA** | 0.94 |
| #100 | **G$^{m}$Co$^{m}$CoAoG**gctggttatg**Ao$^{m}$CoTo$^{m}$CA** | 0.35 |
| #101 | G$^{m}$C$^{m}$CAGgctggttatg**Ao$^{m}$CoTo$^{m}$CoA** | 3.11 |
| #102 | G$^{m}$C$^{m}$CAGgctggttat**GoAo$^{m}$CoTo$^{m}$CoA** | 3.22 |
| #103 | G$^{m}$C$^{m}$CAGgctggtta**ToGoAo$^{m}$CoTo$^{m}$CoA** | 7.44 |
| #104 | G$^{m}$C$^{m}$CAGgctggtt**AoToGoAo$^{m}$CoTo$^{m}$CoA** | 7.20 |
| #105 | G$^{m}$C$^{m}$CAGgctggttatgA$^{m}$CT**$^{m}$CoA** | 0.33 |
| #106 | G$^{m}$C$^{m}$CAGgctggttatgA$^{m}$CT**o$^{m}$CoA** | 0.58 |
| #107 | **Go**$^{m}$C$^{m}$CAGgctggttatgA$^{m}$CTo$^{m}$C**oA** | 0.30 |
| #108 | G$^{m}$C**o$^{m}$Co**AGgctggttatgA$^{m}$C**oTo**$^{m}$CA | 1.03 |
| #109 | G$^{m}$C**o$^{m}$Co**AGgctggttatgA**o$^{m}$Co**T$^{m}$CA | 1.31 |
| #110 | G$^{m}$C$^{m}$CAG**oGo**ctggttat**oGo**A$^{m}$CT$^{m}$CA | 1.71 |
| #111 | G$^{m}$C**o$^{m}$Co**AGgctggttatgA**o$^{m}$CoTo**$^{m}$CA | 3.10 |
| #112 | G$^{m}$C**o$^{m}$CoAo**GgctggttatgA**o$^{m}$CoTo**$^{m}$CA | 3.81 |
| #113 | G**o$^{m}$Co**$^{m}$CAGgctggttatgA$^{m}$CT$^{m}$CA | 0.45 |
| #114 | G$^{m}$C**o$^{m}$Co**AGgctggttatgA$^{m}$CT$^{m}$CA | 0.37 |
| #115 | G$^{m}$C$^{m}$C**oAo**GgctggttatgA$^{m}$CT$^{m}$CA | 0.31 |
| #116 | G$^{m}$C$^{m}$CA**oGo**gctggttatgA$^{m}$CT$^{m}$CA | 0.49 |
| #117 | G$^{m}$C$^{m}$CAG**oGo**ctggttatgA$^{m}$CT$^{m}$CA | 0.59 |
| #118 | G$^{m}$C$^{m}$CAGg**o$^{m}$Co**tggttatgA$^{m}$CT$^{m}$CA | 0.71 |
| #119 | G$^{m}$C$^{m}$CAGgc**oTo**ggttatgA$^{m}$CT$^{m}$CA | 0.88 |
| #120 | G$^{m}$C$^{m}$CAGgct**oGo**gttatgA$^{m}$CT$^{m}$CA | 0.56 |
| #121 | G$^{m}$C$^{m}$CAGgctg**oGo**ttatgA$^{m}$CT$^{m}$CA | 0.69 |

(continued)

| CMP ID NO | Sequence | $IC_{50}$ (N=2) [uM] |
|---|---|---|
| #122 | G$^m$C$^m$CAGgctgg**oTo**tatgA$^m$CT$^m$CA | 0.66 |
| #123 | G$^m$C$^m$CAGgctggt**oTo**atgA$^m$CT$^m$CA | 1.02 |
| #124 | G$^m$C$^m$CAGgctggtt**oAo**tgA$^m$CT$^m$CA | 1.42 |
| #125 | G$^m$C$^m$CAGgctggtta**oTo**gA$^m$CT$^m$CA | 0.97 |
| #126 | G$^m$C$^m$CAGgctggttat**oGo**A$^m$CT$^m$CA | 0.55 |
| #127 | G$^m$C$^m$CAGgctggttatg**oAo**$^m$CT$^m$CA | 0.97 |
| #128 | G$^m$C$^m$CAGgctggttatgA**o$^m$Co**T$^m$CA | 0.91 |
| #129 | G$^m$C$^m$CAGgctggttatgA$^m$C**oTo**$^m$CA | 0.68 |
| #130 | G$^m$C$^m$CAGgctggttatgA$^m$CT**o$^m$Co**A | 0.41 |
| #131 | G$^m$C$^m$CAGgctggttatgA$^m$CT$^m$C**oA** | 0.48 |
| CONTROL #3 | GG$^m$CATatgcagataaTGTT$^m$C | 0.21/0.17 |
| #151 | GG$^m$CAT**oA**tgcagataaTGTT$^m$C | 0.17 |
| #152 | GG$^m$CAT**oAoT**gcagataaTGTT$^m$C | 0.26 |
| #153 | GG$^m$CAT**oAoToG**cagataaTGTT$^m$C | 0.38 |
| #154 | GG$^m$CAT**oAoToGo$^m$C**agataaTGTT$^m$C | 0.56 |
| #155 | GG$^m$CAT**oAoToGo$^m$CoA**gataaTGTT$^m$C | 0.61 |
| #156 | **GG$^m$CAoT**atgcagataaTGTT$^m$C | 0.13 |
| #157 | **GG$^m$CoAoT**atgcagataaTGTT$^m$C | 0.16 |
| #158 | **GGo$^m$CoAoT**atgcagataaTGTT$^m$C | 0.23 |
| #159 | **GoGo$^m$CoAoT**atgcagataaTGTT$^m$C | 0.15 |
| #160 | **GoGo$^m$CoAoToA**tgcagataaTGTT$^m$C | 0.11 |
| #161 | **GoGo$^m$CoAoToAoT**gcagataaTGTT$^m$C | 0.14 |
| #162 | **GoGo$^m$CoAoToAoToG**cagataaTGTT$^m$C | 0.45 |
| #163 | **GoGo$^m$CoAoToAoToGo$^m$C**agataaTGTT$^m$C | 0.57 |
| #164 | **GoGo$^m$CoAoToAoToGo$^m$CoA**gataaTGTT$^m$C | 0.57 |
| #169 | **Go**G$^m$CATatgcagataaTGTT**o$^m$C** | 0.24 |
| #170 | **Go**G$^m$CATatgcagataaTGT**oTo$^m$C** | 0.07 |
| #171 | **GoGo**$^m$CATatgcagataaTGT**oTo$^m$C** | 0.13/0.15 |
| #172 | **GoGo**$^m$CAT**oA**tgcagataaTGT**oTo$^m$C** | 0.26 |
| #173 | **GoGo**$^m$CAT**oAoT**gcagataaTGT**oTo$^m$C** | 0.21 |
| #174 | **GoGo**$^m$CAT**oAoToG**cagataaTGT**oTo$^m$C** | 0.70 |
| #175 | **GoGo**$^m$CAT**oAoToGo$^m$C**agataaTGT**oTo$^m$C** | 1.76 |
| #176 | **GoGo**$^m$CAT**oAoToGo$^m$CoA**gataaTGT**oTo$^m$C** | 4.33 |
| #177 | **GoGo$^m$CoAoT**atgcagataa**ToGoToTo$^m$C** | 0.83 |
| #178 | **GGo$^m$CoAoT**atgcagataa**ToGoToT$^m$C** | 0.65 |

(continued)

| CMP ID NO | Sequence | $IC_{50}$ (N=2) [uM] |
|---|---|---|
| #179 | GG$^m$CATatgcagataa**ToGoToTo$^m$C** | 1.03 |
| #180 | GG$^m$CATatgcagata**AoToGoToTo$^m$C** | 1.72 |
| #181 | GG$^m$CATatgcagat**AoAoToGoToTo$^m$C** | 1.91 |
| #182 | GG$^m$CATatgcaga**ToAoAoToGoToTo$^m$C** | 8.45 |
| #183 | GG$^m$CATatgcagataaTGT**To$^m$C** | 0.22 |
| #184 | GG$^m$CATatgcagataaTGT**oTo$^m$C** | 0.29 |
| #185 | GG**o$^m$C**ATatgcagataaTG**oT**T$^m$C | 1.38 |
| #186 | GG**o$^m$Co**ATatgcagataaTG**oTo**T$^m$C | 1.58 |
| #187 | GG**o$^m$Co**ATatgcagataaT**GoTo**T$^m$C | 2.49 |
| #188 | GG**o$^m$Co**ATatgcagataaT**oGoTo**T$^m$C | 7.33 |
| #189 | GG**o$^m$CoAo**Tatgcagataa T**oGoTo**T$^m$C | 2.86 |
| #190 | GG$^m$CAT**oA**tgcagata**oA**TGTT$^m$C | 0.70 |
| #191 | GG$^m$CAT**oAo**tgcagata**oAo**TGTT$^m$C | 4.29 |
| CONTROL #4 | GGGAGttacttgccaA$^m$CTTG | 0.24/0.36 |
| #192 | GGGAG**oT**tacttgccaA$^m$CTTG | 0.36 |
| #193 | GGGAG**oToT**acttgccaA$^m$CTTG | 0.28 |
| #194 | GGGAG**oToToA**cttgccaA$^m$CTTG | 0.21 |
| #195 | GGGAG**oToToAo$^m$C**ttgccaA$^m$CTTG | 0.23 |
| #196 | GGGAG**oToToAo$^m$CoT**tgccaA$^m$CTTG | 0.47 |
| #197 | GGGAG**oToToAo$^m$CoToT**gccaA$^m$CTTG | 0.41 |
| #198 | **GoGo**GAGttacttgccaA$^m$CT**oToG** | 0.25 |
| #199 | **GoGo**GAG**oT**tacttgccaA$^m$CT**oToG** | 0.40 |
| #200 | **GoGo**GAG**oToT**acttgccaA$^m$CT**oToG** | 0.46 |
| #201 | **GoGo**GAG**oToToA**cttgccaA$^m$CT**oToG** | 0.47 |
| #202 | **GoGo**GAG**oToToAo$^m$C**ttgccaA$^m$CT**oToG** | 1.01 |
| #203 | **GoGo**GAG**oToToAo$^m$CT**tgccaA$^m$CT**oToG** | 2.02 |
| #217 | GGGAGttacttgcca**Ao$^m$CoToToG** | 0.54 |
| #218 | GGGAGtacttgc**$^m$CoAoAo$^m$CoToToG** | 2.22 |
| CONTROL #5 | TG$^m$C$^m$CTttaggattctAGA$^m$CA | 0.34/0.20 |
| #219 | TG$^m$C$^m$CT**oT**taggattctAGA$^m$CA | 1.08 |
| #220 | TG$^m$C$^m$CT**oToT**aggattctAGA$^m$CA | 0.34 |
| #221 | TG$^m$C$^m$CT**oToToA**ggattctAGA$^m$CA | 0.81 |
| #222 | TG$^m$C$^m$CT**oToToAoG**gattctAGA$^m$CA | 0.89 |
| #223 | TG$^m$C$^m$CT**oToToAoGoG**attctAGA$^m$CA | 1.34 |
| #224 | TG$^m$C$^m$CT**oToToAoGoGoA**ttctAGA$^m$CA | 1.73 |

(continued)

| CMP ID NO | Sequence | IC$_{50}$ (N=2) [uM] |
|---|---|---|
| #225 | **ToGo$^{m}$Co$^{m}$CoT**ttaggattct**AoGoAo$^{m}$CoA** | 7.61 |
| #226 | **TGo$^{m}$Co$^{m}$CoT**ttaggattct**AoGoAo$^{m}$CA** | 2.91 |
| #238 | TG$^{m}$C$^{m}$CTttaggattct**AoGoAo$^{m}$CoA** | 1.78 |
| #239 | TG$^{m}$C$^{m}$CTttaggattc**ToAoGoAo$^{m}$CoA** | 5.62 |
| #240 | TG$^{m}$C$^{m}$CTtaggatt**$^{m}$CoToAoGoAo$^{m}$CoA** | 8.39 |
| CONTROL #6 | $^{m}$C$^{m}$CAGGctggttatga$^{m}$CT$^{m}$CAG | 0.88 |
| #241 | **$^{m}$C$^{m}$CAGoG**ctggttatga$^{m}$CT$^{m}$CAG | 0.37 |
| #242 | **$^{m}$C$^{m}$CAoGoG**ctggttatga$^{m}$CT$^{m}$CAG | 0.30 |
| #243 | **$^{mc}$m$^{Co}$AoGoG**ctggttatga$^{m}$CT$^{m}$CAG | 0.56 |
| #244 | **$^{m}$Co$^{m}$CoAoGoG**ctggttatga$^{m}$CT$^{m}$CAG | 0.71 |
| #245 | **$^{m}$Co$^{m}$CoAoGoGo$^{m}$C**tggttatga$^{m}$CT$^{m}$CAG | 1.13 |
| #246 | **$^{m}$Co$^{m}$CoAoGoGo$^{m}$CoT**ggttatga$^{m}$CT$^{m}$CAG | 1.47 |
| #247 | **$^{m}$Co$^{m}$CoAoGoGo$^{m}$CoToG**gttatga$^{m}$CT$^{m}$CAG | 1.07 |
| #248 | **$^{m}$Co$^{m}$CoAoGoGo$^{m}$CoToGoG**ttatga$^{m}$CT$^{m}$CAG | 1.09 |
| CONTROL #7 | TTAT$^{m}$CaattcaccaaGGAG$^{m}$C | 2.26 |
| #249 | **TTATo$^{m}$C**aattcaccaaGGAG$^{m}$C | 0.93 |
| #250 | **TTAoTo$^{m}$C**aattcaccaaGGAG$^{m}$C | 0.61 |
| #251 | **TToAoTo$^{m}$C**aattcaccaaGGAG$^{m}$C | 1.12 |
| #252 | **ToToAoTo$^{m}$C**aattcaccaaGGAG$^{m}$C | 1.25 |
| #253 | **ToToAoTo$^{m}$CoA**attcaccaaGGAG$^{m}$C | 1.68 |
| #254 | **ToToAoTo$^{m}$CoAoA**ttcaccaaGGAG$^{m}$C | 1.28 |
| #255 | **ToToAoTo$^{m}$CoAoAoT**tcaccaaGGAG$^{m}$ | 2.18 |
| #256 | **ToToAoTo$^{m}$CoAoAoToT**caccaaGGAG$^{m}$C | 2.96 |
| #257 | **ToToAoTo$^{m}$CoAoAoToTo$^{m}$C**accaaGGAG$^{m}$C | 6.34 |
| CONTROL #8 | ATGGAggtatgacatATAAT | 0.94 |
| #258 | **ATGGoA**ggtatgacatATAAT | 1.56 |
| #259 | **ATGoGoA**ggtatgacatATAAT | 1.63 |
| #260 | **AToGoGoA**ggtatgacatATAAT | 0.97 |
| #261 | **AoToGoGoA**ggtatgacatATAAT | 0.99 |
| #262 | **AoToGoGoAo**GgtatgacatATAAT | 0.27 |
| #263 | **AoToGoGoAoGoG**tatgacatATAAT | 0.31 |
| #264 | **AoToGoGoAoGoGoT**atgacatATAAT | 0.33 |
| #265 | **AoToGoGoAoGoGoToA**tgacatATAAT | 0.53 |
| #266 | **AoToGoGoAoGoGoToAoT**gacatATAAT | 0.88 |
| #267 | **GToToAoT**gcttattccc**$^{m}$CoAoAoTG** | 0.75 |
| #268 | **$^{m}$CTo$^{m}$CoAoG**cctttatcac**ToCoAoG$^{m}$C** | 1.44 |

(continued)

| CMP ID NO | Sequence | $IC_{50}$ (N=2) [uM] |
|---|---|---|
| #269 | **AAo$^m$CoAoA**atttccttag**ToToGoG$^m$C** | 0.82 |
| #270 | **TAo$^m$CoAo$^m$C**cagtcctttt**AoGoToAG** | 0.48 |
| #271 | **AToGo$^m$CoT**catcacttta**ToGoAoAG** | 0.43 |
| #272 | **AAoAoAoG**gcttag$^m$cgcc**$^m$CoAo$^m$Co$^m$CT** | 1.37 |
| #273 | **AAoGoAoG**aaccacacac**ToAo$^m$Co$^m$CA** | 3.07 |
| #274 | **$^m$CAoTo$^m$CoT**caacctc$^m$cgt**$^m$CoAoToGT** | 0.92 |
| #275 | **AAoGoGoT**ctcatacact**$^m$CoAo$^m$CoTA** | 0.88 |
| #276 | **GToAo$^m$CoT**atcccatcac**ToGoAoAG** | 0.82 |
| #277 | **GAo$^m$Co$^m$Co$^m$C**ctgactttct**GoGoAoAA** | 2.03 |
| #278 | **$^m$CAoGo$^m$CoG**gtacactcct**To$^m$CoToCT** | 0.14 |
| #279 | **G$^m$Co$^m$CoAoA**tatttgcccc**To$^m$Co$^m$Co$^m$C$^m$C** | 1.04 |
| #280 | **TGo$^m$CoAoT**ttacttgcca**Ao$^m$CoAoGA** | 0.51 |
| #281 | **AAoAoGoA**gtaactacca**Go$^m$Co$^m$CoAT** | 1.19 |
| #282 | **AAo$^m$CoAoG**gtcatctatt**$^m$CoAo$^m$CoAA** | 0.38 |
| #283 | **TGoToAo$^m$C**attttgccct**ToAoGo$^m$CT** | 0.14 |
| #284 | **G$^m$Co$^m$CoAoA**gcactcatat**Go$^m$CoAoAT** | 1.59 |
| #285 | GTTAT**oGo$^m$CoToToAoT**tccc$^m$CAATG | 1.55 |
| #286 | GTGTA**oAoToToAo$^m$Co$^m$C**ttttA$^m$CT$^m$CT | 1.05 |
| #287 | $^m$C$^m$CTT$^m$C**oAoGoAoGoAoT**tcaaTG$^m$CTA | 1.03 |
| #288 | G$^m$C$^m$CAA**oToAoToToToG**ccccT$^m$C$^m$C$^m$C$^m$C | 0.58 |
| #289 | TG$^m$CTA**oAoAo$^m$CoAoAoA**tttc$^m$CTTAG | 1.09 |
| #290 | AA$^m$CAA**oAoToToTo$^m$Co$^m$C**ttagTTGG$^m$C | 0.33 |
| #291 | T$^m$CAGT**oGo$^m$CoToAoToT**ttat$^m$C$^m$CAAT | 0.49 |
| #292 | TGTA$^m$C**oAoToToToToG**ccctTAG$^m$CT | 0.62 |
| #293 | TTG$^m$CT**oGoAoAoAoToT**gtct$^m$CAATT | 1.53 |
| #294 | TTGAA**oGo$^m$CoAoToAo$^m$C**cttaA$^m$CAT$^m$C | 0.81 |
| #295 | $^m$CT$^m$C$^m$CA**oAoToAo$^m$CoToT**gtctTAG$^m$CT | 1.46 |
| #296 | G$^m$C$^m$CTT**oAoAoAoGoToT**acatT$^m$CGTT | 1.11 |
| #297 | AAGGT**o$^m$CoTo$^m$CoAoToA**cact$^m$CA$^m$CTA | 0.47 |
| #298 | GTA$^m$CT**oAoTo$^m$Co$^m$Co$^m$CoA**tcacTGAAG | 1.02 |
| #299 | ATG$^m$CA**oAoGoToToAoA**acttAT$^m$CTG | 0.42 |
| #300 | TG$^m$C$^m$CT**o$^m$CoToT$^{om}$CoAoT**tgtaTTT$^m$CT | 0.52 |
| #301 | $^m$CAT$^m$CA**oAoGoGo$^m$CoAo$^m$C**tgat$^m$CA$^m$CTT | 0.48 |
| #302 | G$^m$C$^m$CAA**oGo$^m$CoAo$^m$CoTo$^m$C**atatG$^m$CAAT | 1.22 |
| #303 | GGGAG**oToToAo$^-$CoToT**gccaA$^m$CTTG | 0.53 |

**A, G, $^{m}$C, T (in bold):** represent TNA modification
A, G, $^{m}$C, T (underline): represent MOE modification
A, G, $^{m}$C, T represent LNA nucleotides
a, g, c, $^{m}$C, t represent DNA nucleotides
**o** represents phosphodiester linkages
all other linkages were prepared as phosphorothioates

*Example 4: Caspase 3/7 Activation HepG2 cells (KalyCell)*

Caspase 3/7 Activation HepG2 cells were cultivated at app. 70% confluence in MEM medium with GlutaMax (Gibco #41090), supplemented with 10% heat inactivated fetal calf serum. Cells were detached with 0.25% Trypsin-EDTA solution (Gibco #25200056) and seeded into black, clear 96-well plates P37102-EP 78 (Corning #3904, NY, USA) at a density of 1 $\times$ $10^4$ cells/well. 24h post-seeding HepG2 cells were transiently transfected with Lipofectamine 2000 (Life Technologies #11668019) using 100 nM oligonucleotides dissolved in Opti-MEM (Gibco #31985). Caspase-3/7 activity was determined using the Caspase-Glo® 3/7 Assay (Promega Corporation, Madison WI, USA). Reconstituted Caspase-Glo® 3/7 reagent was added to the cells 24 hours post-transfection, incubated for 60 min, cell lysates were transferred into opaque 96-well plates (Corning #3600, NY, USA) before luminescence was determined on an Enspire multi-mode plate reader (Perkin Elmer) according to the manufacturer's instructions. The results are shown in Table 8, where the percentage (% assay window) indicates the degree of cell apoptosis based on vehicle (cells only treated with PBS). The higher the value, the higher apoptotic activity and thereby *in vitro* cytotoxicity. ASO = antisense oligonucleotide.

Table 8: Caspase 3/7 assay results

| CMP ID NO | Sequence | HepG2 cells 100nM ASO Caspase 3/7 activation after 24 h (%assay window) |
|---|---|---|
| CONTROL #1 | GAGttacttgccaA$^{m}$CT | 294 |
| #29 | G**oAo**GttacttgccaA$^{m}$CT | 0 |
| #30 | GA**oGo**ttacttgccaA$^{m}$CT | 8 |
| #31 | GAG**oTo**tacttgccaA$^{m}$CT | 25 |
| #32 | GAGt**oTo**acttgccaA$^{m}$CT | 25 |
| #33 | GAGtt**oAo**cttgccaA$^{m}$CT | 15 |
| #34 | GAGtta**o$^{m}$Co**ttgccaA$^{m}$CT | 219 |
| #35 | GAGttac**oTo**tgccaA$^{m}$CT | 11 |
| #36 | GAGttact**oTo**gccaA$^{m}$CT | 40 |
| #37 | GAGttactt**oGo**ccaA$^{m}$CT | 128 |
| #38 | GAGttacttg**o$^{m}$Co**caA$^{m}$CT | 86 |
| #39 | GAGttacttgc**o$^{m}$Co**aA$^{m}$CT | 71 |
| #40 | GAGttacttgcc**oAo**A$^{m}$CT | 30 |
| #41 | GAGttacttgcca**oAo**$^{m}$CT | 35 |
| #42 | GAGttacttgccaA**o$^{m}$Co**T | 109 |
| #43 | GAGttacttgccaA$^{m}$C**oT** | 68 |

*Example 5: Thermal melting (Tm) of oligonucleotides containing TNA(PO) modifications hybridized to RNA*

The denaturation point (thermal melting = Tm) were measured according to the following procedure: Gapmer ASOs and complementary RNA were added to 20mM disodium phosphate buffer, 200 mM NaCl and 0.2 mM EDTA (pH 7) resulting in a final concentration of 1.5 $\mu$M. Samples were heated to 95°C for 5 min and then slowly cooled to room temperature over a period of 1 hour. Thermal melting curves were recorded at 260 nm on a Agilent Cary 3500 equipped with a Peltier Temperature Programmer using a temperature gradient that was increased by 5°C/min from 25°C to 95°C

and then decreased to 25°C. The first derivatives of both curves were used to determine the melting temperature (Tm). The values are averaged over three heating and cooling curves (are reported as value t standard deviation).

Table 8: Thermal melting temperature (Tm) values

| CMP ID NO | Sequence | Tm (°C) |
|---|---|---|
| CONTROL #1 | GAGttacttgccaA$^{m}$CT | 61.4 |
| #10 | **GoAoGoToToA**cttgccaA$^{m}$CT | 59.5 |
| #11 | **GoAoGoToToAo$^{m}$C**ttgccaA$^{m}$CT | 61.1 |
| #12 | **GoAoGoToToAo$^{m}$CoT**tgccaA$^{m}$CT | 61.3 |
| #13 | **GoAoGoToToA**cttgccaa$^{m}$CT | 57.3 |
| #14 | **GoAoGoToToA**cttgccaacT | 53.8 |
| #15 | **GoAoGoToToAo$^{m}$C**ttgccaa$^{m}$CT | 58.5 |
| #16 | **GoAoGoToToAo$^{m}$C**ttgccaacT | 54.6 |
| #17 | **GoAoGoToToAo$^{m}$CoT**tgccaa$^{m}$CT | 58.2 |
| #18 | **GoAoGoToToAo$^{m}$CoT**tgccaacT | 55 |
| #22 | GA**oG**ttacttgcca**oAo**$^{m}$CT | 54.3 |
| #23 | GA**oGo**ttacttgcca**oAo**$^{m}$CT | 56 |
| #24 | GA**oG**ttacttgccaA**o$^{m}$C**T | 52.8 |
| #25 | GA**oGo**ttacttgccaA**o$^{m}$C**T | 53.3 |
| #26 | GA**oGo**ttacttgccaA**o$^{m}$Co**T | 53.8 |
| #27 | G**oA**GttacttgccaA**o$^{m}$C**T | 53.3 |
| #28 | G**oAo**GttacttgccaA**o$^{m}$C**T | 54.3 |
| CONTROL #2 | G$^{m}$C$^{m}$CAGgctggttatgA$^{m}$CT$^{m}$CA | 70.8 |
| #80 | GmcmCAG**oG**ctggttatgA$^{m}$CT$^{m}$CA | 68.9 |
| #81 | G$^{m}$C$^{m}$CAG**oGo**ctggttatgA$^{m}$CT$^{m}$CA | 69.3 |
| #82 | G$^{m}$C$^{m}$CAG**oGo$^{m}$C**tggttatgA$^{m}$CT$^{m}$CA | 70.7 |
| #83 | G$^{m}$C$^{m}$CAG**oGo$^{m}$Co**tggttatgA$^{m}$CT$^{m}$CA | 71.2 |
| #84 | G$^{m}$C$^{m}$CAG**oGo$^{m}$CoT**ggttatgA$^{m}$CT$^{m}$CA | 71.3 |
| #85 | G$^{m}$C$^{m}$CAG**oGo$^{m}$CoTo**ggttatgA$^{m}$CT$^{m}$CA | 71.6 |
| #86 | G$^{m}$C$^{m}$CAG**oGo$^{m}$CoToG**gttatgA$^{m}$CT$^{m}$CA | 73.8 |
| #87 | G$^{m}$C$^{m}$CAG**oGo$^{m}$CoToGo**gttatgA$^{m}$CT$^{m}$CA | 73.7 |
| #88 | G$^{m}$C$^{m}$CAG**oGo$^{m}$CoToGoG**ttatgA$^{m}$CT$^{m}$CA | 74.5 |
| #89 | G$^{m}$C$^{m}$CAG**oGo$^{m}$CoToGoGo**ttatgA$^{m}$CT$^{m}$CA | 74.8 |
| #90 | G$^{m}$C$^{m}$CAG**oGo$^{m}$CoToGoGoT**tatgA$^{m}$CT$^{m}$CA | 74.4 |
| #105 | G$^{m}$C$^{m}$CAGgctggttatgA$^{m}$CT**$^{m}$CoA** | 67.6 |
| #106 | G$^{m}$C$^{m}$CAGgctggttatgA$^{m}$CT**o$^{m}$CoA** | 69.1 |
| #107 | **Go**$^{m}$C$^{m}$CAGgctggttatgA$^{m}$CTo$^{m}$C**oA** | 69.3 |
| #108 | G$^{m}$C**o$^{m}$Co**AGgctggttatgA$^{m}$C**oTo**$^{m}$CA | 63.6 |
| #109 | G$^{m}$C**o$^{m}$Co**AGgctggttatgA**o$^{m}$Co**T$^{m}$CA | 63.5 |

(continued)

| CMP ID NO | Sequence | Tm (°C) |
|---|---|---|
| #110 | G$^m$C$^m$CAG**oGo**ctggttat**oGo**A$^m$CT$^m$CA | 72.0 |
| #111 | G$^m$C**o$^m$Co**AGgctggttatgA**o$^m$CoTo**$^m$CA | 61.7 |
| #112 | G$^m$C**o$^m$CoAo**GgctggttatgA**o$^m$CoTo**$^m$CA | 60.8 |
| #132 | **Go**$^m$C$^m$CAGgctggttatgA$^m$CT$^m$CA | 70.6 |
| #133 | G**o$^m$C**$^m$CAGgctggttatgA$^m$CT$^m$CA | 67.3 |
| #134 | G$^m$C**o$^m$C**AGgctggttatgA$^m$CT$^m$CA | 66.5 |
| #135 | G$^m$C$^m$C**oA**GgctggttatgA$^m$CT$^m$CA | 69.4 |
| #136 | G$^m$C$^m$CA**oG**gctggttatgA$^m$CT$^m$CA | 69.1 |
| #137 | G$^m$C$^m$CAG**oG**ctggttatgA$^m$CT$^m$CA | 69.6 |
| #138 | G$^m$C$^m$CAGg**o$^m$C**tggttatgA$^m$CT$^m$CA | 70.1 |
| #139 | G$^m$C$^m$CAGgc**oT**ggttatgA$^m$CT$^m$CA | 70 |
| #140 | G$^m$C$^m$CAGgct**oG**gttatgA$^m$CT$^m$CA | 71.6 |
| #141 | G$^m$C$^m$CAGgctg**oG**ttatgA$^m$CT$^m$CA | 70.5 |
| #142 | G$^m$C$^m$CAGgctgg**oT**tatgA$^m$CT$^m$CA | 70.2 |
| #143 | G$^m$C$^m$CAGgctggt**oT**atgA$^m$CT$^m$CA | 71.3 |
| #144 | G$^m$C$^m$CAGgctggtt**oA**tgA$^m$CT$^m$CA | 70.5 |
| #145 | G$^m$C$^m$CAGgctggtta**oT**gA$^m$CT$^m$CA | 72.4 |
| #146 | G$^m$C$^m$CAGgctggttat**oG**A$^m$CT$^m$CA | 72.3 |
| #147 | G$^m$C$^m$CAGgctggttatg**oA**$^m$CT$^m$CA | 71.1 |
| #148 | G$^m$C$^m$CAGgctggttatgA**o$^m$C**T$^m$CA | 66.8 |
| #149 | G$^m$C$^m$CAGgctggttatgA$^m$C**oT**$^m$CA | 67.9 |
| #150 | G$^m$C$^m$CAGgctggttatgA$^m$CT**o$^m$C**A | 69.9 |
| CONTROL #3 | GG$^m$CATatgcagataaTGTT$^m$C | 58.9 |
| #151 | GG$^m$CAT**oA**tgcagataaTGTT$^m$C | 55.6 |
| #152 | GG$^m$CAT**oAoT**gcagataaTGTT$^m$C | 58.3 |
| #153 | GG$^m$CAT**oAoToG**cagataaTGTT$^m$C | 59.3 |
| #154 | GG$^m$CAT**oAoToGo$^m$C**agataaTGTT$^m$C | 61.8 |
| #163 | **GoGo$^m$CoAoToAoToGo$^m$C**agataaTGTT$^m$C | 61.8 |
| #164 | **GoGo$^m$CoAoToAoToGo$^m$CoA**gataaTGTT$^m$C | 63.3 |
| #165 | **GoGo$^m$CoAoToAoToGo$^m$CoA**gataatGTT$^m$C | 61.5 |
| #166 | **GoGo$^m$CoAoToAoToGo$^m$CoA**gataatgTT$^m$C | 59.5 |
| #167 | **GoGo$^m$CoAoToAoToGo$^m$CoAoG**ataatGTT$^m$C | 65 |
| #168 | **GoGo$^m$CoAoToAoToGo$^m$CoAoG**ataatgTT$^m$C | 62.3 |
| #183 | GG$^m$CATatgcagataaTGT**To$^m$C** | 53.7 |
| #184 | **G**G$^m$CATatgcagataaTGT**oTo$^m$C** | 53.3 |

(continued)

| CMP ID NO | Sequence | Tm (°C) |
|---|---|---|
| #185 | GG**o$^{m}$C**ATatgcagataaTG**oT**T$^{m}$C | 48.9 |
| #186 | GG**o$^{m}$Co**ATatgcagataaTG**oTo**T$^{m}$C | 50.3 |
| #187 | GG**o$^{m}$Co**ATatgcagataaT**GoTo**T$^{m}$C | 50.4 |
| #188 | GG**o$^{m}$Co**ATatgcagataaT**oGoTo**T$^{m}$C | 50.2 |
| #189 | GG**o$^{m}$CoAo**TatgcagataaT**oGoTo**T$^{m}$C | 50.9 |
| #190 | GG$^{m}$CAT**oA**tgcagata**oA**TGTT$^{m}$C | 56.8 |
| #191 | GG$^{m}$CAT**oAo**tgcagata**oAo**TGTT$^{m}$C | 57.9 |

**A, G, $^{m}$C,** T **(in bold):** represent TNA modification
A, G, $^{m}$C, T (underline): represent MOE modification
A, G, $^{m}$C, T represent LNA nucleotides
a, g, c, $^{m}$c, t represent DNA nucleotides
**o** represents phosphodiester linkages
all other linkages were prepared as phosphorothioates

LIST OF REFERENCES

Crooke et al., Nucleic Acids Research 2020;48(10):5235-5253. DOI:10.1093/nar/gkaa299

Eckstein, Antisense and Nucleic Acid Drug Development 2009;10:117-121. DOI: 10.1089/oli.1.2000.10.117.

Liu et al., ACS Appl. Mater. Interfaces 2018;10:9736-9743. DOI:10.1021/acsami.8b01180

Matsuda et al., Poster; XXIII International Round Table on Nucleosides, Nucleotides and Nucleic acids; August 2018. DOI:10.13140/RG.2.2.10627.45605

Zhang and Chaput, Current Protocols in Nucleic Acid Chemistry, 4.51.1-4.51.26, 2012. DOI: 10.1002/0471142700.nc0451s50

WO 2012/078536 (Quark Pharmaceuticals, Inc.)

WO 2012/118911 (Quark Pharmaceuticals, Inc.)

WO 2013/179292 A1 (QBI Enterprises Ltd. and Bio-Lab Ltd.)


## Claims

1. An antisense gapmer oligonucleotide comprising a contiguous nucleotide sequence of formula 5'-F-G-F'-3' (I) which is capable of recruiting ribonuclease (RNase) H, wherein

    G is a gap region of up to 18 linked nucleosides which comprises at least 3 contiguous DNA nucleosides,
    each of F and F' is a flanking region of up to 15 linked nucleosides which independently comprises or consists of 1 to 15 sugar-modified nucleosides,
    at least one of F, F' and G comprises a sugar-modified nucleoside which is an $\alpha$-L-threofuranosyl (TNA) nucleoside and which is linked to an adjacent nucleoside by an internucleoside linkage different than a phosphorothioate (PS) internucleoside linkage.

2. The antisense gapmer oligonucleotide according to claim 1, wherein the TNA nucleoside is linked to an adjacent

nucleoside by a phosphodiester (PO) internucleoside linkage.

3. The antisense gapmer oligonucleotide according to any one of the preceding claims, wherein the TNA nucleoside is linked to an adjacent nucleoside by a 2'-PO internucleoside linkage or a 3'-PO internucleoside linkage.

4. The antisense gapmer oligonucleotide according to any one of the preceding claims, wherein the TNA nucleoside is linked to any adjacent nucleoside by a 2'-PO or 3'-PO internucleoside linkage.

5. The antisense gapmer oligonucleotide according to any one of the preceding claims, wherein the adjacent nucleoside is a sugar-modified nucleoside or a DNA nucleoside.

6. The antisense gapmer oligonucleotide according to any one of the preceding claims, wherein the TNA nucleoside is linked to a first and a second adjacent nucleoside by

(a) a 2'-PO internucleoside linkage and a 3'-modified internucleoside linkage, respectively;
(b) a 2'-modified internucleoside linkage and a 3'-PO internucleoside linkage, respectively; or
(c) a 2'-PO internucleoside linkage and a 3'-PO internucleoside linkage, respectively,

optionally wherein the 3'-modified internucleoside linkage is a PS internucleoside linkage.

7. The antisense gapmer oligonucleotide according to any one of the preceding claims, wherein F comprises at least one TNA nucleoside.

8. The antisense gapmer oligonucleotide according to any one of the preceding claims, wherein F comprises at least one TNA nucleoside linked to an adjacent nucleoside by a PO internucleoside linkage.

9. The antisense gapmer oligonucleotide according to any one of the preceding claims, wherein F comprises at least two, three, four, five, six, seven, eight, nine, ten, eleven or twelve TNA nucleosides linked to an adjacent nucleoside by a PO internucleoside linkage.

10. The antisense gapmer oligonucleotide according to any one of the preceding claims, wherein F comprises at least two, three, four, five, six, seven, eight, nine, ten, eleven or twelve adjacent TNA nucleosides linked by PO internucleoside linkages.

11. The antisense gapmer oligonucleotide according to any one of the preceding claims, wherein F comprises or consists of two, three, four, five, six, seven, eight, nine, ten, eleven or twelve adjacent TNA nucleosides linked by PO internucleoside linkages.

12. The antisense oligonucleotide according to any one of the preceding claims, wherein at least the 3'-most nucleoside in F is a TNA nucleoside.

13. The antisense oligonucleotide according to any one of the preceding claims, wherein at least the two, three, four, five, six, seven, eight, nine, ten, eleven or twelve 3'-most nucleosides in F are TNA nucleosides.

14. The antisense oligonucleotide according to any one of the preceding claims, wherein the two, three, four, five, six, seven, eight, nine, ten, eleven, twelve, thirteen or fourteen 3'-most nucleosides in F are TNA nucleosides.

15. The antisense oligonucleotide according to any one of the preceding claims, wherein at least the 5'-most nucleoside in F is a TNA nucleoside.

16. The antisense oligonucleotide according to any one of the preceding claims, wherein at least the two, three, four, five, six, seven, eight, nine, ten, eleven or twelve 5'-most nucleosides in F are TNA nucleosides.

17. The antisense oligonucleotide according to any one of the preceding claims, wherein the two, three, four, five, six, seven, eight, nine, ten, eleven, twelve, thirteen or fourteen 5'-most nucleosides in F are TNA nucleosides.

18. The antisense gapmer oligonucleotide according to any one of the preceding claims, wherein all sugar-modified nucleosides of F are TNA nucleosides.

**19.** The antisense gapmer oligonucleotide according to any one of the preceding claims, wherein all nucleosides of F are TNA nucleosides.

**20.** The antisense gapmer oligonucleotide according to any one of the preceding claims, wherein

(a) each TNA nucleoside in F is linked to an adjacent nucleoside by a PO internucleoside linkage; or
(b) except for the 5'-most TNA nucleoside in F, each TNA nucleoside in F is linked to an adjacent nucleoside by a PO internucleoside linkage.

**21.** The antisense gapmer oligonucleotide according to any one of claims 1 to 20, wherein F' does not comprise any TNA nucleoside.

**22.** The antisense gapmer oligonucleotide according to any one of claims 1 to 20, wherein F' comprises at least one TNA nucleoside.

**23.** The antisense gapmer oligonucleotide according to any one of the preceding claims, wherein F' comprises at least one TNA nucleoside linked to an adjacent nucleoside by a PO internucleoside linkage.

**24.** The antisense gapmer oligonucleotide according to any one of the preceding claims, wherein F' comprises at least two, three, four, five, six, seven, eight, nine, ten, eleven or twelve TNA nucleosides linked to an adjacent nucleoside by a PO internucleoside linkage.

**25.** The antisense gapmer oligonucleotide according to any one of the preceding claims, wherein F' comprises at least two, three, four, five, six, seven, eight, nine, ten, eleven or twelve adjacent TNA nucleosides linked by PO internucleoside linkages.

**26.** The antisense gapmer oligonucleotide according to any one of the preceding claims, wherein F' comprises or consists of two, three, four, five, six, seven, eight, nine, ten, eleven or twelve adjacent TNA nucleosides linked by PO internucleoside linkages.

**27.** The antisense oligonucleotide according to any one of the preceding claims, wherein at least the 5'-most nucleoside in F' is a TNA nucleoside.

**28.** The antisense oligonucleotide according to any one of the preceding claims, wherein at least the two, three, four, five, six, seven, eight, nine, ten, eleven or twelve 5'-most nucleosides in F' are TNA nucleosides.

**29.** The antisense oligonucleotide according to any one of the preceding claims, wherein the two, three, four, five, six, seven, eight, nine, ten, eleven, twelve, thirteen or fourteen 5'-most nucleosides in F' are TNA nucleosides.

**30.** The antisense oligonucleotide according to any one of the preceding claims, wherein at least the 3'-most nucleoside in F' is a TNA nucleoside.

**31.** The antisense oligonucleotide according to any one of the preceding claims, wherein at least the two, three, four, five, six, seven, eight, nine, ten, eleven or twelve 3'-most nucleosides in F' are TNA nucleosides.

**32.** The antisense oligonucleotide according to any one of the preceding claims, wherein the two, three, four, five, six, seven, eight, nine, ten, eleven, twelve, thirteen or fourteen 3'-most nucleosides in F' are TNA nucleosides.

**33.** The antisense gapmer oligonucleotide according to any one of the preceding claims, wherein all sugar-modified nucleosides of F' are TNA nucleosides.

**34.** The antisense gapmer oligonucleotide according to any one of the preceding claims, wherein all nucleosides of F' are TNA nucleosides.

**35.** The antisense gapmer oligonucleotide according to any one of the preceding claims, wherein

(a) each TNA nucleoside in F' is linked to an adjacent nucleoside by a PO internucleoside linkage; or
(b) except for the 5'-most TNA nucleoside in F', each TNA nucleoside in F' is linked to an adjacent nucleoside

by a PO internucleoside linkage.

**36.** The antisense gapmer oligonucleotide according to any one of claims 1 to 6 and 21 to 35, wherein F does not comprise any TNA nucleoside.

**37.** The antisense gapmer oligonucleotide according to any one of the preceding claims, wherein F and F' each independently comprises one, two, three, four, five, six, seven, eight, nine, ten, eleven, twelve, thirteen, fourteen or fifteen TNA nucleosides linked to an adjacent nucleoside by a PO internucleoside linkage.

**38.** The antisense gapmer oligonucleotide according to any one of the preceding claims, wherein F and F' together comprise at least two, three, four, five, six, seven, eight, nine, ten, eleven, twelve, thirteen, fourteen, fifteen, sixteen, seventeen, eighteen, nineteen or twenty TNA nucleosides linked to an adjacent nucleoside by a PO internucleoside linkage.

**39.** The antisense gapmer oligonucleotide according to any one of the preceding claims, wherein F and F' each independently comprises or consists of two, three, four, five, six, seven, eight, nine, ten, eleven, twelve, thirteen, fourteen, or fifteen adjacent TNA nucleosides linked by PO internucleoside linkages.

**40.** The antisense gapmer oligonucleotide according to any one of the preceding claims, wherein F and F' together comprise two, three, four, five, six, seven, eight, nine, ten, eleven, twelve, thirteen, fourteen, fifteen, sixteen, seventeen, eighteen, nineteen, or twenty TNA nucleosides which are linked to an adjacent TNA nucleoside by a PO internucleoside linkage.

**41.** The antisense gapmer oligonucleotide according to any one of the preceding claims, wherein

(a) each TNA nucleoside in F and F' is linked to an adjacent nucleoside by a PO internucleoside linkage, or
(b) except for the 5'-most TNA nucleoside in F and the 3'-most TNA nucleoside in F', each TNA nucleoside in F and F' is linked to an adjacent nucleoside by a PO internucleoside linkage.

**42.** The antisense gapmer oligonucleotide according to any one of the preceding claims, wherein F, F', or both F and F', further comprise 1 to 8 sugar-modified nucleosides other than TNA(PO) nucleosides, such as three, four or five sugar-modified nucleosides other than TNA(PO) nucleosides.

**43.** The antisense gapmer oligonucleotide according to any one of the preceding claims, wherein the sugar-modified nucleosides of F, F' or both F and F', comprise or consist of at least one sugar-modified nucleoside comprising a modified sugar-moiety selected from the group consisting of:

2'-methoxy-ribose (2'-OMe),
2'-O-methoxyethyl-ribose (2'-O-MOE),
5'-methyl-2'-O-methoxyethyl ribose (5'-Me-2'-O-MOE),
2'-O-[2-(methylthio)ethyl]-ribose (2'-O-MTE),
2-(N-methylcarbamoyl)-ethyl]-ribose (2'-O-MCE),
2'-O-[2-(methylamino)-2-oxoethyl]-ribose (2'-O-NMA),
2'-deoxy-2'-fluoro-ribose (as in 2'-deoxy-2'-fluororibo-nucleic acid; 2'-F-RNA),
2'-fluoro-2'-arabinose (as in 2'-fluoro-2'-arabinose nucleic acid; 2'-F-ANA),
2'-O-benzyl-ribose,
oxy, amino or thio β-D-locked ribose (as in β-D-LNA),
oxy, amino or thio α-L-locked ribose (as in α-L-LNA),
2',4'-constrained 2'-O-ethyl ribose (as in constrained ethyl locked nucleic acid; cEt),
tricyclo-deoxyribose (as in tricyclo-deoxyribose DNA; TcDNA),
3'-deoxy-ribose (as in 3'-deoxy-ribose DNA; 3'-DNA),
unlocked ribose (as in unlocked nucleic acid; UNA),
glycol (as in glycol nucleic acid; GNA),
hexitol (as in hexitol nucleic acid; HNA),
3'-fluoro hexitol (as in 3'-fluoro hexitol nucleic acid; FHNA),
3'-arabino-fluoro hexitol (as in 3'-arabino-fluoro hexitol nucleic acid; Ara-FHNA),
cyclohexene (as in cyclohexene nucleic acid; CeNA), and
fluoro-cyclohexenenyl (as in 2'-fluoro-cyclohexenyl nucleic acid; F-CeNA).

**44.** The antisense gapmer oligonucleotide according to any one of the preceding claims, wherein the sugar-modified nucleosides of F, F’ or both F and F’, comprise or consist of one or more 2’-sugar modified nucleosides, such as high-affinity 2’-sugar-modified nucleosides.

**45.** The antisense gapmer oligonucleotide according to any one of the preceding claims, wherein the sugar-modified nucleosides of F, F’ or both F and F’, comprise one or more LNA nucleosides.

**46.** The antisense gapmer oligonucleotide according to claim 45, wherein, except for any at least one TNA nucleoside, all nucleosides in F’ and F are LNA nucleosides.

**47.** The antisense gapmer oligonucleotide according to any one of the preceding claims, wherein the sugar-modified nucleosides of F, F’, or both F and F’, comprise one or more 2’-O-methoxyethyl-RNA (2’-O-MOE) nucleosides.

**48.** The antisense gapmer oligonucleotide according to claim 47, wherein, except for any at least one TNA nucleoside, all nucleosides in F’ and F are 2’-O-MOE nucleosides.

**49.** The antisense gapmer oligonucleotide according to any one of the preceding claims, wherein G does not comprise any TNA nucleoside.

**50.** The antisense gapmer oligonucleotide according to any one of claims 1 to 48, wherein G comprises at least one TNA nucleoside, such as at least one, two, or three, TNA nucleosides.

**51.** The antisense gapmer oligonucleotide according to any one of the preceding claims, wherein G comprises at least one TNA nucleoside linked to an adjacent nucleoside by a PO internucleoside linkage.

**52.** The antisense gapmer oligonucleotide according to claim 51, wherein the second, third, fourth, fifth, sixth, seventh-most, or eight-most 5’ nucleoside in G is a TNA nucleoside linked to an adjacent nucleoside by a PO internucleoside linkage.

**53.** The antisense gapmer oligonucleotide according to claim 51 or 52, wherein the second, third, fourth, fifth, sixth, seventh-most, or eight-most 3’ nucleoside in G is a TNA nucleoside linked to an adjacent nucleoside by a PO internucleoside linkage.

**54.** The antisense gapmer oligonucleotide according to any one of the preceding claims, wherein G comprises at least two, such as two or three, adjacent TNA nucleosides linked by PO internucleoside linkages.

**55.** The antisense gapmer oligonucleotide according to any one of the preceding claims, wherein at least the 5’-most and 3’-most nucleosides in G are DNA nucleosides.

**56.** The antisense gapmer oligonucleotide according to any one of the preceding claims, wherein, except for any at least one TNA(PO) nucleoside, all nucleosides of G are DNA nucleosides.

**57.** The antisense gapmer oligonucleotide according to any one of the preceding claims, wherein the gap region G comprises at least four DNA nucleosides, such as 4, 5, 6, 7, 8, 9, 10, 11, 12, 13, 14, 15 or 16 contiguous DNA nucleosides.

**58.** The antisense gapmer according to any one of the preceding claims, wherein G comprises at most 10 DNA nucleosides, such as 9, 8, 7, 6, 5, or 4 DNA nucleosides.

**59.** The antisense gapmer oligonucleotide according to any one of the preceding claims, which comprises at least one modified internucleoside linkage.

**60.** The antisense gapmer oligonucleotide according to any one of the preceding claims, which comprises a nuclease resistant modified internucleoside linkage.

**61.** The antisense gapmer oligonucleotide according to any one of the preceding claims, wherein, except for any PO internucleoside linkage between a TNA nucleoside and an adjacent nucleoside, all internucleoside linkages are phosphorothioate internucleoside linkages.

**62.** The antisense gapmer oligonucleotide according to any one of the preceding claims, wherein the contiguous nucleotide sequence of formula 5'-F-G-F'-3' (I) has a length of from 12 to 32 nucleosides, such as from 12 to 28 nucleosides, such as from 12 to 26 nucleosides, such as from 14 to 26 nucleosides, such as from 14 to 24 nucleosides, such as from 14 to 22 nucleosides, such as from 16 to 22 nucleosides, such as from 16 to 20 nucleosides.

**63.** The antisense gapmer oligonucleotide according to claim 62, wherein the contiguous nucleotide sequence is of the formula

$F_{1-15}$-$G_{3-18}$-$F'_{1-15}$ (IV), such as $F_{1-15}$-$G_{3-18}$-$F'_{1-12}$ (IVa) or $F_{1-12}$-$G_{3-18}$-$F'_{1-15}$ (IVb);
$F_{1-12}$-$G_{3-18}$-$F'_{1-12}$ (V), such as $F_{1-12}$-$G_{3-18}$-$F'_{1-9}$ (Va), or $F_{1-9}$-$G_{3-18}$-$F'_{1-12}$ (Vb);
$F_{1-12}$-$G_{4-16}$-$F'_{1-12}$ (VI), such as $F_{1-12}$-$G_{4-16}$-$F'_{1-9}$ (VIa), or $F_{1-9}$-$G_{4-16}$-$F'_{1-12}$ (VIb); or
$F_{3-12}$-$G_{4-10}$-$F'_{3-12}$ (VII), such as $F_{3-12}$-$G_{4-10}$-$F'_{3-9}$ (VIIa) or $F_{3-9}$-$G_{4-10}$-$F'_{3-12}$ (VIIb), wherein the numeric ranges represent the number of linked nucleosides in F, G and F', respectively.

**64.** The antisense gapmer oligonucleotide according to any one of the preceding claims, which can reduce the expression level of a target nucleic acid by at least about 50%, such as at least about 60%, such as at least about 70%, such as at least about 80%, such as at least about 90%, as compared to the normal expression level of the target, optionally when determined in a target cell expressing the target nucleic acid and incubated with the antisense gapmer oligonucleotide at a concentration of about 25 μM for about 3 days.

**65.** The antisense gapmer oligonucleotide according to any one of the preceding claims, which antisense gapmer oligonucleotide according to any one of the preceding claims, which has an IC50 of no more than about 20 μM, such as no more than about 10 μM, such as no more than about 5 μM, for reducing the expression level of a target nucleic acid, optionally when determined in a target cell expressing the target nucleic acid and incubated with the antisense gapmer oligonucleotide at a concentration of about 25 μM for about 3 days.

**66.** The antisense gapmer oligonucleotide according to any one of the preceding claims, which using the Caspase 3/7 assay described in Example 4, the percentage assay window (%AW) is preferably at most about 60%, such as at most about 40%, such as at most about 20%, such as at most about 10%.

**67.** The antisense gapmer oligonucleotide according to any one of the preceding claims, which has, in the form of a duplex of the antisense gapmer oligonucleotide with an RNA target sequence, a melting temperature (Tm) of at least about 50°C, such as at least about 52°C, such as at least about 54°C, such as at least about 56°C, such as at least about 58°C, such as at least about 60°C.

**68.** The antisense gapmer oligonucleotide according to any one of the preceding claims, which is **characterized by** a combination of claims 64 and 65; claims 64 and 66; claims 64 and 67; claims 65 and 66; claims 65 and 67; claims 64, 65 and 66; claims 64, 65 and 67; claims 64, 66 and 67, claims 65, 66, and 67; or claims 64 to 67.

**69.** The antisense gapmer oligonucleotide according to any one of claims 64 to 78, wherein the target nucleic acid is an RNA target sequence which has a nucleobase sequence complementary to the contiguous nucleotide sequence of formula I.

**70.** An antisense gapmer oligonucleotide comprising a contiguous nucleotide sequence of formula 5'-F-G-F'-3' (I) which is capable of recruiting RNase H, wherein the contiguous nucleotide sequence comprises at least one TNA nucleoside which is linked to an adjacent nucleoside by a linkage different than a PS internucleoside linkage.

**71.** The antisense gapmer oligonucleotide according to claim 70, wherein the TNA nucleoside is linked to an adjacent nucleoside by a PO internucleoside linkage.

**72.** The antisense gapmer oligonucleotide according to any one of claim 70 and 71, wherein the contiguous nucleotide sequence of formula 5'-F-G-F'-3' (I) has a length of 12 to 32 nucleosides, such as from 12 to 28 nucleosides, such as from 12 to 26 nucleosides, such as from 14 to 26 nucleosides, such as from 14 to 24 nucleosides, such as from 14 to 22 nucleosides, such as from 16 to 22 nucleosides, such as from 16 to 20 nucleosides.

**73.** The antisense gapmer oligonucleotide according to any one of claims 70 to 72, wherein the contiguous nucleotide sequence is of the formula

$F_{1-15}$-$G_{3-18}$-$F'_{1-15}$ (II), such as $F_{1-15}$-$G_{3-18}$-$F'_{1-12}$ (IIa) or $F_{1-12}$-$G_{3-18}$-$F'_{1-15}$ (IIb);
$F_{1-12}$-$G_{3-18}$-$F'_{1-12}$ (III), such as $F_{1-12}$-$G_{3-18}$-$F'_{1-9}$ (IIIa), or $F_{1-9}$-$G_{3-18}$-$F'_{1-12}$ (IIIb);
$F_{1-12}$-$G_{4-16}$-$F'_{1-12}$ (IV), such as $F_{1-12}$-$G_{4-16}$-$F'_{1-9}$ (IVa), or $F_{1-9}$-$G_{4-16}$-$F'_{1-12}$ (IVb); or
$F_{3-12}$-$G_{4-10}$-$F'_{3-12}$ (V), such as $F_{3-12}$-$G_{4-10}$-$F'_{3-9}$ (Va) or $F_{3-9}$-$G_{4-10}$-$F'_{3-12}$ (Vb), wherein the numeric ranges represent the number of linked nucleosides in F, G and F', respectively.

**74.** The antisense gapmer according to any one of claims 70 to 73, further comprising the features of any one of claims 1 to 69.

**75.** The antisense gapmer oligonucleotide according to any one of claims 63 to 74, wherein the contiguous nucleotide sequence of formula IVa has a length of at least 16 nucleosides and

(a) the 5'-most nucleosides in F and the nucleosides in F' are independently 3, 4 or 5 high-affinity sugar-modified nucleosides,
(b) the remaining nucleosides in F are TNA nucleosides, and
(c) all nucleosides in G are DNA nucleosides.

**76.** The antisense gapmer oligonucleotide according to claim 75, wherein G comprises at most 10 contiguous DNA nucleosides, such as 9, 8, 7, 6, 5, or 4 contiguous DNA nucleosides.

**77.** The antisense gapmer oligonucleotide according to any one of claims 63 to 74, wherein the contiguous nucleotide sequence of formula IV has a length of at least 16 nucleosides and

(a) F and F' each independently consists of 3, 4, or 5 nucleosides,
(b) wherein at least one of the nucleosides in F and F' is a TNA nucleoside and the remaining nucleosides are high-affinity sugar-modified nucleosides,
(c) all nucleosides in G are DNA nucleosides.

**78.** The antisense gapmer oligonucleotide of claim 77, wherein all nucleosides in F and F' are TNA nucleosides.

**79.** The antisense gapmer oligonucleotide according to any one of claims 63 to 74, wherein

(a) each TNA nucleoside in F and F' is linked to an adjacent nucleoside by a PO internucleoside linkage, or
(b) except for the 5'-most TNA nucleoside in F and the 3'-most TNA nucleoside in F', each TNA nucleoside in F and F' is linked to an adjacent nucleoside by a PO internucleoside linkage.

**80.** The antisense gapmer oligonucleotide according to any one of claims 63 to 74, wherein the contiguous nucleotide sequence of formula IV has a length of at least 16 nucleosides and

(a) F and F' each independently comprises or consists of 3, 4, or 5 linked high-affinity sugar-modified nucleosides and does not comprise any TNA nucleoside, and
(b) the second, third, fourth or fifth 5'-most nucleoside in G is a TNA nucleoside and the remaining nucleosides in G are DNA nucleosides.

**81.** The antisense gapmer oligonucleotide according to any one of claims 75 to 80, wherein the high-affinity sugar modified nucleosides are selected from the sugar-modified nucleosides in claim 43.

**82.** The antisense oligonucleotide according to any one of the preceding claims, wherein the antisense oligonucleotide is a single-stranded antisense oligonucleotide.

**83.** A conjugate comprising the antisense gapmer oligonucleotide according to any one of the preceding claims and at least one conjugate moiety covalently attached to said oligonucleotide, optionally via a linker.

**84.** The conjugate according to claim 83, wherein the conjugate moiety is selected from carbohydrates, cell surface receptor ligands, drug substances, hormones, lipophilic substances, polymers, proteins, peptides, toxins, vitamins, viral proteins and combinations thereof.

**85.** The conjugate according to claim 83 or 84, wherein the conjugate moiety facilitates delivery across the blood brain

barrier.

**86.** A pharmaceutically acceptable salt of the antisense gapmer oligonucleotide according to any one of claims 1 to 82, or the conjugate according to any one of claims 83 to 85.

**87.** A pharmaceutical composition comprising the antisense gapmer oligonucleotide according to any one of claims 1 to 82, or the conjugate according to any one of claims 83 to 85, the pharmaceutically acceptable salt according to claim 86, and a pharmaceutically acceptable diluent, solvent, carrier, salt and/or adjuvant.

**88.** An antisense oligonucleotide according to any one of claims 1 to 82, a conjugate according to any one of claims 83 to 85, the pharmaceutically acceptable salt according to claim 86, or a pharmaceutical composition according to claim 87, for use as a medicament.

**89.** A method of preparing a modified version of a parent antisense gapmer oligonucleotide, wherein the parent antisense gapmer comprises a contiguous nucleotide sequence of formula 5' F-G-F' 3' (I) which is capable of recruiting RNase H, wherein G is a gap region of 5 to 18 linked DNA nucleosides and each of F and F' is a flanking region of up to 8 linked nucleosides which independently comprises or consists of 1 to 8 sugar-modified nucleosides other than TNA nucleosides, and wherein, in the modified version, at least one nucleoside in F, F', and/or G of the parent antisense gapmer oligonucleotide has been replaced with a TNA nucleoside linked to an adjacent nucleoside by a PO internucleoside linkage,

the method comprising the step of manufacturing the modified antisense gapmer oligonucleotide by reacting nucleotide units to form covalently linked contiguous nucleotide units comprised in the oligonucleotide, wherein at least one of the nucleotide units comprises a TNA nucleoside, and,
optionally purifying or isolating the modified antisense gapmer oligonucleotide.

**90.** The method according to claim 89, wherein the modified antisense gapmer oligonucleotide has a reduced toxicity, optionally hepatotoxicity, as compared to the parent antisense gapmer oligonucleotide.

**91.** The method according to any one of claims 89 and 90, wherein the modified antisense gapmer oligonucleotide is less toxic to HepG2 cells than the parent antisense gapmer oligonucleotide, optionally as determined by a Caspase 3/7 assay.

**92.** The method according to any one of claims 89 to 91, wherein the modified antisense gapmer oligonucleotide has an increased exonuclease resistance as compared to the parent antisense gapmer oligonucleotide.

**93.** The method according to any one of claims 89 to 92, wherein the modified antisense gapmer oligonucleotide has an increased endonuclease resistance as compared to the parent antisense gapmer oligonucleotide.

**94.** The method according to any one of claims 89 to 93, wherein the parent antisense gapmer oligonucleotide is an LNA gapmer or MOE gapmer, optionally wherein all internucleoside linkages are phosphorothioate linkages.

**95.** The method according to any one of claims 89 to 94, wherein the nucleotide units are nucleoside phosphoramidites.

**96.** The method according to any one of claims 89 to 95, wherein the modified antisense gapmer oligonucleotide comprises the features of any one of claims 1 to 82.

**97.** An antisense gapmer oligonucleotide obtained or obtainable by the method according to any one of claims 89 to 96.

**98.** Use of a TNA nucleotide in the preparation of an antisense gapmer oligonucleotide according to any one of claims 1 to 82 or the conjugate of any one of claims 83 to 85.

Europäisches Patentamt
European Patent Office
Office européen des brevets

# EUROPEAN SEARCH REPORT

Application Number
EP 22 19 2656

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WANG FEI ET AL.: "Synthetic alpha-L-Threose nucleic acids targeting BcL-2 show gene silencing and in vivo antitumor activity for cancer therapy", APPLIED MATERIALS & INTERFACES, vol. 11, no. 42, 26 September 2019 (2019-09-26), pages 38510-38518, XP093031784, US ISSN: 1944-8244, DOI: 10.1021/acsami.9b14324 * the whole document * | 1-15 | INV. C12N15/113 C12N15/11 |
| X | -& Wang Fei ET AL.: "Supporting Information", , 26 September 2019 (2019-09-26), XP93045923, Retrieved from the Internet: URL:https://pubs.acs.org/doi/10.1021/acsami.9b14324# [retrieved on 2023-05-10] * the whole document * ----- | 1-15 | TECHNICAL FIELDS SEARCHED (IPC) C12N |
| X | LIU LING SUM ET AL.: "alpha-L-Threose nucleic acids as biocompatible antisense oligonucleotides for suppressing gene expression in living cells", APPLIED MATERIALS & INTERFACES, vol. 10, no. 11, 23 February 2018 (2018-02-23), pages 9736-9743, XP093031816, US ISSN: 1944-8244, DOI: 10.1021/acsami.8b01180 * the whole document * -/-- | 1-15 | |

The present search report has been drawn up for all claims

1

| Place of search | Date of completion of the search | Examiner |
|---|---|---|
| The Hague | 11 May 2023 | Macchia, Giovanni |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 22 19 2656

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | -& Ling Sum ET AL.: "Supporting Information", , 23 February 2018 (2018-02-23), XP93045921, Retrieved from the Internet: URL:https://pubs.acs.org/doi/10.1021/acsami.8b01180 [retrieved on 2023-05-10] * the whole document * ----- | 1-15 | |
| A | ROBERTS THOMAS C. ET AL.: "Advances in oligonucleotide drug delivery", NATURE REVIEWS DRUG DISCOVERY, NATURE PUBLISHING GROUP, GB, vol. 19, no. 10, 11 August 2020 (2020-08-11), pages 673-694, XP037256878, ISSN: 1474-1776, DOI: 10.1038/S41573-020-0075-7 [retrieved on 2020-08-11] * the whole document * ----- | 1-15 | TECHNICAL FIELDS SEARCHED (IPC) |

The present search report has been drawn up for all claims

1

| Place of search | Date of completion of the search | Examiner |
|---|---|---|
| The Hague | 11 May 2023 | Macchia, Giovanni |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.....
& : member of the same patent family, corresponding document

EPO FORM 1503 03.82 (P04C01)

Europäisches Patentamt
European Patent Office
Office européen des brevets

Application Number
EP 22 19 2656

## CLAIMS INCURRING FEES

The present European patent application comprised at the time of filing claims for which payment was due.

- [ ] Only part of the claims have been paid within the prescribed time limit. The present European search report has been drawn up for those claims for which no payment was due and for those claims for which claims fees have been paid, namely claim(s):

- [x] No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

- [ ] All further search fees have been paid within the fixed time limit. The present European search report has been drawn up for all claims.

- [ ] As all searchable claims could be searched without effort justifying an additional fee, the Search Division did not invite payment of any additional fee.

- [ ] Only part of the further search fees have been paid within the fixed time limit. The present European search report has been drawn up for those parts of the European patent application which relate to the inventions in respect of which search fees have been paid, namely claims:

- [ ] None of the further search fees have been paid within the fixed time limit. The present European search report has been drawn up for those parts of the European patent application which relate to the invention first mentioned in the claims, namely claims:

- [ ] The present supplementary European search report has been drawn up for those parts of the European patent application which relate to the invention first mentioned in the claims (Rule 164 (1) EPC).

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012078536 A
- WO 2012118911 A
- WO 2013179292 A
- WO 2011017521 A
- WO 2013154798 A
- WO 99014226 A
- WO 0066604 A
- WO 98039352 A
- WO 2004046160 A
- WO 00047599 A
- WO 2007134181 A
- WO 2010077578 A
- WO 2010036698 A
- WO 2007090071 A
- WO 2009006478 A
- WO 2011156202 A
- WO 2008154401 A
- WO 2009067647 A
- WO 2008150729 A
- WO 0123613 A
- EP 2009050349 W
- WO 2013022984 A
- WO 2008049085 A
- WO 2012109395 A
- WO 2014076195 A
- WO 2015113922 A
- WO 2013179292 A1
- WO 2007031091 A


### Non-patent literature cited in the description


- **S. T. CROOKE.** Antisense drug technology: principles, strategies, and applications. CRC Press, 2008
- **ECKSTEIN.** *Antisense and Nucleic Acid Drug Development,* 2009, vol. 10, 117-121
- **CROOKE et al.** *Nucleic Acids Research,* 2020, vol. 48 (10), 5235-5253
- **MATSUDA et al.** XXIII International Round Table on Nucleosides. *Nucleotides and Nucleic acids,* 2018
- **LIU et al.** *ACS Appl. Mater. Interfaces,* 2018, vol. 10, 9736-9743
- **HIRAO et al.** *Accounts of Chemical Research,* 2012, vol. 45, 2055
- **BERGSTROM.** *Current Protocols in Nucleic Acid Chemistry,* 2009, (37
- **FREIER ; ALTMANN.** *Nucl. Acid Res.,* 1997, vol. 25, 4429-4443
- **UHLMANN.** *Curr. Opinion in Drug Development,* 2000, vol. 3 (2), 293-213
- **DELEAVEY ; DAMHA.** *Chemistry and Biology,* 2012, vol. 19, 937
- **MORITA et al.** *Bioorganic & Med. Chem. Lett.,* 2002, vol. 12, 73-76
- **SETH et al.** *J. Org. Chem.,* 2010, vol. 75 (5), 1569-81
- **MITSUOKA et al.** *Nucleic Acids Research,* 2009, vol. 37 (4), 1225-1238
- **WAN ; SETH.** *J. Medical Chemistry,* 2016, vol. 59, 9645-9667
- **MERGNY ; LACROIX.** *Oligonucleotides,* 2003, vol. 13, 515-537
- **HANSEN et al.** *Chem. Comm.,* 1965, 36-38
- **HOLDGATE et al.** *Drug Discov Today,* 2005
- **SANTALUCIA.** *Proc Natl Acad Sci USA.,* 1998, vol. 95, 1460-1465
- **SUGIMOTO et al.** *Biochemistry,* 1995, vol. 34, 11211-11216
- **MCTIGUE et al.** *Biochemistry,* 2004, vol. 43, 5388-5405
- **VESTER et al.** *Bioorg. Med. Chem. Lett.,* 2008, vol. 18, 2296-2300
- **MANGOS et al.** *J. AM. CHEM. SOC.,* 2003, vol. 125, 654-661
- **FLUITER et al.** *Mol. Biosyst.,* 2009, vol. 10, 1039
- **RUKOV et al.** *Nucl. Acids Res.,* 2015, vol. 43, 8476-8487
- **CARUTHERS et al.** *Methods in Enzymology,* 1987, vol. 154, 287-313
- **ZHANG ; CHAPUT.** Synthesis of Threose Nucleic Acid (TNA) Phosphoramidite Monomers and Oligonucleotide Polymers. *Current Protocols in Nucleic Acid Chemistry,* 2012, 4.51.1-4.51.26
- **BASTIN.** *Organic Process Research & Development,* 2000, vol. 4, 427-435
- **ANSEL.** Pharmaceutical Dosage Forms and Drug Delivery Systems. 1995, 196, , 1456-1457
- Remington's Pharmaceutical Sciences. Mack Publishing Company, 1985
- **LANGER.** *Science,* 1990, vol. 249, 1527-1533

- Synthesis of Threose Nucleic Acid (TNA) Phosphoramidite Monomers and Oligonucleotide Polymers. *Current Protocols in Nucleic Acid Chemistry,* 2012, 4.51.1-4.51.26
- **ZHANG et al.** *Chem. Inf. Model.,* 2012, vol. 52 (10), 2796-2806
- **MATSUDA et al.** Poster. *XXIII International Round Table on Nucleosides, Nucleotides and Nucleic acids,* August 2018
- **ZHANG ; CHAPUT.** *Current Protocols in Nucleic Acid Chemistry,* 2012, 4.51.1-4.51.26